# EUROPÄISCHE PATENTSCHRIFT

(11) **EP 1 140 802 B1**
(45) Veröffentlichungstag und Bekanntmachung des Hinweises auf die Patenterteilung: **17.03.2004**
(21) Anmeldenummer: 99965464.3
(22) Anmeldetag: 13.12.1999
(51) Int. Cl.: C07C 257/18, C07D 207/08, C07D 277/24, C07D 235/14, C07D 231/12, C07D 233/54, C07D 213/50, C07D 333/22, A61K 31/155, A61K 31/40, A61K 31/415, A61P 7/02

(54) **SUBSTITUIERTE ARYL- UND HETEROARYLAMIDINDERIVATE, DEREN HERSTELLUNG UND DEREN VERWENDUNG ALS ARZNEIMITTEL**
SUBSTITUTED ARYL AND HETEROARYL DERIVATIVES, THEIR PRODUCTION AND THEIR USE AS MEDICINES
DERIVES ARYLE ET HETEROARYLE SUBSTITUES, LEUR PRODUCTION ET LEUR UTILISATION COMME MEDICAMENTS

(30) Priorität: 16.12.1998 DE 19858029; 07.10.1999 DE 19948101
(43) Veröffentlichungstag der Anmeldung: 10.10.2001
(73) Patentinhaber: Boehringer Ingelheim Pharma GmbH & Co.KG, 55218 Ingelheim am Rhein (DE)
(72) Erfinder: PRIEPKE, Henning, D-88447 Warthausen (DE); KAUFFMANN, Iris, D-88448 Attenweiler (DE); HAUEL, Norbert, D-88433 Schemmerhofen (DE); RIES, Uwe, D-88400 Biberach (DE); NAR, Herbert, D-88441 Mittelbiberach (DE); STASSEN, Jean, Marie, D-88447 Warthausen (DE); WIENEN, Wolfgang, D-88400 Biberach (DE)
(74) Vertreter: Laudien, Dieter, Dr.
(86) Internationale Anmeldenummer: PCT/EP1999/009921
(87) Internationale Veröffentlichungsnummer: WO 2000/035859

(56) Entgegenhaltungen:
- EP-A- 0 760 364
- EP-A- 0 805 149
- WO-A-95/18111

## Beschreibung

Gegenstand der vorliegenden Erfindung sind neue substituierte Aryl- und Heteroarylderivate der allgemeinen Formel

Ar - A - (HCR₁) - X - Y , (I)

deren Tautomere, deren Stereoisomere, deren Gemische und deren Salze, insbesondere deren physiologisch verträgliche Salze mit anorganischen oder organischen Säuren oder Basen, welche wertvolle Eigenschaften aufweisen.

In der internationalen Patentanmeldung WO 95/18111 werden bereits Verbindungen als Fibrinogen-Rezeptor-Antagonisten beschrieben, welche basische und saure Endgruppe enthalten.

Die Verbindungen der obigen allgemeinen Formel I, in denen Y keine Cyanogruppe enthält, weisen wertvolle pharmakologische Eigenschaften auf, insbesondere eine antithrombotische Wirkung, und
die Verbindungen der obigen allgemeinen Formel I, in denen Y eine Cyanogruppe enthält, stellen wertvolle Zwischenprodukte zur Herstellung der Verbindungen der allgemeinen Formel I dar, in der R₅ eine gegebenenfalls substituierte Amino-C₁₋₃-alkyl-, Amidino-, Guanidino- oder Guanidino-C₁₋₃-alkylgruppe darstellt.

Gegenstand der vorliegenden Erfindung sind somit die neuen Verbindungen der obigen allgemeinen Formel I sowie deren Her stellung, die die pharmakologisch wirksamen Verbindungen enthaltenden Arzneimittel und deren Verwendung.

In der obigen allgemeinen Formel bedeutet
A eine Ethinylengruppe, eine gegebenenfalls durch eine C₁₋₃-Alkyl- oder Carboxy-C₁₋₃-alkylgruppe oder durch ein Chlor-, Bromoder Jodatom substituierte Vinylen- oder Ethylengruppe,
R₁ ein Wasserstoffatom, eine C₁₋₃-Alkyl- oder Carboxy-C₁₋₃-alkylgruppe,
Ar eine durch die Reste R₂ bis R₄ substituierte Phenylgruppe, wobei
R₂ eine C₁₋₆-Alkyl- oder C₃₋₇-Cycloalkyl-C₁₋₃-alkylgruppe, die jeweils im C₁₋₆- und C₁₋₃-Alkylteil durch eine Carboxy-, Phenyl-, Amino-, C₁₋₃-Alkylamino-, Carboxy-C₁₋₃-alkylamino-, Di-(C₁₋₃-Alkyl)-amino-, N-(Carboxy-C₁₋₃-alkyl)-C₁₋₃-alkylamino-, C₃₋₇-Cycloalkylamino-, Phenylamino-, N-(C₁₋₃-Alkyl)-phenylamino-, N-(C₁₋₄-Alkanoyl)-phenylamino-, Heteroarylamino-, N- (C₁₋₃-Alkyl)-heteroarylamino-, N- (Carboxy-C₁₋₃-alkyl)-phenylamino- oder N-(Carboxy-C₁₋₃-alkyl)-heteroarylaminogruppe substituiert sein können,
eine Carboxy-C₁₋₅-alkylgruppe, die im Alkylteil durch eine C₁₋₃-Alkylamino-, N,N-Di-(C₁₋₃-alkyl)-amino-, Pyrrolidino-, Piperidino- oder Hexamethyleniminogruppe substituiert ist,
eine Carboxy-C₁₋₅-alkylgruppe, in der die Wasserstoffatome einer Methylengruppe durch eine n-C₂₋₅-Alkylenbrücke ersetzt sind,
eine Phenyl-, Phenyloxy- oder Phenylsulfonylgruppe, die jeweils im Phenylteil durch ein Fluor-, Chlor-, Brom- oder Jodatom, durch eine C₁₋₃-Alkyl-, Carboxy-C₁₋₃-alkyl- oder C₁₋₃-Alkoxygruppe substituiert- sein können,
eine C₁₋₅-Alkylamino-, Carboxy-C₁₋₃-alkylamino-, Di-(C₁₋₅-alkyl)-amino-, N-(Carboxy-C₁₋₃-alkyl)-C₁₋₅-alkylamino-, C₃₋₇-Cycloalkylamino-, N-(Carboxy-C₁₋₃-alkyl)-C₃₋₇-cycloalkylamino-, Phenylamino-, N-(C₁₋₃-Alkyl)-phenylamino-, N-(Carboxy-C₁₋₃-alkyl)-phenylamino-, Heteroarylamino-, N-(C₁₋₃-Alkyl)-heteroarylamino- oder N- (Carboxy-C₁₋₃-alkyl)-heteroarylaminogruppe,
eine C₁₋₅-Alkylcarbonylamino-, C₃₋₇-Cycloalkylcarbonylamino-, Arylcarbonylamino-, Heteroarylcarbonylamino-, C₁₋₅-Alkylsulfonylamino-, Arylsulfonylamino-, Heteroarylsulfonylamino-, N- (C₁₋₃-Alkyl)-C₁₋₅-alkylcarbonylamino-, N- (C₁₋₃-Alkyl)-C₃₋₇-cycloalkylcarbonylamino-, N-(C₁₋₃-Alkyl)-arylcarbonylamino-, N- (C₁₋₃-Alkyl)-heteroarylcarbonylamino-, N- (C₁₋₃-Alkyl) -C₁₋₅-alkylsulfonylamino-, N-(C₁₋₃-Alkyl)-arylsulfonylamino- oder N-(C₁₋₃-Alkyl)-heteroarylsulfonylaminogruppe, wobei
die vorstehend erwähnten N-(C₁₋₃-Alkyl)-teile zusätzlich durch eine Carboxy-, Carboxy-C₁₋₃-alkylaminocarbonyl- oder N- (C₁₋₃-Alkyl)-carboxy-C₂₋₃-alkylaminocarbonylgruppe oder mit Ausnahme des α-Kohlenstoffatoms bezogen auf das Stickstoffatom auch durch eine Hydroxy-, Carboxy-C₁₋₃-alkoxy-, Amino-, Carboxy-C₁₋₃-alkylamino- oder N- (C₁₋₃-Alkyl)-carboxy-C₁₋₃-alkylaminogruppe substituiert sein können,
eine 5- bis 7-gliedrige Cycloalkyleniminogruppe,
eine Amino-, C₁₋₅-Alkylamino-, C₃₋₇-Cycloalkylamino-, Arylamino-, Aryl-C₁₋₃-alkylamino-, Heteroarylamino- oder Heteroaryl-C₁₋₃-alkyl-aminogruppe, die jeweils am Aminstickstoffatom durch eine C₁₋₃-Alkylcarbonyl-, Carboxy-C₁₋₃-alkylcarbonyl-, Carboxy-C₁₋₃-alkylaminocarbonyl-, 2-Oxo-pyrrolidinylcarbonyl- oder Piperazinocarbonylgruppe substituiert sind, wobei zusätzlich
(i) die vorstehend erwähnte Aminogruppe, die durch eine C₁₋₃-Alkylcarbonyl-, Carboxy-C₁₋₃-alkylcarbonyl- oder Carboxy-C₁₋₃-alkylaminocarbonylgruppe monosubstituiert ist, durch eine 5- bis 7-gliedrige Cycloalkyleniminogruppe oder durch eine N,N-Di-(C₁₋₅-Alkyl)-aminogruppe substituiert ist, und
(ii) der Alkylteil der vorstehend erwähnten C₁₋₃-Alkylcarbonylgruppe durch eine Carboxy-, Amino-, Hydroxy-, Carboxy-C₁₋₃-alkoxy-, Carboxy-C₁₋₃-alkylaminocarbonyl-, Carboxy-C₁₋₃-alkylamino-, N-(C₁₋₃-Alkyl)-carboxy-C₁₋₃-alkylamino- oder Amino-C₁₋₃-alkylcarbonylaminogruppe oder durch eine Carboxy- oder Hydroxygruppe und durch eine Aminooder Trifluoracetylaminogruppe substituiert ist,
eine Carbiminogruppe, die am Stickstoffatom durch eine Carboxy-C₁₋₃-alkoxy-, Amino-, C₁₋₃-Alkylamino-, Carboxy-C₁₋₃-alkylamino-, Di-(C₁₋₃-Alkyl)-amino- oder N-(Carboxy-C₁₋₃-alkyl)-C₁₋₃-alkylaminogruppe und am Kohlenstoffatom durch eine C₁₋₅-Alkylgruppe, durch eine gegebenenfalls durch eine C₁₋₃-Alkyl- oder C₁₋₃-Alkoxygruppe substituierte Phenylgruppe oder durch eine gegebenenfalls durch eine C₁₋₃-Alkylgruppe substituierte Heteroarylgruppe substituiert ist,
eine Heteroaryl- oder Heteroaryl-C₁₋₃-alkylgruppe, die jeweils im Heteroarylteil zusätzlich auch durch eine Phenyloder Heteroarylgruppe oder durch eine Phenyl- oder Heteroarylgruppe und durch eine Carboxy-C₁₋₃-alkyl- oder C₁₋₃-Alkoxycarbonyl-C₁₋₃-alkylgruppe substituiert sein können,
eine gegebenenfalls durch 1 bis 3 C₁₋₃-Alkylgruppen substituierte 5-Oxo-4,5-dihydro-pyrazolyl- oder 6-Oxo-4,5-dihydropyridazinylgruppe, in der ein Alkylsubstituent gleichzeitig durch eine Carboxy- oder C₁₋₃-Alkoxycarbonylgruppe substituiert sein kann, oder
eine Carbonylgruppe, die
durch ein Wasserstoffatom, durch eine Hydroxy-, C₁₋₅-Alkoxy- oder C₃₋₇-Cycloalkoxygruppe,
durch eine gegebenenfalls durch eine Carboxygruppe substituierte C₁₋₅-Alkyl- oder C₃₋₇-Cycloalkylgruppe,
durch eine durch eine Piperazinogruppe substituierte C₁₋₃-Alkylgruppe,
durch eine Phenylgruppe, die durch ein Fluor-, Chlor-, Brom- oder Jodatom, durch eine C₁₋₃-Alkyl-, Carboxy-C₁₋₃-alkyl-, C₁₋₃-Alkoxy- oder Carboxygruppe substituiert sein kann,
durch eine Amino-, C₁₋₅-Alkylamino-, Carboxy-C₁₋₃-alkylamino-, C₃₋₇-Cycloalkylamino-, Phenylamino- oder Heteroarylaminogruppe, die jeweils zusätzlich am Aminstickstoffatom durch eine C₁₋₅-Alkyl-, C₃₋₇-Cycloalkyl-, Phenyl-C₁₋₃-alkyl-, Carboxy-C₁₋₃-alkyl-, 2-[Di-(C₁₋₃-alkyl)-amino]-ethyl-, 3- [Di-(C₁₋₃-alkyl)-amino]-propyl-, Di- (C₁₋₃-alkyl)-amino-, 2- (N-Carboxy-C₁₋₃-alkyl-C₁₋₃-alkylamino)-ethyl-, 3- (N-Carboxy-C₁₋₃-alkyl-C₁₋₃-alkylamino)-propyl- oder N-Carboxy-C₁₋₃-alkyl-C₁₋₃-alkylamino-, Phenyl-, Pyridyl-, Pyrrolidinyloder Piperidinylgruppe substituiert sein können,
durch eine gegebenenfalls durch eine oder zwei C₁₋₃-Alkylgruppen substituierte Pyrrolyl-, Thienyl-, Imidazolyl-, Pyrazolyl-, Thiazolyl-, Pyridinyl-, Pyrimidinyl-, Pyrazinyl- oder Pyridazinylgruppe, an die jeweils über zwei benachbarte Kohlenstoffatome ein Phenylring ankondensiert sein kann,
durch eine gegebenenfalls durch eine C₁₋₃-Alkyl- oder Carboxy-C₁₋₃-alkylgruppe substituierte C₃₋₆-Cycloalkylenimino-, C₅₋₈-Bicycloalkylenimino-, Morpholino-, Piperazino-, Dihydropyrazolo-, Tetrahydropyrazolo-, Tetrahydroisoxazolo-, Tetrahydropyrazinyl- oder Tetrahydropyridazinylgruppe oder
durch eine gegebenenfalls durch eine C₁₋₃-Alkyl-, Carboxy-C₁₋₃-alkyl-, Hydroxy-, Hydroxy-C₁₋₃-alkyl-, Amino-, Carboxy-, Carboxy-C₁₋₃-alkoxy-C₁₋₃-alkyl-, Carboxy-C₁₋₃-alkylamino-C₁₋₃-alkyl- oder Carboxy-C₁₋₃-alkylaminocarbonyl-C₁₋₃-alkylgruppe substituierte C₃₋₆-Cycloalkyleniminogruppe,
durch eine gegebenenfalls durch eine C₁₋₃-Alkyl- oder Carboxy-C₁₋₃-alkylgruppe substituierte C₅₋₈-Bicycloalkylenimino-, Morpholino-, Piperazino-, Dihydropyrazolo-, Tetrahydropyrazolo-, Tetrahydroisoxazolo-, Tetrahydropyrazinyl- oder Tetrahydropyridazinylgruppe substituiert ist,
R₃ ein Wasserstoff-, Fluor-, Chlor-, Brom- oder Jodatom, eine Formyl- oder Trifluormethylgruppe,
eine C₁₋₃-Alkoxy-, Amino-, C₁₋₃-Alkylamino-, Di-(C₁₋₃-Alkyl)-amino-, C₁₋₄-Alkanoylamino- oder N-(C₁₋₄-Alkanoyl)-C₁₋₃-alkylaminogruppe,
eine gegebenenfalls durch eine Hydroxy-, C₁₋₃-Alkoxy-, Carboxy-, Carboxy-C₁₋₃-alkoxy-, Carboxy-C₁₋₃-alkylamino-, N-(C₁₋₃-Alkyl)-carboxy-C₁₋₃-alkylamino- oder Carboxy-C₁₋₃-alkylaminocarbonylgruppe substituierte C₁₋₃-Alkylgruppe,
eine durch eine Carboxy- oder Carboxy-C₁₋₃-alkylaminocarbonylgruppe substituierte C₂₋₃-Alkenylgruppe oder
eine gegebenenfalls am Kohlenstoffatom durch eine C₁₋₃-Alkylgruppe substituierte Carbiminogruppe, die am Iminostickstoffatom durch eine Carboxy-C₁₋₃-alkoxy- oder Aminocarbonylaminogruppe substituiert ist,
oder R₂ und R₃ zusammen eine -CO-O-CH₂- oder -CO-O-CH₂CH₂-Gruppe und
R₄ ein Wasserstoff-, Fluor-, Chlor-, Brom- oder Iodatom, eine C₁₋₃-Alkyl-, C₃₋₇-Cycloalkyl-, Trifluormethyl- oder C₁₋₃-Alkoxygruppe darstellen,
oder Ar auch eine Heteroarylgruppe, die durch die vorstehend erwähnten Reste R₂ bis R₄ substituiert sein kann, welche wie vorstehend erwähnt definiert sind,
X ein Sauerstoff- oder Schwefelatom, eine gegebenenfalls durch eine oder zwei C₁₋₃-Alkylgruppen substituierte Methylengruppe, eine Carbonyl-, Sulfinyl-, Sulfonyl-, Imino-, N-(C₁₋₃-Alkyl)-imino- oder N-(Carboxy-C₁₋₃-alkyl)-iminogruppe, wobei der Alkylteil der N-(C₁₋₃-Alkyl)-iminogruppe in 2- oder 3-Stellung zusätzlich durch eine Amino-, C₁₋₃-Alkylamino-, Di- (C₁₋₃-Alkyl)-amino-, C₁₋₄-Alkanoylamino- oder N- (C₁₋₄-Alkanoyl)-C₁₋₃-alkylaminogruppe substituiert sein kann, und
Y eine durch eine Aminogruppe substituierte Cyclohexylgruppe oder eine durch den Rest R₅ substituierte Phenyl- oder Heteroarylgruppe, wobei die vorstehend erwähnte Phenylgruppe jeweils durch eip Fluor-, Chlor-, Brom- oder Jodatom oder durch eine C₁₋₃-Alkyl- oder C₁₋₃-Alkoxygruppe sowie die vorstehend erwähnte Heteroarylgruppe durch eine C₁₋₃-Alkylgruppe substituiert sein können und
R₅ ein Wasserstoffatom, eine Cyanogruppe oder eine gegebenenfalls durch eine in vivo abspaltbare Gruppe substituierte Amino-, Amino-C₁₋₃-alkyl-, Amidino-, Guanidino- oder Guanidino-C₁₋₃-alkylgruppe darstellt.

Unter den vorstehend erwähnten Heteroarylgruppen ist eine gegebenenfalls durch eine oder zwei C₁₋₃-Alkylgruppen substituierte 5-gliedrige heteroaromatische Gruppe, die eine gegebenenfalls durch eine C₁₋₃-Alkylgruppe substituierte Iminogruppe, ein Sauerstoff- oder Schwefelatom oder eine gegebenenfalls durch eine C₁₋₃-Alkylgruppe substituierte Iminogruppe, ein Sauerstoff- oder Schwefelatom und eine oder zwei Stickstoffatome sowie deren partiell hydrierten Derivate, insbesondere deren Dihydroderivate, oder eine 6-gliedrige heteroaromatische Gruppe, die ein, zwei oder drei Stickstoffatome enthält, wobei zusätzlich an die vorstehend erwähnten 5- und 6-gliedrigen heteroaromatischen Ringe über zwei benachbarte Kohlenstoffatome ein Phenylring ankondensiert sein kann, zu verstehen.

Ferner können die bei der Definition der Reste vorstehend erwähnten Carboxygruppen durch eine Tetrazolylgruppe oder durch eine in-vivo in eine Carboxygruppe überführbare Gruppe ersetzt sein, z.B. durch eine Hydroxymethyl- oder Formylgruppe, durch eine mit einem Alkohol veresterte Carboxygruppe, in der der alkoholische Teil vorzugsweise ein C₁₋₆-Alkanol, ein Phenyl-C₁₋₃-alkanol, ein C₃₋₉-Cycloalkanol, wobei ein C₅₋₈-Cycloalkanol zusätzlich durch eine oder zwei C₁₋₃-Alkylgruppen substituiert sein kann, ein C₅₋₈-Cycloalkanol, in dem eine Methylengruppe in 3- oder 4-Stellung durch ein Sauerstoffatom oder durch eine gegebenenfalls durch eine C₁₋₃-Alkyl-, Phenyl-C₁₋₃-alkyl-, Phenyl-C₁₋₃-alkoxycarbonyl- oder C₂₋₆-alkoxycarbonyl- oder C₂₋₆-Alkanoylgruppe substituierte Iminogruppe ersetzt ist und der Cycloalkanolteil zusätzlich durch eine oder zwei C₁₋₃-Alkylgruppen substituiert sein kann, ein C₄₋₇-Cycloalkenol, ein C₃₋₅-Alkenol, ein Phenyl-C₁₋₅-alkenol, ein C₃₋₅-Alkinol oder Phenyl-C₃₋₅-alkinol mit der Maßgabe, daß keine Bindung an das Sauerstoffatom von einem Kohlenstoffatom ausgeht, welches eine Doppel- oder Dreifachbindung trägt, ein C₃₋₈-Cycloalkyl-C₁₋₃-alkanol, ein Bicycloalkanol mit insgesamt 8 bis 10 Kohlenstoffatomen, das im Bicycloalkylteil zusätzlich durch eine oder zwei C₁₋₃-Alkylgruppen substituiert ist, ein 1,3-Dihydro-oxo-1-isobenzfuranol oder ein Alkohol der Formel

RₐCO-O-(R_{b}CR_{c})-OH,

in dem Rₐ eine C₁₋₈-Alkyl-, C₅₋₇-Cycloalkyl-, Phenyl- oder Phenyl-C₁₋₃-alkylgruppe,
R_{b} ein Wasserstoffatom, eine C₁₋₃-Alkyl-, C₅₋₇-Cycloalkyl- oder Phenylgruppe und
R_{c} ein Wasserstoffatom oder eine C₁₋₃-Alkylgruppe darstellen,
und die der bei der Definition der Reste erwähnten Imino- oder Aminogruppen durch einen in-vivo abspaltbaren Rest substituiert sein, z.B. durch eine Hydroxygruppe, durch eine Acylgruppe wie die Benzoyl- oder Pyridinoylgruppe oder eine C₁₋₁₆-Alkanoylgruppe wie die Formyl-, Acetyl-, Propionyl-, Butanoyl-, Pentanoyl- oder Hexanoylgruppe, durch eine Allyloxycarbonylgruppe, durch eine C₁₋₁₆-Alkoxycarbonylgruppe wie die Methyloxycarbonyl-, Ethyloxycarbonyl-, Propyloxycarbonyl-, Isopropyloxcarbonyl-, Butyloxycarbonyl-, tert.Butyloxycarbonyl-, Pentyloxycarbonyl-, Hexyloxycarbonyl-, Octyloxycarbonyl-, Nonyloxycarbonyl-, Decyloxycarbonyl-, Undecyloxycarbonyl-, Dodecyloxycarbonyl- oder Hexadecyloxycarbonylgruppe, durch eine Phenyl-C₁₋₁₆-alkoxycarbonylgruppe wie die Benzyloxycarbonyl-, Phenylethyloxycarbonyl- oder Phenylpropyloxycarbonylgruppe, durch eine C₁₋₃-Alkylsulfonyl-C₂₋₄-alkoxycarbonyl-, C₁₋₃-Alkoxy-C₂₋₄-alkoxy-C₂₋₄-alkoxycarbonyl- oder RₐCO-O- (R_{b}CR_{c})-O-CO-Gruppe, in der Rₐ bis R_{b} wie vorstehend erwähnt definiert sind.

Desweiteren schließen die bei der Definition der vorstehend erwähnten gesättigten Alkyl- und Alkoxyteile, die mehr als 2 Kohlenstoffatome enthalten, sowie die Alkanoyl- und ungesättigten Alkylteile, die mehr als 3 Kohlenstoffatome enthalten, auch deren verzweigte Isomere wie beispielsweise die Isopropyl-, tert.Butyl-, Isobutylgruppe etc. ein.

Bevorzugte Verbindungen der allgemeinen Formel I der vorliegenden Erfindung sind diejenigen, in denen
A eine Ethinylengruppe, eine gegebenenfalls durch eine C₁₋₃-Alkyl- oder Carboxy-C₁₋₃-alkylgruppe oder durch ein Chlor-, Bromoder Jodatom substituierte Vinylen- oder Ethylengruppe,
R₁ ein Wasserstoffatom, eine C₁₋₃-Alkyl- oder Carboxy-C₁₋₃-alkylgruppe,
Ar eine durch die Reste R₂ bis R₄ substituierte Phenylgruppe, wobei
R₂ eine C₁₋₃-Alkylgruppe, die durch eine Carboxy-, Phenyl-, Amino-, C₁₋₃-Alkylamino-, Carboxy-C₁₋₃-alkylamino-, Di-(C₁₋₃-Alkyl)-amino-, N- (Carboxy-C₁₋₃-alkyl)-C₁₋₃-alkylamino-, Phenylamino-, N-(C₁₋₃-Alkyl)-phenylamino-, N- (C₁₋₄-Alkanoyl)-phenylamino-, Heteroarylamino-, N-(C₁₋₃-Alkyl)-heteroarylamino-, N-(Carboxy-C₁₋₃-alkyl)-phenylamino- oder N-(Carboxy-C₁₋₃-alkyl)-heteroarylaminogruppe substituiert sein kann,
eine Phenyl- oder Phenylsulfonylgruppe, die jeweils im Phenylteil durch ein Fluor-, Chlor-, Brom- oder Jodatom, durch eine C₁₋₃-Alkyl-, Carboxy-C₁₋₃-alkyl- oder C₁₋₃-Alkoxygruppe substituiert sein können,
eine C₁₋₃-Alkylamino-, Carboxy-C₁₋₃-alkylamino-, Di-(C₁₋₃-alkyl) -amino-, N- (Carboxy-C₁₋₃-alkyl)-C₁₋₃-alkylamino-, Phenylamino-, N- (C₁₋₃-Alkyl)-phenylamino-, N- (Carboxy-C₁₋₃-alkyl)-phenylamino-, Heteroarylamino-, N-(C₁₋₃-Alkyl)-heteroarylamino- oder N- (Carboxy-C₁₋₃-alkyl)-heteroarylaminogruppe,
eine C₁₋₅-Alkylcarbonylamino-, Arylcarbonylamino-, Heteroarylcarbonylamino-, C₁₋₅-Alkylsulfonylamino-, Arylsulfonylamino-, Heteroarylsulfonylamino-, N-(C₁₋₃-Alkyl)-C₁₋₅-alkylcarbonylamino-, N-(C₁₋₃-Alkyl)-arylcarbonylamino-, N- (C₁₋₃-Alkyl)-heteroarylcarbonylamino-, N- (C₁₋₃-Alkyl)-C₁₋₅-alkylsulfonylamino-, N- (C₁₋₃-Alkyl)-arylsulfonylamino-, N- (C₁₋₃-Alkyl)-heteroarylsulfonylamino-, N- (Carboxy-C₁₋₃-alkyl)-C₁₋₅-alkylcarbonylamino-, N-(Carboxy-C₁₋₃-alkyl)-arylcarbonylamino-, N- (Carboxy-C₁₋₃-alkyl)-heteroarylcarbonylamino-, N-(Carboxy-C₁₋₃-alkyl)-C₁₋₅-alkylsulfonylamino-, N-(Carboxy-C₁₋₃-alkyl)-arylsulfonylamino- oder N-(Carboxy-C₁₋₃-alkyl)-heteroarylsulfonylaminogruppe,
eine Carbiminogruppe, die am Stickstoffatom durch eine Carboxy-C₁₋₃-alkoxy-, Amino-, C₁₋₃-Alkylamino-, Carboxy-C₁₋₃-alkylamino-, Di-(C₁₋₃-Alkyl)-amino- oder N-(Carboxy-C₁₋₃-alkyl)-C₁₋₃-alkylaminogruppe und am Kohlenstoffatom durch eine C₁₋₅-Alkylgruppe, durch eine gegebenenfalls durch eine C₁₋₃-Alkyl- oder C₁₋₃-Alkoxygruppe substituierte Phenylgruppe oder durch eine gegebenenfalls durch eine C₁₋₃-Alkylgruppe substituierte Heteroarylgruppe substituiert ist,
eine Heteroaryl- oder Heteroaryl-C₁₋₃-alkylgruppe, die jeweils im Heteroarylteil zusätzlich auch durch eine Phenyloder Heteroarylgruppe oder durch eine Phenyl- oder Heteroarylgruppe und durch eine Carboxy-C₁₋₃-alkyl- oder C₁₋₃-Alkoxycarbonyl-C₁₋₃-alkylgruppe substituiert sein können, oder
eine Carbonylgruppe, die
durch ein Wasserstoffatom, durch eine Hydroxy-, C₁₋₅-Alkoxy- oder C₃₋₇-Cycloalkoxygruppe,
durch eine gegebenenfalls durch eine Carboxygruppe substituierte C₁₋₅-Alkyl- oder C₃₋₇-Cycloalkylgruppe,
durch eine Phenylgruppe, die durch ein Fluor-, Chlor-, Brom- oder Jodatom, durch eine C₁₋₃-Alkyl-, Carboxy-C₁₋₃-alkyl-, C₁₋₃-Alkoxy- oder Carboxygruppe substituiert sein kann,
durch eine Amino-, C₁₋₅-Alkylamino-, C₃₋₇-Cycloalkylamino-, Phenylamino- oder Heteroarylaminogruppe, die jeweils zusätzlich am Aminstickstoffatom durch eine C₁₋₅-Alkyl-, C₅₋₇-Cycloalkyl-, Phenyl-C₁₋₃-alkyl-, Carboxy-C₁₋₃-alkyl-, 2- [Di-(C₁₋₃-alkyl)-amino] -ethyl-, 3- [Di-(C₁₋₃-alkyl)-amino]-propyl-, Di-(C₁₋₃-alkyl)-amino-, 2-(N-Carboxy-C₁₋₃-alkyl-C₁₋₃-alkylamino)-ethyl-, 3-(N-Carboxy-C₁₋₃-alkyl-C₁₋₃-alkylamino)-propyl- oder N-Carboxy-C₁₋₃-alkyl-C₁₋₃-alkylamino-, Pyrrolidinyl- oder Piperidinylgruppe substituiert sein können,
durch eine gegebenenfalls durch eine C₁₋₃-Alkylgruppe substituierte Pyrrolyl-, Thienyl-, Imidazolyl-, Pyrazolyl-, Thiazolyl-, Pyridinyl-, Pyrimidinyl-, Pyrazinyl- oder Pyridazinylgruppe, an die jeweils über zwei benachbarte Kohlenstoffatome ein Phenylring ankondensiert sein kann,
durch eine gegebenenfalls durch eine C₁₋₃-Alkyl- oder Carboxy-C₁₋₃-alkylgruppe substituierte C₃₋₆-Cycloalkylenimino-, C₅₋₈-Bicycloalkylenimino-, Morpholino-, Piperazino-, Dihydropyrazolo-, Tetrahydropyrazolo-, Tetrahydroisoxazolo-, Tetrahydropyrazinyl- oder Tetrahydropyridazinylgruppe oder
durch eine Pyrrolidinogruppe substituiert ist, wobei die vorstehend erwähnte Pyrrolidinogruppe durch eine Hydroxy-, Hydroxy-C₁₋₃-alkyl-, Amino-, Carboxy-, Carboxy-C₁₋₃-alkyl-, Carboxy-C₁₋₃-alkoxy-C₁₋₃-alkyl-, Carboxy-C₁₋₃-alkylamino-C₁₋₃-alkyl- oder Carboxy-C₁₋₃-alkylaminocarbonyl-C₁₋₃-alkylgruppe substituiert ist,
R₃ ein Wasserstoffatom oder eine Formylgruppe,
eine Amino-, C₁₋₃-Alkylamino-, Di-(C₁₋₃-Alkyl)-amino-, C₁₋₄-Alkanoylamino- oder N-(C₁₋₄-Alkanoyl)C₁₋₃-alkylaminogruppe,
eine gegebenenfalls durch eine Hydroxy-, C₁₋₃-Alkoxy-, Carboxy-, Carboxy-C₁₋₃-alkoxy-, Carboxy-C₁₋₃-alkylamino-, N-(C₁₋₃-Alkyl)-carboxy-C₁₋₃-alkylamino- oder Carboxy-C₁₋₃-alkylaminocarbonylgruppe substituierte C₁₋₃-Alkylgruppe,
eine durch eine Carboxy- oder Carboxy-C₁₋₃-alkylaminocarbonylgruppe substituierte C₂₋₃-Alkenylgruppe oder
eine gegebenenfalls am Kohlenstoffatom durch eine C₁₋₃-Alkylgruppe substituierte Carbiminogruppe, die am Iminostickstoffatom durch eine Carboxy-C₁₋₃-alkoxy- oder Aminocarbonylaminogruppe substituiert ist,
oder R₂ und R₃ zusammen eine -CO-O-CH₂- oder -CO-O-CH₂CH₂-Gruppe und
R₄ ein Wasserstoff-, Fluor-, Chlor-, Brom- oder Iodatom, eine C₁₋₃-Alkyl-, C₃₋₇-Cycloalkyl- oder C₁₋₃-Alkoxygruppe darstellen, oder Ar auch eine Heteroarylgruppe, die durch die vorstehend erwähnten Reste R₂ bis R₄ substituiert sein kann,
X ein Sauerstoff- oder Schwefelatom, eine gegebenenfalls durch eine oder zwei C₁₋₃-Alkylgruppen substituierte Methylengruppe, eine Carbonyl-, Sulfinyl-, Sulfonyl-, Imino-, N-(C₁₋₃-Alkyl)-imino- oder N-(Carboxy-C₁₋₃-alkyl)-iminogruppe, wobei der Alkylteil der N-(C₁₋₃-Alkyl)-iminogruppe in 2- oder 3-Stellung zusätzlich durch eine Amino-, C₁₋₃-Alkylamino-, Di-(C₁₋₃-Alkyl)-amino-, C₁₋₄-Alkanoylamino- oder N-(C₁₋₄-Alkanoyl)-C₁₋₃-alkylaminogruppe substituiert sein kann, und
Y eine durch eine Aminogruppe substituierte Cyclohexylgruppe oder eine durch den Rest R₅ substituierte Phenyl- oder Heteroarylgruppe, wobei die vorstehend erwähnte Phenylgruppe jeweils durch ein Fluor-, Chlor-, Brom- oder Jodatom oder durch eine C₁₋₃-Alkyl- oder C₁₋₃-Alkoxygruppe sowie die vorstehend erwähnte Heteroarylgruppe durch eine C₁₋₃-Alkylgruppe substituiert sein können und
R₅ ein Wasserstoffatom, eine Cyanogruppe oder eine gegebenenfalls durch eine in vivo abspaltbare Gruppe substituierte Amino-, Amino-C₁₋₃-alkyl-, Amidino-, Guanidino- oder Guanidino-C₁₋₃-alkylgruppe darstellt.
bedeuten, deren Pro-Drugs, deren Tautomere, deren Stereoisomere, deren Gemische und deren Salze,
insbesondere jedoch diejenigen, in denen
A eine gegebenenfalls durch ein Chlor-, Brom- oder Jodatom substituierte Vinylengruppe, eine Ethylen- oder Ethinylengruppe,
R₁ ein Wasserstoffatom oder eine C₁₋₃-Alkylgruppe,
Ar eine durch eine Benzoylgruppe substituierte Pyridyl- oder Thienylgruppe,
eine durch eine Pyrrolidinocarbonylgruppe substituierte Bromfuranylgruppe oder
eine durch die Reste R₂ bis R₄ substituierte Phenylgruppe, wobei
R₂ eine Phenyl- oder Phenoxygruppe,
eine C₁₋₃-Alkylgruppe, die durch eine Phenyl-, Phenylamino-, N- (C₁₋₃-Alkyl)-phenylamino- oder N- (C₁₋₃-Alkanoyl)-phenylaminogruppe substituiert sein kann,
eine Carboxy- oder C₁₋₃-Alkoxycarbonylgruppe,
eine Benzoyl- oder Phenylsulfonylgruppe, in denen jeweils der Phenylteil zusätzlich durch ein Fluor-, Chlor- oder Bromatom, durch eine Methyl-, Methoxy-, Carboxy- oder C₁₋₃-Alkoxycarbonylgruppe substituiert sein kann, wobei in den vorstehend erwähnten Benzoylgruppen zusätzlich das Sauerstoffatom durch Carboxy-C₁₋₃-alkoxyimino- oder C₁₋₃-Alkoxycarbonyl-C₁₋₃-alkoxyiminogruppe ersetzt sein kann,
eine C₁₋₅-Alkylaminogruppe, die im Alkylteil durch eine Phenyl-, Carboxy-, C₁₋₃-Alkoxycarbonyl-, Carboxy-C₁₋₃-alkylaminocarbonyl-, C₁₋₃-Alkoxycarbonyl-C₁₋₃-alkylaminocarbonyl-, N-(C₁₋₃-Alkyl)-carboxy-C₁₋₃-alkylaminocarbonyl- oder N- (C₁₋₃-Alkyl)-C₁₋₃-alkoxycarbonyl-C₁₋₃-alkylaminocarbonylgruppe substituiert sein kann, oder eine C₃₋₇-Cycloalkylaminogruppe, wobei die vorstehend erwähnten Gruppen jeweils am Aminstickstoffatom zusätzlich durch eine C₃₋₇-Cycloalkanoyl-, Benzoyloder Phenylsulfonylgruppe, durch eine Carboxy-C₁₋₃-alkylcarbonyl- oder C₁₋₃-Alkoxycarbonyl-C₁₋₃-alkylcarbonylgruppe, in der der Alkylteil der Alkylcarbonylgruppe jeweils durch eine Amino- oder Trifluoracetylaminogruppe substituiert sein kann, durch eine C₂₋₄-Alkanoylgruppe, die im Alkanoylteil durch eine Amino-, Carboxy-, C₁₋₃-Alkoxycarbonyl-, Carboxy-C₁₋₃-alkoxy-, C₁₋₃-Alkoxycarbonyl-C₁₋₃-alkoxy-, Carboxy-C₁₋₃-alkylaminocarbonyl-, C₁₋₃-Alkoxycarbonyl-C₁₋₃-alkylaminocarbonyl-, N- (C₁₋₃-Alkyl) -carboxy-C₁₋₃-alkylaminocarbonyl- oder N- (C₁₋₃-Alkyl)-C₁₋₃-alkoxycarbonyl-C₁₋₃-alkylaminocarbonylgruppe substituiert sein kann, durch eine Carboxy-C₁₋₂-alkylaminocarbonyl-, C₁₋₃-Alkoxycarbonyl-C₁₋₂-alkylaminocarbonyl-, Carboxy-C₁₋₃-alkylaminocarbonyl-C₁₋₂-alkylaminocarbonyl- oder C₁₋₃-Alkoxycarbonyl-C₁₋₃-alkylaminocarbonyl-C₁₋₂-alkylaminocarbonylgruppe substituiert ist,
eine Formyl-, Pyridylcarbonyl-, Thienylcarbonyl-, Imidazolylcarbonyl-, 1-Methyl-imidazolylcarbonyl-, Thiazolylcarbonyl- oder Indolylcarbonylgruppe,
eine gegebenenfalls durch eine 1 oder 2 Methylgruppen substituierte Benzimidazol-1-yl-, Benzimidazol-1-yl-methyl- oder 5-Oxo-4,5-dihydro-pyrazol-3-ylgruppe,
eine durch eine Phenylgruppe, durch eine Phenylgruppe und eine C₁₋₄-Alkylgruppe oder durch eine oder zwei C₁₋₄-Alkylgruppen substituierte Pyrazol-1-ylgruppe, in der ein Alkylsubstituent gleichzeitig durch eine Carboxy- oder C₁₋₃-Alkoxycarbonylgruppe substituiert sein kann, oder
eine Carbonylgruppe, die
durch eine gegebenenfalls durch eine Carboxy- oder C₁₋₃-Alkoxycarbonylgruppe substituierte C₁₋₅-Alkylgruppe,
durch eine C₃₋₇-Cycloalkylgruppe,
durch eine Amino- oder C₁₋₅-Alkylaminogruppe, die jeweils am Aminstickstoffatom durch eine C₁₋₃-Alkylgruppe, die durch eine C₃₋₇-Cycloalkyl-, Phenyl-, Pyrrolidinyl- oder Pyridinylgruppe oder in 2- oder 3-Stellung durch eine Di-(C₁₋₃-Alkyl)-aminogruppe substituiert sein kann, oder durch eine Di-(C₁₋₃-Alkyl)-aminogruppe substituiert sein kann,
durch eine Carboxy-C₁₋₃-alkylamino- oder C₁₋₃-Alkoxycarbonyl-C₁₋₃-alkylaminogruppe, die jeweils am Aminstickstoffaton
durch eine gegebenenfalls durch eine C₁₋₃-Alkylgruppe substituierte Pyrazolylgruppe substituiert ist,
durch eine 3- bis 7-gliedrige Cycloalkyleniminogruppe, die durch eine oder zwei C₁₋₃-Alkylgruppen substituiert sein kann, wobei die vorstehend erwähnten gegebenenfalls durch eine Methylgruppe substituierten Pyrrolidinogruppen zusätzlich durch eine Hydroxymethyl-, Carboxy-, C₁₋₃-Alkoxycarbonyl-, Carboxy-C₁₋₃-alkyl-, C₁₋₃-Alkoxycarbonyl-C₁₋₃-alkyl-, Carboxy-C₁₋₃-alkyloxy-C₁₋₃-alkyl-, C₁₋₃-Alkoxycarbonyl-C₁₋₃-alkyloxy-C₁₋₃-alkyl-, Carboxy-C₁₋₃-alkylamino-C₁₋₃-alkyl-, N-(C₁₋₃-Alkyl)-carboxy-C₁₋₃-alkylamino-C₁₋₃-alkyl-, C₁₋₃-Alkoxycarbonyl-C₁₋₃-alkylamino-C₁₋₃-alkyl-, N- (C₁₋₃-Alkyl)-carboxy-C₁₋₃-alkylamino-C₁₋₃-alkyl- oder N-(C₁₋₃-Alkyl)-C₁₋₃-alkoxycarbonyl-C₁₋₃-alkylamino-C₁₋₃-alkylgruppe substituiert sein können,
durch eine Morpholino-, Piperazino-, 4-Methyl-piperazino-, Piperazino-C₁₋₃-alkyl-, Dihydropyrazolo-, Tetrahydropyrazolo-, Tetrahydroisooxazolo- oder 7-Azabicycloheptylgruppe oder
durch eine gegebenenfalls im Alkylteil durch eine Carboxyoder C₁₋₃-Alkoxycarbonylgruppe substituierte N-(C₁₋₃-Alkyl)-phenyl- oder N-(C₁₋₃-Alkyl)-pyridylaminogruppe substituiert ist,
R₃ ein Wasserstoff-, Fluor-, Chlor- oder Bromatom,
eine Hydroxy-, C₁₋₃-Alkoxy-, Trifluormethyl-, Amino- oder C₂₋₃-Alkanoylaminogruppe,
eine C₁₋₃-Alkylgruppe, die durch eine Hydroxy-, Carboxy-, C₁₋₃-Alkoxycarbonyl-, Carboxy-C₁₋₃-alkoxy-, C₁₋₃-Alkoxycarbonyl-C₁₋₃-alkoxy-, Carboxy-C₁₋₃-alkylaminocarbonyl-, C₁₋₃-Alkoxycarbonyl-C₁₋₃-alkylaminocarbonyl-, N-(C₁₋₃-Alkyl)-carboxy-C₁₋₃-alkylaminocarbonyl- oder N-(C₁₋₃-Alkyl)-C₁₋₃-alkoxycarbonyl-C₁₋₃-alkylaminocarbonylgruppe substituiert sein kann,
eine C₁₋₃-Alkylgruppe, die durch eine Carboxy-C₁₋₃-alkylamino-, C₁₋₃-Alkoxycarbonyl-C₁₋₃-alkylamino-, N- (C₁₋₃-Alkyl)-carboxy-C₁₋₃-alkylamino- oder C₁₋₃-Alkoxycarbonyl-C₁₋₃-alkylaminocarbonylgruppe substituiert ist,
eine durch eine Carboxy- oder C₁₋₃-Alkoxycarbonylgruppe substituierte C₂₋₃-Alkenylgruppe oder
eine gegebenenfalls am Kohlenstoffatom durch eine C₁₋₃-Alkylgruppe substituierte Carbiminogruppe, die am Iminostickstoffatom durch eine Carboxy-C₁₋₃-alkoxy-, C₁₋₃-Alkoxycarbonyl-C₁₋₃-alkoxy- oder Aminocarbonylaminogruppe substituiert ist,
oder R₂ und R₃ zusammen eine -CO-O-CH₂-Gruppe und
R₄ ein Wasserstoff-, Fluor-, Chlor- oder Bromatom, eine C₁₋₃-Alkyl- oder Trifluormethylgruppe darstellen,
X ein Sauerstoff- oder Schwefelatom oder eine gegebenenfalls durch eine C₁₋₃-Alkylgruppe substituierte -NH-Gruppe und
Y eine durch eine Aminogruppe substituierte Cyclohexylgruppe, eine durch eine Amidinogruppe, welche durch eine Benzoyl- oder C₁₋₈-Alkoxycarbonylgruppe substituiert sein kann, substituierte Phenylen- oder Pyridinylengruppe, wobei die vorstehend erwähnte Phenylengruppe durch eine Methyl- oder Methoxygruppe und die vorstehend erwähnte Pyridinylengruppe durch eine Methylgruppe substituiert sein kann, bedeuten,
deren Tautomere, deren Stereoisomere, deren Gemische und deren Salze.

Besonders bevorzugte Verbindungen der vorliegenden Erfindung sind die Verbindungen der allgemeinen Formel 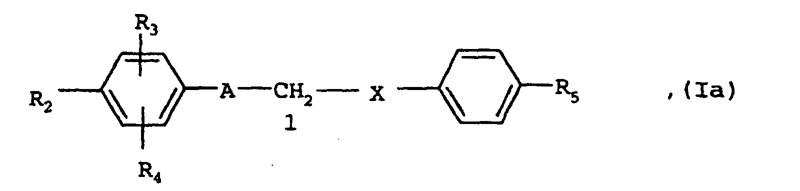 in der
A eine Ethylen- oder Ethinylengruppe,
X ein Sauerstoffatom oder eine gegebenenfalls durch eine Methylgruppe substituierte Iminogruppe,
R₂ eine C₁₋₄-Alkylcarbonylamino- oder C₃₋₅-Cycloalkylcarbonylaminogruppe, die jeweils am Aminstickstoffatom durch eine Carboxy-C₁₋₂-alkyl-, C₁₋₃-Alkoxycarbonyl-C₁₋₂-alkyl-, Carboxy-C₁₋₂-alkylaminocarbonyl-C₁₋₂-alkyl- oder C₁₋₃-Alkoxycarbonyl-C₁₋₂-alkylaminocarbonyl-C₁₋₂-alkylgruppe substituiert ist,
eine C₁₋₄-Alkylamino- oder C₃₋₅-Cycloalkylaminogruppe, die jeweils am Aminstickstoffatom durch eine durch eine gegebenenfalls im Alkylteil durch eine Aminogruppe substituierte Carboxy-C₁₋₃-alkylcarbonyl-, C₁₋₃-Alkoxycarbonyl-C₁₋₃-alkylcarbonyl-, Carboxy-C₁₋₂-alkylaminocarbonyl-C₁₋₂-alkylcarbonyl-, C₁₋₃-Alkoxycarbonyl-C₁₋₂-alkylaminocarbonyl-C₁₋₂-alkylcarbonyl-, Carboxy-C₁₋₂-alkylaminocarbonyl-, C₁₋₃-Alkoxycarbonyl-C₁₋₂-alkylaminocarbonyl-, Carboxy-C₁₋₂-alkylaminocarbonyl-C₁₋₂-alkylaminocarbonyl- oder C₁₋₃-Alkoxycarbonyl-C₁₋₂-alkylaminocarbonyl-C₁₋₂-alkylaminocarbonylgruppe, durch eine Carboxymethyloxymethylcarbonyl-, C₁₋₃-Alkoxycarbonyl-methyloxymethylcarbonyl-, Carboxymethylaminomethylcarbonyl-, C₁₋₃-Alkoxycarbonyl-methylaminomethylcarbonyl-, N-Methyl-carboxymethylaminomethylcarbonyl-, N-Methyl-C₁₋₃-alkoxycarbonyl-carboxymethylaminomethylcarbonyl-, Aminomethylcarbonyl-, 2-Amino-ethylcarbonyl-, Carboxy-C₁₋₂-al kylaminocarbonylmethyloxymethylcarbonyl-, C₁₋₃-Alkoxycarbonyl-C₁₋₂-alkylaminocarbonylmethyloxymethylcarbonyl-, Carboxy-C₁₋₂-alkylaminocarbonylmethylaminomethylcarbonyl-, C₁₋₃-Alkoxycarbonyl-C₁₋₂-alkylaminocarbonylmethylaminomethylcarbonyl-, N-Methyl-carboxy-C₁₋₂-alkylaminocarbonylmethylaminomethylcarbonyloder N-Methyl-C₁₋₃-alkoxycarbonyl-C₁₋₂-alkylaminocarbonylmethylaminomethylcarbonylgruppe substituiert ist, oder
eine Carbonylgruppe, die
durch eine Cyclopentylgruppe,
durch eine C₃₋₅-Alkylgruppe, die zusätzlich durch eine Carboxy- oder C₁₋₃-Alkoxycarbonylgruppe substituiert sein kann,
durch eine am Aminstickstoffatom durch eine C₁₋₄-Alkyl-, Carboxy-C₁₋₃-alkyl- oder C₁₋₃-Alkoxycarbonyl-C₁₋₃-alkylgruppe substituierte C₁₋₄-Alkylamino-, Phenylamino- oder Pyridylaminogruppe,
durch eine durch eine Methyl-, Hydroxymethyl-, Amino-, Carboxy-, C₁₋₃-Alkoxycarbonyl-, Carboxy-C₁₋₂-alkyl-, C₁₋₃-Alkoxycarbonyl-C₁₋₂-alkyl-, Carboxymethyloxymethyl-, C₁₋₃-Alkoxycarbonylmethyloxymethyl-, Carboxymethylaminomethyl-, C₁₋₃-Alkoxycarbonyl-methylaminomethyl-, Carboxymethylaminocarbonylmethyloxymethyl- oder C₁₋₃-Alkoxycarbonylmethylaminocarbonylmethyloxymethylgruppe substituierte Pyrrolidinogruppe substituiert ist,
R₃ ein Wasserstoff-, Fluor-, Chlor- oder Bromatom oder eine Trifluormethylgruppe,
eine gegebenenfalls durch eine Hydroxy-, Carboxy-, C₁₋₃-Alkoxycarbonyl-, Carboxymethyloxy-, C₁₋₃-Alkoxycarbonyl-methyloxy-, Carboxymethylamino-, N-Methyl-carboxymethylamino-, C₁₋₃-Alkoxycarbonylmethylamino-, N-Methyl-C₁₋₃-alkoxycarbonylmethylamino-, Carboxymethylaminocarbonyl- oder C₁₋₃-Alkoxycarbonylmethylaminocarbonylgruppe substituierte C₁₋₂-Alkylgruppe, eine durch eine Carboxy- oder C₁₋₃-Alkoxycarbonylgruppe substituierte Vinylgruppe,
R₄ ein Wasserstoff-, Fluor-, Chlor- oder Bromatom, eine Methyl-, Ethyl- oder Trifluormethylgruppe und
R₅ eine gegebenenfalls durch eine C₁₋₈-Alkoxycarbonyl- oder Benzoylgruppe substituierte Amidinogruppe bedeuten,
deren Tautomere, deren Stereoisomere, deren Gemische und deren Salze.

Ganz besonders bevorzugte Verbindungen der vorliegenden Erfindung sind die Verbindungen der allgemeinen Formel Ia, in der
A eine Ethylen- oder Ethinylengruppe,
X eine Iminogruppe,
R₂ eine C₁₋₄-Alkylaminocarbonylgruppe, die jeweils am Aminstickstoffatom durch eine Carboxy-C₁₋₂-alkyl- oder C₁₋₃-Alkoxycarbonyl-C₁₋₂-alkylgruppe substituiert ist,
eine C₁₋₄-Alkylaminogruppe, die am Aminstickstoffatom durch eine Carboxy-C₁₋₃-alkylcarbonyl-, C₁₋₃-Alkoxycarbonyl-C₁₋₃-alkylcarbonyl-, Carboxy-C₁₋₂-alkylaminocarbonyl- oder C₁₋₃-Alkoxycarbonyl-C₁₋₂-alkylaminocarbonylgruppe gruppe substituiert ist, oder
eine Carbonylgruppe, die
durch eine C₃₋₅-Alkylgruppe, die zusätzlich durch eine Carboxy- oder C₁₋₃-Alkoxycarbonylgruppe substituiert sein kann,
durch eine am Aminstickstoffatom durch eine Carboxy-C₁₋₃-alkyl- oder C₁₋₃-Alkoxycarbonyl-C₁₋₃-alkylgruppe substituierte C₁₋₄-Alkylamino- oder Pyridylaminogruppe,
durch eine gegebenenfalls durch eine Methylgruppe substituierte Pyrrolidinogruppe substituiert ist,
R₃ eine gegebenenfalls durch eine Carboxy- oder C₁₋₃-Alkoxycarbonylgruppe substituierte C₁₋₂-Alkylgruppe,
R₄ ein Wasserstoffatom oder eine Methylgruppe und
R₅ eine gegebenenfalls durch eine C₁₋₈-Alkoxycarbonyl- oder Benzoylgruppe substituierte Amidinogruppe bedeuten,
deren Tautomere, deren Stereoisomere, deren Gemische und deren Salze.

Als besonders bevorzugte Verbindungen seien beispielsweise folgende erwähnt:
(a) rac-4-{3-[5-Ethoxycarbonylmethyl-2-methyl-4-(2-methylpyrrolidinocarbonyl)-phenyl]-propargylamino}benzamidin,
(b) rac-4-{3-[2,5-Dimethyl-4-(2-methyl-pyrrolidinocarbonyl)-phenyl]-propargylamino}benzamidin,
(c) 4-[3-(2,5-Dimethyl-4-isopropylcarbonyl-phenyl)propargylamino]benzamidin,
(d) 4-{3-[2,5-Dimethyl-4-(N-methyl-N-pyridin-2-yl-aminocarbonyl)-phenyl]propargylamino}benzamidin,
(e) 4-{3-[2,5-Dimethyl-4-(N-methyl-N-pyridin-2-yl-aminocarbonyl)-phenyl]prop-1-ylamino}benzamidin,
(f) 4-[3-(3-Methyl-4-pyrrolidinocarbonyl-phenyl)-propargylamino]-benzamidin,
(g) 4-{3-[2,5-Dimethyl-4-(N-(2-methoxycarbonyl-ethyl)-N-ethylcarbonylamino)-phenyl]-propargylamino}benzamidin,
(h) 4-{3-[2,5-Dimethyl-4-(N-isopropyl-N-hydroxycarbonylmethylaminocarbonyl-amino)-phenyl]-propargylamino}-benzamidin und
(i) 4-{3-[2,5-Dimethyl-4-(N-isopropyl-N-hydroxycarbonylmethylcarbonyl-amino)-phenyl]-propargylamino}-benzamidin
sowie deren Salze.

Erfindungsgemäß erhält man die Verbindungen der allgemeinen Formel I nach bekannten Verfahren beispielsweise nach folgenden Verfahren:
a. Umsetzung einer Verbindung der allgemeinen Formel

   Ar - Z₁ , (II)

   in der
   Ar wie eingangs erwähnt definiert ist und
   Z₁ eine Austrittsgruppe wie ein Halogenatom oder eine Sulfonyloxygruppe darstellt, z.B. ein Chlor-, Brom- oder Iodatom oder eine Trifluormethylsulfonyloxygruppe, mit einer Verbindung der allgemeinen Formel

   H - A' - HCR₁ - X - Y' , (III)

   in der
   R₁ und X wie eingangs erwähnt definiert sind,
   Y' die für Y eingangs erwähnten Bedeutungen mit der Maßgabe besitzt, daß eine vorhandene Amino- oder Iminogruppe durch einen üblichen Schutzrest geschützt ist, und
   A' eine Ethinylgruppe darstellt, gegebenenfalls anschließende katalytische Hydrierung und/oder Abspaltung eines verwendeten Schutzrestes.
   Die Umsetzung wird vorzugsweise in einem Lösungsmittel wie Acetonitril, Diethylether, Tetrahydrofuran oder Dimethylformamid in Gegenwart eines Palladium-Katalysators wie Bis(triphenylphosphin)-palladium(II)chorid oder Tetrakis-(triphenylphosphin)-palladium(0) in Gegenwart einer tertiären oder anorganischen Base wie Triethylamin, N-Isopropyl-diethylamin, Kalium-tert.butylat, Natriumcarbonat oder Cäsiumcarbonat und in Gegenwart eines Reaktionsbeschleunigers wie einem Kupferhalogenid wie Kupfer(I)iodid und bei Temperaturen zwischen 20 und 120°C, vorzugsweise bei Temperaturen zwischen 40 und 100°C, durchgeführt (siehe auch K. Sonogashira, Comprehensive Organic Synthesis, Vol. 3, Seite 52ff., Pergamon Press, Oxford 1991).
   Die gegebenenfalls verwendeten Schutzreste und deren Abspaltung wird später beschrieben (siehe auch T. Greene, Protective Groups in Organic Synthesis, Wiley Interscience, New York 1981).
b. Zur Herstellung einer Verbindung der allgemeinen Formel I, in der der Ar-A-Rest eine Carboxygruppe enthält und R₅ wie eingangs erwähnt definiert ist oder der Ar-A-Rest wie eingangs erwähnt definiert ist und R₅ eine Amino-, Amino-C₁₋₃-alkyl-, Amidino- oder Guanidinogruppe darstellt oder der Ar-A-Rest eine Carboxygruppe enthält und R₅ eine Amino-, Amino-C₁₋₃-alkyl-, Amidino- oder Guanidinogruppe darstellt:
   Überführung einer Verbindung der allgemeinen Formel

   Ar' - A - HCR₁ - X - Y" , (IV)

   in der
   A, R₁, und X wie eingangs erwähnt definiert sind,
   Ar' und Y" die für Ar und Y eingangs erwähnten Bedeutungen mit der Maßgabe besitzen, daß
   Ar' eine durch Hydrolyse, Behandeln mit einer Säure oder Base, Thermolyse oder Hydrogenolyse in eine Carboxygruppe überführbare Gruppe enthält und Y" die für Y eingangs erwähnt Bedeutungen aufweist oder
   Ar' die für Ar eingangs erwähnten Bedeutungen aufweist und Y'' eine durch Hydrolyse, Behandeln mit einer Säure oder Base, Thermolyse oder Hydrogenolyse in eine Amino-, Amino-C₁₋₃-alkyl-, Amidino- oder Guanidinogruppe überführbare Gruppe oder
   Ar' eine durch Hydrolyse, Behandeln mit einer Säure oder Base, Thermolyse oder Hydrogenolyse in eine Carboxygruppe überführbare Gruppe enthält und Y" eine durch Hydrolyse, Behandeln mit einer Säure oder Base, Thermolyse oder Hydrogenolyse in eine Amino-, Amino-C₁₋₃-alkyl-, Amidino- oder Guanidinogruppe überführbare Gruppe enthalten,
   mittels Hydrolyse, Behandeln mit einer Säure oder Base, Thermolyse oder Hydrogenolyse in eine Verbindung der allgemeinen Formel I übergeführt wird, in der der Ar-A-Rest eine Carboxygruppe enthält und R₅ wie eingangs erwähnt definiert ist oder der Ar-A-Rest wie eingangs erwähnt definiert ist und R₅ eine Amino-, Amino-C₁₋₃-alkyl-, Amidino- oder Guanidinogruppe darstellt oder der Ar-A-Rest eine Carboxygruppe enthält und R₅ eine Amino-, Amino-C₁₋₃-alkyl-, Amidino- oder Guanidinogruppe darstellt.
   Als eine in eine Carboxygruppe überführbare Gruppe kommt beispielsweise eine durch einen Schutzrest geschützte Carboxylgruppe wie deren funktionelle Derivate, z. B. deren unsubstituierte oder substituierte Amide, Ester, Thioester, Trimethylsilylester, Orthoester oder Iminoester, welche zweckmäßigerweise mittels Hydrolyse in eine Carboxylgruppe übergeführt werden,
   deren Ester mit tertiären Alkoholen, z.B. der tert. Butylester, welche zweckmäßigerweise mittels Behandlung mit einer Säure oder Thermolyse in eine Carboxylgruppe übergeführt werden, und
   deren Ester mit Aralkanolen, z.B. der Benzylester, welche zweckmäßigerweise mittels Hydrogenolyse in eine Carboxylgruppe übergeführt werden, in Betracht.
   Die Hydrolyse wird zweckmäßigerweise entweder in Gegenwart einer Säure wie Salzsäure, Schwefelsäure, Phosphorsäure, Essigsäure, Trichloressigsäure, Trifluoressigsäure oder deren Gemischen oder in Gegenwart einer Base wie Lithiumhydroxid, Natriumhydroxid oder Kaliumhydroxid in einem geeigneten Lösungsmittel wie Wasser, Wasser/Methanol, Wasser/Ethanol, Wasser/-Isopropanol, Methanol, Ethanol, Wasser/Tetrahydrofuran oder Wasser/Dioxan bei Temperaturen zwischen -10 und 120°C, z.B. bei Temperaturen zwischen Raumtemperatur und der Siedetemperatur des Reaktionsgemisches, durchgeführt.
   Enthält eine Verbindung der allgemeinen Formel IV beispielsweise die tert.Butyl- oder tert.Butyloxycarbonylgruppe, so können diese auch durch Behandlung mit einer Säure wie Trifluoressigsäure, Ameisensäure, p-Toluolsulfonsäure, Schwefelsäure, Salzsäure, Phosphorsäure oder Polyphosphorsäure gegebenenfalls in einem inerten Lösungsmittel wie Methylenchlorid, Chloroform, Benzol, Toluol, Diethylether, Tetrahydrofuran oder Dioxan vorzugsweise bei Temperaturen zwischen -10 und 120°C, z.B. bei Temperaturen zwischen 0 und 60°C, oder auch thermisch gegebenenfalls in einem inerten Lösungsmittel wie Methylenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40 und 120°C, abgespalten werden.
   Enthält eine Verbindung der allgemeinen Formel IV beispielsweise die Benzyloxy- oder Benzyloxycarbonylgruppe, so können diese auch hydrogenolytisch in Gegenwart eines Hydrierungskatalysators wie Palladium/Kohle in einem geeigneten Lösungsmittel wie Methanol, Ethanol, Ethanol/Wasser, Eisessig, Essigsäureethylester, Dioxan oder Dimethylformamid vorzugsweise bei Temperaturen zwischen 0 und 50°C, z.B. bei Raumtemperatur, und einem Wasserstoffdruck von 1 bis 5 bar abgespalten werden.
c. Zur Herstellung einer Verbindung der allgemeinen Formel I, in der R₅ eine Amidinogruppe darstellt:
   Umsetzung einer gegebenenfalls im Reaktionsgemisch gebildeten Verbindung der allgemeinen Formel

   Ar - A - HCR₁ - X - Y" , (V)

   in der
   A, Ar, R₁ und X wie eingangs erwähnt definiert sind und Y" einen der für Y eingangs erwähnten Reste mit der Maßgabe bedeutet, daß R₅ eine Z₁-(HN=)C-Gruppe darstellt, in der
   Z₁ eine Alkoxy- oder Aralkoxygruppe wie die Methoxy-, Ethoxy-, n-Propoxy-, Isopropoxy- oder Benzyloxygruppe oder eine Alkylthio- oder Aralkylthiogruppe wie die Methylthio-, Ethylthio-, n-Propylthio- oder Benzylthiogruppe darstellt,
   mit einem Ammoniumsalz wie Diammoniumcarbonat oder Ammoniumacetat.
   Die Umsetzung wird zweckmäßigerweise in einem Lösungsmittel wie Methanol, Ethanol, n-Propanol, Tetrahydrofuran oder Dioxan bei Temperaturen zwischen 0 und 150°C, vorzugsweise bei Temperaturen zwischen 0 und 80°C, durchgeführt.
   Eine Verbindung der allgemeinen Formel V erhält man beispielsweise durch Umsetzung einer entsprechenden Cyanoverbindung mit einem entsprechenden Alkohol wie Methanol, Ethanol, n-Propanol, Isopropanol oder Benzylalkohol in Gegenwart einer Säure wie Salzsäure oder durch Umsetzung eines entsprechenden Amids mit einem Trialkyloxoniumsalz wie Triethyloxonium-tetrafluorborat in einem Lösungsmittel wie Methylenchlorid, Tetrahydrofuran oder Dioxan bei Temperaturen zwischen 0 und 50°C, vorzugsweise jedoch bei 20°C, oder eines entsprechenden Nitrils mit Schwefelwasserstoff zweckmäßigerweise in einem Lösungsmittel wie Pyridin oder Dimethylformamid und in Gegenwart einer Base wie Triethylamin und anschließender Alkylierung des gebildeten Thioamids mit einem entsprechenden Alkyl- oder Aralkylhalogenid.
   Bei der vorstehend beschriebenen Umsetzung kann gleichzeitig an eine elektronenreiche oder elektronenarme Dreifachbindung eine Halogenwasserstoffaddition erfolgen.
d) Zur Herstellung einer Verbindung der allgemeinen Formel I, in der R₅ eine Amidinogruppe darstellt, die durch eine Hydroxygruppe substituiert ist:
   Umsetzung einer gegebenenfalls im Reaktionsgemisch gebildeten Verbindung der allgemeinen Formel

   Ar - A - HCR₁ - X - Y" , (V)

   in der
   A, Ar, R₁ und X wie eingangs erwähnt definiert sind und Y" einen der für Y eingangs erwähnten Reste mit der Maßgabe bedeutet, daß R₅ eine Z₁-(HN=)C-Gruppe darstellt, in der
   Z₁ eine Alkoxy- oder Aralkoxygruppe wie die Methoxy-, Ethoxy-, n-Propoxy-, Isopropoxy- oder Benzyloxygruppe oder eine Alkylthio- oder Aralkylthiogruppe wie die Methylthio-, Ethylthio-, n-Propylthio- oder Benzylthiogruppe darstellt,
   mit Hydroxylamin oder dessen Salzen.
   Die Umsetzung wird zweckmäßigerweise in einem Lösungsmittel wie Methanol, Ethanol, n-Propanol, Wasser, Methanol/Wasser, Tetrahydrofuran, Tetrahydrofuran/Wasser, Dioxan oder Dioxan/-Wasser in Gegenwart einer Base wie Triethylamin bei Temperaturen zwischen 0 und 150°C, vorzugsweise bei Temperaturen zwischen 0 und 80°C, durchgeführt.
e. Zur Herstellung einer Verbindung der allgemeinen Formel I, in der X ein Sauerstoff- oder Schwefelatom, eine Carbonyl-, Imino- oder N-(C₁₋₃-Alkyl)-iminogruppe darstellt:
   Umsetzung einer Verbindung der allgemeinen Formel I

   Ar - A - HCR₁ - Z₂ , (VI)

   in der
   A, Ar und R₁ wie eingangs erwähnt definiert sind und
   Z₂ eine Austrittsgruppe wie ein Halogenatom oder eine Sulfonyloxygruppe, z.B. ein Brom- oder Iodatom, eine Methansulfonyloxy- oder p-Toluolsulfonyloxygruppe, mit einer Verbindung der allgemeinen Formel

   U - Y , (VII)

   in der
   Y wie eingangs erwähnt definiert ist und
   U eine Hydroxy-, Mercapto-, Hydroxycarbonyl-, Imino- oder N-(C₁₋₃-Alkyl)-iminogruppe bedeutet.
   Die Umsetzung wird vorzugsweise in einem Lösungsmittel wie Methanol, Ethanol, Methylenchlorid, Tetrahydrofuran, Toluol, Dioxan, Dimethylsulfoxid oder Dimethylformamid gegebenenfalls in Gegenwart einer anorganischen oder einer tertiären organischen Base, vorzugsweise bei Temperaturen zwischen 20°C und der Siedetemperatur des verwendeten Lösungsmittel, durchgeführt.
f. Zur Herstellung einer Verbindung der allgemeinen Formel I, in der Ar und/oder Y einen in-vivo abspaltbaren Rest enthalten:
   Umsetzung einer Verbindung der allgemeinen Formel

   Ar" - A - HCR₁ - X - Y' " , (VIII)

   in der
   A, R₁, und X wie eingangs erwähnt definiert sind,
   Ar" und Y' " die für Ar und Y eingangs erwähnten Bedeutungen mit der Maßgabe besitzen, daß
   Ar" eine Carboxygruppe enthält und Y' " die für Y eingangs erwähnt Bedeutungen aufweist oder
   Ar" die für Ar eingangs erwähnten Bedeutungen aufweist und Y'" eine Amino-, Amino-C₁₋₃-alkyl-, Amidino- oder Guanidinogruppe enthält oder
   Ar" eine Carboxygruppe und Y'" eine Amino-, Amino-C₁₋₃-alkyl-, Amidino- oder Guanidinogruppe überführbare Gruppe enthalten, mit einer Verbindung der allgemeinen Formel

   Z₃ - R₇ , (IX)

   in der
   R₇ eine C₁₋₈-Alkoxycarbonylgruppe, eine RₐCO-O-(R_{b}CR_{c})-Gruppe oder den Acylrest einer der eingangs erwähnten in vivo abspaltbaren Reste, wobei Rₐ bis R_{c} wie eingangs erwähnt definiert sind, und
   Z₃ eine nukleofuge Austrittsgruppe wie ein Halogenatom, z.B. ein Chlor-, Brom- oder Jodatom, oder eine p-Nitrophenylgruppe oder auch, wenn Ar" eine Carboxygruppe enthält, eine Hydroxygruppe bedeuten.

Die Umsetzung wird vorzugsweise in einem Lösungsmittel wie Methanol, Ethanol, Methylenchlorid, Tetrahydrofuran, Toluol, Dioxan, Dimethylsulfoxid oder Dimethylformamid gegebenenfalls in Gegenwart eines die Säure aktivierenden Mittels oder eines wasserentziehenden Mittels und gegebenenfalls in Gegenwart einer anorganischen oder einer tertiären organischen Base, vorzugsweise bei Temperaturen zwischen 20°C und der Siedetemperatur des verwendeten Lösungsmittel, durchgeführt.

Mit einer Verbindung der allgemeinen Formel IX, in der Z₃ eine nukleofuge Austrittsgruppe darstellt, wird die Umsetzung vorzugsweise in einem Lösungsmittel wie Methylenchlorid, Acetonitril, Tetrahydrofuran, Toluol, Dimethylformamid oder Dimethylsulfoxid gegebenenfalls in Gegenwart einer Base wie Natriumhydrid, Kaliumcarbonat, Kalium-tert.butylat oder N-Ethyl-diisopropylamin bei Temperaturen zwischen 0 und 60°C, durchgeführt.

Mit einer Verbindung der allgemeinen Formel IX, in der Z₃ eine Hydroxygruppe darstellt, wird die Umsetzung gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan in Gegenwart eines wasserentziehenden Mittels, z.B. in Gegenwart von Chlorameisensäureisobutylester, Orthokohlensäuretetraethylester, Orthoessigsäuretrimethylester, 2,2-Dimethoxypropan, Tetramethoxysilan, Thionylchlorid, Trimethylchlorsilan, Phosphortrichlorid, Phosphorpentoxid, N,N'-Dicyclohexylcarbodiimid, N,N'-Dicyclohexylcarbodiimid/N-Hydroxysuccinimid, N,N'-Dicyclohexylcarbodiimid/1-Hydroxy-benztriazol, 2-(1H-Benzotriazol-1-yl)-1,1,3,3-tetramethyluronium-tetrafluorborat, 2-(1H-Benzotriazol-1-yl)-1,1,3,3-tetramethyluronium-tetrafluorborat/1-Hydroxy-benztriazol, N,N'-Carbonyldiimidazol oder Triphenylphosphin/Tetrachlorkohlenstoff, und gegebenenfalls unter Zusatz einer Base wie Pyridin, 4-Dimethylaminopyridin, N-Methyl-morpholin oder Triethylamin zweckmäßigerweise bei Temperaturen zwischen 0 und 150°C, vorzugsweise bei Temperaturen zwischen 0 und 100°C, durchgeführt.

Bedeutet in einer Verbindung der allgemeinen Formel IX Z₃ eine Hydroxygruppe, so kann die Umsetzung auch mit einem seiner reaktionsfähigen Derivate wie deren Ester, Imidazolide oder Halogeniden vorzugsweise in einem Lösungsmittel wie Methylenchlorid oder Ether und vorzugsweise in Gegenwart einer tertiären organische Base wie Triethylamin, N-Ethyl-diisopropylamin oder N-Methyl-morpholin bei Temperaturen zwischen 0 und 150°C, vorzugsweise bei Temperaturen zwischen 50 und 100°C, durchgeführt.

Erhält man erfindungsgemäß eine Verbindung der allgemeinen Formel I, in der R₅ eine Amidinogruppe darstellt, so kann diese durch Alkylierung mit einem Halogenessigsäurederivat, durch anschließende Hydrolyse und Decarboxylierung in eine durch eine oder zwei Methylgruppen substituierte entsprechende Amidinoverbindung übergeführt werden und/oder
eine Verbindung der allgemeinen Formel I, in der R₅ eine Hydroxyamidinogruppe darstellt, so kann diese mittels katalytischer Hydrierung in eine entsprechende Amidinoverbindung übergeführt werden und/oder
eine Verbindung der allgemeinen Formel I, die eine Doppeloder Dreifachbindung enthält, so kann diese mittels katalytischer Hydrierung in eine entsprechende gesättigte Verbindung übergeführt werden und/oder
eine Verbindung der allgemeinen Formel I, in der X ein Schwefelatom darstellt, so kann diese mittels Oxidation in eine entsprechende Sulfinyl- oder Sulfonylverbindung übergeführt werden und/oder
eine Verbindung der allgemeinen Formel I, in der R₂ eine Tetrahydropyrazolocarbonylgruppe darstellt, so kann diese mittels Oxidation in eine entsprechende 4,5-Dihydropyrazolocarbonyl-Verbindung übergeführt werden und/oder
eine Verbindung der allgemeinen Formel I, die eine Carbonylgruppe enthält, so kann diese mittels einem entsprechenden Oxim in eine entsprechende Oximverbindung übergeführt werden und/oder
eine Verbindung der allgemeinen Formel I, die eine Carboxygruppe enthält, so kann diese mittels eines entsprechenden Amins in ein entsprechendes Amid übergeführt werden.

Die anschließende Alkylierung wird zweckmäßigerweise in einem Lösungsmittel wie Methylenchlorid, Tetrahydrofuran, Dioxan, Dimethylsulfoxid, Dimethylformamid oder Aceton gegebenenfalls in Gegenwart eines Reaktionsbeschleunigers wie Natrium- oder Kaliumiodid und vorzugsweise in Gegenwart einer Base wie Natriumcarbonat oder Kaliumcarbonat oder in Gegenwart einer tertiären organischen Base wie N-Ethyl-diisopropylamin oder N-Methyl-morpholin, welche gleichzeitig auch als Lösungsmittel dienen können, oder gegebenenfalls in Gegenwart von Silberkarbonat oder Silberoxid bei Temperaturen zwischen -30 und 100°C, vorzugsweise jedoch bei Temperaturen zwischen -10 und 80°C, durchgeführt.

Die anschließende Hydrolyse wird zweckmäßigerweise entweder in Gegenwart einer Säure wie Salzsäure, Schwefelsäure, Phosphorsäure, Essigsäure, Trichloressigsäure, Trifluoressigsäure oder deren Gemischen oder in Gegenwart einer Base wie Lithiumhydroxid, Natriumhydroxid oder Kaliumhydroxid in einem geeigneten Lösungsmittel wie Wasser, Wasser/Methanol, Wasser/Ethanol, Wasser/Isopropanol, Methanol, Ethanol, Wasser/Tetrahydrofuran oder Wasser/Dioxan, durchgeführt.

Die anschließende Decarboxylierung wird in Gegenwart einer Säure wie vorstehend beschrieben bei Temperaturen zwischen -10 und 120°C, z.B. bei Temperaturen zwischen Raumtemperatur und der Siedetemperatur des Reaktionsgemisches, durchgeführt.

Die nachträgliche katalytische Hydrierung wird vorzugsweise in Gegenwart eines Hydrierungskatalysators wie Palladium/Kohle und in einem geeigneten Lösungsmittel wie Methanol, Ethanol, Ethanol/Wasser, Eisessig, Essigsäureethylester, Dioxan oder Dimethylformamid vorzugsweise bei Temperaturen zwischen 0 und 50°C, z.B. bei Raumtemperatur, und einem Wasserstoffdruck von 1 bis 5 bar durchgeführt.

Die nachträgliche Oxidation wird vorzugsweise in einem Lösungsmittel oder Lösungsmittelgemisch, z.B. in Wasser, Wasser/Pyridin, Aceton, Methylenchlorid, Essigsäure, Essigsäure/-Acetanhydrid, verdünnter Schwefelsäure oder Trifluoressigsäure, je nach dem verwendeten Oxidationsmittel zweckmäßigerweise bei Temperaturen zwischen -80 und 100°C durchgeführt.

Zur Herstellung einer entsprechenden Sulfinylverbindung der allgemeinen Formel I wird die Oxidation zweckmäßigerweise mit einem Äquivalent des verwendeten Oxidationsmittels durchgeführt, z.B. mit Wasserstoffperoxid in Eisessig, Trifluoressigsäure oder Ameisensäure bei 0 bis 20°C oder in Aceton bei 0 bis 60°C, mit einer Persäure wie Perameisensäure in Eisessig oder Trifluoressigsäure bei 0 bis 50°C oder mit m-Chlorperbenzoesäure in Methylenchlorid, Chloroform oder Dioxan bei -20 bis 80°C, mit Natriummetaperjodat in wäßrigem Methanol oder Ethanol bei -15 bis 25°C, mit Brom in Eisessig oder wäßriger Essigsäure gegebenenfalls in Gegenwart einer schwachen Base wie Natriumacetat, mit N-Bromsuccinimid in Ethanol, mit tert.-Butylhypochlorit in Methanol bei -80 bis -30°C, mit Jodbenzodichlorid in wäßrigem Pyridin bei 0 bis 50°C, mit Salpetersäure in Eisessig bei 0 bis 20°C, mit Chromsäure in Eisessig oder in Aceton bei 0 bis 20°C und mit Sulfurylchlorid in Methylenchlorid bei -70°C, der hierbei erhaltene Thioether-Chlor-Komplex wird zweckmäßigerweise mit wäßrigem Ethanol hydrolysiert.

Zur Herstellung einer Sulfonylverbindung der allgemeinen Formel I wird die Oxidation ausgehend von einer entsprechenden Sulfinylverbindung zweckmäßigerweise mit einem oder mehr Äquivalenten des verwendeten Oxidationsmittels oder ausgehend von einer entsprechenden Sulfenylverbindung zweckmäßigerweise mit zwei oder mehr Äquivalenten des verwendeten Oxidationsmittels durchgeführt, z.B. mit Wasserstoffperoxid in Eisessig/Acetanhydrid, Trifluoressigsäure oder in Ameisensäure bei 20 bis 100°C oder in Aceton bei 0 bis 60°C, mit einer Persäure wie Perameisensäure oder m-Chlorperbenzoesäure in Eisessig, Trifluoressigsäure, Methylenchlorid oder Chloroform bei Temperaturen zwischen 0 und 60°C, mit Salpetersäure in Eisessig bei 0 bis 20°C, mit Chromsäure, Natriumperjodat oder Kaliumpermanganat in Essigsäure, Wasser/Schwefelsäure oder in Aceton bei 0 bis 20°C.

Zur Herstellung einer 4,5-Dihydropyrazolocarbonylverbindung der allgemeinen Formel I kann die Oxidation auch mittels Luftsauerstoff in einem der oben erwähnten Lösungsmittel bei Raumtemperatur durchgeführt werden.

Die nachträgliche Oximbildung wird zweckmäßigerweise in einem Lösungsmittel wie Methanol/Toluol in Gegenwart eines wasserentziehenden Mittels wie Molekularsieb vorzugsweise bei der Siedetemperatur des verwendeten Lösungsmittels durchgeführt.

Die nachträgliche Amidbildung wird vorzugsweise in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan in Gegenwart eines wasserentziehenden Mittels, z.B. in Gegenwart von Chlorameisensäureisobutylester, Orthokohlensäuretetraethylester, Orthoessigsäuretrimethylester, 2,2-Dimethoxypropan, Tetramethoxysilan, Thionylchlorid, Trimethylchlorsilan, Phosphortrichlorid, Phosphorpentoxid, N,N'-Dicyclohexylcarbodiimid, N,N'-Dicyclohexylcarbodiimid/N-Hydroxysuccinimid, N,N'-Dicyclohexylcarbodiimid/1-Hydroxy-benztriazol, 2-(1H-Benzotriazol-1-yl)-1,1,3,3-tetramethyluronium-tetrafluorborat, 2-(1H-Benzotriazol-1-yl)-1,1,3,3-tetramethyluronium-tetrafluorborat/1-Hydroxy-benztriazol, N,N'-Carbonyldiimidazol oder Triphenylphosphin/Tetrachlorkohlenstoff, und gegebenenfalls unter Zusatz einer Base wie Pyridin, 4-Dimethylaminopyridin, N-Methyl-morpholin oder Triethylamin zweckmäßigerweise bei Temperaturen zwischen 0 und 150°C, vorzugsweise bei Temperaturen zwischen 0 und 100°C, durchgeführt.

Bei den vorstehend beschriebenen Umsetzungen können gegebenenfalls vorhandene reaktive Gruppen wie Hydroxy-, Carboxy-, Amino-, Alkylamino- oder Iminogruppen während der Umsetzung durch übliche Schutzgruppen geschützt werden, welche nach der Umsetzung wieder abgespalten werden (siehe auch T. Greene, Protective Groups in Organic Synthesis, Wiley Interscience, New York 1981).

Beispielsweise kommt als Schutzrest für eine Hydroxygruppe die Trimethylsilyl-, Acetyl-, Benzoyl-, tert.Butyl-, Trityl-, Benzyl- oder Tetrahydropyranylgruppe,
als Schutzreste für eine Carboxylgruppe die Trimethylsilyl-, Methyl-, Ethyl-, tert.Butyl-, Benzyl- oder Tetrahydropyranylgruppe und
als Schutzrest für eine Amino-, Alkylamino- oder Iminogruppe die Acetyl-, Trifluoracetyl-, Benzoyl-, Ethoxycarbonyl-, tert.Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wäßrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid oder mittels Etherspaltung, z.B. in Gegenwart von Jodtrimethylsilan, bei Temperaturen zwischen 0 und 100°C, vorzugsweise bei Temperaturen zwischen 10 und 50°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure bei Temperaturen zwischen 0 und 50°C, vorzugsweise jedoch bei Raumtemperatur, und bei einem Wasserstoffdruck von 1 bis 7 bar, vorzugsweise jedoch von 3 bis 5 bar.

Die Abspaltung eines tert.Butyl- oder tert.Butyloxycarbonylrestes erfolgt vorzugsweise durch Behandlung mit einer Säure wie Trifluoressigsäure oder Salzsäure gegebenenfalls unter Verwendung eines Lösungsmittels wie Wasser, Methylenchlorid, Diethylether, Tetrahydrofuran oder Dioxan.

Die Abspaltung eines Allyloxycarbonylrestes erfolgt durch Behandlung mit einer katalytischen Menge Tetrakis-(triphenylphosphin)-palladium(O) vorzugsweise in einem Lösungsmittel wie Tetrahydrofuran und vorzugsweise in Gegenwart eines Überschusses von einer Base wie Morpholin oder 1,3-Dimedon bei Temperaturen zwischen 0 und 100°C, vorzugsweise bei Raumtemperatur und unter Inertgas, oder durch Behandlung mit einer katalytischen Menge von Tris-(triphenylphosphin)-rhodium(I)chlorid in einem Lösungsmittel wie wässrigem Ethanol und gegebenenfalls in Gegenwart einer Base wie 1,4-Diazabicyclo[2.2.2]-octan bei Temperaturen zwischen 20 und 70°C.

Die als Ausgangsstoffe verwendeten Verbindungen der allgemeinen Formeln II bis IX, welche teilweise literaturbekannt sind, erhält man nach literaturbekannten Verfahren, des weiteren wird ihre Herstellung in den Beispielen beschrieben.

So erhält man beispielsweise eine Verbindung der allgemeinen Formel II durch Umsetzung eines entsprechenden substituierten Halogenbenzols mit einer entsprechenden Verbindung,
eine Verbindung der allgemeinen Formel III durch Umsetzung eines entsprechenden Anilins mit einem Propargylhalogenid und anschließende Überführung des so erhaltenen substituierten Anilins in eine Verbindung der allgemeinen Formel III nach bekannten Methoden, z.B. durch Pinner-Reaktion, sowie
die Verbindungen der allgemeinen Formeln IV, V, VI und VIII zweckmäßigerweise nach üblichen Methoden wie sie in der vorliegenden Erfindung beschrieben werden.

Ferner können die erhaltenen Verbindungen der allgemeinen Formel I in ihre Enantiomeren und/oder Diastereomeren sowie die erhaltenen Verbindungen der allgemeinen Fotmel I, die eine Doppelbindung enthalten in ihre cis/trans-Isomere aufgetrennt werden.

So lassen sich beispielsweise die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestes 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren-Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Apfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise der (+)- oder (-)-Menthyloxycarbonylrest in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Methansulfonsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine Carboxygruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Cyclohexylamin, Ethanolamin, Diethanolamin und Triethanolamin in Betracht.

Wie bereits eingangs erwähnt, weisen die neuen Verbindungen der allgemeinen Formel I und deren Salze wertvolle Eigenschaften auf.

So weisen die Verbindungen der allgemeinen Formel I, in denen Y keine Cyanogruppe enthält, wertvolle pharmakologische Eigenschaften auf, insbesondere eine antithrombotische Wirkung, welche vorzugsweise auf einer Thrombin oder Faktor Xa beeinflussenden Wirkung beruht, beispielsweise auf einer thrombinhemmenden oder Faktor Xa-hemmenden Wirkung, auf einer die aPTT-Zeit verlängernden Wirkung und auf einer Hemmwirkung auf verwandte Serinproteasen wie z. B. Trypsin, Urokinase Faktor VIIa; Faktor IX, Faktor XI und Faktor XII, und die Verbindungen der allgemeinen Formel I, in denen Y eine Cyanogruppe enthält, stellen wertvolle zwischenprodukte zur Herstellung der Verbindungen der allgemeinen Formel I dar, in der R₅ eine gegebenenfalls substituierte Aminomethyl-, Amidino- oder Guanidinomethylgruppe darstellt.

### Beispielsweise wurden die Verbindungen

A = rac-4-{3-[5-Ethoxycarbonylmethyl-2-methyl-4-(2-methylpyrrolidinocarbonyl)-phenyl]-propargylamino}benzamidin,
B = rac-4-{3-[2,5-Dimethyl-4-(2-methyl-pyrrolidinocarbonyl)-phenyl]-propargylamino}benzamidin,
C = 4-[3-(2,5-Dimethyl-4-isopropylcarbonyl-phenyl)propargylamino]benzamidin,
D = 4-{3-[2,5-Dimethyl-4-(N-methyl-N-pyridin-2-yl-aminocarbonyl)-phenyl]propargylamino}benzamidin,
E = 4-{3-[2,5-Dimethyl-4-(N-methyl-N-pyridin-2-yl-aminocarbonyl)-phenyl]prop-1-ylamino}benzamidin,
F = 4-[3-(3-Methyl-4-pyrrolidinocarbonyl-phenyl)-propargylamino]-benzamidin,
G = 4-{3-[2,5-Dimethyl-4-(N-(2-methoxycarbonyl-ethyl)-N-ethylcarbonylamino)-phenyl]-propargylamino}benzamidin,
H = 4-{3-[2,5-Dimethyl-4-(N-isopropyl-N-hydroxycarbonylmethylaminocarbonyl-amino)-phenyl]-propargylamino}-benzamidin und
I = 4-{3-[2,5-Dimethyl-4-(N-isopropyl-N-hydroxycarbonylmethylcarbonyl-amino)-phenyl]-propargylamino}-benzamidin
auf ihre Wirkung auf die aPTT-Zeit-Verlängerung wie folgt untersucht:
Material:-Plasma, aus humanem Citratblut,
- PTT-Reagenz, Boehringer Mannheim (524298),
- Calcium-Lösung (0.025 Mol/l), Behring Werke, Marburg (ORH 056/57),
- Diethylbarbituratacetat-Puffer, Behring Werke, Marburg (ORWH 60/61),
- Biomatic B10 Koagulometer, Desaga, Wiesloch.

### Durchführung:

Die Bestimmung der aPTT-Zeit erfolgte mit einem Biomatic B10-Koagulometer der Firma Desaga.

Die Testsubstanz wird in die vom Hersteller vorgeschriebenen Testgefäßen mit 0,1 ml humanem Citrat-Plasma und 0,1 ml PTT-Reagenz gegeben. Der Ansatz wird für drei Minuten bei 37°C inkubiert. Durch Zugabe von 0.1 ml Calcium-Lösung wird die Gerinnungsreaktion gestartet. Gerätebedingt erfolgt mit der Eingabe der Calcium-Lösung die Messung der Zeit bis zur Gerinnung des Ansatzes. Als Kontrolle dienten Ansätze bei denen 0,1 ml DBA-Puffer zugegeben wird.

Gemäß der Definition wird über eine Dosis-Wirkungskurve die effektive Substanzkonzentration ermittelt, bei der die aPTT-Zeit gegenüber der Kontrolle verdoppelt wird.

Die nachfolgende Tabelle enthält die gefundenen Werte:

| Substanz | aPTT-Zeit (ED₂₀₀ in µM) |
|---|---|
| A | 0.23 |
| B | 0.45 |
| C | 0.97 |
| D | 0.23 |
| E | 0.69 |
| F | 0.54 |
| G | 0.29 |
| H | 0.20 |
| I | 0.45 |

Aufgrund ihrer pharmakologischen Eigenschaften eignen sich die neuen Verbindungen und deren physiologisch verträglichen Salze zur Vorbeugung und Behandlung venöser und arterieller thrombotischer Erkrankungen, wie zum Beispiel der Behandlung von tiefen Beinvenen-Thrombosen, der Verhinderung von Reocclusionen nach Bypass-Operationen oder Angioplastie (PT(C)A), sowie der Occlusion bei peripheren arteriellen Erkrankungen wie Lungenembolie, der disseminierten intravaskulären Gerinnung, der Prophylaxe der Koronarthrombose, der Prophylaxe des Schlaganfalls und der Verhinderung der Occlusion von Shunts. Zusätzlich sind die erfindungsgemäßen Verbindungen zur antithrombotischen Unterstützung bei einer thrombolytischen Behandlung, wie zum Beispiel mit rt-PA oder Streptokinase, zur Verhinderung der Langzeitrestenose nach PT(C)A, zur Verhinderung der Metastasierung und des Wachstums von koagulationsabhängigen Tumoren und von fibrinabhängigen Entzündungsprozessen geeignet.

Die zur Erzielung einer entsprechenden Wirkung erforderliche Dosierung beträgt zweckmäßigerweise bei intravenöser Gabe 0,1 bis 30 mg/kg, vorzugsweise 0,3 bis 10 mg/kg, und bei oraler Gabe 0,1 bis 50 mg/kg, vorzugsweise 0,3 bis 30 mg/kg, jeweils 1 bis 4 x täglich. Hierzu lassen sich die erfindungsgemäß hergestellten Verbindungen der Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen oder Zäpfchen einarbeiten.

Die nachfolgenden Beispiele sollen die Erfindung näher erläutern:

### Beispiel 1

### rac-N-tert.Butoxycarbonyl-4-{3-[5-ethoxycarbonylmethyl-2-methyl-4-(2-methyl-pyrrolidinocarbonyl)-phenyl]propargylamino}benzamidin

### a. 4-Brom-2,5-dimethyl-benzoesäure

Bei -78°C wird zu einer Lösung aus 43.63 g (0.162 Mol) 2,5-Dibrom-p-xylol, 100 ml (0.16 Mol) einer 1.6 molaren n-Butyl-lithium-Lösung in Hexan zugetropft und 1 Stunde nachgerührt. Anschließend leitet man 4 Stunden lang trockenes Kohlendioxid in die Lösung ein. Es wird langsam auf Raumtemperatur erwärmt und 16 Stunden nachgerührt. Nach langsamer Zugabe von 210 ml 2N Salzsäure, trennt man die Phasen, extrahiert die Wasserphase 2 x mit je 200 ml Ether, wäscht die vereinigten organischen Phasen mit konz. NaCl-Lösung und trocknet mit Na₂SO₄. Nach dem Entfernen des Lösungsmittels im Vakuum wird das Rohprodukt mit 200 ml 2N NaOH versetzt und die erhaltene braune Lösung 3 x mit 100 ml Diethylether extrahiert. Die wäßrige Phase wird mit konz. HCl angesäuert und der daraufhin ausfallende Niederschlag abgesaugt, mit Eiswasser gewaschen und getrocknet.
Ausbeute: 34.99 g (94 % der Theorie),
R_{f}-Wert: 0.55 (Kieselgel; Essigester/Petrolether = 2:1)

### b. 4-Brom-2-ethoxycarbonylmethyl-5-methyl-benzoesäure

Zu einer auf -78°C gekühlten Lösung, welche aus 4.2 ml (30 mMol) Diiisopropylamin und 19 ml einer 1.6 molaren n-Butyl-lithium-Lösung in Hexan hergestellt wird, in 35 ml Tetrahydrofuran wird innerhalb von 2.5 Stunden eine Lösung von 1.7 ml (14 mMol) Diethylcarbonat und 2.3 g (10 mMol) 4-Brom-2,5-dimethyl-benzoesäure in 15 ml Tetrahydrofuran zugetropft. Anschließend wird auf 0°C erwärmt, in 200 ml 3%ige NH₄Cl-Lösung gegossen, mit Essigsäure auf pH 6 gebracht und mit Essigester ausgeschüttelt. Die Essigesterphase wird mit 14%iger NaCl- Lösung gewaschen und mit Na₂SO₄ getrocknet. Das Lösungsmittel wird abdestilliert und der verbleibende Rückstand mehrmals in wenig Diisopropylether/Petrolether digeriert und anschließend getrocknet.
Ausbeute: 1.95 g (65 % der Theorie),
R_{f}-Wert: 0.35 (Kieselgel; Essigester/Petrolether = 3:7 + 1 Tropfen Eisessig)

### c. rac-N-(4-Brom-2-ethoxycarbonylmethyl-5-methyl-benzoyl)-2-methyl-pyrrolidin

Zu einer Lösung aus 1.9 g (6.31 mMol) 4-Brom-2-ethoxycarbonylmethyl-5-methyl-benzoesäure in 660 ml Tetrahydrofuran/H₂O (9:1) wird nacheinander 2.2 g (6.85 mMol) O-(Benzotriazol-1-yl)-N,N,N',N'-tetramethyluroniumtetrafluoroborat, 2.41 ml (13.9 mMol) N,N-Diisopropyl-ethylamin und 0.27 g (2.0 mMol) 1-Hydroxy-1H-benzotriazol zugegeben. Nach 10-minütigem Rühren gibt man 0.59 g (6.94 mMol) rac-2-Methyl-pyrrolidin zu, rührt 19 Stunden nach und verdünnt anschließend mit 200 ml Essigester. Die erhaltene Lösung wird mit 14%iger NaCl-Lösung gewaschen und 2 x mit Essigester extrahiert. Die vereinigten organischen Phasen werden mit 14%iger NaCl-Lösung gewaschen und mit Na₂SO₄ getrocknet. Nach Abdestillieren des Lösungsmittels und Flash-Chromatographie (Kieselgel; Methylenchlorid/-Ethanol = 98:2) erhält man die gewünschte Verbindung. Ausbeute: 2.00 g (86 % der Theorie),
R_{f}-Wert: 0.3 (Kieselgel; Essigester/Petrolether = 3:7 + 1 Tropfen Eisessig)
C₁₇H₂₂BrNO₃ (368.27)
Massenspektrum: M⁺ = 367/369 (Bromisotope)
(M+H)⁺ = 368/370 (Bromisotope)
(M-H)⁻ = 366/368 (Bromisotope)

### d. 4-Propargylamino-benzonitril

Eine Lösung aus 23.6 g (0.20 Mol) 4-Amino-benzonitril, 16.6 ml (0.22 Mol) Propargylbromid und 38.3 ml (0.22 Mol) Diisopropylethylamin werden in 500 ml Toluol 27 Stunden auf 90°C erwärmt. Anschließend wird mit Essigester verdünnt, 3 x mit H₂O gewaschen und über MgSO₄ getrochnet. Nach Abdestillieren des Lösungsmittels wird das Rohprodukt durch Flash-Chromatographie (Kieselgel; Essigester/Petrolether = 20:80 bis 75:25) gereinigt.
Ausbeute: 22.9 g (73 % der Theorie),
R_{f}-Wert: 0.38 (Kieselgel; Essigester/Petrolether = 3:7)

### e. 4-Propargylamino-benzamidin

Eine Lösung aus 5.0 g (32 mMol) 4-Propargylamino-benzonitril wird in 150 ml mit Chlorwasserstoffgas gesättigtem Ethanol erst 3 Stunden bei.0°C, dann 21 Stunden bei Raumtemperatur gerührt. Das Lösungsmittel wird bei maximal 30°C Badtemperatur im Vakuum entfernt und durch 250 ml absolutem Ethanol ersetzt. Anschließend setzt man 10.7 g (0.11 Mol) Ammoniumcarbonat zu und rührt 36 Stunden. Das Lösungsmittel wird abdestilliert, der Rückstand wird in 200 ml Methylenchlorid/Ethanol (94:6) aufgenommen, von unlöslichen Bestandteilen abfiltriert, konzentriert und durch Flash-Chromatographie (Kieselgel; Methylenchlorid/Ethanol = 90:10 bis 75:25) gereinigt.
Ausbeute: 6.52 g (97 % der Theorie),
R_{f}-Wert: 0.22 (Kieselgel; Methylenchlorid/Ethanol = 80:20)

### f. N-(4-tert.Butoxycarbonylamidino-phenyl)-propargylamin

Eine Lösung aus 1.9 g (9.06 mMol) 4-Propargylamino-benzamidin und 2.35 g (10.8 mMol) Pyrokohlensäure-di-tert.butylester in 50 ml Tetrahydrofuran wird bei 5°C langsam mit 45 ml 0.2N NaOH versetzt und 2.5 Stunden bei Raumtemperatur nachgerührt. Das Lösungsmittel wird abdestilliert, der feste Rückstand wird mit H₂O gewaschen und getrocknet.
Ausbeute: 2.2 g (88 % der Theorie),
R_{f}-Wert: 0.41 (Reversed Phase Kieselgel RP-8; Methanol/5%ige NaCl-Lösung = 60:40)

### g. rac-N-tert.Butoxycarbonyl-4-{3-[5-ethoxycarbonylmethyl-2-methyl-4-(2-methyl-pyrrolidinocarbonyl)-phenyl]propargylamino}benzamidin

Eine Lösung aus 1.00 g (2.72 mMol) N-(4-Brom-2-ethoxycarbonylmethyl-5-methyl-benzoyl)-2-methyl-pyrrolidin, 1.11 g (4.07 mMol) N-tert.Butoxycarbonyl-4-propargylamino-benzamidin und 10.4 ml (13.5 mMol) Triethylamin in 3.0 ml Acetonitril wird 15 Minuten unter Stickstoff gerührt, dann nacheinander mit 0.32 g (0.277 mMol) Tetrakis-(triphenylphosphin)-palladium(0) und 0.11 g (0.578 mMol) Kupfer(I)iodid versetzt und 1 Stunden bei 90°C gerührt. Anschließend wird das Lösungsmittel abdestilliert und das Rohprodukt durch Flash-Chromatographie (Kieselgel; Methylenchlorid/Ethanol = 99:1 bis 95:5) gereinigt.
Ausbeute: 0.18 g (12 % der Theorie),
R_{f}-Wert: 0.3 (Kieselgel; Methylenchlorid:Ethanol = 19:1) C₃₂H₄₀N₄O₅ (560.69)
Massenspektrum: (M+H)⁺ = 561
(M-H)⁻ = 559

### Beispiel 2

### rac-4-{3-[5-Ethoxycarbonylmethyl-2-methyl-4-(2-methyl-pyrrolidinocarbonyl)-phenyl]-propargylamino}benzamidin

Eine Lösung aus 0.36 g (0.642 mMol) rac-N-tert.Butoxycarbonyl-4-{3-[5-ethoxycarbonylmethyl-2-methyl-4-(2-methyl-pyrrolidinocarbonyl)-phenyl]propargylamino}benzamidin in 20 ml Methylenchlorid und 3 ml Trifluoressigsäure wird 4 Stunden gerührt. Anschließend wird das Lösungsmittel abdestilliert und das Rohprodukt durch Flash-Chromatographie (Kieselgel; Methylenchlorid/Ethanol = 95:5 bis 80:20) gereinigt.
Ausbeute: 0.20 g (54 % der Theorie),
R_{f}-Wert: 0.3 (Reversed Phase Kieselgel RP-8; Methanol/5%ige NaCl-Lösung = 60:40)
C₂₇H₃₂N₄O₃ x CF₃COOH (460.58/574.61)
Massenspektrum: (M+H)⁺ = 461

### Beispiel 3

### rac-4-{3-[5-Hydroxycarbonylmethyl-2-methyl-4-(2-methyl-pyrrolidinocarbonyl)-phenyl]-propargylamino}benzamidin

Ein Gemisch aus 146 mg (0.254 mMol) rac-4-{3-[5-Ethoxycarbonylmethyl-2-methyl-4-(2-methyl-pyrrolidinocarbonyl)-phenyl]-propargylamino)benzamidin, 3.5 ml Tetrahydrofuran, 2.5 ml H₂O und 1.3 ml 1N LiOH-Lösung wird 5 Stunden gerührt. Dann gibt man 78 mg (1.45 mMol) Ammoniumchlorid zu, rührt 16 Stunden nach und destilliert das Lösungsmittel ab. Der Rückstand wird mit H₂O verrieben, abgesaugt und mit wenig H₂O gewaschen.
Ausbeute: 35 mg (29 % der Theorie),
R_{f}-Wert: 0.35 (Reversed Phase Kieselgel RP-8; Methanol/5%ige NaCl-Lösung = 60:40)
C₂₅H₂₈N₄O₃ x HCl (432.53/468.99)
Massenspektrum: (M+H)⁺ = 433
(M-H)⁻ = 431

### Beispiel 4

### 4-{3-Methyl-4-[(thiazol-2-yl)carbonyl]-phenyl]-propargylamino}benzamidin

### a. 2-(4-Brom-2-methyl-benzoyl)-thiazol

2.0 g (13 mMol) 2-Trimethylsilylthiazol und 6.1 g (26 mMol) 4-Brom-2-methyl-benzoesäurechlorid werden unter Eisbadkühlung vereinigt und 3 Stunden auf 80°C erhitzt. Das Rohprodukt wird in 30 ml Essigester aufgenommen, mit H₂O, gesättigter NaHCO₃-Lösung und H₂O gewaschen, getrocknet und durch Flash-Chromatographie (Kieselgel; Methylenchlorid) gereinigt.
Ausbeute: 1.4 g (40 % der Theorie),
R_{f}-Wert: 0.65 (Kieselgel; Essigester/Petrolether = 20:80)

### b. 4-{3-[3-Methyl-4-[(thiazol-2-yl)carbonyl]-phenyl]-propargylamino}benzonitril

Hergestellt analog Beispiel 1g aus 2-(4-Brom-2-methyl-benzoyl)-thiazol, 4-Propargylamino-benzonitril, Tetrakis(triphenylphosphin)palladium(0), Kupfer(I)iodid und Triethylamin in Acetonitril.
Ausbeute: 72% der Theorie,
R_{f}-Wert: 0.43 (Kieselgel; Essigester/Petrolether = 20:80)

### c. 4-{3-[3-Methyl-4-[(thiazol-2-yl)carbonyl]-phenyl]-propargylamino}benzamidin

0.7 g (2 mMol) 4-{3-[3-Methyl-4-[(thiazol-2-yl)carbonyl]-phenyl]-propargylamino}benzonitril wird in 50 ml mit Chlorwasserstoffgas gesättigtem Ethanol zuerst 2 Stunden bei 0°C, dann 6 Stunden bei Raumtemperatur gerührt. Das Lösungsmittel wird bei maximal 30°C Badtemperatur im Vakuum entfernt und durch 50 ml absolutem Ethanol ersetzt. Man setzt 1.4 g Ammoniumcarbonat zu und rührt 16 Stunden. Anschließend wird das Lösungsmittel abdestilliert und der erhaltene Rückstand durch Flash-Chromatographie (Kieselgel; Methylenchlorid/Ethanol = 98:2 bis 80:20) gereinigt.
Ausbeute: 0.7 g (88 % der Theorie),
R_{f}-Wert: 0.31 (Kieselgel; Essigester/Ethanol/Ammoniak = 90:10:1)
C₂₁H₁₈N₄OS x HCl (374.47/410.93)
Massenspektrum: (M+H)⁺ = 375

### Beispiel 5

### 4-[3-(4-Biphenyl)-propargylamino]benzamidin

### a 4-[3-(4-Biphenyl)-propargylamino]benzonitril

Hergestellt analog Beispiel 1g aus 4-Brombiphenyl, 4-Propargylaminobenzonitril, Tetrakis(triphenylphosphin)palladium(0), Kupfer(I)iodid und Triethylamin in Acetonitril.
Ausbeute: 29% der Theorie,
R_{f}-Wert: 0.48 (Kieselgel; Essigester/Petrolether = 25:75)

### b. 4-[3-(4-Biphenyl)-propargylamino]benzamidin

In eine Lösung aus 0.50 g (1.6 mMol) 4-[3-(4-Biphenyl)-propargylamino]benzonitril und 0.78 ml (5.6 mMol) Triethylamin in 25 ml absolutem Pyridin wird solange Schwefelwasserstoff eingeleitet, bis kein Ausgangsprodukt laut Dünnschichtchromatographie mehr nachweisbar ist. Anschließend wird das Lösungsmittel abdestilliert, der erhaltene Rückstand in MethylenChlorid aufgenommen und mit 2N HCl und H₂O gewaschen. Die organische Phase wird getrocknet und das Lösungsmittel abdestilliert. Der Rückstand wird in 25 ml Aceton aufgenommen und mit 2.0 ml (32 mMol) Methyliodid versetzt. Nach 20 Stunden werden die flüchtigen Bestandteile abdestilliert, das Rohprodukt in 35 ml Ethanol und 15 ml Methylenchlorid aufgenommen und mit 2.8 g (36 mMol) Ammcniumacetat versetzt. Das Reaktionsgemisch wird 8 Stunden bei 40°C und 60 Stunden bei Raumtemperatur gerührt, im Vakuum konzentriert und durch Flash-Chromatographie (Kieselgel; Methylenchlorid/Ethanol = 98:2 bis 80:20) gereinigt.
Ausbeute: 0.57 g (78 % der Theorie),
R_{f}-Wert: 0.21 (Kieselgel; Methylenchlorid/Ethanol = 80:20) C₂₂H₁₉N₃ x HI (325.41/453.32)
Massenspektrum: (M+H)⁺ = 326

### Beispiel 6

### 4-{3-[3-(2-Methyl-benzimidazol-1-yl-methyl)-phenyl]-propargylamino}benzamidin

### a. 1-(3-Brombenzyl)-2-methylbenzimidazol

Zu einer Lösung aus 1.32 g (10 mMol) 2-Methyl-benzimidazol in 10 ml absolutem Dimethylsulfoxid gibt man zuerst 1.23 g (11 mMol) Kalium-tert.butylat und nach 45 Minuten 2.62 g (10.5 mMol) 3-Brom-benzylbromid zu und rührt 4 Stunden. Anschließend wird das Reaktionsgemisch mit Essigester verdünnt, 3 x mit 14%iger NaCl-Lösung gewaschen, getrocknet, konzentriert und durch Flash-Chromatographie (Kieselgel; Petrolether/Essigester = 9:1 bis Essigester) gereinigt.
Ausbeute: 2.4 g (80 % der Theorie),
C₁₅H₁₃BrN₂ (301.19)
Massenspektrum: M⁺ = 300/302 (Bromisotope)

### b. 4-{3-[3-(2-Methylbenzimidazol-1-yl-methyl)-phenyl]-propargylamino}benzamidin

Hergestellt analog Beispiel 1g aus 1-(3-Brombenzyl)-2-methylbenzimidazol, N-tert.Butoxycarbonyl-4-propargylamino-benzamidin, Tetrakis(triphenylphosphin)palladium(0), Kupfer(I)-iodid und Triethylamin in Acetonitril und anschließende Abspaltung des tert.Butoxycarbonylrestes durch Trifluoressigsäure analog Beispiel 2.
Ausbeute: 55% der Theorie,
R_{f}-Wert: 0.13 (Kieselgel; Methylenchlorid/Ethanol = 80:20)
C₂₅H₂₃N₅ x CF₃COOH (393.49/507.51)
Massenspektrum: (M+H)⁺ = 394
(M+2H)⁺⁺ = 197.6

### Beispiel 7

### 4-{3-[3-Methyl-4-(2-methyl-benzimidazol-1-yl)-phenyl]-propargylamino}benzamidin

### a. N-(2-Nitrophenyl)-4-brom-2-methyl-anilin

Ein Gemisch aus 2.9 ml (27.5 mMol) 2-Fluor-nitrobenzol, 10.55 g (55 mMol) 4-Brom-2-methyl-anilin und 1.60 g (27.5 mMol) sprühgetrocknetes Kaliumfluorid wird auf 180°C erhitzt. Nach dem Abkühlen wird das Reaktionsgemisch in Methylenchlorid aufgenommen, mit H₂O, mit 10%iger Salzsäure und wiederum H₂O gewaschen, mit Na₂SO₄ getrocknet, konzentriert und durch Flash-Chromatographie (Kieselgel; Petrolether/Essigester = 75:25) gereinigt.
Ausbeute: 5.95 g (70 % der Theorie),
R_{f}-Wert: 0.69 (Kieselgel; Petrolether/Essigester = 75:25)

### b. N-(2-Aminophenyl)-4-brom-2-methyl-anilin

Eine Suspension aus 5.34 g (17.4 mMol) N-(2-Nitrophenyl)-4-brom-2-methyl-anilin und 1.7 g Platin auf Kohle wird in 100 ml Dichlormethan und 100 ml Methanol bei einem WasserStoffdruck von 3 bar 1 Stunde gerührt. Anschließend wird der Katalysator abfiltriert und das Filtrat wird eingedampft.
Ausbeute: 4.8 g (100 % der Theorie),
R_{f}-Wert: 0.27 (Kieselgel; Petrolether/Essigester = 75:25)

### c. 1-(4-Brom-2-methyl-phenyl)-2-methyl-benzimidazol

Ein Gemisch aus 5.22 g (18.8 mMol) N-(2-Aminophenyl)-4-brom-2-methyl-anilin und 7.1 ml (75.2 mMol) Essigsäureanhydrid wird 30 Stunden zum Sieden erhitzt, mit 15 ml Eisessig versetzt und weitere 30 Stunden zum Sieden erhitzt. Anschließend wird das Reaktionsgemisch konzentriert und durch Flash-Chromatographie (Kieselgel; Methylenchlorid) gereinigt.
R_{f}-Wert: 0.20 (Kieselgel; Petrolether/Essigester = 75:25)

### d. 4-{3-[3-Methyl-4-(2-methyl-benzimidazol-1-yl)-phenyl]-propargylamino}benzamidin

Hergestellt analog Beispiel 1g aus 1-(4-Brom-2-methyl-phenyl)-2-methyl-benzimidazol, N-tert.Butoxyca=bonyl-4-propargylaminobenzamidin, Tetrakis(triphenylphosphin)palladium(0), Kupfer-(I)iodid und Triethylamin in Acetonitril und anschließende Abspaltung des tert.Butoxycarbonylrestes durch Trifluoressigsäure analog Beispiel 2.
Ausbeute: 41 % der Theorie,
R_{f}-Wert: 0.13 (Kieselgel; Methylenchlorid/Ethanol = 80:20) C₂₅H₂₃N₅ x CF₃COOH (393.49/507.51)
Massenspektrum: (M+H)⁺ = 394
(M+2H)⁺⁺ = 197.6

### Beispiel 8

### 4-{3-[4-(3-(2-Ethoxycarbonyl-ethyl)-5-phenyl-pyrazol-1-yl)-3-methyl-phenyl]-propargylamino}benzamidin

### a. 1-(4-Iod-2-methyl-phenyl)-3-(2-hydroxycarbonyl-ethyl)-5-phenyl-pyrazol

Eine Mischung aus 2.5 g (8.78 mMol) 4-Iod-2-methyl-phenylhydrazin (hergestellt analog J. Am. Chem. Soc. 78, 5854-5857 (1956)), 1.93 g (8.78 mMol) 4,6-Dioxo-6-phenyl-hexansäure (hergestellt analog Synthesis 1991, 18-20) und 1.22 ml (8.78 mMol) Triethylamin in 70 ml Methanol wird 3 Stunden bei Raumtemperatur gerührt. Anschließend werden die flüchtigen Bestandteile abdestilliert, das erhaltene Rohprodukt in 100 ml Ether aufgenommen, mit 1N HCl gewaschen und die wäßrige Phase mit 50 ml Ether und 50 ml Methylenchlorid extrahiert. Die vereinigten organischen Phasen werden getrocknet, konzentriert und das Rohprodukt durch Flash-Chromatographie (Kieselgel; Methylenchlorid/Ethanol = 98:2) gereinigt.
Ausbeute: 2.26 g (60 % der Theorie),
R_{f}-Wert: 0.45 (Kieselgel; Methylenchlorid/Ethanol = 95:5)
C₁₉H₁₇IN₂O₂ (432.26)
Massenspektrum: (M+H)⁺ = 433
(M+Na)⁺ = 455
(M-H)⁻ = 431

### b. 3-(2-Ethoxycarbonylethyl)-1-(4-iod-2-methyl-phenyl)-5-phenyl-pyrazol

Ein Gemisch aus 2.26 g (5.23 mMol) 1-(4-Iod-2-methyl-phenyl)-3-(2-hydroxycarbonyl-ethyl)-5-phenyl-pyrazol und 0.93 g (5.75 mMol) N,N'-Carbonyldiimidazol in 50 ml Ethanol wird 1 Stunde gerührt, dann mit 5.0 ml absolutem Ethanol versetzt, 1 Stunden zum Sieden erhitzt, konzentriert und durch Flash-Chromatographie (Kieselgel; Methylenchlorid bis Methylenchlorid/Ethanol = 98:2) gereinigt.
Ausbeute: 1.4 g (58 % der Theorie),
R_{f}-Wert: 0.35 (Kieselgel; Methylenchlorid/Ethanol = 99:1)
C₂₁H₂₁IN₂O₂ (460.32)
Massenspektrum: (M+H)⁺ = 461
(2M+Na)⁺ = 943

### d. 4-{3-[4-(3-(2-Ethoxycarbonyl-ethyl)-5-phenyl-pyrazol-1-yl)-3-methyl-phenyl]-propargylamino}benzamidin

Hergestellt analog Beispiel 1g aus 3-(2-Ethoxycarbonyl-ethyl)-1-(4-iod-2-methyl-phenyl)-5-phenyl-pyrazol, N-tert.Butoxycarbonyl-4-propargylamino-benzamidin, Tetrakis(triphenylphosphin)palladium(0), Kupfer(I)iodid und Triethylamin in Acetonitril und anschließende Abspaltung des tert.Butoxycarbonylrestes durch Trifluoressigsäure analog Beispiel 2.
Ausbeute: 71% der Theorie,
R_{f}-Wert: 0.13 (Reversed Phase Kieselgel RP-8; Methanol/5%ige NaCl-Lösung = 6:4)
C₃₁H₃₁N₅O₂ x CF₃COOH (505.63/619.65)
Massenspektrum: (M+H)⁺ = 506

### Beispiel 9

### 4-[3-(3-Methyl-4-morpholinocarbonyl-phenyl)propargylthio]-benzamidin

### a. N-[4-(3-Hydroxy-propin-1-yl)-2-methyl-benzoyl]-morpholin

Hergestellt analog Beispiel 1g aus N-(4-Brom-2-methyl-benzoyl)-morpholin, Propargylalkohol, Tetrakis(triphenylphosphin)-palladium(0), Kupfer(I)iodid und Triethylamin in Acetonitril. Ausbeute: 46% der Theorie,
R_{f}-Wert: 0.40 (Kieselgel; Essigester/Petrolether = 6:4)

### b. N-[4-(3-Methylsulfonyl-propin-1-yl)-2-methyl-benzoyl]-morpholin

Ein Gemisch aus 0.90 g (3.5 mMol) N-[4-(3-Hydroxy-propin-1-yl)-2-methyl-benzoyl]-morpholin, 0.45 g (3.9 mMol) Methansulfonsäurechlorid und 1.0 ml (7 mMol) Triethylamin in 20 ml Tetrahydrofuran wird 1 Stunde bei Raumtemperatur gerührt. Dann wird Eiswasser zugegeben, mit Essigester extrahiert, die organische Phase mit Wasser gewaschen und konzentriert.
Ausbeute: 2.0 g braunes Öl (83 % der Theorie),
R_{f}-Wert: 0.72 (Kieselgel; Methylenchlorid/Ethanol/Ammoniak = 90:10:1)

### c. 4-[3-(3-Methyl-4-morpholinocarbonyl-phenyl)propargylthio]benzonitril

Eine Lösung aus 1.0 g (3mMol) N-[4-(3-Methylsulfonyl-propin-1-yl)-2-methyl-benzoyl]-morpholin, 4-Cyano-thiophenol und 5 ml N,N-Diisopropyl-ethylamin in 10 ml Dimethylformamid wird 30 Minuten auf 100°C erhitzt. Anschließend wird mit 50 ml Essigester verdünnt, 2 x mit 14%iger NaCl Lösung gewaschen, getrocknet, konzentriert und durch Flash-Chromatographie (Kieselgel; Gradientenelution: Methylenchlorid bis Methylenchlorid/Ethanol = 98:2) gereinigt.
Ausbeute: 0.8 g (73 % der Theorie),
R_{f}-Wert: 0.62 (Kieselgel; Methylenchlorid/Ethanol = 95:5)

### d. 4-[3-(3-Methyl-4-morpholinocarbonyl-phenyl]propargylthio}benzamidin

Hergestellt analog Beispiel 4c aus 4-[3-(3-Methyl-4-morpholinocarbonyl-phenyl)propargylthio]benzonitril, mit Chlorwasserstoffgas gesättigtem Ethanol und Ammoniumcarbonat. Ausbeute: 87% der Theorie,
R_{f}-Wert: 0.38 (Kieselgel; Essigester/Ethanol/Ammoniak = 50:45:5)
C₂₂H₂₃N₃O₂S x HCl (393.53/429.99)
Massenspektrum: (M+H)⁺ = 394

### Beispiel 10

### E-4-{3-[3-(2-Ethoxycarbonyl-vinyl)-4-pyrrolidinocarbonylphenyl]propargylamino}benzamidin

### a. 5-Brom-1,3-dihydro-isobenzofuran-1-on

Eine Lösung aus 0.43 g (2.0 mMol) 4-Brom-2-methyl-benzoesäure, 0.34 g (1.9 mMol) N-Brom-succinimid und 20 mg Azaisobuttersäurenitril in 7 ml Propionsäuremethylester wird unter Stickstoffatmosphäre 1 Stunde zum Sieden erhitzt und mit einer Quecksilber-Dampflampe bestrahlt. Das Reaktionsgemisch wird konzentriert, in Methylenchlorid aufgenommen, mit H₂O gewaschen, mit Na₂SO₄ getrocknet und durch Flash-Chromatographie (Kieselgel; Essigester/Petrolether = 5:95 bis 15:85) gereinigt.
Ausbeute: 0.23 g (54 % der Theorie),
R_{f}-Wert: 0.52 (Kieselgel; Essigester/Petrolether = 20:80) C₈H₅BrO₂ (213.03)
Massenspektrum: M⁺ = 212/214 (Bromisotope)

### b. N-(4-Brom-2-hydroxymethyl-benzoyl)-pyrrolidin

Ein Gemisch aus 2.55 g (11.9 mMol) 5-Brom-1,3-dihydro-isobenzofuran-1-on und 1.3 ml (15.5 mMol) Pyrrolidin in 15 ml Ethanol wird 8 Stunden zum Sieden erhitzt: Dann wird erneut 1.3 ml Pyrrolidin zugegeben und weitere 22 Stunden erhitzt. Anschließend wird das Lösungsmittel entfernt, in Essigester aufgenommen, mit H₂O gewaschen, mit Na₂SO₄ getrocknet und durch Flash-Chromatographie (Kieselgel; Methylenchlorid/Ethanol = 99:1 bis 99:2) gereinigt.
Ausbeute: 3.01 g (89 % der Theorie),
R_{f}-Wert: 0.45 (Kieselgel; Methylenchlorid/Ethanol = 95:5)

### c. N-(4-Brom-2-formyl-benzoyl)-pyrrolidin

Zu einer Lösung aus 4.0 g (14 mMol) N-(4-Brom-2-hydroxymethylbenzoyl)-pyrrolidin in 80 ml Methylenchlorid gibt man portionsweise -verteilt über mehrere Stunden- insgesamt 25 g Mangandioxid zu und rührt insgesamt 30 Stunden. Anschließend wird über Kieselgur filtriert und das Lösungsmittel abdestilliert. Das Rohprodukt wird ohne weitere Reinigung umgesetzt. Ausbeute 3.3 g (84 % der Theorie),
R_{f}-Wert: 0.31 (Kieselgel; Methylenchlorid/Ethanol = 95:5)

### d. E-N-[4-Brom-2-(2-ethoxycarbonyl-vinyl)-benzoyl]-pyrrolidin

Eine Lösung aus 1.27 g (4.5 mMol) N-(4-Brom-2-formyl-benzoyl)-pyrrolidin und 1.65 g (4.5 mMol) Carboethoxymethylentriphenylphosphoran in 45 ml Toluol wird 4 Stunden auf 80°C erwärmt Nach Entfernen des Lösungsmittel wird durch Flash-Chromatographie (Kieselgel; Methylenchlorid/Ethanol = 99:1 bis 99:2) gereinigt.
Ausbeute: 1.07 g (67 % der Theorie),
R_{f}-Wert: 0.33 (Kieselgel; Petrolether/Essigester = 1:1).

### e. E-4-{3-[3-(2-Ethoxycarbonyl-vinyl)-4-pyrrolidinocarbonylphenyl]propargylamino}benzamidin

Hergestellt analog Beispiel 1g aus E-N-[4-Brom-2-(2-ethoxycarbonyl-vinyl)-benzoyl]-pyrrolidin, N-tert.Butoxycarbonyl-4-propargylamino-benzamidin, Tetrakis(triphenylphosphin)palladium(0), Kupfer(I)iodid und Triethylamin in Acetonitril und anschließende Abspaltung des tert.Butoxycarbonylrestes durch Trifluoressigsäure analog Beispiel 2.
Ausbeute: 56% der Theorie,
R_{f}-Wert: 0.17 (Kieselgel; Methylenchlorid/Ethanol = 4:1) C₂₆H₂₈N₄O₃ x CF₃COOH (444.54/558.56)
Massenspektrum: (M+H)⁺ = 445

### Beispiel 11

### N-tert.Butoxycarbonyl-4-[3-(2,5-dimethyl-4-isopropylcarbonylphenyl)propargylamino]benzamidin

### a. 4-Brom-2,5-dimethyl-1-isopropylcarbonyl-benzol

Zu einer auf -78°C gekühlten Lösung aus 13.5 g (50 mMol) 2,5-Dibrom-p-xylol in 100 ml Tetrahydrofuran tropft man 31.2 ml (50 mMol) einer 1.6 molaren n-Butyl-lithium-Lösung in Hexan zu, rührt 30 Minuten und versetzt dann mit 4.5 ml (50 mMol) Isobutyronitril. Man läßt das Reaktionsgemisch langsam auf Raumtemperatur erwärmen, rührt 1 Stunde, versetzt dann mit 50 ml 2N HCl und 70 ml Diethylether und rührt weitere 16 Stunden. Man trennt die wäßrige Phase ab und extrahiert diese 2 x mit Diethylether. Die vereinigten organischen Phasen werden mit Na₂SO₄ getrocknet, eingeengt und der Rückstand durch Flash-Chromatographie (Kieselgel; Petrolether bis Petrolether/Essigester = 9:1) gereinigt.
Ausbeute: 6.08 g (48 % der Theorie),
R_{f}-Wert: 0.58 (Kieselgel; Petrolether/Essigester = 9:1). C₁₂H₁₅BrO (255.16)
Massenspektrum: M⁺ = 254/256 (Bromisotope)

### b. N-tert.Butoxycarbonyl-4-[3-(2,5-dimethyl-4-isopropylcarbonyl-phenyl)propargylamino]benzamidin

Hergestellt analog Beispiel 1g aus 4-Brom-2,5-dimethyl-1-isopropylcarbonyl-benzol, N-tert.Butoxycarbonyl-4-propargylaminobenzamidin, Tetrakis(triphenylphosphin)palladium(0), Kupfer-(I)iodid und Triethylamin in Acetonitril.
Ausbeute: 57 % der Theorie,
R_{f}-Wert: 0.3 (Kieselgel; Methylenchlorid/Ethanol = 98:2)
C₂₇H₃₃N₃O₃ (447.58)
Massenspektrum: (M+H)⁺ = 448

### Beispiel 12

### 4-[3-(2,5-dimethyl-4-isopropylcarbonyl-phenyl)propargylamino]benzamidin

Hergestellt analog Beispiel 2 aus N-tert.Butoxycarbonyl-4-[3-(2,5-dimethyl-4-isopropylcarbonyl-phenyl)propargylamino]benzamidin und Trifluoressigsäure.
Ausbeute: 34 % der Theorie,
R_{f}-Wert: 0.30 (Kieselgel; Methylenchlorid/Ethanol = 4:1) C₂₂H₂₅N₃O x CF₃COOH (347.46/461.88)
Massenspektrum: (M+H)⁺ = 348

### Beispiel 13

### 4-{3-[4-[(1-Methyl-imidazol-2-yl)carbonyl]-phenyl]-propargylamino}benzamidin

### a. 2-(4-Iod-benzoyl)-1-methyl-imidazol

0.82 g (10 mMol) 1-Methyl-imidazol und 2.7 g (10 mMol) 4-Iodbenzoesäurechlorid werden unter Eisbadkühlung in 10 ml Acetonitril vereinigt, mit 1.4 ml (10 mMol) Triethylamin versetzt und anschließend 16 Stunden bei Raumtemperatur gerührt. Das Rohprodukt wird in 30 ml Essigester aufgenommen, mit H₂O gewaschen, getrocknet, im Vakuum konzentriert und durch Flash-Chromatographie (Kieselgel; Methylenchlorid) gereinigt. Ausbeute: 1.9 g (51 % der Theorie),
R_{f}-Wert: 0.62 (Kieselgel; Essigester/Petrolether = 60:40)

### b. 4-{3-[4-[(1-Methyl-imidazol-2-yl)carbonyl]-phenyl]-propargylamino}benzonitril

Hergestellt analog Beispiel 1g aus 2-(4-Iod-benzoyl)-1-methylimidazol, 4-Propargylamino-benzonitril, Tetrakis(triphenylphosphin)palladium(0), Kupfer(I)iodid und Triethylamin in Acetonitril.
Ausbeute: 39 % der Theorie,
R_{f}-Wert: 0.28 (Kieselgel; Essigester/Petrolether = 60:40)

### c. 4-{3-[4-[(1-Methyl-imidazol-2-yl)carbonyl]-phenyl]-propargylamino}benzamidin

Hergestellt analog Beispiel 4c aus 4-{3-[4-[(1-Methyl-imidazol-2-yl)carbonyl]-phenyl]-propargylamino}benzonitril, mit Chlorwasserstoffgas gesättigtem Ethanol und Ammoniumcarbonat. Ausbeute: 77 % der Theorie,
R_{f}-Wert: 0.37 (Kieselgel; Essigester/Ethanol/Ammoniak = 50:45:5)
C₂₁H₁₉N₅O x HCl (357.42/393.89)
Massenspektrum: (M+H)⁺ = 358
(M+2H)⁺⁺ = 179.6
(M+HCl)⁺ = 394/396 (Chlorisotope)

### Beispiel 14

### 4-{3-[3-Methyl-4-[(1-methyl-imidazol-2-yl)carbonyl]-phenyl]-propargylamino}benzamidin

Hergestellt analog Beispiel 4c aus 4-{3-[3-Methyl-4-[(1-methyl-imidazol-2-yl)carbonyl]-phenyl]-propargylamino}benzonitril (hergestellt analog Beispiel 13b) mit Chlorwasserstoffgas gesättigtem Ethanol und Ammoniumcarbonat.
Ausbeute: 62 % der Theorie,
R_{f}-Wert: 0.39 (Kieselgel; Essigester/Ethanol/Ammoniak = 50:45:5)
C₂₂H₂₁N₅O x HCl (371.45/407.91)
Massenspektrum: (M+H)⁺ = 372

### Beispiel 15

### 4-{3-[4-[(Imidazol-2-yl)carbonyl]-phenyl]-propargylamino}benzamidin

Hergestellt analog Beispiel 4c aus 4-{3-[4-[(Imidazol-2-yl)-carbonyl]-phenyl]-propargylamino}benzonitril, mit Chlorwasserstoffgas gesättigtem Ethanol und Ammoniumcarbonat.
Ausbeute: 60 % der Theorie,
R_{f}-Wert: 0.35 (Kieselgel; Essigester/Ethanol/Ammoniak = 50:45:5)
C₂₂H₂₁N₅O x HCl (343.41/379.87)
Massenspektrum: (M+H)⁺ = 344
(M+2H)⁺⁺ = 172.7

### Beispiel 16

### 4-{3-[4-[(Thiophen-2-yl)carbonyl]-phenyl]-propargylamino}benzamidin

Hergestellt analog Beispiel 4c aus 4-{3-[4-[(Thiophen-2-yl)-carbonyl]-phenyl]-propargylamino}benzonitril, mit Chlorwasserstoffgas gesättigtem Ethanol und Ammoniumcarbonat.
Ausbeute: 85 % der Theorie,
R_{f}-Wert: 0.45 (Kieselgel; Essigester/Ethanol/Ammoniak = 50:45:5)
C₂₁H₁₇N₃OS x HCl (359.45/395.91)
Massenspektrum: (M+H)⁺ = 360

### Beispiel 17

### 4-{3-[4-(2-Methylphenylcarbonyl)-phenyl]-propargylamino}benzamidin

Hergestellt analog Beispiel 4c aus 4-{3-[4-(2-Methylphenylcarbonyl)-phenyl]-propargylamino}benzonitril, mit Chlorwasserstoffgas gesättigtem Ethanol und Ammoniumcarbonat.
Ausbeute: 80 % der Theorie,
R_{f}-Wert: 0.45 (Kieselgel; Methylenchlorid/Ethanol = 4:1) C₂₄H₂₁N₃O x HCl (367.45/403.91)
Massenspektrum: (M+H)⁺ = 368

### Beispiel 18

### 4-{3-[4-(4-Methylphenylcarbonyl)-phenyl]-propargylamino}benzamidin

Hergestellt analog Beispiel 4c aus 4-{3-[4-(4-Methylphenylcarbonyl)-phenyl]-propargylamino}benzonitril, mit Chlorwasserstoffgas gesättigtem Ethanol und Ammoniumcarbonat.
Ausbeute: 65 % der Theorie,
R_{f}-Wert: 0.15 (Kieselgel; Methylenchlorid/Ethanol = 4:1) C₂₄H₂₁N₃O x HCl (367.45/403.91)
Massenspektrum: (M+H)⁺ = 368
(2M+H)⁺⁺ = 735

### Beispiel 19

### 4-{3-[4-(2-Chlorphenylcarbonyl)-phenyl)-propargylamino}benzamidin

Hergestellt analog Beispiel 2 aus N-tert.Butoxycarbonyl-4-{3-[4-(2-chlorphenylcarbonyl)-phenyl]-propargylamino}benzamidin und Trifluoressigsäure.
Ausbeute: 52 % der Theorie,
R_{f}-Wert: 0.29 (Kieselgel; Methylenchlorid/Ethanol = 4:1 und 1 Tropfen Essigsäure)
C₂₃H₁₈ClN₃O x CF₃COOH (387.87/501.90)
Massenspektrum: (M+H)⁺ = 388/390 (Chlorisotope)

### Beispiel 20

### 4-{3-[4-(3-Chlorphenylcarbonyl)-phenyl]-propargylamino}benzamidin

Hergestellt analog Beispiel 4c aus 4-{3-[4-(3-Chlorphenylcarbonyl)-phenyl]-propargylamino}benzonitril, mit Chlorwasserstoffgas gesättigtem Ethanol und Ammoniumcarbonat.
Ausbeute: 61 % der Theorie,
R_{f}-Wert: 0.25 (Kieselgel; Methylenchlorid/Ethanol = 4:1) C₂₃H₁₈ClN₃O x HCl (387.87/424.33)
Massenspektrum: (M+H)⁺ = 388/390 (Chlorisotope)

### Beispiel 21

### 4-{3-[4- (4-Chlorphenylcarbonyl) -phenyl] -propargylamino}benzamidin

Hergestellt analog Beispiel 2 aus N-tert.Butoxycarbonyl-4-{3-[4-(2-chlorphenylcarbonyl)-phenyl]-propargylamino}benzamidin und Trifluoressigsäure.
Ausbeute: 42 % der Theorie,
R_{f}-Wert: 0.28 (Kieselgel; Methylenchlorid/Ethanol = 4:1 und 1 Tropfen Essigsäure)
C₂₃H₁₈ClN₃O x CF₃COOH (387.87/501.90)
Massenspektrum: (M+H)⁺ = 388/390 (Chlorisotope)

### Beispiel 22

### 4-{3-[4-(Pyrid-2-yl-carbonyl)-phenyl]-propargylamino}benzamidin

Hergestellt analog Beispiel 4c aus 4-{3-[4-(Pyrid-2-yl-carbonyl)-phenyl]-propargylamino}benzonitril, mit Chlorwasserstoffgas gesättigtem Ethanol und Ammoniumcarbonat.
Ausbeute: 47 % der Theorie,
R_{f}-Wert: 0.48 (Kieselgel; Essigester/Ethanol/Ammoniak = 50:45:5)
C₂₂H₁₈N₄O x HCl (354.42/390.88)
Massenspektrum: (M+H)⁺ = 355

### Beispiel 23

### 4-{3-[4-(Pyrid-3-yl-carbonyl)-phenyl]-propargylamino}benzamidin

Hergestellt analog Beispiel 2 aus N-tert.Butoxycarbonyl-4-{3-[4-(pyrid-3-yl-carbonyl)-phenyl]-propargylamino}benzamidin und Trifluoressigsäure.
Ausbeute: 89 % der Theorie,
Rₜ-Wert: 0.37 (Kieselgel; Essigester/Ethanol/Ammoniak = 50:45:5)
C₂₂H₁₈N₄O x CF₃COOH (354.42/468.44)
Massenspektrum: (M+H)⁺ = 355

### Beispiel 24

### 4-{3-[4-(Pyrid-4-yl-carbonyl)-phenyl]-propargylamino}benzamidin

Hergestellt analog Beispiel 2 aus N-tert.Butoxycarbonyl-4-{3-[4-(pyrid-4-yl-carbonyl)-phenyl]-propargylamino}benzamidin und Trifluoressigsäure.
Ausbeute: 84 % der Theorie,
R_{f}-Wert: 0.37 (Kieselgel; Essigester/Ethanol/Ammoniak = 50:45:5)
C₂₂H₁₈N₄O x CF₃COOH (354.42/468.44)
Massenspektrum: (M+H)⁺ = 355

### Beispiel 25

### 4-[3-(2-Methyl-4-phenylcarbonyl-phenyl)-propargylamino}benzamidin

Hergestellt analog Beispiel 4c aus 4-[3-(2-Methyl-4-phenylcarbonyl-phenyl]-propargylamino}benzonitril, mit Chlorwasserstoffgas gesättigtem Ethanol und Ammoniumcarbonat. Ausbeute: 47 % der Theorie,
R_{f}-Wert: 0.24 (Kieselgel; Methylenchlorid/Ethanol = 4:1) C₂₄H₂₁N₃O x HCl (367.45/403.91)
Massenspektrum: (M+H)⁺ = 368

### Beispiel 26

### 4-[3-(3-Methyl-4-phenylcarbonyl-phenyl)-propargylamino]benzamidin

Hergestellt analog Beispiel 4c aus 4-[3-(3-Methyl-4-phenylcarbonyl-phenyl)-propargylamino]benzonitril, mit Chlorwasserstoffgas gesättigtem Ethanol und Ammoniumcarbonat.
Ausbeute: 50 % der Theorie;
R_{f}-Wert: 0.16 (Kieselgel; Methylenchlorid/Ethanol = 4:1) C₂₄H₂₁N₃O x HCl (367.45/403.91)
Massenspektrum: (M+H)⁺ = 368

### Beispiel 27

### 4-[3-(4-Phenylcarbonyl-phenyl)-propargylamino]benzamidin

Hergestellt analog Beispiel 4c aus 4-[3-(4-Phenylcarbonylphenyl)-propargylamino]benzonitril, mit Chlorwasserstoffgas gesättigtem Ethanol und Ammoniumcarbonat.
Ausbeute: 78 % der Theorie,
R_{f}-Wert: 0.13 (Kieselgel; Methylenchlorid/Ethanol = 4:1) C₂₃H₁₉ N₃O x HCl (353.42/389. 88)
Massenspektrum: (M+H)⁺ = 354

### Beispiel 28

### 4-[3-(2-Amino-4-phenylcarbonyl-phenyl)-propargylamino]benzamidin

### a. 4-Iod-3-nitro-benzophenon

Unter Eisbadkühlung werden nacheinander 5.3 g (17 mMol) 4-Iod-3-nitrobenzoesäure und 8.0 g (60 mMol) Aluminiumtrichlorid in 70 ml Benzol eingetragen. Anschließend wird 2 Stunden bei Raumtemperatur gerührt, dann in Eiswasser gegossen, mit Methylenchlorid extrahiert, mit Natriumsulfat getrochnet und im Vakuum konzentriert.
Ausbeute: 5.4 g (90 % der Theorie),
R_{f}-Wert: 0.83 (Kieselgel; Essigester/Petrolether = 3:7)

### b. 3-Amino-4-iod-benzophenon

Ein Gemisch aus 5.0 g (14 mMol) 4-Iod-3-nitro-benzophenon, 7.5 g (42 mMol) Natiumdithionit, 40 ml Pyridin und 15 ml Wasser wird 2 Stunden auf 40°C erwärmt. Anschließend wird im Vakuum konzentriert, mit Eiswasser versetzt und mit Essigester extrahiert. Die organische Phase wird mit Natiumsulfat getrocknet und das Lösungsmittel im Vakuum abdestilliert.
Ausbeute: 3.4 g (76 % der Theorie),
R_{f}-Wert: 0.60 (Kieselgel; Essigester/Petrolether = 3:7)

### c. 4-[3-(2-Amino-4-phenylcarbanyl-phenyl)-propargylamino]-benzamidin

Hergestellt analog Beispiel 1g aus 3-Amino-4-iod-benzophenon, N-tert.Butoxycarbonyl-4-propargylamino-benzamidin, Tetrakis-(triphenylphosphin)palladium(0), Kupfer(I)iodid und Triethylamin in Acetonitril und anschließende Abspaltung des tert.Butoxycarbonylrestes durch Trifluoressigsäure analog Beispiel 2. Ausbeute: 54 % der Theorie,
Rₜ-Wert: 0.60 (Kieselgel; Essigester/Ethanol/Ammoniak = 50:45:5)
C₂₃H₂₀N₄O x CF₃COOH (368.46/482.48)
Massenspektrum: (M+H)⁺ = 369

### Beispiel 29

### 4-[3-(2-Acetamido-4-phenylcarbonyl-phenyl)-propargylamino]-benzamidin

Hergestellt analog Beispiel 1g aus 3-Acetamido-4-iod-benzophenon, N-tert.Butoxycarbonyl-4-propargylamino-benzamidin, Tetrakis(triphenylphosphin)palladium(0), Kupfer(I)iodid und

Triethylamin in Acetonitril und anschließende Abspaltung des tert.Butoxycarbonylrestes durch Trifluoressigsäure analog Beispiel 2.
Ausbeute: 61 % der Theorie,
R_{f}-Wert: 0.30 (Kieselgel; Essigester/Ethanol/Ammoniak = 50:45:5)
C₂₅H₂₂N₄O₂ x CF₃COOH (410.49/524.51)
Massenspektrum: (M+H)⁺ = 411

### Beispiel 30

### 4-{3-[4-(2-Methoxycarbonyl-phenylcarbonyl)-phenyl]-propargyl- amino benzamidin

Hergestellt analog Beispiel 4c aus 4-{3-[4-(2-Methoxycarbonylphenylcarbonyl)-phenyl]-propargylamino}benzonitril, Chlorwasserstoffgas gesättigtem Methanol und Ammoniumcarbonat. Ausbeute: 18 % der Theorie,
R_{f}-Wert: 0.1 (Kieselgel; Methylenchlorid/Ethanol = 4:1) C₂₅H₂₁N₃O₃ x HCl (411.46/447.92)
Massenspektrum: (M+H)⁺ = 412

### Beispiel 31

### 4-{3-[4-(2-Hydroxycarbonyl-phenylcarbonyl)-phenyl]-propargylamino}benzamidin

Hergestellt analog Beispiel 3 aus 4-{3-[4-(2-Methoxycarbonylphenylcarbonyl)-phenyl]-propargylamino}benzamidin und Lithiumhydroxid und anschließender Behandlung mit Ammoniumchlorid.
Ausbeute: 41 % der Theorie,
C₂₄H₁₉N₃O₃ x HCl (397.43/433.89)
Massenspektrum: (M+H)⁺ = 398
(M+Na)⁺ = 420
(M+2Na)⁺⁺ = 221.6

### Beispiel 32

### 4-[1-Methyl-3-(4-phenylcarbonyl-phenyl)-propargylamino]benzamidin

Hergestellt analog Beispiel 4c aus 4-[1-Methyl-3-(4-phenylcarbonyl-phenyl)-propargylamino]benzonitril, mit Chlorwasserstoffgas gesättigtem Ethanol und Ammoniumcarbonat.
Ausbeute: 80 % der Theorie,
R_{f}-Wert: 0.52 (Kieselgel; Essigester/Ethanol/Ammoniak = 50:45:5)
C₂₄H₂₁N₃O x HCl (367.47/403.93)
Massenspektrum: (M+H)⁺ = 368

### Beispiel 33

### 3-Methoxy-4-[3-(4-phenylcarbonyl-3-methyl-phenyl)-propargylamino]benzamidin

Hergestellt analog Beispiel 4c aus 3-Methoxy-4-[3-(4-phenylcarbonyl-3-methyl-phenyl)-propargylamino]benzonitril, mit Chlorwasserstoffgas gesättigtem Ethanol und Ammoniumcarbonat. Ausbeute: 29 % der Theorie,
R_{f}-Wert: 0.24 (Kieselgel; Methylenchlorid/Ethanol = 4:1) C₂₅H₂₃N₃O₂ x HCl (397.48/433.94)
Massenspektrum: (M+H)⁺ = 398

### Beispiel 34

### 4-[3-(5-Phenylcarbonyl-pyrid-2-yl)-propargylamino]benzamidin

Hergestellt analog Beispiel 1g aus 2-Chlor-5-phenylcarbonylpyridin, N-tert.Butoxycarbonyl-4-propargylamino-benzamidin, Tetrakis(triphenylphosphin)palladium(0), Kupfer(I)iodid und

Triethylamin in Acetonitril und anschließende Abspaltung des tert.Butoxycarbonylrestes durch Trifluoressigsäure analog Beispiel 2.
Ausbeute: 47 % der Theorie,
R_{f}-Wert: 0.55 (Kieselgel; Essigester/Ethanol/Ammoniak = 50:45:5)
C₂₂H₁₈N₄O x CF₃COOH (354.42/468.44)
Massenspektrum: (M+H)⁺ = 355

### Beispiel 35

### 4-[3-(5-Phenylcarbonyl-thiophen-2-yl)-propargylamino]benzamidin

Eine Lösung aus 1.2 g (2.6 mMol) N-tert.Butoxycarbonyl-4-[3-(5-phenylcarbonyl-thiophen-2-yl)-propargylamino]benzamidin und 4 ml Trimethylsilyliodid in 50 ml Methylenchlorid wird 3 Stunden gerührt, dann mit 50 ml Methylenchlorid und 25 ml Ethanol verdünnt und mit Wasser gewaschen. Die organische Phase wird getrocknet, konzentriert und durch Flash-Chromatographie (Kieselgel; Methylenchlorid/Ethanol = 49:1 bis 9:1) gereinigt.
Ausbeute: 20 % der Theorie,
R_{f}-Wert: 0.28 (Kieselgel; Essigester/Ethanol/Ammoniak = 50:45:5)
C₂₁H₁₇N₃OS x HI (359.46/487.37)
Massenspektrum: (M+H)⁺ = 360

### Beispiel 36

### 4-[3-(4-Isopropylcarbonyl-phenyl)propargylamino]benzamidin

Hergestellt analog Beispiel 4c aus 4-[3-(4-Isopropylcarbonylphenyl)propargylamino]benzonitril, mit Chlorwasserstoffgas gesättigtem Ethanol und Ammoniumcarbonat.
Ausbeute: 24 % der Theorie,
Rₜ-Wert: 0.13 (Kieselgel; Methylenchlorid/Ethanol = 4:1) C₂₀H₂₁N₃O x HCl (319.41/355.87)
Massenspektrum: (M+H)⁺ = 320
(M+H+HCl)⁺ = 356/358 (Chlorisotope)

### Beispiel 37

### 4-[3-(4-Cyclopentylcarbonyl-phenyl)propargylamino]benzamidin

Hergestellt analog Beispiel 4c aus 4-[3-(4-Cyclopentylcarbonyl-phenyl)propargylamino]benzonitril, mit Chlorwasserstoffgas gesättigtem Ethanol und Ammoniumcarbonat.
Ausbeute: 53 % der Theorie,
R_{f}-Wert: 0.20 (Kieselgel; Methylenchlorid/Ethanol = 4:1) C₂₂H₂₃N₃O x HCl (345.44/381.90)
Massenspektrum: (M+H)⁺ = 346

### Beispiel 38

### 4-[3-(4-tert.Butylcarbonyl-2,5-dimethyl-phenyl)propargylamino]benzamidin

Hergestellt aus N-tert.Butoxycarbonyl-4-[3-(4-tert.butylcarbonyl-2,5-dimethyl-phenyl)propargylamino]benzamidin und Trifluoressigsäure analog Beispiel 2.
Ausbeute: 18 % der Theorie,
R_{f}-Wert: 0.35 (Kieselgel; Methylenchlorid/Ethanol = 4:1 + 1 Tropfen Essigsäure)
C₂₃H₂₇N₃O x CF₃COOH (361.49/475.51)
Massenspektrum: (M+H)⁺ = 362

### Beispiel 39

### 4-{3-[4-(1,1-Dimethyl-2-ethoxycarbonyl-ethylcarbonyl)-2,5-dimethyl-phenyl]propargylamino}benzamidin

### a. 4-(4-Brom-2,5-dimethyl-phenyl)-3,3-dimethyl-4-oxo-butansäure

Zu einer Suspension aus 1.44 g (ca. 30 mMol) 50%iges Natriumhydrid in Öl in 300 ml Tetrahydrofuran wird innerhalb von 5 Minuten eine Lösung aus 2.85 g (10 mMol) 4-(4-Brom-2,5-dimethyl-phenyl)-4-oxo-butansäure zugetropft und 2 Stunden zum Sieden erhitzt. Anschließend wird auf Raumtemperatur abgekühlt, 2.8 ml Methyliodid zugetropft und erneut 2.5 Stunden zum Sieden erhitzt. Es wird in 150 ml Wasser gegossen und das organische Lösungsmittel wird abdestilliert. Die wäßrige Phase wird 2x mit Petrolether gewaschen, mit Salzsäure angesäuert und mit Methylenchlorid extrahiert. Die organische Phase wird mit Natriumsulfat getrocknet und konzentriert.
Ausbeute: 2.45 g (78 % der Theorie),
R_{f}-Wert: 0.35 (Kieselgel; Essigester/Petrolether 30:70 + 1 Tropfen Essigsäure)
C₁₄H₁₇BrO₃ (313.19)
Massenspektrum: (M-H)⁻ = 311/313 (Bromisotope)

### b. 4-(4-Brom-2,5-dimethyl-phenyl)-3,3-dimethyl-4-oxo-butansäureethylester

Eine Lösung aus 3.2 g (10 mMol) 4-(4-Brom-2,5-dimethyl-phenyl)-3,3-dimethyl-4-oxo-butansäure in Tetrahydrofuran wird mit 3.60 g (11 mMol) Carbonyldiimidazol versetzt und 1 Stunde bei Raumtemperatur gerührt. Das Lösungsmittel wird durch 20 ml Ethanol ersetzt und es wird 2 Stunden zum Sieden erhitzt. Dann wird das Lösungsmittel abdestilliert, das Rohprodukt wird in Methylenchlorid aufgenommen, mit Wasser gewaschen, mit Natriumsulfat getrocknet, konzentriert und durch Flash-Chromatographie (Kieselgel; Essigester/Petrolether = 3:97) gereinigt.
Ausbeute: 2.95 g (87 % der Theorie),
R_{f}-Wert: 0.55 (Kieselgel; Essigester/Petrolether = 1:9) C₁₆H₂₁BrO₃ (341.25)
Massenspektrum: (M+H)⁺ = 341/343 (Bromisotope)
(M+Na)⁺ = 363/365 (Bromisotope)

### c. 4-{3-[4-(1,1-Dimethyl-2-ethoxycarbonyl-ethylcarbonyl)-2,5-dimethyl-phenyl]propargylamino}benzamidin

Hergestellt analog Beispiel 1g aus 4-(4-Brom-2,5-dimethylphenyl)-3,3-dimethyl-4-oxo-butansäureethylester, N-tert.Butoxycarbonyl-4-propargylamino-benzamidin, Tetrakis(triphenylphosphin)palladium(0), Kupfer(I)iodid und Triethylamin in Acetonitril und anschließende Abspaltung des tert.Butoxycarbonylrestes durch Trifluoressigsäure analog Beispiel 2. Ausbeute: 9 % der Theorie,
R_{f}-Wert: 0.35 (Kieselgel; Methylenchlorid/Ethanol = 4:1) C₂₆H₃₁N₃O₃ x CF₃COOH (433.46/547.58)
Massenspektrum: (M+H)⁺ = 434

### Beispiel 40

### 4-{3-[4-(1,1-Dimethyl-2-hydroxycarbonyl-ethylcarbonyl)-2,5-dimethyl-phenyl]propargylamino}benzamidin

Hergestellt analog Beispiel 3 aus 4-{3-[4-(1,1-Dimethyl-2-ethoxycarbonyl-ethylcarbonyl)-2,5-dimethyl-phenyl]propargylamino}benzamidin und Lithiumhydroxid und anschließender Behandlung mit Ammoniumchlorid.
Ausbeute: 50 % der Theorie,
R_{f}-Wert: 0.3 (Reversed Phase Kieselgel RP-8; Methanol/5%ige NaCl-Lösung = 60:40)
C₂₄H₂₇N₃O₃ x HCl (405.50/441.96)
Massenspektrum: (M+H)⁺ = 406

### Beispiel 41

### 4-[3-(4-Pyrrolidinocarbonyl-phenyl)-propargylamino]benzamidin

Hergestellt analog Beispiel 2 aus N-tert.Butoxycarbonyl-4-[3-(4-pyrrolidinocarbonyl-phenyl)-propargylamino)benzamidin und Trifluoressigsäure.
Ausbeute: 37 % der Theorie,
R_{f}-Wert: 0.29 (Kieselgel; Methylenchlorid/Ethanol = 4:1 und 1 Tropfen Essigsäure)
C₂₁H₂₂N₄O x CF₃COOH (346.44/460.46)
Massenspektrum: (M+H)⁺ = 347

### Beispiel 42

### 4-[3-(3-Methyl-4-pyrrolidinocarbonyl-phenyl)-propargylamino]-benzamidin

Hergestellt analog Beispiel 2 aus N-tert.Butoxycarbonyl-4-[3-(3-methyl-4-pyrrolidinocarbonyl-phenyl)-propargylamino]benzamidin und Trifluoressigsäure. Ausbeute: 52 % der Theorie,
R_{f}-Wert: 0.27 (Kieselgel; Methylenchlorid/Ethanol = 4:1 und 1 Tropfen Essigsäure)
C₂₂H₂₄N₄O x CF₃COOH (360.46/474.48)
Massenspektrum: (M+H)⁺ = 361
(M+2H)⁺⁺ = 181

### Beispiel 43

### 4-[3-(2,5-Dimethyl-4-pyrrolidinocarbonyl-phenyl)-propargylamino]benzamidin

Hergestellt analog Beispiel 2 aus N-tert.Butoxycarbonyl-4-[3-(2,5-dimethyl-4-pyrrolidinocarbonyl-phenyl)-propargylamino]benzamidin und Trifluoressigsäure.
Ausbeute: 20 % der Theorie,
R_{f}-Wert: 0.3 (Kieselgel; Methylenchlorid/Ethanol = 4:1) C₂₃H₂₆N₄O x CF₃COOH (374.49/488.51)
Massenspektrum: (M+H)⁺ = 375

### Beispiel 44

### 4-[N-Methyl-3-(3-methyl-4-pyrrolidinocarbonyl-phenyl)-propargylamino]benzamidin

Hergestellt analog Beispiel 4c aus 4-[N-Methyl-3-(3-methyl-4-pyrrolidinocarbonyl-phenyl)-propargylamino]benzonitril, Chlorwasserstoffgas gesättigtem Ethanol und Ammoniumcarbonat.
Ausbeute: 67 % der Theorie,
R_{f}-Wert: 0.19 (Kieselgel; Methylenchlorid/Ethanol = 4:1)
C₂₃H₂₆N₄O x HCl (374.49/410.95)
Massenspektrum: (M+H)⁺ = 375

### Beispiel 45

### rac-4-{3-[4-(2-Methyl-pyrrolidinocarbonyl)-phenyl]-propargylamino}benzamidin

Hergestellt analog Beispiel 2 aus rac-N-tert.Butoxycarbonyl-4-{3-[4-(2-methyl-gyrrolidinocarbonyl)-phenyl]-propargylamino}benzamidin und Trifluoressigsäure.
Ausbeute: 32 % der Theorie,
R_{f}-Wert: 0.34 (Kieselgel; Methylenchlorid/Ethanol = 4:1 und 1 Tropfen Essigsäure)
C₂₂H₂₄N₄O x CF₃COOH (360.48/474.48)
Massenspektrum: (M+H)⁺ = 361
(M+2H)⁺⁺ = 181

### Beispiel 46

### rac-4-{3-[3-Methyl-4-(2-methyl-pyrrolidinocarbonyl)-phenyl]-propargylamino}benzamidin

Hergestellt analog Beispiel 2 aus rac-N-tert.Butoxycarbonyl-4-{3-[3-methyl-4-(2-methyl-pyrrolidinocarbonyl)-phenyl]-propargylamino}benzamidin und Trifluoressigsäure. Ausbeute: 90 % der Theorie,
R_{f}-Wert: 0.31 (Kieselgel; Methylenchlorid/Ethanol = 4:1 und 1 Tropfen Essigsäure)
C₂₃H₂₆N₄O x CF₃COOH (374.49/488.51)
Massenspektrum: (M+H)⁺ = 375

### Beispiel 47

### rac-4-{3-[2-Methyl-4-(2-methyl-pyrrolidinocarbonyl)-phenyl]-propargylamino}benzamidin

Hergestellt analog Beispiel 2 aus rac-N-tert.Butoxycarbonyl-4-{3-[2-methyl-4-(2-methyl-pyrrolidinocarbonyl)-phenyl]-propargylamino}benzamidin und Trifluoressigsäure.
Ausbeute: 63 % der Theorie,
R_{f}-Wert: 0.2 (Kieselgel; Methylenchlorid/Ethanol = 4:1) C₂₃H₂₆N₄O x CF₃COOH (374.49/488.51)
Massenspektrum: (M+H)⁺ = 375

### Beispiel 48

### 4-[3-(2-Methyl-4-pyrrolidinocarbonyl-phenyl)-propargylaminol benzamidin

Hergestellt analog Beispiel 2 aus N-tert.Butoxycarbonyl-4-[3-(2-methyl-4-pyrrolidinocarbonyl-phenyl)-propargylamino]benzamidin und Trifluoressigsäure.
Ausbeute: 86 % der Theorie,
R_{f}-Wert: 0.25 (Kieselgel; Methylenchlorid/Ethanol = 4:1) C₂₂H₂₄N₄O x CF₃COOH (360.46/474.48)
Massenspektrum: (M+H)⁺ = 361

### Beispiel 49

### rac-2-Methoxy-4-{3-[3-methyl-4-(2-methyl-pyrrolidinocarbonyl)-phenyl]-propargylamino}benzamidin

Hergestellt analog Beispiel 4c aus rac-2-Methoxy-4-{3-[3-methyl-4-(2-methyl-pyrrolidinocarbonyl)-phenyl)-propargylamino}benzonitril, mit Chlorwasserstoffgas gesättigtem Ethanol und Ammoniumcarbonat.
Ausbeute: 23 % der Theorie,
R_{f}-Wert: 0.33 (Kieselgel; Methylenchlorid/Ethanol = 4:1 und 1 Tropfen Essigsäure)
C₂₄H₂₈N₄O₂ x HCl (404.52/440.98)
Massenspektrum: (M+H)⁺ = 405
(2M+H)⁺ = 809

### Beispiel 50

### 4-[1-Methyl-3-(3-methyl-4-pyrrolidinocarbonyl-phenyl)-propargylamino]benzamidin

Hergestellt analog Beispiel 4c aus 4-[1-Methyl-3-(3-methyl-4-pyrrolidinocarbonyl-phenyl)-propargylamino]benzonitril, Chlorwasserstoffgas gesättigtem Ethanol und Ammoniumcarbonat. Ausbeute: 79 % der Theorie,
R_{f}-Wert: 0.43 (Kieselgel; Essigester/Ethanol/Ammoniak = 50:45:5)
C₂₃H₂₆N₄O₂ x HCl (374.49/410.96)
Massenspektrum: (M+H)⁺ = 375

### Beispiel 51

### rac-4-{3-[2,5-Dimethyl-4-(2-methyl-pyrrolidinocarbonyl)-phenyl]-propargylamino}benzamidin

Hergestellt analog Beispiel 2 aus rac-N-tert.Butoxycarbonyl-4-{3-[2,5-dimethyl-4-(2-methyl-pyrrolidinocarbonyl)-phenyl]-propargylamino}benzamidin und Trifluoressigsäure.
Ausbeute: 4 % der Theorie,
R_{f}-Wert: 0.1 (Kieselgel; Methylenchlorid/Ethanol = 4:1) C₂₄H₂₈N₄O x CF₃COOH (388.51/502.54)
Massenspektrum: (M+H)⁺ = 389

### Beispiel 52

### 2-[3-(3-Methyl-4-pyrrolidinocarbonyl-phenyl]-propargylamino]-5-amidino-pyridin

Hergestellt analog Beispiel 4c aus 2-[3-(3-Methyl-4-pyrrolidinocarbonyl-phenyl)-propargylamino]-5-cyano-pyridin, mit Chlorwasserstoffgas gesättigtem Ethanol und Ammoniumcarbonat. Ausbeute: 43 % der Theorie,
R_{f}-Wert: 0.20 (Kieselgel; Methylenchlorid/Ethanol = 4:1) C₂₁H₂₃N₅O x HCl (361.45/397.91)
Massenspektrum: (M+H)⁺ = 362

### Beispiel 53

### 4-[3-(3-Methyl-4-tetrahydropyrazolocarbonyl-phenyl)-propargylamino]benzamidin

Hergestellt analog Beispiel 2 aus N-tert.Butoxycarbonyl-4-{3-[3-methyl-4-(2-butoxycarbonyl-tetrahydropyrazolocarbonyl)-phenyl]-propargylamino}benzamidin und Trifluoressigsäure.
Ausbeute: 28 % der Theorie,
R_{f}-Wert: 0.45 (Reversed Phase Kieselgel RP-8; Methanol/5%ige NaCl-Lösung = 60:40)
C₂₁H₂₃N₅O x 2 CF₃COOH (361.46/589.50)
Massenspektrum: (M+H)⁺ = 362

### Beispiel 54

### 4-{3-[3-Methyl-4-(4,5-dihydropyrazolocarbonyl)-phenyl]-propargylamino}benzamidin

Hergestellt analog Beispiel 2 durch mit Luftsauerstoff oxidiertem altem N-tert.Butoxycarbonyl-4-{3-[3-methyl-4-(tetrahydropyrazolocarbonyl)-phenyl]-propargylamino}benzamidin und Trifluoressigsäure.
Ausbeute: 5 % der Theorie,
R_{f}-Wert: 0.49 (Reversed Phase Kieselgel RP-8; Methanol/5%ige NaCl-Lösung = 60:40)
C₂₁H₂₁N₅O x CF₃COOH (359.44/473.46)
Massenspektrum: (M+H)⁺ = 360

### Beispiel 55

### 4-{3-[2,5-Dimethyl-4-(7-azabicyclo[2,2,1]hept-7-yl-carbonyl)-phenyl]-propargylamino}benzamidin

Hergestellt analog Beispiel 2 aus N-tert.Butoxycarbonyl-4-{3-[2,5-dimethyl-4-(7-azabicyclo[2,2,1]hept-7-yl-carbonyl)-phenyl]-propargylamino}benzamidin und Trifluoressigsäure.
Ausbeute: 58 % der Theorie,
R_{f}-Wert: 0.49 (Kieselgel; Methylenchlorid/Ethanol = 19:1) C₂₅H₂₈N₄O x CF₃COOH (400.53/514.55)
Massenspektrum: (M+H)⁺ = 401

### Beispiel 56

### rac-4-{3-[4-(3-Amino-pyrrolidinocarbonyl)-3-methyl-phenyl]-propargylamino}benzamidin

Hergestellt analog Beispiel 2 aus rac-N-tert.Butoxycarbonyl-4-{3-[4-(3-(tert.butoxycarbonyl)amino-pyrrolidinocarbonyl)-3-methyl-phenyl]-propargylamino}benzamidin und Trifluoressigsäure.
Ausbeute: 100 % der Theorie,
R_{f}-Wert: 0.60 (Reversed Phase Kieselgel RP-8; Methanol/5%ige NaCl-Lösung = 60:40)
C₂₂H₂₅N₅O x 2CF₃COOH (375.48/603.52)
Massenspektrum: (M+H)⁺ = 376
(M+2H)⁺⁺ = 188.5

### Beispiel 57

### 4-{3-[4-(Indolin-1-yl-carbonyl)-phenyl]-propargylamino}benzamidin

Hergestellt analog Beispiel 2 aus N-tert.Butoxycarbonyl-4-{3-[4-(indolin-1-yl-carbonyl)-phenyl]-propargylamino}benzamidin und Trifluoressigsäure.
Ausbeute: 42 % der Theorie,
R_{f}-Wert: 0.23 (Reversed Phase Kieselgel RP-8; Methanol/5%ige NaCl-Lösung = 60:40)
C₂₅H₂₂N₄O x CF₃COOH (394.48/508.50)
Massenspektrum: (M+H)⁺ = 395

### Beispiel 58

### rac-4-{3-[4-(2-Hydroxymethyl-pyrrolidinocarbonyl)-phenyl]-propargylamino}benzamidin

Hergestellt analog Beispiel 2 aus rac-N-tert.Butoxycarbonyl-4-{3-[4-(2-hydroxymethyl-pyrrolidinocarbonyl)-phenyl]-propargylamino}benzamidin und Trifluoressigsäure.
Ausbeute: 51 % der Theorie,
R_{f}-Wert: 0.26 (Kieselgel; Methylenchlorid/Ethanol = 4:1 und 1 Tropfen Essigsäure)
C₂₂H₂₄N₄O₂ x CF₃COOH (376.46/490.48)
Massenspektrum: (M+H)⁺ = 377
(M+2H)⁺⁺ = 189
(M+Na+H)⁺⁺ = 200

### Beispiel 59

### rac-4-{3-[4-(2-Ethoxycarbonylmethyl-pyrrolidinocarbonyl)-3-methyl-phenyl]-propargylamino}benzamidin

Hergestellt analog Beispiel 2 aus rac-N-tert.Butoxycarbonyl-4-{3-[4-(2-ethoxycarbonylmethyl-pyrrolidinocarbonyl)-3-methylphenyl]-propargylamino}benzamidin und Trifluoressigsäure. Ausbeute: 66 % der Theorie,
R_{f}-Wert: 0.27 (Kieselgel; Methylenchlorid/Ethanol = 4:1 und 1 Tropfen Essigsäure)
C₂₆H₃₀N₄O₃ x CF₃COOH (446.55/560.57)
Massenspektrum: (M+H)⁺ = 447
(M+2H)⁺⁺ = 224
(M+Na+H)⁺⁺ = 235

### Beispiel 60

### rac-4-{3-[4-(2-(2-Ethoxycarbonyl-ethyl)-pyrrolidinocarbonyl)-3-methyl-phenyl]-propargylamino}benzamidin

Hergestellt analog Beispiel 2 aus rac-N-tert.Butoxycarbonyl-4-{3-[4-(2-(2-ethoxycarbonyl-ethyl)-3-methyl-pyrrolidinocarbonyl)-phenyl]-propargylamino}benzamidin und Trifluoressigsäure.

Ausbeute: 56 % der Theorie,
R_{f}-Wert: 0.28 (Kieselgel; Methylenchlorid/Ethanol = 4:1 und 1 Tropfen Essigsäure)
C₂₇H₃₂N₄O₃ x CF₃COOH (460.58/574.61)
Massenspektrum: (M+H)⁺ = 461
(M+Na+H)⁺⁺ = 242

### Beispiel 61

### rac-4-{3-[4-(2-Methoxycarbonyl-pyrrolidinocarbonyl)-phenyl]-propargylamino}benzamidin

Hergestellt analog Beispiel 2 aus rac-N-tert.Butoxycarbonyl-4-{3-[4-(2-methoxycarbonyl-pyrrolidinocarbonyl)-phenyl]-propargylamino}benzamidin und Trifluoressigsäure.
Ausbeute: 69 % der Theorie,
R_{f}-Wert: 0.26 (Kieselgel; Methylenchlorid/Ethanol = 4:1 und 1 Tropfen Essigsäure)
C₂₃H₂₄N₄O₃ x CF₃COOH (404.47/518.49)
Massenspektrum: (M+H)⁺ = 405
(M+Na+H)⁺⁺ = 214
(M+2H)⁺⁺ = 203

### Beispiel 62

### rac-4-{3-[4-(2-Ethoxycarbonylmethyloxymethyl-pyrrolidinocarbonyl)-phenyl]-3-methyl-propargylamino}benzamidin

Hergestellt analog Beispiel 2 aus rac-N-tert.Butoxycarbonyl-4-{3-[4-(2-ethoxycarbonylmethyloxymethyl-pyrrolidinocarbonyl)-3-methyl-phenyl]-propargylamino}benzamidin und Trifluoressigsäure.
Ausbeute: 76 % der Theorie,
R_{f}-Wert: 0.26 (Kieselgel; Methylenchlorid/Ethanol = 4:1 und 1 Tropfen Essigsäure)
C₂₇H₃₂N₄O₄ x CF₃COOH (476.58/590.61)
Massenspektrum: (M+H)⁺ = 477
(M+Na+H)⁺⁺ = 250

### Beispiel 63

### rac-4-{3-[4-(2-Ethoxycarbonylmethylaminocarbonylmethyl-pyrrolidinocarbonyl)-3-methyl-phenyl]-propargylamino}benzamidin

Hergestellt analog Beispiel 2 aus rac-N-tert.Butoxycarbonyl-4-{3-[4-(2-ethoxycarbonylmethylaminocarbonylmethyl-pyrrolidinocarbonyl)-3-methyl-phenyl]-propargylamino}benzamidin und Trifluoressigsäure.
Ausbeute: 60 % der Theorie,
R_{f}-Wert: 0.21 (Kieselgel; Methylenchlorid/Ethanol = 4:1 und ein Tropfen Essigsäure)
C₂₈H₃₃N₅O₄ x CF₃COOH (503.61/617.63)
Massenspektrum: (M+H)⁺ = 504
(M+Na+H)⁺⁺ = 263.7
(M+2H)⁺⁺ = 252.7

### Beispiel 64

### 4-{3-[3-(2-Ethoxycarbonyl-ethyl)-4-pyrrolidinocarbonyl-phenyl]propargylamino}benzamidin

Hergestellt analog Beispiel 2 aus N-tert.Butoxycarbonyl-4-{3-[3-(2-ethoxycarbonyl-ethyl)-4-pyrrolidinocarbonylphenyl]propargylamino}benzamidin und Trifluoressigsäure. Ausbeute: 30 % der Theorie,
R_{f}-Wert: 0.16 (Kieselgel; Methylenchlorid/Ethanol = 4:1) C₂₆H₃₀N₄O₃ x CF₃COOH (446.55/560.57)
Massenspektrum: (M+H)⁺ = 447
(M+2H)⁺⁺ = 224
(M+Na+H)⁺⁺ = 235

### Beispiel 65

### rac-4-{3-[3-(N-Ethoxycarbonylmethyl-N-methyl-aminomethyl)-4-(2-methyl-pyrrolidinocarbonyl)-phenyl]propargylamino}benzamidin

Hergestellt analog Beispiel 2 aus rac-N-tert.Butoxycarbonyl-4-{3-[3-(N-ethoxycarbonylmethyl-N-methyl-aminomethyl)-4-(2-methyl-pyrrolidinocarbonyl)-phenyl]propargylamino}benzamidin und Trifluoressigsäure.
Ausbeute: 49 % der Theorie,
R_{f}-Wert: 0.23 (Reversed Phase Kieselgel RP-8; Methanol/5%ige NaCl-Lösung = 60:40)
C₂₆H₃₅N₅O₃ x CF₃COOH (489.62/603.64)
Massenspektrum: (M+H) ⁺ = 490
(M+2H)⁺⁺ = 245.5
(M+Na+H)⁺⁺ = 256.5

### Beispiel 66

### 4-[3-(3-Ethoxycarbonylmethyloxymethyl-4-pyrrolidinocarbonylphenyl)propargylamino]benzamidin

Hergestellt analog Beispiel 2 aus rac-N-tert.Butoxycarbonyl-4-[3-(3-ethoxycarbonylmethyloxymethyl-4-pyrrolidinocarbonylphenyl)propargylamino]benzamidin und Trifluoressigsäure. Ausbeute: 96 % der Theorie,
Rₜ-Wert: 0.23 (Reversed Phase Kieselgel RP-8; Methanol/5%ige NaCl-Lösung = 60:40)
C₂₆H₃₀N₄O₄ x CF₃COOH (462.55/576.57)
Massenspektrum: (M+H) ⁺ = 463
(M+2H)⁺⁺ = 232
(M+Na+H)⁺⁺ = 243

### Beispiel 67

### 4-[3-(3-Ethoxycarbonylmethyl-4-pyrrolidinocarbonyl-phenyl)propargylaminolbenzamidin

Hergestellt analog Beispiel 2 aus N-tert.Butoxycarbonyl-4-[3-(3-ethoxycarbonylmethyl-4-pyrrolidinocarbonyl-phenyl)-propargylamino]benzamidin und Trifluoressigsäure.
Ausbeute: 85 % der Theorie,
R_{f}-Wert: 0.45 (Kieselgel; Methylenchlorid/Ethanol = 4:1 + 1 Tropfen Essigsäure)
C₂₅H₂₈N₄O₃ x CF₃COOH (432.53/546.55)
Massenspektrum: (M+H)⁺ = 433

### Beispiel 68

### 4-[3-(3-Ethoxycarbonylmethylaminocarbonylmethyl-4-pyrrolidinocarbonyl-phenyl)propargylamino]benzamidin

Hergestellt analog Beispiel 2 aus rac-N-tert.Butoxycarbonyl-4-[3-(3-ethoxycarbonylmethylaminocarbonylmethyl-4-pyrrolidinocarbonyl-phenyl)propargylamino]benzamidin und Trifluoressigsäure.
Ausbeute: 91 % der Theorie,
R_{f}-Wert: 0.35 (Kieselgel; Methylenchlorid/Ethanol = 4:1 + 1 Tropfen Essigsäure)
C₂₇H₃₁N₅O₄ x CF₃COOH (489.58/603.60)
Massenspektrum: (M+H)⁺ = 490

### Beispiel 69

### 4-{3-[4-[Phenyl-(ethoxycarbonylmethyloxyimino)-methylen]-phenyl]propargylamino}benzamidin

### a) 4-{3-[4-[Phenyl-(hydroxycarbonylmethyloxyimino)-methylen]-phenyl]propargylamino}benzonitril

Ein Gemisch aus 0.50 g (1.5 mMol) 4-[3-(4-Phenylcarbonyl-phenyl)-propargylamino]benzonitril, 0.50 g (2.3 mMol) Carboxymethoxylamin-hydrochlorid, 0.32 ml (2.3 mMol) Triethylamin, 3 g 3-Angstöm-Molekularsieb und 3 g 4-Angström-Molekularsieb in 45 ml Methanol/Toluol (2:1) wird eine Woche zum Sieden erhitzt. Anschließend wird filtriert, konzentriert und durch Flash-Chromatographie (Kieselgel; Petrolether/Essigester = 2:1 bis Essigester/Essigsäure = 200:1) gereinigt.
Ausbeute: 75 % der Theorie,
R_{f}-Wert: <0.1 (Kieselgel; Essigester/Petrolether = 1:1)

### b) 4-{3-[4-[Phenyl-(ethoxycarbonylmethyloxyimino)-methylen]-phenyl]propargylamino}benzamidin

Hergestellt analog Beispiel 4c aus 4-{3-[4-[Phenyl-(hydroxycarbonylmethyloxyimino)-methylen]-phenyl]propargylamino}benzonitril, mit Chlorwasserstoffgas gesättigtem Ethanol und Ammoniumcarbonat.
Ausbeute: 27 % der Theorie,
R_{f}-Wert: 0.23 (Kieselgel; Methylenchlorid/Ethanol = 4:1 + 1 Tropfen Essigsäure)
C₂₇H₂₆N₄O₃ x HCl (454.53/490.99)
Massenspektrum: (M+H)⁻ = 455

### Beispiel 70

### 4-{3-[4-[N-(2-Ethoxycarbonyl-ethyl)-N-isopropyl-aminocarbonyl]-3-methyl-phenyl]propargylamino}benzamidin

Hergestellt analog Beispiel 2 aus rac-N-tert.Butoxycarbonyl-4-{3-[4-[N-(2-ethoxycarbonyl-ethyl)-N-isopropyl-aminocarbonyl]-3-methyl-phenyl]propargylamino}benzamidin und Trifluoressigsäure.
Ausbeute: 44 % der Theorie,
R_{f}-Wert: 0.1 (Kieselgel; Methylenchlorid/Ethanol = 4:1) C₂₁H₃₂N₄O₃ x CF₃COOH (448.56/562.59)
Massenspektrum: (M+H)⁺ = 449

### Beispiel 71

### 4-{3-[4-[N-(2-Ethoxycarbonyl-ethyl)-N-methyl-aminocarbonyl]-3-methyl-phenyl]propargylamino}benzamidin

Hergestellt analog Beispiel 2 aus rac-N-tert.Butoxycarbonyl-4-{3-[4-[N-(2-ethoxycarbonyl-ethyl)-N-isopropyl-aminocarbonyl]-3-methyl-phenyl]propargylamino}benzamidin und Trifluoressigsäure.
Ausbeute: 16 % der Theorie,
R_{f}-Wert: 0.1 (Kieselgel; Methylenchlorid/Ethanol = 4:1) C₂₄H₂₈N₄O₃ x CF₃COOH (420.52/534.54)
Massenspektrum: (M+H)⁺ = 421

### Beispiel 72

### 4-{3-[4-[N-(2-Methoxycarbonyl-ethyl)-N-pyridin-2-yl-aminocarbonyl]-2,5-dimethyl-phenyl]propargylamino}benzamidin

Hergestellt analog Beispiel 2 aus rac-N-tert.Butoxycarbonyl-4-{3-[4-[N-(2-methoxycarbonyl-ethyl)-N-pyridin-2-yl-aminocarbonyl]-2,5-dimethyl-phenyl]propargylamino}benzamidin und Trifluoressigsäure.
Ausbeute: 48 % der Theorie,
R_{f}-Wert: 0.25 (Kieselgel; Methylenchlorid/Ethanol = 4:1) C₂₈H₂₉N₅O₃ x 2CF₃COOH (483.58/711.62)
Massenspektrum: (M+H)⁺ = 484

### Beispiel 73

### rac-4-{3-[4-(2-Hydroxycarbonylmethyl-pyrrolidinocarbonyl)-3-methyl-phenyl]-propargylamino}benzamidin

Hergestellt analog Beispiel 3 aus rac-4-{3-[4-(2-Ethoxycarbonylmethyl-pyrrolidinocarbonyl)-3-methyl-phenyl]-propargylamino}benzamidin, Lithiumhydroxid und anschließender Behandlung mit Ammoniumchlorid.
Ausbeute: 53 % der Theorie,
R_{f}-Wert: 0.36 (Reversed Phase Kieselgel RP-8; Methanol/5%ige NaCl-Lösung = 60:40)
C₂₄H₂₆N₄O₃ x HCl (418.50/454.96)
Massenspektrum: (M+H)⁺ = 419
(M+Na)⁺ = 441
(M+Na+H)⁺⁺ = 232

### Beispiel 74

### rac-4-{3-[4-(2-(2-Hydroxycarbonyl-ethyl)-pyrrolidinocarbonyl)-3-methyl-phenyl]-propargylamino}benzamidin

Hergestellt analog Beispiel 3 aus rac-4-{3-[4-(2-(2-Ethoxycarbonyl-ethyl)-pyrrolidinocarbonyl)-3-methyl-phenyl]-propargylamino}benzamidin, Lithiumhydroxid und anschließender Behandlung mit Ammoniumchlorid.
Ausbeute: 67 % der Theorie,
R_{f}-Wert: 0.39 (Reversed Phase Kieselgel RP-8; Methanol/5%ige NaCl-Lösung = 60:40)
C₂₅H₂₈N₄O₃ x HCl (432.52/468.98)
Massenspektrum: (M+H)⁺ = 433
(M+Na) ⁺ = 455
(M+2Na)⁺⁻ = 239

### Beispiel 75

### rac-4-{3-[4-(2-Hydroxycarbonyl-pyrrolidinocarbonyl)-phenyl]-propargylamino}benzamidin

Hergestellt analog Beispiel 3 aus rac-4-{3-{4-(2-Methoxycarbonyl-pyrrolidinocarbonyl)-phenyl]-propargylamino}benzamidin, Lithiumhydroxid und anschließender Behandlung mit Ammoniumchlorid.
Ausbeute: 42 % der Theorie,
R_{f}-Wert: 0.32 (Reversed Phase Kieselgel RP-8; Methanol/5%ige NaCl-Lösung = 60:40)
C₂₂H₂₂N₄O₃ x HCl (390.45/426.91)
Massenspektrum: (M+H)⁺ = 391
(M+Na)⁺ = 413
(M+2Na)⁺⁺ = 218

### Beispiel 76

### rac-4-{3-[4-(2-Hydroxycarbonylmethyloxymethyl-pyrrolidinocarbonyl)-3-methyl-phenyl]-propargylamino}benzamidin

Hergestellt analog Beispiel 3 aus rac-4-{3-[4-(2-Ethoxycarbonylmethyloxymethyl-pyrrolidinocarbonyl)-3-methyl-phenyl]-propargylamino}benzamidin, Lithiumhydroxid und anschließender Behandlung mit Ammoniumchlorid.
Ausbeute: 30 % der Theorie,
R_{f}-Wert: 0.4 (Reversed Phase Kieselgel RP-18; Methanol/5%ige NaCl-Lösung = 60:40)
C₂₅H₂₈N₄O₄ x HCl (448.53/484.99)
Massenspektrum: (M+H)⁺ = 449
(M-H)⁻ = 447

### Beispiel 77

### rac-4-{3-[4-(2-Hydroxycarbonylmethylaminocarbonylmethyl-pyrrolidinocarbonyl)-3-methyl-phenyl]-propargylamino}benzamidin

Hergestellt analog Beispiel 2 aus rac-4-{3-[4-(2-Ethoxycarbonylmethylaminocarbonylmethyl-pyrrolidinocarbonyl)-3-methylphenyl]-propargylamino}benzamidin, Lithiumhydroxid und anschließender Behandlung mit Ammoniumchlorid.
Ausbeute: 80 % der Theorie,
R_{f}-Wert: 0.38 (Reversed Phase Kieselgel RP-8; Methanol/5%ige NaCl-Lösung = 60:40)
C₂₆H₂₉N₅O₄ x HCl (475.55/512.01)
Massenspektrum: (M+H)⁺ = 476
(M-H)⁻ = 474

### Beispiel 78

### 4-{3-[3-(2-Hydroxycarbonyl-ethyl)-4-pyrrolidinocarbonyl-phenyl]propargylamino}benzamidin

Hergestellt analog Beispiel 3 aus 4-{3-[3-(2-Ethoxycarbonylethyl)-4-pyrrolidinocarbonyl-phenyl]propargylamino}benzamidin, Lithiumhydroxid und anschließender Behandlung mit Ammoniümchlorid.
Ausbeute: 84 % der Theorie,
R_{f}-Wert: >0.1 (Kieselgel; Methylenchlorid/Ethanol = 4:1) C₂₄H₂₆N₄O₃ x HCl (418.50/454.96)
Massenspektrum: (M+H)⁺ = 419
(M+2H)⁺⁺ = 210
(M+Na+H)⁺⁺ = 221
(M+2Na)⁺⁺ = 232

### Beispiel 79

### 4-{3-[3-(2-Hydroxycarbonyl-vinyl)-4-pyrrolidinocarbonyl-phenyl]propargylamino}benzamidin

Hergestellt analog Beispiel 3 aus 4-{3-[3-(2-Ethoxycarbonylvinyl)-4-pyrrolidinocarbonyl-phenyl]propargylamino}benzamidin, Lithiumhydroxid und anschließender Behandlung mit Ammoniumchlorid.
Ausbeute: 45 % der Theorie,
R_{f}-Wert: >0.1 (Kieselgel; Methylenchlorid/Ethanol = 4:1) C₂₄H₂₄N₄O₃ x HCl (416.48/452.94)
Massenspektrum: (M+H)⁻ = 417
(M+Na)⁺ = 439
(M+Na+H)⁺⁺ = 220
(M+2Na)⁺⁺ = 231

### Beispiel 80

### rac-4-{3-[3-(N-Hydroxycarbonylmethyl-N-methyl-aminomethyl)-4-(2-methyl-pyrrolidinocarbonyl)-phenyl]propargylamino}benzamidin

Hergestellt analog Beispiel 3 aus rac-4-{3-[3-(N-Ethoxycarbonylmethyl-N-methyl-aminomethyl)-4-(2-methyl-pyrrolidinocarbonyl)-phenyl)propargylamino}benzamidin, Lithiumhydroxid und anschließender Behandlung mit Ammoniumchlorid.
Ausbeute: 30 % der Theorie,
R_{f}-Wert: 0.42 (Reversed Phase Kieselgel RP-8; Methanol/5%ige NaCl-Lösung = 60:40)
C₂₆H₃₁N₅O₃ x HCl (461.57/498.03)
Massenspektrum: (M+H)⁺ = 462
(M-H)⁻ = 460

### Beispiel 81

### 4-[3-(3-Hydroxycarbonylmethyloxymethyl-4-pyrrolidinocarbonylphenyl)propargylamino]benzamidin

Hergestellt analog Beispiel 3 aus 4-[3-(3-Ethoxycarbönylmethyloxymethyl-4-pyrrolidinocarbonyl-phenyl)propargylamino]-benzamidin, Lithiumhydroxid und anschließender Behandlung mit Ammoniumchlorid.
Ausbeute: 21 % der Theorie,
R_{f}-Wert: 0.23 (Reversed Phase Kieselgel RP-8; Methanol/5%ige NaCl-Lösung = 60:40)
C₂₄H₂₆N₄O₄ x HCl (434.50/470.96)
Massenspektrum: (M+H)⁺ = 435
(M-H)⁻ = 433

### Beispiel 82

### 4-[3-(3-Hydroxycarbonylmethyl-4-pyrrolidinocarbonyl-phenyl)-propargylamino]benzamidin

Hergestellt analog Beispiel 3 aus 4-[3-(3-Ethoxycarbonylmethyl-4-pyrrolidinocarbonyl-phenyl)propargylamino]benzamidin,

Lithiumhydroxid und anschließender Behandlung mit Ammoniumchlorid.
Ausbeute: 62 % der Theorie,
R_{f}-Wert: 0.45 (Reversed Phase Kieselgel RP-8; Methanol/5%ige NaCl-Lösung = 60:40)
C₂₃H₂₄N₄O₃ x HCl (404.47/440.93)
Massenspektrum: (M+H)⁺ = 405
(M-H)⁻ = 403

### Beispiel 83

### 4-[3-(3-Hydroxycarbonylmethylaminocarbonylmethyl-4-pyrrolidinocarbonyl-phenyl)propargylamino]benzamidin

Hergestellt analog Beispiel 3 aus 4-[3-(3-Ethoxycarbonylmethylaminocarbonylmethyl-4-pyrrolidinocarbonyl-phenyl)propargylamino}benzamidin, Lithiumhydroxid und anschließender Behandlung mit Ammoniumchlorid.
Ausbeute: 42 % der Theorie,
R_{f}-Wert: 0.45 (Reversed Phase Kieselgel RP-8; Methanol/5%ige NaCl-Lösung = 60:40)
C₂₅H₂₇N₅O₄ x HCl (461.53/497.99)
Massenspektrum: (M+H)⁺ = 462
(M+Na)⁺ = 484

### Beispiel 84

### 4-{3-[3-(Hydroxycarbonylmethyloxyimino)methylen-4-pyrrolidinocarbonyl-phenyl]propargylamino}benzamidin

Hergestellt analog Beispiel 2 aus rac-N-tert.Butoxycarbonyl-4-{3-[3-(hydroxycarbonylmethyloxyimino)methylen-4-pyrrolidinocarbonyl-phenyl]propargylamino}benzamidin und Trifluoressigsäure.
Ausbeute: 42 % der Theorie,
R_{f}-Wert: 0.4 (Reversed Phase Kieselgel RP-8; Methanol/5%ige NaCl-Lösung = 60:40)
C₂₄H₂₅N₅O₄ x CF₃COOH (447.50/561.52)
Massenspektrum: (M+H)⁺ = 448
(M+Na)⁻ = 470
(M+2H)⁺⁺ = 224.5
(M+Na+H)⁺⁺ = 235.7
(M+2Na)⁻⁺ = 246.6

### Beispiel 85

### rac-4-{3-[2-Methoxy-4-(2-methyl-pyrrolidinocarbonyl)-phenyl]-propargylamino}benzamidin

Hergestellt analog Beispiel 2 aus rac-N-tert.Butoxycarbonyl-4-{3-[2-methoxy-4-(2-methyl-pyrrolidinocarbonyl)-phenyl]propargylamino}benzamidin und Trifluoressigsäure.
Ausbeute: 90 % der Theorie,
R_{f}-Wert: 0.38 (Kieselgel; Methylenchlorid/Ethanol = 4:1 und ein Tropfen Essigsäure)
C₂₃H₂₆N₄O₂ x CF₃COOH (390.49/504.51)
Massenspektrum: (M+H)⁺ = 391
(M+2H)⁺⁺ = 196

### Beispiel 86

### 4-[3-(3-Methoxy-4-pyrrolidinocarbonyl-phenyl)propargylamino]-benzamidin

Hergestellt analog Beispiel 2 aus N-tert.Butoxycarbonyl-4-[3-(3-methoxy-4-pyrrolidinocarbonyl-phenyl)propargylamino]benzamidin und Trifluoressigsäure.
Ausbeute: 58 % der Theorie,
R_{f}-Wert: 0.20 (Kieselgel; Methylenchlorid/Ethanol = 4:1 und ein Tropfen Essigsäure)
C₂₂H₂₄N₄O₂ x CF₃COOH (376.46/490.48)
Massenspektrum: (M+H)⁺ = 377

### Beispiel 87

### 4-[3-(3-Hydroxy-4-pyrrolidinocarbonyl-phenyl)propargylamino]-benzamidin

Hergestellt analog Beispiel 2 aus N-tert.Butoxycarbonyl-4-[3-(3-hydroxy-4-pyrrolidinocarbonyl-phenyl)propargylamino]benzamidin und Trifluoressigsäure.
Ausbeute: 54 % der Theorie,
R_{f}-Wert: 0.46 (Reversed Phase Kieselgel RP-8; Methanol/5%ige NaCl-Lösung = 60:40)
C₂₁H₂₂N₄O₂ x CF₃COOH (362.44/476.46)
Massenspektrum: (M+H)⁺ = 363

### Beispiel 88

### rac-4-{3-[3-Hydroxymethyl-4-(2-methyl-pyrrolidinocarbonyl)-phenyl]propargylamino}benzamidin

Hergestellt analog Beispiel 2 aus rac-N-tert.Butoxycarbonyl-4-{3-[3-hydroxymethyl-4-(2-methyl-pyrrolidinocarbonyl)-phenyl]propargylamino}benzamidin und Trifluoressigsäure. Ausbeute: 57 % der Theorie,
R_{f}-Wert: 0.54 (Reversed Phase Kieselgel RP-8; Methanol/5%ige NaCl-Lösung = 60:40)
C₂₃H₂₆N₄O₂ x CF₃COOH (390.49/504.51)
Massenspektrum: (M+H)⁺ = 391

### Beispiel 89

### 4-[3-(3-Formyl-4-pyrrolidinocarbonyl-phenyl)propargylamino]-benzamidin

Hergestellt analog Beispiel 2 aus N-tert.Butoxycarbonyl-4-[3-(3-formyl-4-pyrrolidinocarbonyl-phenyl)propargylamino]benzamidin und Trifluoressigsäure.
Ausbeute: 71 % der Theorie,
R_{f}-Wert: 0.4 (Reversed Phase Kieselgel RP-8; Methanol/5%ige NaCl-Lösung = 60:40)
C₂₂H₂₂N₄O₂ x CF₃COOH (374.45/488.47)
Massenspektrum: (M+H)⁺ = 375

### Beispiel 90

### 4-[3-(3-Aminocarbonylaminoiminomethylen-4-pyrrolidinocarbonylphenyl)propargylamino]benzamidin

Hergestellt analog Beispiel 2 aus N-tert.Butoxycarbonyl-4-[3-(3-aminocarbonylaminoiminomethylen-4-pyrrolidinocarbonylphenyl)propargylamino]benzamidin und Trifluoressigsäure. Ausbeute: 83 % der Theorie,
R_{f}-Wert: 0.4 (Reversed Phase Kieselgel RP-8; Methanol/5%ige. NaCl-Lösung = 60:40)
C₂₃H₂₅N₇O₂ x CF₃COOH (431.50/545.53)
Massenspektrum: (M+H)⁺ = 432
(M+Na+H)⁺⁺ = 227.8

### Beispiel 91

### 4-[1-Methyl-3-(4-pyrrolidinocarbonyl-phenyl)propargylamino]-benzamidin

Hergestellt analog Beispiel 4c aus 4-[1-Methyl-3-(4-pyrrolidinocarbonyl-phenyl)propargylamino]benzonitril, mit Chlorwasserstoffgas gesättigtem Ethanol und Ammoniumcarbonat.
Ausbeute: 78 % der Theorie,
R_{f}-Wert: 0.57 (Kieselgel; Essigester/Ethanol/Ammoniak = 50:45:5)
C₂₂H₂₄N₄O x HCl (360.47/396.93)
Massenspektrum: (M+H)⁺ = 361
(M+Cl)⁻ = 395/397
(M+Cl+HCl)⁻ = 431/433/435N

### Beispiel 92

### 4-[3-(4-Piperidinocarbonyl-phenyl)propargylamino]benzamidin

Hergestellt analog Beispiel 4c aus 4-[3-(4-Piperidinocarbonylphenyl)propargylamino]benzonitril, mit Chlorwasserstoffgas gesättigtem Ethanol und Ammoniumcarbonat.
Ausbeute: 65 % der Theorie,
R_{f}-Wert: 0.25 (Reversed Phase Kieselgel RP-8; Methanol/5%ige NaCl-Lösung = 60:40)
C₂₂H₂₄N₄O x HCl (360.47/396.93)
Massenspektrum: M⁺ = 360

### Beispiel 93

### rac-4-{3-[4-(4-Methylpiperidinocarbonyl)-phenyl]propargylamino}benzamidin

Hergestellt analog Beispiel 2 aus rac-N-tert.Butoxycarbonyl-4-{3-[4-(4-methylpiperidinocarbonyl)-phenyl]propargylamino}benzamidin und Trifluoressigsäure.
Ausbeute: 70 % der Theorie,
R_{f}-Wert: 0.27 (Reversed Phase Kieselgel RP-8; Methanol/5%ige NaCl-Lösung = 60:40)
C₂₃H₂₆N₄O x CF₃COOH (374.49/488.51)
Massenspektrum: (M+H)⁺ = 375

### Beispiel 94

### 4-[3-(4-Azetidinocarbonyl-phenyl)propargylamino]benzamidin

Hergestellt analog Beispiel 2 aus N-tert.Butoxycarbonyl-4-[3-(4-azetidinocarbonyl-phenyl)propargylamino]benzamidin und Trifluoressigsäure.
Ausbeute: 72 % der Theorie,
R_{f}-Wert: 0.21 (Kieselgel; Methylenchlorid/Ethanol 4:1 und ein Tropfen Essigsäure)
C₂₀H₂₀N₄O x CF₃COOH (332.41/446.43)
Massenspektrum: (M+H)⁺ = 333

### Beispiel 95

### 4-[3-(3-Methyl-4-morpholinocarbonyl-phenyl)propargylamino]-benzamidin

Hergestellt analog Beispiel 2 aus N-tert.Butoxycarbonyl-4-[3-(3-methyl-4-morpholinocarbonyl-phenyl)propargylamino]-benzamidin und Trifluoressigsäure.
Ausbeute: 90 % der Theorie,
R_{f}-Wert: 0.32 (Kieselgel; Essigester/Ethanol/Ammoniak = 50:45:5)
C₂₂H₂₄N₄O₂ x CF₃COOH (376.47/490.49)
Massenspektrum: (M+H)⁺ = 377

### Beispiel 96

### 4-[3-(2-Methyl-4-morpholinocarbonyl-phenyl)propargylamino]-benzamidin

Hergestellt analog Beispiel 2 aus N-tert.Butoxycarbonyl-4-[3-(2-methyl-4-morpholinocarbonyl-phenyl)propargylamino]-benzamidin und Trifluoressigsäure.
Ausbeute: 80 % der Theorie,
R_{f}-Wert: 0.38 (Kieselgel; Essigester/Ethanol/Ammoniak = 50:45:5)
C₂₂H₂₄N₄O₂ x CF₃COOH (376.47/4.90.49)
Massenspektrum: (M+H)⁺ = 377

### Beispiel 97

### 4-{3-[4-(4-Methylpiperazinocarbonyl)-phenyl]propargylamino}benzamidin

Hergestellt analog Beispiel 2 aus N-tert.Butoxycarbonyl-4-{3-[4-(4-methylpiperazinocarbonyl)-phenyl]propargylamino}benzamidin und Trifluoressigsäure.
Ausbeute: 87 % der Theorie,
R_{f}-Wert: 0.59 (Reversed Phase Kieselgel RP-8; Methanol/5%ige NaCl-Lösung = 60:40)
C₂₂H₂₅N₅O x 2CF₃COOH (375.48/603.52)
Massenspektrum: (M+H)⁺ = 376

### Beispiel 98

### 4-[3-(4-Dimethylaminocarbonyl-phenyl)propargylamino]benzamidin

Hergestellt analog Beispiel 2 aus N-tert.Butoxycarbonyl-4-[3-(4-dimethylaminocarbonyl-phenyl)propargylamino]benzamidin und Trifluoressigsäure.
Ausbeute: 66 % der Theorie,
R_{f}-Wert: 0.21 (Kieselgel; Methylenchlorid/Ethanol 4:1 und ein Tropfen Essigsäure)
C₁₉H₂₀N₄O x CF₃COOH (320.40/434.42)
Massenspektrum: (M+H)⁺ = 321

### Beispiel 99

### 4-[3-(2,5-Dimethyl-4-dimethylaminocarbonyl-phenyl)propargylaminolbenzamidin

Hergestellt analog Beispiel 2 aus N-tert.Butoxycarbonyl 4-[3-(2,5-dimethyl-4-dimethylaminocarbonyl-phenyl)propargylämino]benzamidin und Trifluoressigsäure.
Ausbeute: 54 % der Theorie,
R_{f}-Wert: 0.13 (Kieselgel; Methylenchlorid/Ethanol = 4:1) C₂₁H₂₄N₄O x CF₃COOH (348.46/462.49)
Massenspektrum: (M+H)⁺ = 349

### Beispiel 100

### 4-[3-(4-Diethylaminocarbonyl-3-methyl-phenyl)propargylamino]-benzamidin

Hergestellt analog Beispiel 2 aus N-tert.Butoxycarbonyl-4-[3-(4-diethylaminocarbonyl-3-methyl-phenyl)propargylamino]-benzamidin und Trifluoressigsäure.
Ausbeute: 80 % der Theorie,
R_{f}-Wert: 0.29 (Kieselgel; Methylenchlorid/Ethanol = 4:1 und ein Tropfen Essigsäure)
C₂₂H₂₆N₄O x CF₃COOH (362.46/476.50)
Massenspektrum: (M+H)⁺ = 363

### Beispiel 101

### 4-{3-[4-(N-Isopropyl-N-methyl-aminocarbonyl)-2,5-dimethylphenyl]propargylamino}benzamidin

Hergestellt analog Beispiel 2 aus N-tert.Butoxycarbonyl-4-{3-[4-(N-isopropyl-N-methyl-aminocarbonyl)-2,5-dimethyl phenyl]propargylamino}benzamidin und Trifluoressigsäure.
Ausbeute: 92 % der Theorie,
R_{f}-Wert: 0.15 (Kieselgel; Methylenchlorid/Ethanol = 4:1)
C₂₃H₂₈N₄O x CF₃COOH (376.51/490.53)
Massenspektrum: (M+H)⁺ = 377

### Beispiel 102

### 4-{3-[4-(N-tert.Butyl-N-methyl-aminocarbonyl)-2,5-dimethylphenyl]propargylamino}benzamidin

Hergestellt analog Beispiel 2 aus N-tert.Butoxycarbonyl-4-{3-[4-(N-tert.butyl-N-methyl-aminocarbonyl)-2,5-dimethylphenyl]propargylamino}benzamidin und Trifluoressigsäure.
Ausbeute: 41 % der Theorie,
R_{f}-Wert: 0.3 (Kieselgel; Methylenchlorid/Ethanol = 4:1)
C₂₄H₃₀N₄O x CF₃COOH (390.54/504.56)
Massenspektrum: (M+H)⁺ = 391

### Beispiel 103

### 4-[3-(4-Trimethylhydrazinocarbonyl-3-methyl-phenyl)propargylamino]benzamidin

Hergestellt analog Beispiel 2 aus N-tert.Butoxycarbonyl-4-[3-(4-trimethylhydrazinocarbonyl-3-methyl-phenyl)propargylamino]benzamidin und Trifluoressigsäure.
Ausbeute: 7 % der Theorie,
R_{f}-Wert: 0.3 (Reversed Phase Kieselgel RP-8; Methanol/5%ige NaCl-Lösung = 60:40)
C₂₁H₂₅N₅O x 2CF₃COOH (363.47/591.51)
Massenspektrum: (M+H)⁺ = 364

### Beispiel 104

### 4-{3-[4-(N-(2-Dimethylamino-ethyl)-N-methyl-aminocarbonyl)-phenyl]propargylamino}benzamidin

Hergestellt analog Beispiel 2 aus N-tert.Butoxycarbonyl-4-{3-[4-(N-(2-dimethylamino-ethyl)-N-methyl-aminocarbonyl)-phenyl]propargylamino}benzamidin und Trifluoressigsäure.
Ausbeute: 23 % der Theorie,
R_{f}-Wert: 0.51 (Reversed Phase Kieselgel RP-8; Methanol/5%ige NaCl-Lösung = 60:40)
C₂₂H₂₇N₅O x 2CF₃COOH (377.50/605.54)
Massenspektrum: (M+H)⁺ = 378
(M+2H)⁺⁺ = 189.7

### Beispiel 105

### 4-{3-[4-(N-(3-Dimethylamino-propyl)-N-methyl-aminocarbonyl)-3-methyl-phenyl]propargylamino}benzamidin

Hergestellt analog Beispiel 2 aus N-tert.Butoxycarbonyl-4-{3-[4-(N-(3-dimethylamino-propyl)-N-methyl-aminocarbonyl)-3-methyl-phenyl]propargylamino}benzamidin und Trifluoressigsäure.
Ausbeute: 5 % der'Theorie,
R_{f}-Wert: 0.5 (Reversed Phase Kieselgel RP-8; Methanol/5%ige NaCl-Lösung = 60:40)
C₂₄H₃₁N₅O x 2CF₃COOH (405.56/633.60)
Massenspektrum: (M+2H)⁺⁺ = 203.8

### Beispiel 106

### 4-{3-[4-(N-Cyclopentyl-N-methyl-aminocarbonyl)-3-methylphenyl]propargylamino}benzamidin

Hergestellt analog Beispiel 2 aus N-tert.Butoxycarbonyl-4-{3-[4-(N-cyclopentyl-N-methyl-aminocarbonyl)-3-methylphenyl]propargylamino}benzamidin und Trifluoressigsäure.
Ausbeute: 84 % der Theorie,
R_{f}-Wert: 0.3 (Kieselgel; Methylenchlorid/Ethanol = 4:1)
C₂₄H₂₈N₄O x CF₃COOH (388.52/502.54)
Massenspektrum: (M+H)⁺ = 389

### Beispiel 107

### 4-{3-[4-(Pyrrolidin-3-ylamino-carbonyl)-3-methylphenyl]propargylamino}benzamidin

Hergestellt analog Beispiel 2 aus N-tert.Butoxycarbonyl-4-{3-[4-(1-tert.butoxycarbonylpyrrolidin-3-yl-aminocarbonyl)-3-methyl-phenyl]propargylamino}benzamidin und Trifluoressigsäure, anschließendes Aufnehmen in Ethanol und Ausfällen mit etherischer Salzsäure. Ausbeute: 33 % der Theorie,
R_{f}-Wert: 0.41 (Reversed Phase Kieselgel RP-8; Methanol/5%ige NaCl-Lösung = 60:40)
C₂₂H₂₅N₅O x 2HCl (375.49/448.41)
Massenspektrum: (M+H)⁺ = 376
(M+2H)⁺ = 185.5

### Beispiel 108

### 4-{3-[5-(N-Cyclopentyl-N-methyl-aminocarbonyl)-2-methylphenyllpropargylamino}benzamidin

Hergestellt analog Beispiel 2 aus N-tert.Butoxycarbonyl-4-{3-[5-(N-cyclopentyl-N-methyl-amino-carbonyl)-2-methylphenyl]propargylamino}benzamidin und Trifluoressigsäure. Ausbeute: 70 % der Theorie,
R_{f}-Wert: 0.21 (Kieselgel; Methylenchlorid/Ethanol = 4:1) C₂₄H₂₈N₄O x CF₃COOH (388.52/502.54)
Massenspektrum: (M+H)⁺ = 389

### Beispiel 109

### 4-{3-[5-(N-Methyl-N-(2-phenyl-ethyl)-aminocarbonyl)-2-methylphenyl]propargylamino}benzamidin

Hergestellt analog Beispiel 2 aus N-tert.Butoxycarbonyl-4-{3-[5-(N-methyl-N-(2-phenyl-ethyl)-aminocarbonyl)-2-methylphenyl]propargylamino}benzamidin und Trifluoressigsäure.
Ausbeute: 54 % der Theorie,
Rₜ-Wert: 0.22 (Kieselgel; Methylenchlorid/Ethanol = 4:1) C₂₇H₂₈N₄O x CF₃COOH (424.56/538.58)
Massenspektrum: (M+H)⁺ = 425

### Beispiel 110

### 4-{3-[5-(N-Methyl-N-benzyl-aminocarbonyl)-2-methyl-phenyl]-propargylamino}benzamidin

Hergestellt analog Beispiel 2 aus N-tert.Butoxycarbonyl-4-{3-[5-(N-methyl-N-benzyl-aminocarbonyl)-2-methyl-phenyl]-propargylamino}benzamidin und Trifluoressigsäure.
Ausbeute: 55 % der Theorie,
R_{f}-Wert: 0.25 (Kieselgel; Methylenchlorid/Ethanol = 4:1) C₂₆H₂₆N₄O x CF₃COOH (410.53/524.55)
Massenspektrum: (M+H)⁺ = 411

### Beispiel 111

### 4-{3-[5-(2-Phenyl-ethylaminocarbonyl)-2-methyl-phenyl]propargylamino}benzamidin

Hergestellt analog Beispiel 2 aus N-tert.Butoxycarbonyl-4-{3-[5-(2-phenyl-ethylaminocarbonyl)-2-methyl-phenyl]propargylamino}benzamidin und Trifluoressigsäure.
Ausbeute: 54 % der Theorie,
R_{f}-Wert: 0.13 (Kieselgel; Methylenchlorid/Ethanol = 4:1) C₂₆H₂₆N₄O x CF₃COOH (410.53/524.55)
Massenspektrum: (M+H)⁺ = 411

### Beispiel 112

### 4-{3-[4-(N-Methyl-N-phenyl-aminocarbonyl)-phenyl]propargylamino}benzamidin

Hergestellt analog Beispiel 4c aus 4-{3-[4-(N-Methyl-N-phenylaminocarbonyl)-phenyl]propargylamino}benzonitril, mit Chlorwasserstoffgas gesättigtem Ethanol und Ammoniumcarbonat. Ausbeute: 97 % der Theorie,
R_{f}-Wert: 0.20 (Kieselgel; Methylenchlorid/Ethanol = 4:1 + 1 Tropfen Essigsäure)
C₂₄H₂₂N₄O x HCl (382.47/418.93)
Massenspektrum: (M+H)⁺ = 383

### Beispiel 113

### 4-{3-[2,5-Dimethyl-4-(N-methyl-N-phenyl-aminocarbonyl)-phenyl]propargylamino}benzamidin

Hergestellt analog Beispiel 2 aus N-tert.Butoxycarbonyl-4-{3-[2,5-dimethyl-4-(N-methyl-N-phenyl-aminocarbonyl)-phenyl]propargylamino}benzamidin und Trifluoressigsäure. Ausbeute: 30 % der Theorie,
R_{f}-Wert: 0.18 (Kieselgel; Methylenchlorid/Ethanol = 4:1) C₂₆H₂₆N₄O x CF₃COOH (410.53/524.55)
Massenspektrum: (M+H)⁺ = 411

### Beispiel 114

### 4-{3-[2,5-Dimethyl-4-(N-methyl-N-phenyl-aminocarbonyl)-phenyl]-N-methyl-propargylamino}benzamidin

Hergestellt analog Beispiel 4c aus 4-{3-[2,5-Dimethyl-4-(N-methyl-N-phenyl-aminocarbonyl)-phenyl]-N-methyl-propargylamino}benzonitril, mit Chlorwasserstoffgas gesättigtem Ethanol und Ammoniumcarbonat.
Ausbeute: 54 % der Theorie,
R_{f}-Wert: 0.2 (Kieselgel; Methylenchlorid/Ethanol = 4:1) C₂₇H₂₈N₄O x HCl (424.55/461.01)
Massenspektrum: (M+H)⁺ = 425

### Beispiel 115

### 4-{3-[2-Methyl-4-(N-methyl-N-phenyl-aminocarbonyl)-phenyl]propargylamino}benzamidin

Hergestellt analog Beispiel 2 aus N-tert.Butoxycarbonyl-4-{3-[2-methyl-4-(N-methyl-N-phenyl-aminocarbonyl)-phenyl]-propargylamino}benzamidin und Trifluoressigsäure.
Ausbeute: 71 % der Theorie,
R_{f}-Wert: 0.22 (Kieselgel; Methylenchlorid/Ethanol = 4:1) C₂₅H₂₄N₄O x CF₃COOH (396.50/510.52)
Massenspektrum: (M+H)⁺ = 397

### Beispiel 116

### 4-{3-[2,5-Dimethyl-4-(N-methyl-N-pyridin-2-yl-aminocarbonyl)-phenyl]propargylamino}benzamidin

Hergestellt analog Beispiel 2 aus N-tert.Butoxycarbonyl-4-{3-[2,5-dimethyl-4-(N-methyl-N-pyridin-2-yl-aminocarbonyl)-phenyl]propargylamino}benzamidin und Trifluoressigsäure. Ausbeute: 50 % der Theorie,
R_{f}-Wert: 0.18 (Kieselgel; Methylenchlorid/Ethanol = 4:1) C₂₅H₂₅N₅O x 2CF₃COOH (411.51/639.55)
Massenspektrum: (M+H)⁻ = 412

### Beispiel 117

### 4-{3-[2-Methyl-5-(N-methyl-N-phenyl-aminocarbonyl)-phenyl]propargylamino}benzamidin

Hergestellt analog Beispiel 2 aus N-tert.Butoxycarbonyl-4-{3-[2-methyl-5-(N-methyl-N-phenyl-aminocarbonyl)-phenyl]propargylamino}benzamidin und Trifluoressigsäure. Ausbeute: 75 % der Theorie,
R_{f}-Wert: 0.13 (Kieselgel; Methylenchlorid/Ethanol = 4:1) C₂₅H₂₄N₄O x CF₃COOH (396.50/510.52)
Massenspektrum: (M+H)⁺ = 397

### Beispiel 118

### 4-{3-[4-(N-Methyl-N-phenyl-aminomethyl)-phenyl]propargylamino}benzamidin

Hergestellt analog Beispiel 4c aus 4-{3-(4-(N-Methyl-N-phenylaminomethyl)-phenyl]propargylamino}benzonitril, mit Chlorwasserstoffgas gesättigtem Ethanol und Ammoniumcarbonat.
Ausbeute: 70 % der Theorie,
R_{f}-Wert: 0.22 (Kieselgel; Methylenchlorid/Ethanol = 4:1 + 1 Tropfen Essigsäure)
C₂₄H₂₄N₄ x HCl (368.49/404.95)
Massenspektrum: (M+H)⁺ = 369

### Beispiel 119

### 4-{3-[4-(N-Acetyl-N-phenyl-aminomethyl)-phenyl]propargylamino}benzamidin

Hergestellt analog Beispiel 4c aus 4-{3-[4-(N-Acetyl-N-phenylaminomethyl)-phenyl]propargylamino}benzonitril, mit Chlorwasserstoffgas gesättigtem Ethanol und Ammoniumcarbonat. Ausbeute: 48 % der Theorie,
R_{f}-Wert: 0.25 (Kieselgel; Methylenchlorid/Ethanol = 4:1 + 1 Tropfen Essigsäure)
C₂₅H₂₄N₄O x HCl (396.50/432.96)
Massenspektrum: (M+H)⁺ = 397

### Beispiel 120

### 4-{3-[3-(N-Methyl-N-phenyl-amino)-phenyl]propargylamino}benzamidin

Hergestellt analog Beispiel 4c aus 4-{3-[3-(N-Methyl-N-phenylamino)-phenyl]propargylamino}benzonitril, mit Chlorwasserstoffgas gesättigtem Ethanol und Ammoniumcarbonat.
Ausbeute: 61 % der Theorie,
R_{f}-Wert: 0.37 (Kieselgel; Methylenchlorid/Ethanol = 4:1 + 1 Tropfen Essigsäure)
C₂₃H₂₂N₄ x HCl (354.46/390.92)
Massenspektrum: M⁺ = 354

### Beispiel 121

### 4-[3-(4-Benzyl-phenyl)propargylamino]benzamidin

Hergestellt analog Beispiel 2 aus N-tert.Butoxycarbonyl-4-[3-(4-benzyl-phenyl)propargylamino]benzamidin und Trifluoressigsäure.
Ausbeute: 38 % der Theorie,
R_{f}-Wert: 0.26 (Kieselgel; Methylenchlorid/Ethanol = 4:1 + 1 Tropfen Essigsäure)
C₂₃H₂₁N₃ x CF₃COOH (339.44/453.46)
Massenspektrum: (M+H)⁺ = 340

### Beispiel 122

### 4-[3-(4-Phenylsulfonyl-phenyl)propargylamino]benzamidin

Hergestellt analog Beispiel 4c aus 4-[3-(4-Phenylsulfonylphenyl)propargylamino]benzonitril, mit Chlorwasserstoffgas gesättigtem Ethanol und Ammoniumcarbonat.
Ausbeute: 64 % der Theorie,
R_{f}-Wert: 0.14 (Kieselgel; Methylenchlorid/Ethanol = 4:1) C₂₂H₁₉N₃SO₂ x HCl (389.47/425.93)
Massenspektrum: (M+H)⁺ = 390

### Beispiel 123

### 4-{3-[4-(4-Methylphenylsulfonyl)-phenyl]propargylamino}benzamidin

Hergestellt analog Beispiel 4c aus 4-{3-[4-(4-Methylphenylsulfonyl)phenyl]propargylamino}benzonitril, mit Chlorwasserstoffgas gesättigtem Ethanol und Ammoniumcarbonat.
Ausbeute: 88 % der Theorie,
R_{f}-Wert: 0.13 (Kieselgel; Methylenchlorid/Ethanol = 4:1) C₂₃H₂₁N₃SO₂ x HCl (403.50/439.96)
Massenspektrum: (M+H)⁺ = 404

### Beispiel 124

### 4-[3-(4-Methyl-phenyl)propargylamino]benzamidin und 4-[2-Chlor-3-(4-methyl-phenyl)propenylamino]benzamidin als 4: 6-Gemisch

Hergestellt analog Beispiel 4c aus 4-[3-(4-Methyl-phenyl)propargylamino]benzonitril, mit Chlorwasserstoffgas gesättigtem Ethanol und Ammoniumcarbonat.
Ausbeute: 47 % der Theorie,
R_{f}-Wert: 0.2 (Kieselgel; Methylenchlorid/Ethanol = 4:1) C₁₇H₁₇N₃ x HCl (263.34/299.80)
Massenspektrum: (M+H)⁺ = 264
C₁₇H₁₈ClN₃ x HCl (299.80/336.26)
Massenspektrum: (M+H)⁺ = 300/302 (Chlorisotope)

### Beispiel 125

### 4-[3-(3-Methyl-phenyl)propargylamino]benzamidin

Hergestellt analog Beispiel 4c aus 4-[3-(3-Methyl-phenyl)propargylamino]benzonitril, mit Chlorwasserstoffgas gesättigtem Ethanol und Ammoniumcarbonat.
Ausbeute: 38 % der Theorie,
R_{f}-Wert: 0.28 (Kieselgel; Methylenchlorid/Ethanol = 4:1 und ein Tropfen Essigsäure)
C₁₇H₁₇N₃ x HCl (263.34/299.80)
Massenspektrum: M⁺ = 263

### Beispiel 126

### 4-[3-(3-Biphenyl)-propargylamino]benzamidin

Hergestellt analog Beispiel 4c aus 4-[3-(3-Biphenyl)-propargylamino]benzonitril, mit Chlorwasserstoffgas gesättigtem Ethanol und Ammoniumcarbonat.
Ausbeute: 74 % der Theorie,
R_{f}-Wert: 0.29 (Kieselgel; Methylenchlorid/Ethanol = 4:1 und ein Tropfen Essigsäure)
C₂₂H₁₉N₃ x HCl (325.42/361.88)
Massenspektrum: M⁺ = 326

### Beispiel 127

### 4-[3-(4-Ethoxycarbonyl-3-methyl-phenyl)-propargylaminolbenzamidin

Hergestellt analog Beispiel 4c aus 4-[3-(4-Imidazol-1-yl-carbonyl-3-methyl-phenyl)-propargylamino]benzonitril, Chlorwasserstoffgas gesättigtem Ethanol und Ammoniumcarbonat.
Ausbeute: 46 % der Theorie,
R_{f}-Wert: 0.37 (Kieselgel; Essigester/Ethanol/Ammoniak = 50:45:5)
C₂₀H₂₁N₃O₂ x HCl (335.41/371.67)
Massenspektrum: (M+H)⁺ = 336
(2M+H)⁺ = 671

### Beispiel 128

### d. 4-{3-[4-(3-(2-Hydroxycarbonyl-ethyl)-5-phenyl-pyrazol-1-yl)-3-methyl-phenyl]-propargylamino}benzamidin

Hergestellt analog Beispiel 3 aus 4-{3-[4-(3-(2-Ethoxycarbonyl-ethyl)-5-phenyl-pyrazol-1-yl)-3-methyl-phenyl]-propargylamino}benzamidin, Lithiumhydroxid und anschließender Behandlung mit Ammoniumchlorid.
Ausbeute: 58 % der Theorie,
R_{f}-Wert: 0.24 (Reversed Phase Kieselgel RP-8; Methanol/5%ige NaCl-Lösung = 6:4)
C₂₉H₂₇N₅O₂ x CF₃COOH (477.58/591.60)
Massenspektrum: (M+H)⁻ = 478
(M-H)⁻ = 476

### Beispiel 129

### 4-{3-[4-(3,5-Diethyl-pyrazol-1-yl)-3-methyl-phenyl)-propargylamino}benzamidin

Hergestellt analog Beispiel 2 aus N-tert.Butoxycarbonyl-4-{3-(4-(3,5-diethyl-pyrazol-1-yl)-3-methyl-phenyl)-propargylamino}benzamidin und Trifluoressigsäure.
Ausbeute: 40 % der Theorie,
R_{f}-Wert: 0.3 (Kieselgel; Methylenchlorid/Ethanol = 4:1)
C₂₄H₂₇N₅ x CF₃COOH (385.52/499.54)
Massenspektrum: (M+H)⁺ = 386
M⁺ = 385
(M-H)⁻ = 384

### Beispiel 130

### 4-{3-[2-Methyl-5-(N-methyl-N-pyrid-2-yl-aminocarbonyl)-phenyl]propargylamino}benzamidin

Hergestellt analog Beispiel 2 aus N-tert.Butoxycarbonyl-4-{3-[2-methyl-5-(N-methyl-N-pyrid-2-yl-aminocarbonyl)-phenyl]propargylamino}benzamidin und Trifluoressigsäure.
Ausbeute: 54 % der Theorie,
R_{f}-Wert: 0.17 (Kieselgel; Methylenchlorid/Ethanol = 4:1)
C₂₄H₂₃N₅O x CF₃COOH (397.49/511.51)
Massenspektrum: (M+H)⁺ = 398
(M-H)⁻ = 396
(M+CF₃COOH-H)⁻ = 510

### Beispiel 131

### 4-{3-[4-[N-(2-Hydroxycarbonyl-ethyl)-N-pyridin-2-yl-aminocarbonyl]-2.5-dimethyl-phenyl]propargylamino}benzamidin

Hergestellt analog Beispiel 3 aus 4-{3-[4-[N-(2-Methoxycarbonyl-ethyl)-N-pyridin-2-yl-aminocarbonyl]-2,5-dimethyl-phenyl]propargylamino}benzamidin, Lithiumhydroxid und anschließender Behandlung mit Ammoniumchlorid.
Ausbeute: 13 % der Theorie,
R_{f}-Wert: 0.5 (Reversed Phase Kieselgel RP-8; Methanol/5%ige NaCl-Lösung = 6:4)
C₂₇H₂₇N₅O₃ x 2 HCl (469.55/542.47)
Massenspektrum: (M+H)⁺ = 470
(M-H)⁻ = 468

### Beispiel 132

### 4-{3-[5-Ethexycarbonylmethyl-2-methyl-4-(N-methyl-N-phenylaminocarbonyl)-phenyl]propargylamino}benzamidin

Hergestellt analog Beispiel 2 aus N-tert.Butoxycarbonyl-4-{3-[5-ethoxycarbonylmethyl-2-methyl-4-(N-methyl-N-phenylaminocarbonyl)-phenyl]propargylamino}benzamidin und Trifluoressigsäure.
Ausbeute: 34 % der Theorie,
R_{f}-Wert: 0.25 (Reversed Phase Kieselgel RP-8; Methanol/5%ige NaCl-Lösung = 6:4)
C₂₉H₃₀N₄O₃ x CF₃COOH (482.59/596.61)
Massenspektrum: (M+H)⁺ = 483

### Beispiel 133

### 4-[3-(1,3-Dihydro-isobenzofuran-1-on-5-yl)propargylamino]benzamidin

Hergestellt als Nebenprodukt bei der Trifluoressigsäurebehandlung von rac-N-tert.Butoxycarbonyl-4-{3-[3-hydroxymethyl-4-(2-methyl-pyrrolidinocarbonyl)-phenyl]propargylamino}benzamidin gemäß Beispiel 88.
Ausbeute: 7 % der Theorie,
R_{f}-Wert: 0.59 (Reversed Phase Kieselgel RP-8; Methanol/5%ige NaCl-Lösung = 6:4)
C₁₈H₁₅N₃O₂ x CF₃COOH (305.34/419.36)
Massenspektrum: (M+H)⁺ = 306

### Beispiel 134

### 4-[3-(3-Methyl-4-pyrrolidinocarbonyl-phenyl)-propargylamino]-pyridin

### a. 4-Propargylaminopyridin

Eine Lösung aus 1.9 g (20 mMol) 4-Aminopyridin in 40 ml Tetrahydrofuran wird tropfenweise mit 10 ml (30 mMol) einer 3 molaren etherischen Methylmagnesiumbromid-Lösung versetzt und 2 Stunden gerührt. Dann wird 3.7 g (28 mMol) Propargylmethansulfonat in 40 ml Toluol zugetropft und es wird langsam auf 110°C erhitzt, hierbei werden flüchtige Bestandteile abdestilliert. Nach 48 Stunden bei 110°C wird mit 100 ml Essigester versetzt, mit 100 ml einer 14%igen Kochsalzlösung gewaschen, filtriert und mit Natriumsulfat getrocknet. Nach dem Abdestillieren des Lösungsmittels wird das Rohprodukt chromatographisch gereinigt (Aluminiumoxid; Methylenchlorid/Ethanol 97:3).
Ausbeute: 0.60 g (22 % der Theorie),
R_{f}-Wert: 0.48 (Aluminiumoxid; Methylenchlorid/Ethanol 19:1)

### b. 4-[3-(3-Methyl-4-pyrrolidinocarbonyl-phenyl)-propargylamino]pyridin

Hergestellt analog Beispiel 1 g aus N-(4-Brom-2-methyl-benzoyl)pyrrolidin, 4-Propargylaminopyridin, Tetrakis(triphenylphosphin)palladium(0), Kupferiodid und Triethylamin in Acetonitril.
Ausbeute: 31 % der Theorie,
R_{f}-Wert: 0.35 (Kieselgel; Essigester/Ethanol/Ammoniak 80:40:2)
C₂₀H₂₁N₃O (319.41)
Massenspektrum: M⁺ = 319

### Beispiel 135

### Trans-4-[3-(3-Methyl-4-pyrrolidinocarbonyl-phenyl)-propargylamino]-cyclohexylamin

### a. 3-(3-Methyl-4-pyrrolidinocarbonyl-phenyl)-propargylbromid

Zu einer Lösung aus 3.1 g (13 mMol) 3-(3-Methyl-4-pyrrolidinocarbonyl-phenyl)-propargylalkohol (hergestellt analog Beispiel 9a) und 2.27 g (14 mMol) 1,1'-Carbonyldiimidazol in 90 ml Acetonitril werden 7.71 g (64 mmol) Allylbromid zugetropft und erst 30 Minuten bei Raumtemperatur, anschließend 4 Stunden bei 80°C gerührt. Danach wird mit 350 ml Essigester verdünnt, mit 100 ml Wasser und 100 ml gesättigter NaCl-Lösung gewaschen, mit Natriumsulfat getrocknet, konzentriert und durch Flash-Chromatographie (Kieselgel; Methylenchlorid bis Methylenchlorid/Ethanol 49:1) gereinigt.
Ausbeute: 1.75 g (45 % der Theorie),
R_{f}-Wert: 0.6 (Kieselgel; Essigester)
C₁₅H₁₆BrNO (306)
Massenspektrum: M⁺ = 305/307 (Bromisotope)

### b. Trans-4-[3-(3-Methyl-4-pyrrolidinocarbonyl-phenyl)-propargylamino]-cyclohexylamin

Zu einer Lösung aus 0.50 g (1.63 mMol) 3-(3-Methyl-4-pyrrolidinocarbonyl-phenyl)-propargylbromid und 0.88 g (4.10 mMol) Trans-4-tert.Butoxycarbonylaminocyclohexylamin in 50 ml THF wird bei 0°C 0,53 g (4.09 mmol) N-Ethyl-diisopropylamin zugegeben und dann 2 Stunden bei 0°C, 2 Stunden bei 50°C und 15 Stunden bei Raumtemperatur gerührt. Anschließend wird 2 mal mit je 50 ml gesättigter Natriumhydrogencarbonatlösung und mit 50 ml Natriumchloridlösung gewaschen, die wäßrigen Phasen mit 50 ml Essigester extrahiert und die vereinigten organischen Phasen mit Natriumsulfat getrocknet und im Vakuum konzentriert. Das Rohprodukt wird direkt analog Beispiel 2 mit Trifluoressigsäure in Methylenchlorid zur Titelverbindung umgesetzt.
Ausbeute: 0.38 g (41 % der Theorie),
R_{f}-Wert: 0.3 (Aluminiumoxid; Essigester/Ethanol/Ammoniak = 50:45:5)
C₂₁H₂₉N₃O x 2 CF₃COOH (339.49/567.53)
Massenspektrum: M⁺ = 339

### Beispiel 136

### 4-{3-[5-(2-Phenyl-ethylaminocarbonyl)-2-methyl-phenyl]prop-1-ylamino}benzamidin

Eine Suspension aus 100 mg (0.19 mMol) 4-{3-[5-(2-Phenylethylaminocarbonyl)-2-methyl-phenyl]propargylamino}benzamidin und 40 mg 10% Palladium auf Kohle in 20 ml Ethanol wurde 15 Minuten bei 3 bar Wasserstoffdruck hydriert. Anschließend wurde der Katalysator abfiltriert und das Filtrat eingedampft. Ausbeute: 100 % der Theorie,
R_{f}-Wert: 0.26 (Reversed Phase Kieselgel RP-8; Methanol/5%ige NaCl-Lösung = 6:4)
C₂₆H₃₀N₄O x CF₃COOH (414.56/528.58)
Massenspektrum: (M+H)⁺ = 415

### Beispiel 137

### 4-[3-(2,5-Dimethyl-4-isopropylcarbonyl-phenyl)prop-1-ylamino]-benzamidin

Hergestellt analog Beispiel 136 aus 4-[3-(2,5-Dimethyl-4-isopropylcarbonyl-phenyl)propargylamino)benzamidin, mit 10% Palladium auf Kohle und Wasserstoff in Ethanol.
Ausbeute: 100 % der Theorie,
R_{f}-Wert: 0.13 (Reversed Phase Kieselgel RP-8; Methanol/5%ige NaCl-Lösung = 6:4)
C₂₂H₂₉N₃O x CF₃COOH (351.50/465.52)
Massenspektrum: (M+H)⁺ = 352

### Beispiel 138

### 4-[3-(2,5-Dimethyl-4-pyrrolidinocarbonyl-phenyl)-prop-1-ylamino]benzamidin

Hergestellt analog Beispiel 136 aus 4-[3-(2,5-Dimethyl-4-pyrrolidinocarbonyl-phenyl)-propargylamino]benzamidin, mit 10% Palladium auf Kohle und Wasserstoff in Ethanol.
Ausbeute: 69 % der Theorie,
R_{f}-Wert: 0.21 (Reversed Phase Kieselgel RP-8; Methanol/5%ige NaCl-Lösung = 6:4)
C₂₃H₃₀N₄O x CF₃COOH (378.53/492.55)
Massenspektrum: (M+H)⁺ = 379

### Beispiel 139

### rac-4-{3-[3-Methyl-4-(2-methyl-pyrrolidinocarbonyl)-phenyl]-prop-1-ylamino}benzamidin

Hergestellt analog Beispiel 136 aus rac-4-{3-[3-Methyl-4-(2-methyl-pyrrolidinocarbonyl)-phenyl]-propargylamino}benzamidin, mit 10% Palladium auf Kohle und Wasserstoff in Ethanol.
Ausbeute: 99 % der Theorie,
R_{f}-Wert: 0.2 (Kieselgel; Methylenchlorid/Ethanol = 4:1)
C₂₃H₃₀N₄O x CF₃COOH (378.52/492.55)
Massenspektrum: (M+H)⁺ = 379

### Beispiel 140

### 4-{3-[3-(2-Hydroxycarbonyl-ethyl)-4-pyrrolidinocarbonyl-phenyl]prop-1-ylamino}benzamidin

Hergestellt analog Beispiel 136 aus 4-{3-[3-(2-Hydroxycarbonyl-ethyl)-4-pyrrolidinocarbonyl-phenyl]propargylamino}benzamidin, mit 10% Palladium auf Kohle und Wasserstoff in Ethanol.
Ausbeute: 100 % der Theorie,
R_{f}-Wert: 0.1 (Kieselgel; Methylenchlorid/Ethanol = 4:1) C₂₄H₃₀N₄O₃ x CF₃COOH (422.54/536.56)
Massenspektrum: (M+H)⁺ = 423

### Beispiel 141

### 4-[3-(3-Methyl-4-phenylcarbonyl-phenyl)-prop-1-ylamino]benzamidin

Hergestellt analog Beispiel 136 aus 4-[3-(3-Methyl-4-phenylcarbonyl-phenyl)-propargylamino]benzamidin, mit 10% Palladium auf Kohle und Wasserstoff in Ethanol.
R_{f}-Wert: 0.18 (Kieselgel; Methylenchlorid/Ethanol = 4:1) C₂₄H₂₅N₃O x HCl (371.43/407.95)
Massenspektrum: (M+H)⁺ = 372
(M+HCl-H)⁺ = 406/408 (Chlorisotope)

### Beispiel 142

### 4-{3-[4-(N-(3-Dimethylamino-propyl)-N-methyl-aminocarbonyl)-3-methyl-phenyl]prop-1-ylamino}benzamidin

Hergestellt analog Beispiel 136 aus 4-{3-[4-(N-(3-Dimethylamino-propyl)-N-methyl-aminocarbonyl)-3-methyl-phenyl]propargylamino)benzamidin, mit 10% Palladium auf Kohle und Wasserstoff in Ethanol.
Ausbeute: 100 % der Theorie,
R_{f}-Wert: 0.52 (Reversed Phase Kieselgel RP-8; Methanol/5%ige NaCl-Lösung = 60:40)
C₂₄H₃₅N₅O x 2 CF₃COOH (409.59/637.63)
Massenspektrum: (M+H) ⁺ = 410

### Beispiel 143

### 4-{3-[2,5-Dimethyl-4-(N-methyl-N-phenyl-aminocarbonyl)-phenyl]prop-1-ylamino}benzamidin

Hergestellt analog Beispiel 136 aus 4-(3-[2,5-Dimethyl-4-(N-methyl-N-phenyl-aminocarbonyl)-phenyl]propargylamino}benzamidin, mit 10% Palladium auf Kohle und Wasserstoff in Ethanol.
Ausbeute: 100 % der Theorie,
R_{f}-Wert: 0.34 (Reversed Phase Kieselgel RP-8; Methanol/5%ige NaCl-Lösung = 60:40)
C₂₆H₃₀N₄O x CF₃COOH (414.56/528.58)
Massenspektrum: (M+H)⁺ = 415

### Beispiel 144

### 4-{3-[2,5-Dimethyl-4-(N-methyl-N-phenyl-aminocarbonyl)-phenyl]-N-methyl-prop-1-ylamino}benzamidin

Hergestellt analog Beispiel 136 aus 4-{3-[2,5-Dimethyl-4-(N-methyl-N-phenyl-aminocarbonyl)-phenyl]-N-methyl-propargylamino}benzamidin, mit 10% Palladium auf Kohle und Wasserstoff in Ethanol.
Ausbeute: 59 % der Theorie;
R_{f}-Wert: 0.2 (Kieselgel; Methylenchlorid/Ethanol = 4:1)
C₂₇H₃₂N₄O x HCl (428.53/465.03)
Massenspektrum: (M+H)⁺ = 429

### Beispiel 145

### 4-{3-[5-(N-Methyl-N-(2-phenyl-ethyl)-aminocarbonyl)-2-methylphenyl]prop-1-ylamino}benzamidin

Hergestellt analog Beispiel 136 aus 4-{3-[5-(N-Methyl-N-(2-phenyl-ethyl)-aminocarbonyl)-2-methyl-phenyl]propargylamino}benzamidin, mit 10% Palladium auf Kohle und Wasserstoff in Ethanol.
Ausbeute: 97 % der Theorie,
R_{f}-Wert: 0.20 (Kieselgel; Methylenchlorid/Ethanol = 4:1) C₂₇H₃₂N₄O x CF₃COOH (428.59/542.61)
Massenspektrum: (M+H)⁺ = 429

### Beispiel 146

### 4-{3-[5-(2-Phenyl-ethylaminocarbonyl)-2-methyl-phenyl]prop-1-ylamino}benzamidin

Hergestellt analog Beispiel 136 aus 4-{3-[5-(2-Phenyl-ethylaminocarbonyl)-2-methyl-phenyl]propargylamino}benzamidin, mit 10% Palladium auf Kohle und Wasserstoff in Ethanol.
Ausbeute: 100 % der Theorie,
R_{f}-Wert: 0.26 (Reversed Phase Kieselgel RP-8; Methanol/5%ige NaCl-Lösung = 6:4)
C₂₆H₃₀N₄O x CF₃COOH (414.56/528.58)
Massenspektrum: (M+H)⁺ = 415

### Beispiel 147

### 4-{3-[2-Methyl-5-(N-methyl-N-phenyl-aminocarbonyl)-phenyl]-prop-1-ylamino}benzamidin

Hergestellt analog Beispiel 136 aus 4-{3-[2-Methyl-5-(N-methyl-N-phenyl-aminocarbonyl)-phenyl]propargylamino}benzamidin, mit 10% Palladium auf Kohle und Wasserstoff in Ethanol. Ausbeute: 79 % der Theorie;
Rₜ-Wert: 0.27 (Reversed Phase Kieselgel RP-8; Methanol/5%ige NaCl-Lösung = 6:4)
C₂₅H₂₈N₄O x CF₃COOH (400.52/514.54)
Massenspektrum: (M+H)⁺ = 401

### Beispiel 148

### 4-{3-[2,5-Dimethyl-4-(N-methyl-N-pyridin-2-yl-aminocarbonyl)-phenyl]prop-1-ylamino}benzamidin

Hergestellt analog Beispiel 136 aus 4-{3-[2,5-Dimethyl-4-(N-methyl-N-pyridin-2-yl-aminocarbonyl)-phenyl]propargylamino}benzamidin, mit 10% Palladium auf Kohle und Wasserstoff in Ethanol.
Ausbeute: 60 % der Theorie,
R_{f}-Wert: 0.12 (Reversed Phase Kieselgel RP-8; Methanol/5%ige NaCl-Lösung = 6:4)
C₂₅H₂₉N₅O x 2 CF₃COOH (415.53/643.59)
Massenspektrum: (M+H)⁺ = 416

### Beispiel 149

### 4-[2-Iod-1-(5-phenylcarbonyl-pyrid-2-yl)prop-1-en-3-yl-amino]-benzamidin

Hergestellt aus N-tert.Butoxycarbonyl-4-[3-(5-phenylcarbonylpyrid-2-yl)-propargylamino]benzamidin durch sukzessive Behandlung mit Trimethylsilyliodid analog Beispiel 35 und Trifluoressigsäure analog Beispiel 2.
Ausbeute: 66 % der Theorie,
R_{f}-Wert: 0.28 (Kieselgel; Essigester/Ethanol/Ammoniak = 50:45:5)
C₂₂H₁₉IN₄O x CF₃COOH (482.35/596.37)
Massenspektrum: (M+H)⁺ = 483

### Beispiel 150

### 4-[2-Chlor-1-(5-phenylcarbonyl-pyrid-2-yl)prop-1-en-3-ylamino]benzamidin

Hergestellt analog Beispiel 4c aus 4-[3-(5-Phenylcarbonylpyrid-2-yl)-propargylamino)benzonitril, Chlorwasserstoffgas gesättigtem Ethanol und Ammoniumcarbonat.
Ausbeute: 76 % der Theorie,
R_{f}-Wert: 0.3 (Kieselgel; Essigester/Ethanol/Ammoniak = 50:45:5) C₂₂H₁₉ClN₄O x HCl (390.88/427.34)
Massenspektrum: (M+H)⁺ = 391/393 (Chlorisotope)

### Beispiel 151

### E-4-[1-Chlor-1-(4-phenyloxy-phenyl)prop-1-en-3-yl-amino]-benzamidin und Z-4-[1-Chlor-1-(4-phenyloxy-phenyl)prop-1-en-3-yl-amino]-benzamidin

Hergestellt analog Beispiel 4c aus 4-[3-(4-Phenyloxy-phenyl)-propargylamino]benzonitril, Chlorwasserstoffgas gesättigtem Ethanol und Ammoniumcarbonat.
Ausbeute: 26 % eines E/Z-Isomerengemisches, welches durch präparative HPLC aufgetrennt wird (Lichrospher RP; 250x8mm; Laufmittel: Komponente A: Methanol/Acetonitril = 5:1, Komponente B: 1% Ammoniumformiatpuffer pH 4.6, Komponente A:B 65:35)
1. Isomer (Rₜ = 19.05 Minuten, cis-HCl-Additionsprodukt):
   E-4-[1-Chlor-1-(4-phenyloxy-phenyl)prop-1-en-3-yl-amino]benzamidin)
2. Isomer (Rₜ = 23.53 Minuten, trans-HCl-Additionsprodukt):
   Z-4-[1-Chlor-1-(4-phenyloxy-phenyl)prop-1-en-3-yl-amino]benzamidin)
   C₂₂H₂₀ClN₃O x HCl (377.87/414.33)
   Massenspektrum des Gemischs: (M+H)⁺ = 378/380 (Chlorisotope)

Analog den vorstehenden Beispielen werden folgende Verbindungen erhalten:
(1) 4-{3-[4-(Isoxazolidin-2-ylcarbonyl)-2,5-dimethyl-phenyl]-propargylamino}benzamidin
(2) 4-{3-[4-[N-(2-Ethoxycarbonyl-ethyl)-N-pyrrolidino-aminocarbonyl]-2,5-dimethyl-phenyl]propargylamino}benzamidin
(3) 4-{3-[4-[N-(2-Hydroxycarbonyl-ethyl)-N-pyrrolidino-aminocarbonyl]-2,5-dimethyl-phenyl]propargylamino}benzamidin
(4) 4-{3-[2,5-Dimethyl-4-(N-methyl-N-phenyl-amino-carbonyl)-phenyl]propargyloxy}benzamidin
(5) 4-[3-(3-Methyl-4-pyrrolidinocarbonyl-phenyl)propargylamino]benzamidin
(6) 4-{3-[2,5-Dimethyl-4-(N-benzoyl-N-(2-hydroxycarbonylethyl)-amino)-phenyl]propargyloxy}benzamidin
(7) 4-{3-[2,5-Dimethyl-4-(N-benzoyl-N-(2-ethoxycarbonylethyl)-amino)-phenyl]propargyloxy}benzamidin
(8) 4-{3-[2,5-Dimethyl-4-(N-isopropylcarbonyl-N-(2-hydroxycarbonyl-ethyl)-amino)-phenyl]propargyloxy}benzamidin
(9) 4-{3-[2,5-Dimethyl-4-(N-isopropylcarbonyl-N-(2-ethoxycarbonyl-ethyl)-amino)-phenyl]propargyloxy}benzamidin
(10) 4-{3-[2,5-Dimethyl-4-(N-phenylsulfonyl-N-(2-hydroxycarbonyl-ethyl)-amino)-phenyl]propargyloxy}benzamidin
(11) 4-{3-[2,5-Dimethyl-4-(N-phenylsulfonyl-N-(2-ethoxycarbonyl-ethyl)-amino)-phenyl]propargyloxy}benzamidin

### Beispiel 152

### 4-{3-[5-Hydroxycarbonylmethyl-2-methyl-4-(N-methyl-N-phenylaminocarbonyl)-phenyl]propargylamino}benzamidin

Hergestellt analog Beispiel 3 aus 4-{3-[5-Ethoxycarbonylmethyl-2-methyl-4-(N-methyl-N-phenyl-aminocarbonyl)-phenyl]propargylamino}benzamidin, Lithiumhydroxid und anschließender Behandlung mit Trifluoressigsäure.
Ausbeute: 5 % der Theorie,
R_{f}-Wert: 0.42 (Reversed Phase Kieselgel RP-8; Methanol/5%ige NaCl-Lösung = 6:4)
C₂₇H₂₆N₄O₃ x CF₃COOH (454.53/568.45)
Massenspektrum: (M+H)⁺ = 455
(M-H)⁻ = 453

### Beispiel 153

### 4-{3-[4-[N-(2-Ethoxycarbonyl-ethyl)-N-(1-ethylpyrazol-5-yl)-aminocarbonyl]-2,5-dimethyl-phenyl]propargylamino}benzamidin

Hergestellt analog Beispiel 2 aus N-tert.Butoxycarbonyl-4-{3-[4-[N-(2-ethoxycarbonyl-ethyl)-N-(1-ethylpyrazol-5-yl)-aminocarbonyl]-2,5-dimethyl-phenyl]propargylamino}benzamidin und Trifluoressigsäure.
Ausbeute: 24 % der Theorie,
R_{f}-Wert: 0.22 (Kieselgel; Methylenchlorid/Ethanol = 4:1) C₂₉H₃₄N₆O₃ x CF₃COOH (514.63/628.65)
Massenspektrum: (M+H)⁺ = 515

### Beispiel 154

### 4-{3-[4-[N-(2-Hydroxycarbonyl-ethyl)-N-(1-ethylpyrazol-5-yl)-aminocarbonyl]-2,5-dimethyl-phenyl]propargylamino}benzamidin

Hergestellt analog Beispiel 3 aus 4-{3-[4-[N-(2-Ethoxycarbonyl-ethyl)-N-(1-ethylpyrazol-5-yl)-aminocarbonyl]-2,5-dimethyl-phenyl]propargylamino}benzamidin, Lithiumhydroxid und anschließender Behandlung mit Trifluoressigsäure.
Ausbeute: 6 % der Theorie,
C₂₇H₃₀N₆O₃ x CF₃COOH (486.58/600.60)
Massenspektrum: (M+H)⁺ = 487

### Beispiel 155

### 4-{3-[4-(Isoxazolidin-2-ylcarbonyl)-3-methyl-phenyl]propargylamino}benzamidin

Hergestellt analog Beispiel 2 aus N-tert.Butoxycarbonyl-4-{3-[4-(isoxazolidin-2-ylcarbonyl)-3-methyl-phenyl]-propargylamino}benzamidin und Trifluoressigsäure.
Ausbeute: 42 % der Theorie,
R_{f}-Wert: 0.09 (Kieselgel; Methylenchlorid/Ethanol = 4:1) C₂₁H₂₂N₄O₂ x CF₃COOH (362.44/476.46)
Massenspektrum: (M+H)⁺ = 363

### Beispiel 156

### 4-{3-[4-(Diethylaminocarbonyl)-2,5-dimethyl-phenyl]propargylamino}benzamidin

Hergestellt analog Beispiel 2 aus N-tert.Butoxycarbonyl-4-{3-[4-(diethylaminocarbonyl)-2,5-dimethyl-phenyl]propargylamino}benzamidin und Trifluoressigsäure.
Ausbeute: 36 % der Theorie,
R_{f}-Wert: 0.1 (Kieselgel; Methylenchlorid/Ethanol = 4:1) C₂₃H₂₈N₄O x CF₃COOH (376.51/490.53)
Massenspektrum: (M+H)⁺ = 377

### Beispiel 157

### 4-{3-[2,5-Dimethyl-4-(N-(2-methoxycarbonyl-ethyl)-N-ethylcarbonyl-amino)-phenyl]-propargylamino}benzamidin

### a. 2,5-Dimethyl-4-(2-methoxycarbonyl-ethyl-amino)-1-iod-benzol 15.0 g (0.061 Mol) 2,5-Dimethyl-4-iod-anilin, 55 ml

(0.611 Mol) Acrylsäuremethylester, 6 ml Benzyltrimethylammoniumhydroxid und 0.3 g (3 mMol) Hydrochinon werden 11 Tage zum Rückfluß erhitzt. Anschließend wird der überschüssige Acrylester abdestilliert und der Rückstand an Kieselgel chromatographiert, wobei mit Methylenchlorid eluiert wird.
Ausbeute: 11.7 g (58 % der Theorie),
R_{f}-Wert: 0.65 (Kieselgel; Essigester/Petrolether = 4:6)

### b. 2,5-Dimethyl-4-(N-(2-methoxycarbonyl-ethyl)-N-ethylcarbonyl-amino)-1-iod-benzol

0.5 ml (6 mMol) Propionsäurechlorid wird in 30 ml Tetrahydrofuran vorgelegt, unter Eiskühlung 2.0 g (6 mMol) 2,5-Dimethyl-4-(2-methoxycarbonyl-ethyl-amino)-1-iod-benzol in 30 ml Tetrahydrofuran zugetropft und 30 Minuten unter Eiskühlung nachgerührt. Über Nacht wird bei Raumtemperatur gerührt, anschließend mit 14%iger NaCl-Lösung verdünnt und mit Essigester extrahiert. Die vereinigten organischen Extrakte werden über Natriumsulfat getrocknet und eingedampft. Der Rückstand wird an Kieselgel chromatographiert, wobei mit Petrolether/Essigester (3:1) eluiert wird.
Ausbeute: 2.08 g (89 % der Theorie),
R_{f}-Wert: 0.38 (Kieselgel; Dichlormethan/Ethanol = 98:2)

### c. N-tert.Butoxycarbonyl-4-{3-[2,5-dimethyl-4-(N-(2-methoxycarbonyl-ethyl)-N-ethylcarbonyl-amino)-phenyl]-propargylamino}benzamidin

Hergestellt analog Beispiel 1g aus 2,5-Dimethyl-4-(N-(2-methoxycarbonyl-ethyl)-N-ethylcarbonyl-amino)-iod-benzol, N-tert.Butoxycarbonyl-4-propargylamino-benzamidin, Tetrakis(triphenyl-phosphin)palladium(0), Kupfer(I)iodid und Triethylamin in Acetonitril.
Ausbeute: 0.4 g (20 % der Theorie),
C₃₀H₃₈N₄O₅ (534.66)
Massenspektrum: (M+H)⁺ = 535
(M+Na)⁺ = 557

### d. 4-{3-[2,5-Dimethyl-4-(N-(2-methoxycarbonyl-ethyl)-N-ethylcarbonyl-amino)-phenyl]-propargylamino}benzamidin

Hergestellt analog Beispiel 2 aus N-tert.Butoxycarbonyl-4-{3-[2,5-dimethyl-4-(N-(2-methoxycarbonyl-ethyl)-N-ethylcarbonyl-amino)-phenyl]-propargylamino}benzamidin und Trifluoressigsäure.
Ausbeute: 77 % der Theorie,
C₂₅H₃₀N₄O₃ x CF₃COOH (434.57/548.57)
Massenspektrum: (M+H)⁺ = 435
(M-H)⁻ = 433

### Beispiel 158

### 4-{3-[2,5-Dimethyl-4-(N-(3-ethoxycarbonyl-propionyl)-N-isopropyl-amino)-phenyl]-propargylamino}benzamidin

### a. 2,5-Dimethyl-4-iod-anilin

Zu einer Lösung aus 8.8 ml (70.8 mMol) 2,5-Dimethylanilin in 250 ml Methanol und 600 ml Dichlormethan werden 25.0 g (71.8 mMol) Benzyltrimethylammoniumdichloriodat und 12.8 g (92.5 mMol) Kaliumcarbonat gegeben und 1 Stunde bei Raumtemperatur gerührt. Anschließend werden die anorganischen Salze abgesaugt und das Solvens abdestilliert. Der Rückstand wird mit einer Lösung aus 13.5 g (70.8 mMol) Natriumpyrosulfit in 640 ml Wasser versetzt und mit Ether extrahiert. Die vereinigten organischen Extrakte werden über Natriumsulfat getrocknet und eingedampft. Das Rohprodukt wird mit Petrolether verrieben, abgesaugt und getrocknet.
Ausbeute: 13.3 g (76 % der Theorie),
R_{f}-Wert: 0.65 (Kieselgel; Essigester/Petrolether = 3:7)

### b. 2,5-Dimethyl-4-iod-N-isopropyl-anilin 4.1 g (0.017 Mol) 2,5-Dimethyl-4-iod-anilin, 1.4 ml

(0.019 Mol) Aceton, 1.4 ml (0,024 Mol) Eisessig und 0.1 g (0.001 Mol) p-Toluolsulfonsäure werden in 30 ml Tetrahydrofuran gelöst und 30 Minuten bei Raumtemperatur gerührt. Anschließend werden 4.7 g (0.022 Mol) Natriumtriacetoxyborhydrid zugesetzt und 20 Stunden bei Raumtemperatur nachgerührt. Nach Zugabe von 150 ml Wasser wird soviel Natriumcarbonat zugesetzt, bis keine CO₂-Entwicklung mehr feststellbar ist. Danach wird mit Essigester extrahiert, die vereinigten organischen Extrakte über Natriumsulfat getrocknet und eingedampft. Der Rückstand wird an Kieselgel chromatographiert, wobei mit Essigester/Petrolether (2:98) eluiert wird.
Ausbeute: 4.4 g (91.3 % der Theorie),
R_{f}-Wert: 0.50 (Kieselgel; Essigester/Petrolether = 3:7)

### c. 2,5-Dimethyl-N-(3-ethoxycarbonyl-propionyl)-N-isopropyl-4-iod-anilin

2.0 g (6.9 mMol) 2,5-Dimethyl-4-iod-N-isopropyl-anilin und 2.4 ml (13.8 mMol) N-Ethyl-diisopropylamin werden in 30 ml Tetrahydrofuran gelöst und nach Zugabe von 1.5 ml (10.5 mMol) Bernsteinsäureethylesterchlorid 2 Stunden zum Rückfluß erhitzt. Nach dem Abkühlen auf Raumtemperatur wird mit Essigester verdünnt und sukzessiv mit 1 molarer Salzsäure und 1-molarer Natronlauge gewaschen. Die organische Phase wird über Natriumsulfat getrocknet und eingedampft.
Ausbeute: 2.9 g (100 % der Theorie),
R_{f}-Wert: 0.85 (Kieselgel; Essigester/Petrolether = 3:7)

### d. N-tert.Butoxycarbonyl-4-{3-[2,5-dimethyl-4-(N-(3-ethoxycarbonyl-propionyl)-N-isopropyl-amino)-phenyl]-propargylamino}benzamidin

Hergestellt analog Beispiel 1g aus 2,5-Dimethyl-N-(3-ethoxycarbonyl-propionyl)-N-isopropyl-4-iod-anilin, N-tert.Butoxycarbonyl-4-propargylamino-benzamidin, Tetrakis(triphenylphosphin)palladium(0), Kupfer(I)iodid und Triethylamin in Acetonitril.
Ausbeute: 39 % der Theorie,
R_{f}-Wert: 0.3 (Kieselgel; Dichlormethan/Ethanol = 19:1)
C₃₂H₄₂N₄O₅ (562.72)
Massenspektrum: (M+H)⁺ = 563
(M+Na)⁺ = 585

### e. 4-{3-[2,5-Dimethyl-4-(N-(3-ethoxycarbonyl-propionyl)-N-isopropyl-amino)-phenyl]-propargylamino}benzamidin

Hergestellt analog Beispiel 2 aus N-tert.Butoxycarbonyl-4-{3-[2,5-dimethyl-4-(N-(3-ethoxycarbonyl-propionyl)-N-isopropyl-amino)-phenyl]-propargylamino}benzamidin und Trifluoressigsäure.
Ausbeute: 47 % der Theorie,
C₂₇H₃₄N₄O₃ x CF₃COOH (462.62/576.62)
Massenspektrum: (M+H)⁺ = 463
(M-H)⁻ = 461

### Beispiel 159

### 4-{3-[5-(N-(2-Ethoxycarbonylethyl)-N-(2-pyridyl)-aminocarbonyl)-2-ethyl-phenyl]propargylamino}benzamidin

Hergestellt analog Beispiel 2 aus N-tert.Butoxycarbonyl-4-{3-[5-(N-(2-ethoxycarbonylethyl)-N-(2-pyridyl)-aminocarbonyl)-2-ethyl-phenyl]propargylamino}benzamidin und Trifluoressigsäure.
Ausbeute: 47 % der Theorie,
R_{f}-Wert: 0.19 (Kieselgel; Dichlormethan/Ethanol = 4:1) C₂₉H₃₁N₅O₃ x CF₃COOH (497.60/611.62)
Massenspektrum: (M+H)⁺ = 498

### Beispiel 160

### 4-{3-[5-(N-(2-Hydroxycarbonylethyl)-N-(2-pyridyl)-aminocarbonyl)-2-ethyl-phenyl]propargylamino}benzamidin

Hergestellt analog Beispiel 3 aus 4-{3-[5-(N-(2-Ethoxycarbonylethyl)-N-(2-pyridyl)-aminocarbonyl]-2-ethyl-phenyl]propargylamino}benzamidin, Natriumhydroxid und anschließender Behandlung mit Trifluoressigsäure.
Ausbeute: 55 % der Theorie,
R_{f}-Wert: 0.31 (Kieselgel; Dichlormethan/Ethanol = 4:1) C₂₇H₂₇N₅O₃ x CF₃COOH (469.54/583.57)
Massenspektrum: (M+H)⁺ = 470
(M-H)⁻ = 468

### Beispiel 161

### 4-{3-[2,5-Dimethyl-4-(N-(2-methoxycarbonyl-ethyl)-N-benzoylamino)-phenyl]-propargylamino}benzamidin

Hergestellt analog Beispiel 2 aus N-tert.Butoxycarbonyl-4-{3-[2,5-dimethyl-4-(N-(2-methoxycarbonyl-ethyl)-N-benzoylamino)-phenyl]-propargylamino}benzamidin und Trifluoressigsäure.
Ausbeute: 29 % der Theorie,
C₂₉H₃₀N₄O₃ x CF₃COOH (482.59/596.61) _{.}
Massenspektrum: (M+H)⁺ = 483

### Beispiel 162

### 4-{3-[2,5-Dimethyl-4-(N-(2-pyridyl)-N-methyl-aminocarbonyl)-phenyl]-N-methyl-propargylamino}benzamidin

Hergestellt analog Beispiel le aus 4-{3-[2,5-Dimethyl-4-(N-(2-pyridyl)-N-methyl-aminocarbonyl)-phenyl]-N-methylpropargylamino}benzonitril und Salzsäure/Ammoniumcarbonat in Ethanol.
Ausbeute: 6 % der Theorie,
C₂₆H₂₇N₅O x HCl (425.54/462.00)
Massenspektrum: (M+H)⁺ = 426

### Beispiel 163

### 4-{3-[4-(3,5-Diethyl-pyrazol-1-yl)-2,5-dimethyl-phenyl]-propargylamino}benzamidin

Hergestellt analog Beispiel 2 aus N-tert.Butoxycarbonyl-4-{3-[4-(3,5-Diethyl-pyrazol-1-yl)-2,5-dimethyl-phenyl]-propargylamino}benzamidin und Trifluoressigsäure.
Ausbeute: 96 % der Theorie,
C₂₅H₂₉N₅ x CF₃COOH (399.55/513.57)
Massenspektrum: (M+H)⁺ = 400

### Beispiel 164

### 4-{3-[2,5-Dimethyl-4-(N-ethylcarbonyl-N-(2-hydroxycarbonylethyl)-amino)-phenyl]propargylamino}benzamidin

Hergestellt analog Beispiel 3 aus 4-{3-[2,5-Dimethyl-4-(N-ethylcarbonyl-N-(2-methoxycarbonyl-ethyl)-amino)-phenyl]-propargylamino}benzamidin und Natriumhydroxid.
Ausbeute: 59 % der Theorie,
C₂₄H₂₈N₄O₃ (420.52)
Massenspektrum: (M+H)⁺ = 421
(M+Na)⁺ = 443

### Beispiel 165

### 4-{3-[2,5-Dimethyl-4-(N-phenylsulfonyl-N-(2-methoxycarbonylethyl)-amino)-phenyl]-propargylamino}-benzamidin

Hergestellt analog Beispiel 2 aus N-tert.Butoxycarbonyl-4-{3-[2,5-dimethyl-4-(N-phenylsulfonyl-N-(2-methoxycarbonylethyl)-amino)-phenyl]-propargylamino}-benzamidin und Trifluoressigsäure.
Ausbeute: 63 % der Theorie,
C₂₈H₃₀N₄O₄S x CF₃COOH (518.64/632.66)
Massenspektrum: (M+H)⁺ = 519

### Beispiel 166

### 4-{3-[2,5-Dimethyl-4-(N-phenylsulfonyl-N-(2-hydroxycarbonylethyl)-amino)-phenyl]-propargylamino}-benzamidin

Hergestellt analog Beispiel 3 aus 4-{3-[2,5-Dimethyl-4-(N-phenylsulfonyl-N-(2-methoxycarbonyl-ethyl)-amino)-phenyl]-propargylamino}-benzamidin, Lithiumhydroxid und anschließender Behandlung mit Eisessig.
Ausbeute: 37 % der Theorie,
C₂₇H₂₈N₄O₄S (504.61)
Massenspektrum: (M+H)⁺ = 505
(M+Na)⁺ = 527

### Beispiel 167

### 4-{3-[2,5-Dimethyl-4-(N-phenylsulfonyl-N-(2-methoxycarbonylethyl)-amino)-phenyl]-prop-1-ylamino}-benzamidin

Hergestellt analog Beispiel 136 aus 4-{3-[2,5-Dimethyl-4-(N-phenylsulfonyl-N-(2-methoxycarbonyl-ethyl)-amino)-phenyl]-propargylamino}-benzamidin, 10 % Palladium auf Aktivkohle und Wasserstoff in Ethanol.
Ausbeute: 65 % der Theorie,
C₂₈H₃₄N₄O₄S x CF₃COOH (522.68/636.70)
Massenspektrum: (M+H)⁺ = 523

### Beispiel 168

### 4-{3-[2,5-Dimethyl-4-(N-(2-methoxycarbonyl-ethyl)-N-propylcarbonyl-amino)-phenyl]-propargylamino}-benzamidin

Hergestellt analog Beispiel 2 aus N-tert.Butoxycarbonyl-4-{3-[2,5-dimethyl-4-(N-(2-methoxycarbonyl-ethyl)-N-propylcarbonyl-amino)-phenyl]-propargylamino}-benzamidin und Trifluoressigsäure.
Ausbeute: 82 % der Theorie,
C₂₆H₃₂N₄O₃ x CF₃COOH (448.57/562.59)
Massenspektrum: (M+H)⁺ = 449

### Beispiel 169

### 4-{3-[2,5-Dimethyl-4-(N-(2-methoxycarbonyl-ethyl)-N-cyclopropylcarbonyl-amino)-phenyl]-propargylamino}-benzamidin

Hergestellt analog Beispiel 2 aus N-tert.Butoxycarbonyl-4-{3-[2,5-dimethyl-4-(N-(2-methoxycarbonyl-ethyl)-N-cyclopropylcarbonyl-amino)-phenyl]-propargylamino}-benzamidin und Trifluoressigsäure.
Ausbeute: 37 % der Theorie,
C₂₆H₃₀N₄O₃ x CF₃COOH (446.56/560.58)
Massenspektrum: (M+H)⁺ = 447
(M-H)⁻ = 445

### Beispiel 170

### 4-{3-[2,5-Dimethyl-4-(N-(2-methoxycarbonyl-ethyl)-N-methylcarbonyl-amino)-phenyl]-propargylamino}-benzamidin

Hergestellt analog Beispiel 2 aus N-tert.Butoxycarbonyl-4-{3-[2,5-dimethyl-4-(N-(2-methoxycarbonyl-ethyl)-N-methylcarbonyl-amino)-phenyl]-propargylamino}-benzamidin und Trifluoressigsäure.
Ausbeute: 90 % der Theorie,
C₂₄H₂₈N₄O₃ x CF₃COOH (420.52/534.54)
Massenspektrum: (M+H)⁺ = 421

### Beispiel 171

### 4-{3-[2,5-Dimethyl-4-(N-(2-hydroxycarbonyl-ethyl)-N-methylcarbonyl-amino)-phenyl]-propargylamino}-benzamidin

Hergestellt analog Beispiel 3 aus 4-{3-[2,5-Dimethyl-4-(N-(2-methoxycarbonyl-ethyl)-N-methylcarbonyl-amino)-phenyl]-propargylamino}-benzamidin, Lithiumhydroxid und anschließender Behandlung mit Eisessig.
Ausbeute: 57 % der Theorie;
C₂₃H₂₆N₄O₃ (406.49)
Massenspektrum: (M-H)⁻ = 405

### Beispiel 172

### 4-{3-[2,5-Dimethyl-4-(N-(2-hydroxycarbonyl-ethyl)-N-propylcarbonyl-amino)-phenyl]-propargy]amino}-benzamidin

Hergestellt analog Beispiel 3 aus 4-{3-[2,5-Dimethyl-4-(N-(2-methoxycarbonyl-ethyl)-N-propylcarbonyl-amino)-phenyl]-propargylamino}-benzamidin, Lithiumhydroxid und anschließender Behandlung mit Eisessig.
Ausbeute: 65 % der Theorie,
C₂₅H₃₀N₄O₃ (434.54)
Massenspektrum: (M-H)⁻ = 433
(M+H)⁺ = 435

### Beispiel 173

### 4-{3-[2,5-Dimethyl-4-(N-2-hydroxycarbonyl-ethyl-N-cyclopropylcarbonyl-amino)-phenyl]-propargylamino}-benzamidin

Hergestellt analog Beispiel 3 aus 4-{3-[2,5-Dimethyl-4-(N-(2-methoxycarbonyl-ethyl)-N-cyclopropylcarbonyl-amino)-phenyl]-propargylamino}-benzamidin, Lithiumhydroxid und anschließender Behandlung mit Eisessig.
Ausbeute: 75 % der Theorie,
C₂₅H₂₈N₄O₃ (432.53)
Massenspektrum: (M+Na)⁺ = 455
(M+H)⁺ = 433

### Beispiel 174

### 4-{3-[2,5-Dimethyl-4-(N-(3-hydroxycarbonyl-propionyl)-N-isopropyl-amino)-phenyl]-propargylamino}-benzamidin

Hergestellt analog Beispiel 3 aus 4-{3-[2,5-Dimethyl-4-(N-(3-ethoxycarbonyl-propionyl)-N-isopropyl-amino)-phenyl]-propargylamino}-benzamidin, Lithiumhydroxid und anschließender Behandlung mit Eisessig.
Ausbeute: 73 % der Theorie,
C₂₅H₃₀N₄O₃ (434.54)
Massenspektrum: (M+H)⁺ = 435
(M-H)⁻ = 433

### Beispiel 175

### 4-{3-[2,5-Dimethyl-4-(N-ethylcarbonyl-N-(2-methoxycarbonylethyl)-amino)-phenyl]-prop-1-ylamino}-benzamidin

Hergestellt analog Beispiel 136 aus 4-{3-[2,5-Dimethyl-4-(N-ethylcarbonyl-N-(2-methoxycarbonyl-ethyl)-amino)-phenyl]-propargylamino}benzamidin, 10 % Palladium auf Aktivkohle und Wasserstoff in Ethanol.
Ausbeute: 99 % der Theorie,
C₂₅H₃₄N₄O₃ x CF₃COOH (438.58/552.60)
Massenspektrum: (M+H)⁺ = 439

### Beispiel 176

### 4-{3-[2,5-Dimethyl-4-(N-(3-hydroxycarbonyl-propionyl)-N-isopropyl-amino)-phenyl]-prop-1-ylamino}-benzamidin

Hergestellt analog Beispiel 136 aus 4-{3-(2,5-Dimethyl-4-(N-(3-hydroxycarbonyl-propionyl)-N-isopropyl-amino)-phenyl]-propargylamino}-benzamidin, 10 % Palladium auf Aktivkohle und Wasserstoff in Ethanol.
Ausbeute: 99 % der Theorie,
C₂₅H₃₄N₄O₃ (438.58/552.60)
Massenspektrum: (M+H)⁺ = 439
(M-H)⁻ = 437

### Beispiel 177

### 4-{3-[2,5-Dimethyl-4-(N-(3-ethoxycarbonyl-propionyl)-N-benzylamino)-phenyl]-propargylamino}-benzamidin

Hergestellt analog Beispiel 2 aus N-tert.Butoxycarbonyl-4-{3-[2,5-dimethyl-4-(N-(3-ethoxycarbonyl-propionyl)-N-benzylamino)-phenyl]-propargylamino}-benzamidin und Trifluoressigsäure.
Ausbeute: 64 % der Theorie,
C₃₁H₃₄N₄O₃ x CF₃COOH (510.65/624.67)
Massenspektrum: (M+H)⁺ = 511

### Beispiel 178

### 4-{3-[2,5-Dimethyl-4-(N-(3-hydroxycarbonyl-propionyl)-N-benzyl-amino)-phenyl]-propargylamino}-benzamidin

Hergestellt analog Beispiel 3 aus 4-{3-[2,5-Dimethyl-4-(N-(3-ethoxycarbonyl-propionyl)-N-benzyl-amino)-phenyl]-propargylamino}-benzamidin, Lithiumhydroxid und anschließender Behandlung mit Trifluoressigsäure.
Ausbeute: 73 % der Theorie,
C₂₉H₃₀N₄O₃ x CF₃COOH (482.59/596.61)
Massenspektrum: (M+H)⁺ = 483
(M-H)⁻ = 481

### Beispiel 179

### 4-{3-[2,5-Dimethyl-4-(N-ethylcarbonyl-N-methoxycarbonylmethylamino)-phenyl]-propargylamino}-benzamidin

Hergestellt analog Beispiel 2 aus N-tert.Butoxycarbonyl-4-{3-[2,5-dimethyl-4-(N-ethylcarbonyl-N-methoxycarbonylmethylamino)-phenyl]-propargylamino}-benzamidin und Trifluoressigsäure.
Ausbeute: 67 % der Theorie,
C₂₄H₂₈N₄O₃ x CF₃COOH (420.52/534.54)
Massenspektrum: (M+CF₃COOH-H)⁻ = 533

### Beispiel 180

### 4-{3-[4-(4,4-Dimethyl-5-oxo-4,5-dihydro-1H-pyrazol-3-yl)-2,5-dimethyl-phenyl]-propargylamino}-benzamidin

### a. 4-Brom-2,5-dimethylbenzoesäurechlorid

10.3 g (45 mMol) 4-Brom-2,5-dimethylbenzoesäure werden in 250 ml Dichlormethan gelöst und nach Zugabe von 9.9 ml (135 mMol) Thionylchlorid zwei Stunden zum Rückfluß erhitzt. Anschließend wird zur Trockene eingedampft.
Ausbeute: 3.2 g (100 % der Theorie).

### b. 3-(4-Brom-2,5-dimethyl-phenyl)-2,2-dimethyl-3-oxo-propionsäuremethylester

5.4 g (0.022 Mol) 4-Brom-2,5-dimethylbenzoesäurechlorid, 4.5 ml (0.022 Mol) 1-Methoxy-2-methyl-1-(trimethylsilyloxy)-1-propen und 8.2 ml (0.066 Mol) Bortrifluoridetherat werden in 50 ml Diethylether unter Stickstoffatmosphäre 20 Stunden zum Rückfluß erhitzt. Anschließend wird 2x mit je 50 ml 1N Natronlauge und 1x mit 50 ml Wasser gewaschen. Die organische Phase wird mit Natriumsulfat getrocknet und das Lösungsmittel wird abdestilliert.
Ausbeute: 3.6 g (53 % der Theorie),
R_{f}-Wert: 0.6 (Kieselgel; Petrolether/Essigester = 4:1)

### c. 3-(4-Brom-2,5-dimethyl-phenyl)-4,4-dimethyl-4,5-dihydro-1Hpyrazol-5-on

3.5 g (11.7 mMol) 3-(4-Brom-2,5-dimethyl-phenyl)-2,2-dimethyl-3-oxo-propionsäure-methyl ester und 28 ml (28 mMol) 1-molare Hydrazinlösung in Tetrahydrofuran werden in 50 ml Ethanol 24 Stunden zum Rückfluß erhitzt. Das Lösemittel wird abdestilliert und der Rückstand aus Ethanol umkristallisiert. Ausbeute: 2.1 g (64 % der Theorie),
R_{f}-Wert: 0.9 (Kieselgel; Petrolether/Essigester = 7:3)

### d. N-tert.Butoxycarbonyl-4-{3-[4-(4,4-dimethyl-5-oxo-4,5-dihydro-1H-pyrazol-3-yl)-2,5-dimethyl-phenyl]-propargylamino}benzamidin

Hergestellt analog Beispiel 1g aus 3-(4-Brom-2,5-dimethylphenyl)-4,4-dimethyl-4,5-dihydro-1H-pyrazol-5-on, N-tert.Butoxycarbonyl-4-propargylamino-benzamidin, Tetrakis-(triphenylphosphin)-palladium(0), Kupfer-I-iodid und Triethylamin in Acetonitril.
Ausbeute: 19 % der Theorie,
R_{f}-Wert: 0.2 (Kieselgel; Dichlormethan/Ethanol = 19:1)

### e. 4-{3-[4-(4,4-Dimethyl-5-oxo-4,5-dihydro-1H-pyrazol-3-yl)-2,5-dimethyl-phenyl]-propargylamino}-benzamidin

Hergestellt analog Beispiel 2 aus N-tert.Butoxy-4-{3-[4-(4,4-dimethyl-5-oxo-4,5-dihydro-1H-pyrazol-3-yl)-2,5-dimethylphenyl]-propargylamino}-benzamidin und Trifluoressigsäure.
Ausbeute: 93 % der Theorie,
C₂₃H₂₅N₅O x CF₃COOH (387.49/501.51)
Massenspektrum: (M+H)⁺ = 388

### Beispiel 181

### 4-{3-[2,5-Dimethyl-4-(N-ethylcarbonyl-N-hydroxycarbonylmethylamino)-phenyl]-propargylamino}-benzamidin

Hergestellt analog Beispiel 3 aus 4-{3-[2,5-Dimethyl-4-(N-ethylcarbonyl-N-methoxycarbonylmethyl-amino)-phenyl]-propargylamino}-benzamidin, Lithiumhydroxid und anschließender Behandlung mit Eisessig.
Ausbeute: 74 % der Theorie,
C₂₃H₂₆N₄O₃ (406.49)
Massenspektrum: (M+H)⁺ = 407
(M+Na)⁺ = 429
(M-H)⁻ = 405

### Beispiel 182

### 4-{3-[2,5-Dimethyl-4-(N-ethylcarbonyl-N-ethoxycarbonylmethylaminocarbonylmethyl-amino)-phenyl]-propargylamino}-benzamidin

Hergestellt analog Beispiel 2 aus N-tert.Butoxycarbonyl-4-{3-[2,5-dimethyl-4-(N-ethylcarbonyl-N-ethoxycarbonylmethylaminocarbonylmethyl-amino)-phenyl]-propargylamino}-benzamidin und Trifluoressigsäure.
Ausbeute: 61 % der Theorie,
C₂₇H₃₃N₅O₄ x CF₃COOH (491.60/605.62)
Massenspektrum: (M+H)⁺ = 492

### Beispiel 183

### 4-{3-[2,5-Dimethyl-4-(N-ethylcarbonyl-N-hydroxycarbonylmethylaminocarbonylmethyl-amino)-phenyl]-propargylamino}-benzamidin

Hergestellt analog Beispiel 3 aus 4-{3-[2,5-Dimethyl-4-(N-ethylcarbonyl-N-ethoxycarbonylmethylaminocarbonylmethylamino)-phenyl]-propargylamino}-benzamidin, Lithiumhydroxid und anschließender Behandlung mit Eisessig.
Ausbeute: 48 % der Theorie,
C₂₅H₂₉N₅O₄ (463.54)
Massenspektrum: (M+H)⁺ = 464
(M-H)⁻ = 462

### Beispiel 184

### 4-{3-[2,5-Dimethyl-4-(N-isopropyl-N-ethoxycarbonylmethylaminocarbonyl-amino)-phenyl]-propargylamino}-benzamidin

Hergestellt analog Beispiel 2 aus N-tert.Butoxycarbonyl-4-{3-[2,5-dimethyl-4-(N-isopropyl-N-ethoxycarbonylmethylaminocarbonyl-amino)-phenyl]-propargylamino}-benzamidin und Trifluoressigsäure.
Ausbeute: 67 % der Theorie,
C₂₆H₃₃N₅O₃ x CF₃COOH (463.59/577.61)
Massenspektrum: (M+H)⁺ = 464

### Beispiel 185

### 4-{3-[2,5-Dimethyl-4-(N-isopropyl-N-hydroxycarbonylmethylaminocarbonyl-amino)-phenyl]-propargylamino}-benzamidin

Hergestellt analog Beispiel 3 aus 4-{3-[2,5-Dimethyl-4-(N-isopropyl-N-methoxycarbonylmethylaminocarbonyl-amino)-phenyl]-propargylamino}-benzamidin, Lithiumhydroxid und anschließender Behandlung mit Eisessig.
Ausbeute: 68 % der Theorie,
C₂₄H₂₉N₅O₃ (435.53)
Massenspektrum: (M+H)⁺ = 436
(M-H)⁻ = 434

### Beispiel 186

### 4-{3-[2,5-Dimethyl-4-(N-isopropyl-N-(3-trifluoracetylamino-3-methoxycarbonyl-propionyl)-amino)-phenyl]-propargylamino}benzamidin

Hergestellt analog Beispiel 2 aus N-tert.Butoxycarbonyl-4-{3-[2,5-dimethyl-4-(N-isopropyl-N-(3-trifluoracetylamino-3-methoxycarbonyl-propionyl)-amino)-phenyl]-propargylamino}benzamidin und Trifluoressigsäure.
Ausbeute: 65 % der Theorie,
C₂₈H₃₂F₃N₅O₄ x CF₃COOH (559.59/673.61)
Massenspektrum: (M+H)⁺ = 560
(M-H)⁻ = 558

### Beispiel 187

### 4-{3-[2,5-Dimethyl-4-(N-isopropyl-N-methoxycarbonylmethoxy methylcarbonyl-amino)-phenyl]-propargylamino}-benzamidin

Hergestellt analog Beispiel 2 aus N-tert.Butoxycarbonyl-4-{3-[2,5-dimethyl-4-(N-isopropyl-N-methoxycarbonylmethoxymethylcarbonyl-amino)-phenyl]-propargylamino}-benzamidin und Trifluoressigsäure.
Ausbeute: 65 % der Theorie,
C₂₆H₃₂N₄O₄ x CF₃COOH (464.47/578.59)
Massenspektrum: M⁺ = 464

### Beispiel 188

### 4-{3-[2,5-Dimethyl-4-(N-isopropyl-N-hydroxycarbonylmethoxymethylcarbonyl-amino)-phenyl]-propargylamino}-benzamidin

Hergestellt analog Beispiel 3 aus 4-{3-[2,5-Dimethyl-4-(N-isopropyl-N-methoxycarbonylmethoxymethylcarbonyl-amino)-phenyl]-propargylamino}-benzamidin, Lithiumhydroxid und anschließender Behandlung mit Eisessig.
Ausbeute: 12 % der Theorie,
C₂₅H₃₀N₄O₄ (450.54)
Massenspektrum: (M+H)⁺ = 451
(M-H)⁻ = 449

### Beispiel 189

### 4-{3-[2,5-Dimethyl-4-(N-isopropyl-N-methoxycarbonylmethylcarbonyl-amino)-phenyl]-propargylamino}-benzamidin

Hergestellt analog Beispiel 2 aus N-tert.Butoxycarbonyl-4-{3-[2,5-dimethyl-4-(N-isopropyl-N-methoxycarbonylmethylcarbonyl-amino)-phenyl]-propargylamino}-benzamidin und Trifluoressigsäure.
Ausbeute: 74 % der Theorie,
C₂₅H₃₀N₄O₃ x CF₃COOH (434.54/548.56)
Massenspektrum: (M+H)⁺ = 435
(M-H)⁻ = 433

### Beispiel 190

### 4-{3-[2,5-Dimethyl-4-(N-isopropyl-N-hydroxycarbonylmethylcarbonyl-amino)-phenyl]-propargylamino}-benzamidin

Hergestellt analog Beispiel 3 aus 4-(3-[2,5-Dimethyl-4-(N-isopropyl-N-methoxycarbonylmethylcarbonyl-amino)-phenyl]-propargylamino)-benzamidin, Lithiumhydroxid und anschließender Behandlung mit Eisessig.
Ausbeute: 80 % der Theorie,
C₂₄H₂₈N₄O₃ (420.51)
Massenspektrum: (M+H)⁺ = 421
(M-H)⁻ = 419
(M+Na)⁺ = 443

### Beispiel 191

### 4-{3-[2,5-Dimethyl-4-(N-isopropyl-N-methoxycarbonylmethylaminocarbonylmethylcarbonyl-amino)-phenyl]-propargylamino}benzamidin

0.3 g (0.71 Mol) 4-{3-[2,5-Dimethyl-4-(N-isopropyl-N-hydroxycarbonylmethylcarbonyl-amino)-phenyl]-propargylamino}-benzamidin und 0.1 g (0.71 Mol) Glycinmethylester werden in 10 ml Dimethylformamid gelöst und nach Zugabe von 0.2 g (0.78 Mol) N,N'-Dicyclohexylcarbodiimid 20 Stunden bei Raumtemperatur gerührt. Anschließend wird der ausgefallene Niederschlag abgesaugt und die Mutterlauge zur Trockene eingedampft. Der Rückstand wird an Kieselgel chromatographiert, wobei mit Dichlormethan/5 bis 14 % Ethanol eluiert wird.
Ausbeute: 88 % der Theorie,
C₂₇H₃₃N₅O₄ x HCl (491.60/528.06)
Massenspektrum: (M+H)⁺ = 492
(M-H)⁻ = 490

### Beispiel 192

### 4-{3-[2,5-Dimethyl-4-(N-propyl-N-(3-ethoxycarbonyl-propionyl)-amino)-phenyl]-propargylamino}-benzamidin

Hergestellt analog Beispiel 2 aus N-tert.Butoxycarbonyl-4-{3-[2,5-dimethyl-4-(N-propyl-N-(3-ethoxycarbonyl-propionyl)-amino)-phenyl]-propargylamino}-benzamidin und Trifluoressigsäure.
Ausbeute: 58 % der Theorie,
C₂₇H₃₄N₄O₃ x CF₃COOH (462.59/576.62)
R_{f}-Wert: 0.2 (Kieselgel; Dichlormethan/Methanol = 4:1) Massenspektrum: (M+H)⁺ = 463

### Beispiel 193

### 4-{3-[2,5-Dimethyl-4-(N-cyclobutyl-N-(3-ethoxycarbonyl-propionyl)-amino)-phenyl]-propargylamino}-benzamidin

Hergestellt analog Beispiel 2 aus N-tert.Butoxycarbonyl-4-{3-[2,5-dimethyl-4-(N-cyclobutyl-N-(3-ethoxycarbonyl-propionyl)-amino)-phenyl]-propargylamino}-benzamidin und Trifluoressigsäure.
Ausbeute: 49 % der Theorie,
C₂₈H₃₄N₄O₃ x CF₃COOH (474.61/588.63)
Massenspektrum: (M+H)⁺ = 475

### Beispiel 194

### 4-{3-[2,5-Dimethyl-4-(N-propyl-N-(3-hydroxycarbonyl-propionyl)-amino)-phenyl]-propargylamino}-benzamidin

Hergestellt analog Beispiel 3 aus 4-{3-[2,5-Dimethyl-4-(N-propyl-N-(3-ethoxycarbonyl-propionyl)-amino)-phenyl]-propargylamino}-benzamidin, Lithiumhydroxid und anschließender Behandlung mit Ammoniumchlorid.
Ausbeute: 63 % der Theorie,
C₂₅H₃₀N₄O₃ x HCl (434.54/471.00)
Massenspektrum: (M+H)⁺ = 435
(M-H)⁻ = 433

### Beispiel 195

### 4-{3-[2,5-Dimethyl-4-(N-ethyl-N-(3-ethoxycarbonyl-propionyl)-amino)-phenyl]-propargylamino}-benzamidin

Hergestellt analog Beispiel 2 aus N-tert.Butoxycarbonyl-4-{3-[2,5-dimethyl-4-(N-ethyl-N-(3-ethoxycarbonyl-propionyl)-amino)-phenyl]-propargylamino}-benzamidin und Trifluoressigsäure.
Ausbeute: 49 % der Theorie,
C₂₆H₃₂N₄O₃ x CF₃COOH (448.57/562.59)
Massenspektrum: (M+H)⁺ = 449

### Beispiel 196

### 4-{3-[2,5-Dimethyl-4-(N-ethyl-N-(3-hydroxycarbonyl-propionyl)-amino)-phenyl]-propargylamino}-benzamidin

0.2 g (0.267 Mol) 4-{3-(2,5-Dimethyl-4-(N-ethyl-N-(3-ethoxycarbonyl-propionyl)-amino)-phenyl]-propargylamino)-benzamidin werden in 30 ml 6 molarer Salzsäure 19 Stunden bei Raumtemperatur gerührt. Anschließend wird im Vakuum eingedampft, der Rückstand mit Aceton verrieben und abgesaugt.
Ausbeute: 98 % der Theorie,
C₂₄H₂₈N₄O₃ x HCl (420.52/456.98)
Massenspektrum: (M+H)⁺ = 421
(M-H)⁻ = 419

### Beispiel 197

### 4-{3-[2,5-Dimethyl-4-(N-isopropyl-N-(N'-ethoxycarbonylmethyl-N'-methyl-aminomethylcarbonyl)-amino)-phenyl]-propargylamino}benzamidin

Hergestellt analog Beispiel 2 aus N-tert.Butoxycarbonyl-4-{3-[2,5-dimethyl-4-(N-isopropyl-N-(ethoxycarbonylmethyl-(N-methylamino)methylcarbonyl)-amino)-phenyl]-propargylamino}benzamidin und Trifluoressigsäure.
Ausbeute: 66 % der Theorie,
C₂₈H₃₇N₅O₃ x 2 CF₃COOH (491.64/719.68)
Massenspektrum: (M+H)⁺ = 492
(M-H)⁻ = 490

### Beispiel 198

### 4-{3-[2,5-Dimethyl-4-(N-isopropyl-N-(3-ethoxycarbonyl-propionyl)-amino)-phenyl]-propargyloxy}-benzamidin

Hergestellt analog Beispiel le aus 4-{3-[2,5-Dimethyl-4-(N-isopropyl-N-(3-ethoxycarbonyl-propionyl)-amino)-phenyl]-propargyloxy}-benzonitril und Salzsäure/Ammoniumcarbonat in Ethanol.
Ausbeute: 9 % der Theorie,
C₂₇H₃₃N₃O₄ x HCl (463.59/500.047)
R_{f}-Wert: 0.62 (Reversed Phase Kieselgel RP-8; Methanol/6%ige Kochsalzlösung = 4:1)
Massenspektrum: (M+H)⁺ = 464

### Beispiel 199

### 4-{3-[2,5-Dimethyl-4-(N-isopropyl-N-(3-hydroxycarbonyl-propionyl)-amino)-phenyl]-propargyloxy}-benzamidin

Hergestellt analog Beispiel le aus 4-{3-[2,5-Dimethyl-4-(N-isopropyl-N-(3-ethoxycarbonyl-propionyl)-amino)-phenyl]-propargyloxy}-benzamidin, Lithiumhydroxid und anschließender Behandlung mit Eisessig.
Ausbeute: 65 % der Theorie,
C₂₅H₂₉N₃O₄ (435.53)
R_{f}-Wert: 0.62 (Reversed Phase Kieselgel RP-8; Methanol/6%ige Kochsalzlösung = 4:1)
Massenspektrum: (M+H)⁺ = 436
(M-H)⁻ = 434

### Beispiel 200

### 4-{3-[2,5-Dimethyl-4-(N-isopropyl-N-hydroxycarbonylmethylaminocarbonylmethylcarbonyl-amino)-phenyl]-propargylamino}benzamidin

Hergestellt analog Beispiel 3 aus 4-{3-[2,5-Dimethyl-4-(N-isopropyl-N-methoxycarbonylmethylaminocarbonylmethylcarbonylamino)-phenyl]-propargylamino}-benzamidin, Lithiumhydroxid und anschließender Behandlung mit Eisessig.
Ausbeute: 3 % der Theorie,
C₂₆H₃₁N₅O₄ (477.568)
R_{f}-Wert: 0.74 (Reversed Phase Kieselgel RP-8; Methanol/6%ige Kochsalzlösung = 4:1)
Massenspektrum: (M+H)⁺ = 478
(M+Na)⁺ = 500
(M-H)⁻ = 476

### Beispiel 201

### 4-{3-[2,5-Dimethyl-4-(N-isopropyl-N-(3-amino-3-ethoxycarbonylpropionyl)-amino)-phenyl]-propargylamino}-benzamidin

Hergestellt analog Beispiel 2 aus N-tert.Butoxycarbonyl-4-{3-[2,5-dimethyl-4-(N-isopropyl-N-(3-tert.butoxycarbonylamino-3-ethoxycarbonyl-propionyl)-amino)-phenyl]-propargylamino}-benzamidin und Trifluoressigsäure und anschließender Behandlung mit ethanolischer Salzsäure.
Ausbeute: 86 % der Theorie,
C₂₇H₃₅N₅O₃ x 2HCl (477.62/550.54)
R_{f}-Wert: 0.77 (Reversed Phase Kieselgel RP-8; Methanol/6%ige Kochsalzlösung = 4:1)
Massenspektrum: (M+H)⁺ = 478

### Beispiel 202

### 4-{3-[2,5-Dimethyl-4-(N-isopropyl-N-(3-amino-3-hydroxycarbonyl-propionyl)-amino)-phenyl]-propargylamino}-benzamidin

Hergestellt analog Beispiel 3 aus 4-{3-[2,5-Dimethyl-4-(N-isopropyl-N-(3-amino-3-ethoxycarbonyl-propionyl)-amino)-phenyl]-propargylamino}-benzamidin und Kaliumhydroxid und anschließender Behandlung mit ethanolischer Salzsäure.
Ausbeute: 12 % der Theorie,
C₂₅H₃₁N₅O₃ x 2HCl (449.56/522.49)
R_{f}-Wert: 0.69 (Reversed Phase Kieselgel RP-8; Methanol/6%ige Kochsalzlösung = 4:1)
Massenspektrum: (M+H)⁺ = 450
(M-H)⁻ = 448

### Beispiel 203

### 4-{3-[5-Fluor-2-methyl-4-(N-isopropyl-N-ethoxycarbonylmethylaminocarbonyl-amino)-phenyl]-propargylamino}-benzamidin

Hergestellt analog Beispiel 2 aus N-tert.Butoxycarbonyl-4-{3-[5-fluor-2-methyl-4-(N-isopropyl-N-ethoxycarbonylmethylaminocarbonyl-amino)-phenyl]-propargylamino}-benzamidin und Trifluoressigsäure und anschließender Behandlung mit ethanolischer Salzsäure.
Ausbeute: 43 % der Theorie,
C₂₅H₃₀FN₅O₃ x HCl (467.55/504.01)
R_{f}-Wert: 0.2 (Kieselgel; Dichlormethan/Methanol = 3:1) Massenspektrum: (M+H)⁺ = 468
(M-H)⁻ = 466

### Beispiel 204

### 4-{3-[5-Fluor-2-methyl-4-(N-isopropyl-N-hydroxycarbonylmethylaminocarbonyl-amino)-phenyl]-propargylamino}-benzamidin

Hergestellt analog Beispiel 3 aus 4-{3-[5-Fluor-2-methyl-4-(N-isopropyl-N-ethoxycarbonylmethylaminocarbonyl-amino)-phenyl]-propargylamino}-benzamidin, Natriumhydroxid und anschließender Behandlung mit Eisessig.
Ausbeute: 92 % der Theorie,
C₂₃H₂₆FN₅O₃ (439.49)
Massenspektrum: (M+H)⁺ = 440
(M+Na)⁺ = 462
(M-H)⁻ = 438

### Beispiel 205

### 4-{3-[2-Methyl-4-(N-isopropyl-N-ethoxycarbonylmethylaminocarbonyl-amino)-phenyl]-propargylamino}-benzamidin

Hergestellt analog Beispiel 2 aus N-tert.Butoxycarbonyl-4-{3-[2-methyl-4-(N-isopropyl-N-ethoxycarbonylmethylaminocarbonyl-amino)-phenyl]-propargylamino}-benzamidin und Trifluoressigsäure.
Ausbeute: 56 % der Theorie,
C₂₅H₃₁N₅O₃ x CF₃COOH (449.55/563.58)
Massenspektrum: (M+H)⁺ = 450
(M+CF₃COOH-H)⁻ = 562

### Beispiel 206

### 4-{3-[2,5-Dimethyl-4-(N-cyclobutyl-N-methoxycarbonylmethylcarbonyl-amino)-phenyl]-propargylamino}-benzamidin

Hergestellt analog Beispiel 2 aus N-tert.Butoxycarbonyl-4-{3-[2,5-dimethyl-4-(N-cyclobutyl-N-methoxycarbonylmethylcarbonyl-amino)-phenyl]-propargylamino}-benzamidin und Trifluoressigsäure.
Ausbeute: 65 % der Theorie,
C₂₆H₃₀N₄O₃ x CF₃COOH (446.55/560.56)
Massenspektrum: (M+H)⁺ = 447

### Beispiel 207

### 4-{3-[2,5-Dimethyl-4-(N-isopropyl-N-aminomethylcarbonyl-amino)-phenyl]-propargylamino}-benzamidin

Hergestellt analog Beispiel 2 aus N-tert.Butoxycarbonyl-4-{3-[2,5-dimethyl-4-(N-isopropyl-N-tert.butoxycarbonylaminomethylcarbonyl-amino)-phenyl]-propargylamino}-benzamidin und Trifluoressigsäure.
Ausbeute: 61 % der Theorie,
C₂₃H₂₉N₅O x 2 CF₃COOH (391.53/619.57)
R_{f}-Wert: 0.70 (Reversed Phase Kieselgel RP-8; Methanol/6%ige Kochsalzlösung = 4:1)
Massenspektrum: (M+H)⁺ = 392

### Beispiel 208

### 4-{3-[2-Methyl-4-(N-isopropyl-N-hydroxycarbonylmethylaminocarbonyl-amino)-phenyl]-propargylamino}-benzamidin

Hergestellt analog Beispiel 3 aus 4-{3-[2-Methyl-4-(N-isopropyl-N-ethoxycarbonylmethylaminocarbonyl-amino)-phenyl]-propargylamino}-benzamidin, Natriumhydroxid und anschließender Behandlung mit Eisessig.
Ausbeute: 42 % der Theorie,
C₂₃H₂₇N₅O₃ (421.50)
Massenspektrum: (M+H)⁺ = 422
(M+Na)⁺ = 444
(M-H)⁻ = 420

### Beispiel 209

### 4-{3-[2-Chlor-5-methyl-4-(N-isopropyl-N-ethoxycarbonylmethylaminocarbonyl-amino)-phenyl]-propargylamino}-benzamidin

Hergestellt analog Beispiel 2 aus N-tert.Butoxycarbonyl-4-{3-[2-chlor-5-methyl-4-(N-isopropyl-N-ethoxycarbonylmethylaminocarbonyl-amino)-phenyl]-propargylamino}-benzamidin und Trifluoressigsäure.
Ausbeute: 38 % der Theorie,
C₂₅H₃₀ClN₅O₃ x CF₃COOH (484.00/598.02)
R_{f}-Wert: 0.4 (Kieselgel; Dichlormethan/Methanol = 4:1) Massenspektrum: (M+H)⁺ = 484/486 (Chlorisotope)

### Beispiel 210

### 4-{3-[2-Chlor-5-methyl-4-(N-isopropyl-N-hydroxycarbonylmethylaminocarbonyl-amino)-phenyl]-propargylamino}-benzamidin

Hergestellt analog Beispiel 3 aus 4-{3-[2-Chlor-5-methyl-4-(N-isopropyl-N-ethoxycarbonylmethylaminocarbonyl-amino)-phenyl]-propargylamino}-benzamidin, Natriumhydroxid und anschließender Behandlung mit Eisessig.
Ausbeute: 7 % der Theorie,
C₂₃H₂₆ClN₅O₃ (455.94)
Massenspektrum: (M+H)⁺ = 456/458 (Chlorisotope)
(M+Na)⁺ = 478/480 (Chlorisotope)
(M-H)⁻ = 454/456 (Chlorisotope)

### Beispiel 211

### 4-{3-[2,5-Dimethyl-4-(N-isopropyl-N-(3-amino-propionyl)-amino)-phenyl]-propargylamino}-benzamidin

Hergestellt analog Beispiel 2 aus N-tert.Butoxycarbonyl-4-{3-[2,5-dimethyl-4-(N-isopropyl-N-(3-tert.butoxycarbonylamino-propionyl)-amino)-phenyl]-propargylamino}-benzamidin und Trifluoressigsäure.
Ausbeute: 71 % der Theorie,
R_{f}-Wert: 0.39 (Kieselgel; Dichlormethan/Ethanol = 4:1)
R_{f}-Wert: 0.36 (Reversed Phase Kieselgel RP-8; Methanol/6%ige Kochsalzlösung = 4:1)
C₂₄H₃₁N₅O x 2 CF₃COOH (405.55/633.59)

### Beispiel 212

### 4-{3-[3-Methyl-4-(N-isopropyl-N-ethoxycarbonylmethylaminocarbonyl-amino)-phenyl]-propargylamino}-benzamidin

Hergestellt analog Beispiel 2 aus N-tert.Butoxycarbonyl-4-{3-[3-methyl-4-(N-isopropyl-N-ethoxycarbonylmethylaminocarbonyl-amino)-phenyl]-propargylamino}-benzamidin und Trifluoressigsäure.
Ausbeute: 46 % der Theorie,
R_{f}-Wert: 0.21 (Kieselgel; Dichlormethan/Ethanol = 4:1) C₂₅H₃₁N₅O₃ x CF₃COOH (449.56/563.58)
Massenspektrum: (M+H)⁺ = 450
(M-H)⁻ = 448

### Beispiel 213

### 4-{3-[3-Methyl-4-(N-isopropyl-N-hydroxycarbonylmethylaminocarbonyl-amino)-phenyl]-propargylamino}-benzamidin

Hergestellt aus 4-{3-[3-Methyl-4-(N-isopropyl-N-ethoxycarbonylmethylaminocarbonyl-amino)-phenyl]-propargylamino}-benzamidin durch 18-stündiges Einwirken von 20 ml 6N HCl und anschließendes Abdestillieren der flüchtigen Bestandteile. Ausbeute: 82 % der Theorie,
R_{f}-Wert: 0.46 (Reversed Phase Kieselgel RP-8; Methanol/5%ige Kochsalzlösung = 6:4)
C₂₃H₂₇N₅O₃ x HCl (421.50/457.96)
Massenspektrum: (M+H)⁺ = 422
(M+Na)⁺ = 444
(M-H)⁻ = 420
(M+HCl-H)⁻ = 456/458 (Chlorisotope)

### Beispiel 214

### 4-{3-[2-Methyl-4-(N-isopropyl-N-(2-trifluoracetylamino-3-methoxycarbonyl-propionyl)-amino)-phenyl]-propargylamino}-benzamidin

Hergestellt analog Beispiel 2 aus N-tert.Butoxycarbonyl-4-{3-[2-methyl-4-(N-isopropyl-N-(2-trifluoracetylamino-3-methoxycarbonyl-propionyl)-amino)-phenyl]-propargylamino}benzamidin und Trifluoressigsäure.
Ausbeute: 100 % der Theorie,
R_{f}-Wert: 0.17 (Kieselgel; Dichlormethan/Ethanol = 4:1) C₂₇H₃₀F₃N₅O₄ x CF₃COOH (545.57/659.59)
Massenspektrum: (M+H)⁺ = 546
(M-H)⁻ = 544
(M+CF3COOH-H)⁻ = 658

### Beispiel 215

### 4-{3-[2-Methyl-4-(N-isopropyl-N-(2-trifluoracetylamino-3-hydroxycarbonyl-propionyl)-amino)-phenyl]-propargylamino}-benzamidin

Hergestellt analog Beispiel 213 aus 4-{3-[2-Methyl-4-(N-isopropyl-N-(2-trifluoracetylamino-3-methoxycarbonyl-propionyl)-amino)-phenyl]-propargylamino}-benzamidin und 6N HCl. Ausbeute: 100 % der Theorie,
R_{f}-Wert: 0.42 (Reversed Phase Kieselgel RP-8; Methanol/5%ige Kochsalzlösung = 6:4)
C₂₆H₂₈F₃N₅O₄ x HCl (531.540/568.00)
Massenspektrum: (M+H)⁺ = 532
(M-H)⁻ = 530

### Beispiel 216

### 4-{3-[2-Methyl-4-(N-isopropyl-N-(2-amino-3-hydroxycarbonylpropionyl)-amino)-phenyl]-propargylamino}-benzamidin

Hergestellt aus 0.160 g (0.282 mmol) 4-{3-[2-Methyl-4-(N-isopropyl-N-(2-trifluoracetylamino-3-hydroxycarbonyl-propionyl)-amino)-phenyl]-propargylamino}-benzamidin und 120 mg Natriumcarbonat in 20 ml Methanol durch 5-tägiges Rühren bei Raumtemperatur. Nach anschließendem 1-tägigen Erhitzen auf 60°C werden die flüchtigen Bestandteile abdestilliert.
Ausbeute: 98 % der Theorie,
R_{f}-Wert: 0.46 (Reversed Phase Kieselgel RP-8; Methanol/5%ige Kochsalzlösung = 6:4)
C₂₄H₂₉N₅O₃ x HCl (435.53/471.99)

### Beispiel 217

### 4-{3-[2,5-Dimethyl-4-(N-isopropyl-N-(N'-hydroxycarbonylmethyl-N'-methyl-aminomethylcarbonyl)-amino)-phenyl)-propargylamino}benzamidin

Hergestellt analog Beispiel 3 aus 4-{3-[2,5-Dimethyl-4-(N-isopropyl-N-(N'-ethoxycarbonylmethyl-N'-methyl-aminomethylcarbonyl)-amino)-phenyl]-propargylamino}-benzamidin, Natriumhydroxid, anschließender Fällung mit Eisessig und mehrmaliger chromatographischer Reinigung mittels HPLC-Säule.
Ausbeute: 2 % der Theorie,
C₂₆H₃₃N₅O₃ x HCl (463.58/500.04)
Massenspektrum: (M+H)⁺ = 464

### Beispiel 218

### 4-{3-[2-Methyl-4-(N-isopropyl-N-(3-trifluoracetylamino-3-methoxycarbonyl-propionyl)-amino)-phenyl]-propargylamino}-benzamidin

Hergestellt analog Beispiel 2 aus N-tert.Butoxycarbonyl-4-{3-[2-methyl-4-(N-isopropyl-N-(3-trifluoracetylamino-3-methoxycarbonyl-propionyl)-amino)-phenyl]-propargylamino}benzamidin und Trifluoressigsäure.
Ausbeute: 89 % der Theorie,
R_{f}-Wert: 0.21 (Kieselgel; Dichlormethan/Ethanol = 4:1) C₂₇H₃₀F₃N₅O₄ x CF₃COOH (545.57/659.59)
Massenspektrum: (M+H)⁺ = 546
(M-H)⁻ = 544

### Beispiel 219

### 4-[3-(3-Brom-5-(2-methyl-pyrrolidinocarbonyl)-furan-2-yl)-propargylamino]benzamidin

Hergestellt analog Beispiel 1g aus 2,3-Dibrom-5-(2-methylpyrrolidinocarbonyl)-furan, N-tert.Butoxycarbonyl-4-propargylamino-benzamidin, Tetrakis(triphenylphosphin)palladium(0), Kupfer(I)iodid und Triethylamin in Acetonitril und anschließende Abspaltung des tert.Butyloxycarbonylrestes durch Trifluoressigsäure analog Beispiel 2.
Ausbeute: 17 % der Theorie,
R_{f}-Wert: 0.28 (Kieselgel; Dichlormethan/Ethanol = 4:1) C₂₀H₂₁BrN₄O₂ x CF₃COOH (429.32/543.34)
Massenspektrum: (M+H)⁺ = 429/431 (Bromisotope)

### Beispiel 220

### 4-{3-[2-Methyl-4-(N-isopropyl-N-(3-amino-3-methoxycarbonylpropionyl)-amino)-phenyl]-propargylamino}-benzamidin

Hergestellt analog Beispiel 216 aus 4-{3-[2-Methyl-4-(N-isopropyl-N-(3-trifluoracetylamino-3-methoxycarbonyl-propionyl)-amino)-phenyl]-propargylamino}-benzamidin und Natriumcarbonat in Methanol.
Ausbeute: 93 % der Theorie,
R_{f}-Wert: 0.45 (Reversed Phase Kieselgel RP-8; Methanol/5%ige Kochsalzlösung = 6:4)
C₂₅H₃₁N₅O₃ x CF₃COOH (449.56/563.58)

### Beispiel 221

### 4-{3-[3-Methyl-4-(N-isopropyl-N-methoxycarbonylmethylcarbonylamino)-phenyl]-propargylamino}-benzamidin

Hergestellt analog Beispiel 2 aus N-tert.Butoxycarbonyl-4-{3-[3-methyl-4-(N-isopropyl-N-methoxycarbonylmethylcarbonylamino)-phenyl]-propargylamino}-benzamidin und Trifluoressigsäure.
Ausbeute: 55 % der Theorie,
R_{f}-Wert: 0.21 (Kieselgel; Dichlormethan/Ethanol = 4:1)
C₂₄H₂₈N₄O₃ x CF₃COOH (420.52/534.54)
Massenspektrum: (M+H)⁺ = 421
(M+CF₃COOH-H)⁻ = 533

### Beispiel 222

### 4-{3- [3-Methyl-4- (N-cyclopentyl-N-methoxycarbonylmethylcarbonyl-amino)-phenyl]-propargylamino}-benzamidin

Hergestellt analog Beispiel 2 aus N-tert.Butoxycarbonyl-4-{3-[3-methyl-4-(N-cyclopentyl-N-methoxycarbonylmethylcarbonyl-amino)-phenyl]-propargylamino}-benzamidin und Trifluoressigsäure.
Ausbeute: 85 % der Theorie,
R_{f}-Wert: xxxx (Kieselgel; Dichlormethan/Ethanol = 4:1)
C₂₆H₃₀N₄O₃ x CF₃COOH (446.55/560.57)
Massenspektrum: (M+H)⁺ = 447
(M+CF₃COOH-H)⁻ = 559

### Beispiel 223

### 4-{3-[3-Methyl-4-(N-isopropyl-N-hydroxycarbonylmethylcarbonylamino)-phenyl]-propargylamino}-benzamidin

Hergestellt analog Beispiel 213 aus 4-{3-[3-Methyl-4-(N-isopropyl-N-methoxycarbonylmethylcarbonyl-amino)-phenyl]-propargylamino}-benzamidin und 6N HCl. Ausbeute: 100 % der Theorie,
R_{f}-Wert: 0.40 (Kieselgel; Dichlormethan/Ethanol = 4:1)
C₂₃H₂₆N₄O₃ x HCl (406.48/442.94)
Massenspektrum: (M+H)⁺ = 407
(M-H)⁻ = 405

### Beispiel 224

### 4-{3-[3-Methyl-4-(N-cyclopentyl-N-hydroxycarbonylmethylcarbonyl-amino)-phenyl]-propargylamino}-benzamidin

Hergestellt analog Beispiel 213 aus 4-{3-[3-Methyl-4-(N-cyclopentyl-N-methoxycarbonylmethylcarbonyl-amino)-phenyl]-propargylamino}-benzamidin und 6N HCl.
Ausbeute: 92 % der Theorie,
R_{f}-Wert: 0.23 (Kieselgel; Dichlormethan/Ethanol = 4:1)
C₂₅H₂₈N₄O₃ x HCl (432.52/468.99)
Massenspektrum: (M+H)⁺ = 433
(M-H)⁻ = 431

Analog den vorstehenden Beispielen können folgende Verbindungen hergestellt werden:
4-{3-[2-Brom-5-methyl-4-(N-isopropyl-N-(2-ethoxycarbonylethylcarbonyl)-amino)-phenyl]-propargylamino}-benzamidin
4-{3-[2,5-Dimethyl-4-(N-isopropyl-N-(2-ethoxycarbonyl-2-aminoacetylamino)-phenyl]-propargylamino}-benzamidin
4-{3-[2,5-Dimethyl-4-(N-isopropyl-N-(2-hydroxycarbonyl-2-amino-acetylamino)-phenyl]-propargylamino}-benzamidin
4-{3-[2,5-Bis(trifluormethyl)-4-(N-isopropyl-N-ethoxycarbonylmethylaminocarbonyl-amino)-phenyl]-propargylamino}benzamidin
4-{3-[2,5-Bis(trifluormethyl)-4-(N-isopropyl-N-hydroxycarbonylmethylaminocarbonyl-amino)-phenyl]-propargylamino}-benzamidin
4-{3-[2-Trifluormethyl-5-methyl-4-(N-isopropyl-N-ethoxycarbonylmethylaminocarbonyl-amino)-phenyl]-propargylamino}-benzamidin
4-{3-[2-Trifluormethyl-5-methyl-4-(N-isopropyl-N-hydroxycarbonylmethylaminocarbonyl-amino)-phenyl]-propargylamino}-benzamidin
4-{3-[2-Trifluormethyl-4-(N-isopropyl-N-ethoxycarbonylmethylaminocarbonyl-amino)-phenyl]-propargylamino}-benzamidin
4-{3-[2-Trifluormethyl-4-(N-isopropyl-N-hydroxycarbonylmethylaminocarbonyl-amino)-phenyl]-propargylamino}-benzamidin
4-{3-[2,5-Dimethyl-4-(N-isopropyl-N-(2-ethoxycarbonylethylaminocarbonyl)-amino)-phenyl]-propargylamino}-benzamidin
4-{3-[2,5-Dimethyl-4-(N-isopropyl-N-(2-hydroxycarbonylethylaminocarbonyl)-amino)-phenyl]-propargylamino}-benzamidin
4-{3-[2,5-Dimethyl-4-(N-isopropyl-N-(ethoxycarbonylmethylaminomethylcarbonyl)-amino)-phenyl]-propargylamino}-benzamidin
4-{3-[2,5-Dimethyl-4-(N-isopropyl-N-(hydroxycarbonylmethylaminomethylcarbonyl)-amino)-phenyl]-propargylamino}-benzamidin
4-{3-[2,5-Dimethyl-4-(N-isopropyl-N-(4-amino-4-ethoxycarbonylbutanoyl)-amino)-phenyl]-propargylamino}-benzamidin
4-{3-[2,5-Dimethyl-4-(N-isopropyl-N-(4-amino-4-hydroxycarbonyl-butanoyl)-amino)-phenyl]-propargylamino}-benzamidin
4-{3-[2,5-Dimethyl-4-(N-isopropyl-N-(2-amino-4-ethoxycarbonylbutanoyl)-amino)-phenyl]-propargylamino}-benzamidin
4-{3-[2,5-Dimethyl-4-(N-isopropyl-N-(2-amino-4-hydroxycarbonyl-butanoyl)-amino)-phenyl]-propargylamino}-benzamidin
4-{3-[2,5-Dimethyl-4-(N-isopropyl-N-(2-ethoxycarbonylethylaminocarbonyl)-amino)-phenyl]-propargylamino}-benzamidin
4-{3-[2,5-Dimethyl-4-(N-isopropyl-N-(2-hydroxycarbonylethylaminocarbonyl)-amino)-phenyl]-propargylamino}-benzamidin
4-{3-[2-Methyl-4-(N-isopropyl-N-(3-amino-3-hydroxycarbonylpropionyl)-amino)-phenyl]-propargylamino}-benzamidin
4-{3-[2-Methyl-4-(N-isopropyl-N-(2-amino-3-methoxycarbonylpropionyl)-amino)-phenyl]-propargylamino}-benzamidin
4-{3-[2,5-Dimethyl-4-(N-isopropyl-N-aminocarbonyl-amino)-phenyl]-propargylamino}-benzamidin
4-{3-[2,5-Dimethyl-4-(N-isopropyl-N-(piperazin-1-yl-carbonyl)-amino)-phenyl]-propargylamino}-benzamidin
4-{3-[2,5-Dimethyl-4-(N-isopropyl-N-(piperazin-1-yl-methylcarbonyl)-amino)-phenyl]-propargylamino}-benzamidin
4-{3-[2,5-Dimethyl-4-(N-isopropyl-N-(2-pyrrolidinon-5-yl-carbonyl)-amino)-phenyl]-propargylamino}-benzamidin
4-{3-[3-Brom-5-pyrrolidinocarbonyl-furan-2-yl]-propargylamino}-benzamidin

### Beispiel 225

### Trockenampulle mit 75 mg Wirkstoff pro 10 ml

### Zusammensetzung:

| | |
|---|---|
| Wirkstoff | 75,0 mg |
| Mannitol | 50,0 mg |
| Wasser für Injektionszwecke | ad 10,0 ml |

### Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

### Beispiel 226

### Trockenampulle mit 35 mg Wirkstoff pro 2 ml

### Zusammensetzung:

| | |
|---|---|
| Wirkstoff | 35,0 mg |
| Mannitol | 100,0 mg |
| Wasser für Injektionszwecke | ad 2,0 ml |

### Herstellung:

Wirkstoff und Mannitol werden in Wasser gelöst. Nach Abfüllung wird gefriergetrocknet.

Die Auflösung zur gebrauchsfertigen Lösung erfolgt mit Wasser für Injektionszwecke.

### Beispiel 227

### Tablette mit 50 mg Wirkstoff

### Zusammensetzung:

| | |
|---|---|
| (1) Wirkstoff | 50,0 mg |
| (2) Milchzucker | 98,0 mg |
| (3) Maisstärke | 50,0 mg |
| (4) Polyvinylpyrrolidon | 15,0 mg |
| (5) Magnesiumstearat | 2,0 mg |
| | 215,0 mg |

### Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreBt, biplan mit beidseitiger Facette und einseitiger Teilkerbe. Durchmesser der Tabletten: 9 mm.

### Beispiel 228

### Tablette mit 350 mg Wirkstoff

### Zusammensetzung:

| | |
|---|---|
| (1) Wirkstoff | 350,0 mg |
| (2) Milchzucker | 136,0 mg |
| (3) Maisstärke | 80,0 mg |
| (4) Polyvinylpyrrolidon | 30,0 mg |
| (5) Magnesiumstearat | 4.0 mg |
| | 600,0 mg |

### Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 12 mm.

### Beispiel 229

### Kapseln mit 50 mg Wirkstoff

### Zusammensetzung:

| | |
|---|---|
| (1) Wirkstoff | 50,0 mg |
| (2) Maisstärke getrocknet | 58,0 mg |
| (3) Milchzucker pulverisiert | 50,0 mg |
| (4) Magnesiumstearat | 2.0 mg |
| | 160,0 mg |

### Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 3 abgefüllt.

### Beispiel 230

### Kapseln mit 350 mg Wirkstoff

### Zusammensetzung:

| | |
|---|---|
| (1) Wirkstoff | 350,0 mg |
| (2) Maisstärke getrocknet | 46,0 mg |
| (3) Milchzucker pulverisiert | 30,0 mg |
| (4) Magnesiumstearat | 4,0 mg |
| | 430,0 mg |

### Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Gr6Be 0 abgefüllt.

### Beispiel 231

### Suppositorien mit 100 mg Wirkstoff

| | |
|---|---|
| 1 Zäpfchen enthält: | |
| Wirkstoff | 100,0 mg |
| Polyethylenglykol (M.G. 1500) | 600,0 mg |
| Polyethylenglykol (M.G. 6000) | 460,0 mg |
| Polyethylensorbitanmonostearat | 840,0 mg |
| | 2 000,0 mg |

### Herstellung:

Das Polyethylenglykol wird zusammen mit Polyethylensorbitanmonostearat geschmolzen. Bei 40°C wird die gemahlene Wirksubstanz in der Schmelze homogen dispergiert. Es wird auf 38°C abgekühlt und in schwach vorgekühlte Suppositorienformen ausgegossen.

## Patentansprüche

1. Substituierte Aryl- und Heteroarylderivate der allgemeinen Formel
Ar - A - (HCR₁) - X - Y , (I)
in der
A eine Ethinylengruppe, eine gegebenenfalls durch eine C₁₋₃-Alkyl- oder Carboxy-C₁₋₃-alkylgruppe oder durch ein Chlor-, Bromoder Jodatom substituierte Vinylen- oder Ethylengruppe,
R₁ ein Wasserstoffatom, eine C₁₋₃-Alkyl- oder Carboxy-C₁₋₃-alkylgruppe,
Ar eine durch die Reste R₂ bis R₄ substituierte Phenylgruppe, wobei
R₂ eine C₁₋₆-Alkyl- oder C₃₋₇-Cycloalkyl-C₁₋₃-alkylgruppe, die jeweils im C₁₋₆- und C₁₋₃-Alkylteil durch eine Carboxy-, Phenyl-, Amino-, C₁₋₃-Alkylamino-, Carboxy-C₁₋₃-alkylamino-, Di- (C₁₋₃-Alkyl) -amino-, N- (Carboxy-C₁₋₃-alkyl)-C₁₋₃-alkylamino-, C₃₋₇-Cycloalkylamino-, Phenylamino-, N-(C₁₋₃-Alkyl)-phenylamino-, N-(C₁₋₄-Alkanoyl)-phenylamino-, Heteroarylamino-, N- (C₁₋₃-Alkyl) -heteroarylamino-, N- (Carboxy-C₁₋₃-alkyl)-phenylamino- oder N- (Carboxy-C₁₋₃-alkyl)-heteroarylaminogruppe substituiert sein können,
eine Carboxy-C₁₋₅-alkylgruppe, die im Alkylteil durch eine C₁₋₃-Alkylamino-, N,N-Di-(C₁₋₃-alkyl)-amino-, Pyrrolidino-, Piperidino- oder Hexamethyleniminogruppe substituiert ist,
eine Carboxy-C₁₋₅-alkylgruppe, in der die Wasserstoffatome einer Methylengruppe durch eine n-C₂₋₅-Alkylenbrücke ersetzt sind,
eine Phenyl-, Phenyloxy- oder Phenylsulfonylgruppe, die jeweils im Phenylteil durch ein Fluor-, Chlor-, Brom- oder Jodatom, durch eine C₁₋₃-Alkyl-, Carboxy-C₁₋₃-alkyl- oder C₁₋₃-Alkoxygruppe substituiert sein können,
eine C₁₋₅-Alkylamino-, Carboxy-C₁₋₃-alkylamino-, Di-(C₁₋₅-alkyl)-amino-, N-(Carboxy-C₁₋₃-alkyl)-C₁₋₅-alkylamino-, C₃₋₇-Cycloalkylamino-, N-(Carboxy-C₁₋₃-alkyl)-C₃₋₇-cycloalkylamino-, Phenylamino-, N-(C₁₋₃-Alkyl)-phenylamino-, N- (Carboxy-C₁₋₃-alkyl) -phenylamino-, Heteroarylamino-, N- (C₁₋₃-Alkyl)-heteroarylamino- oder N- (Carboxy-C₁₋₃-alkyl)-heteroarylaminogruppe,
eine C₁₋₅-Alkylcarbonylamino-, C₃₋₇-Cycloalkylcarbonylamino-, Arylcarbonylamino-, Heteroarylcarbonylamino-, C₁₋₅-Alkylsulfonylamino-, Arylsulfonylamino-, Heteroarylsulfonylamino-, N- (C₁₋₃-Alkyl)-C₁₋₅-alkylcarbonylamino-, N- (C₁₋₃-Alkyl)-C₃₋₇-cycloalkylcarbonylamino-, N-(C₁₋₃-Alkyl)-arylcarbonylamino-, N-(C₁₋₃-Alkyl)-heteroarylcarbonylamino-, N-(C₁₋₃-Alkyl)-C₁₋₅-alkylsulfonylamino-, N- (C₁₋₃-Alkyl)-arylsulfonylamino- oder N-(C₁₋₃-Alkyl)-heteroarylsulfonylaminogruppe, wobei
die vorstehend erwähnten N-(C₁₋₃-Alkyl)-teile zusätzlich durch eine Carboxy-, Carboxy-C₁₋₃-alkylaminocarbonyl- oder N- (C₁₋₃-Alkyl)-carboxy-C₁₋₃-alkylaminocarbonylgruppe oder mit Ausnahme des α-Kohlenstoffatoms bezogen auf das Stickstoffatom auch durch eine Hydroxy-, Carboxy-C₁₋₃-alkoxy-, Amino-, Carboxy-C₁₋₃-alkylamino- oder N-(C₁₋₃-Alkyl)-carboxy-C₁₋₃-alkylaminogruppe substituiert sein können,
eine 5- bis 7-gliedrige Cycloalkyleniminogruppe,
eine Amino-, C₁₋₅-Alkylamino-, C₃₋₇-Cycloalkylamino-, Arylamino-, Aryl-C₁₋₃-alkylamino-, Heteroarylamino- oder Heteroaryl-C₁₋₃-alkyl-aminogruppe, die jeweils am Aminstickstoffatom durch eine C₁₋₃-Alkylcarbonyl-, Carboxy-C₁₋₃-alkylcarbonyl-, Carboxy-C₁₋₃-alkylaminocarbonyl-, 2-Oxo-pyrrolidinylcarbonyl- oder Piperazinocarbonylgruppe substituiert sind, wobei zusätzlich
(i) die vorstehend erwähnte Aminogruppe, die durch eine C₁₋₃-Alkylcarbonyl-, Carboxy-C₁₋₃-alkylcarbonyl- oder Carboxy-C₁₋₃-alkylaminocarbonylgruppe monosubstituiert ist, durch eine 5- bis 7-gliedrige Cycloalkyleniminogruppe oder durch eine N,N-Di-(C₁₋₅-Alkyl)-aminogruppe substituiert ist, und
(ii) der Alkylteil der vorstehend erwähnten C₁₋₃-Alkylcarbonylgruppe durch eine Carboxy-, Amino-, Hydroxy-, Carboxy-C₁₋₃-alkoxy-, Carboxy-C₁₋₃-alkylaminocarbonyl-, Carboxy-C₁₋₃-alkylamino-, N-(C₁₋₃-Alkyl)-carboxy-C₁₋₃-alkylamino- oder Amino-C₁₋₃-alkylcarbonylaminogruppe oder durch eine Carboxy- oder Hydroxygruppe und durch eine Aminooder Trifluoracetylaminogruppe substituiert ist,
eine Carbiminogruppe, die am Stickstoffatom durch eine Carboxy-C₁₋₃-alkoxy-, Amino-, C₁₋₃-Alkylamino-, Carboxy-C₁₋₃-alkylamino-, Di- (C₁₋₃-Alkyl) -amino- oder N- (Carboxy-C₁₋₃-alkyl)-C₁₋₃-alkylaminogruppe und am Kohlenstoffatom durch eine C₁₋₅-Alkylgruppe, durch eine gegebenenfalls durch eine C₁₋₃-Alkyl- oder C₁₋₃-Alkoxygruppe substituierte Phenylgruppe oder durch eine gegebenenfalls durch eine C₁₋₃-Alkylgruppe substituierte Heteroarylgruppe substituiert ist,
eine Heteroaryl- oder Heteroaryl-C₁₋₃-alkylgruppe, die jeweils im Heteroarylteil zusätzlich auch durch eine Phenyloder Heteroarylgruppe oder durch eine Phenyl- oder Heteroarylgruppe und durch eine Carboxy-C₁₋₃-alkyl- oder C₁₋₃-Alkoxycarbonyl-C₁₋₃-alkylgruppe substituiert sein können,
eine gegebenfalls durch 1 bis 3 C₁₋₃-Alkylgruppen substituierte 5-Oxo-4,5-dihydro-pyrazolyl- oder 6-Oxo-4,5-dihydropyridazinylgruppe, in der ein Alkylsubstituent gleichzeitig durch eine Carboxy- oder C₁₋₃-Alkoxycarbonylgruppe substituiert sein kann, oder
eine Carbonylgruppe, die
durch ein Wasserstoffatom, durch eine Hydroxy-, C₁₋₅-Alkoxy- oder C₃₋₇-Cycloalkoxygruppe,
durch eine gegebenenfalls durch eine Carboxygruppe substituierte C₁₋₅-Alkyl- oder C₃₋₇-Cycloalkylgruppe,
durch eine durch eine Piperazinogruppe substituierte C₁₋₃-Alkylgruppe,
durch eine Phenylgruppe, die durch ein Fluor-, Chlor-, Brom- oder Jodatom, durch eine C₁₋₃-Alkyl-, Carboxy-C₁₋₃-alkyl-, C₁₋₃-Alkoxy- oder Carboxygruppe substituiert sein kann,
durch eine Amino-, C₁₋₅-Alkylamino-, Carboxy-C₁₋₃-alkylamino-, C₃₋₇-Cycloalkylamino-, Phenylamino- oder Heteroarylaminogruppe, die jeweils zusätzlich am Aminstickstoffatom durch eine C₁₋₅-Alkyl-, C₃₋₇-Cycloalkyl-, Phenyl-C₁₋₃-alkyl-, Carboxy-C₁₋₃-alkyl-, 2-[Di-(C₁₋₃-alkyl)-amino]-ethyl-, 3- [Di-(C₁₋₃-alkyl) -amino] -propyl-, Di- (C₁₋₃-alkyl) -amino-, 2- (N-Carboxy-C₁₋₃-alkyl-C₁₋₃-alkylamino) -ethyl-, 3- (N-Carboxy-C₁₋₃-alkyl-C₁₋₃-alkylamino) -propyl- oder N-Carboxy-C₁₋₃-alkyl-C₁₋₃-alkylamino-, Phenyl-, Pyridyl-, Pyrrolidinyloder Piperidinylgruppe substituiert sein können,
durch eine gegebenenfalls durch eine oder zwei C₁₋₃-Alkylgruppen substituierte Pyrrolyl-, Thienyl-, Imidazolyl-, Pyrazolyl-, Thiazolyl-, Pyridinyl-, Pyrimidinyl-, Pyrazinyl- oder Pyridazinylgruppe, an die jeweils über zwei benachbarte Kohlenstoffatome ein Phenylring ankondensiert sein kann,
durch eine gegebenenfalls durch eine C₁₋₃-Alkyl- oder Carboxy-C₁₋₃-alkylgruppe substituierte C₃₋₆-Cycloalkylenimino-, C₅₋₈-Bicycloalkylenimino-, Morpholino-, Piperazino-, Dihydropyrazolo-, Tetrahydropyrazolo-, Tetrahydroisoxazolo-, Tetrahydropyrazinyl- oder Tetrahydropyridazinylgruppe oder
durch eine gegebenenfalls durch eine C₁₋₃-Alkyl-, carboxy-C₁₋₃-alkyl-, Hydroxy-, Hydroxy-C₁₋₃-alkyl-, Amino-, Carboxy-, Carboxy-C₁₋₃-alkoxy-C₁₋₃-alkyl-, Carboxy-C₁₋₃-alkylamino-C₁₋₃-alkyl- oder Carboxy-C₁₋₃-alkylaminocarbonyl-C₁₋₃-alkylgruppe substituierte C₃₋₆-Cycloalkyleniminogruppe,
durch eine gegebenenfalls durch eine C₁₋₃-Alkyl- oder Carboxy-C₁₋₃-alkylgruppe substituierte C₅₋₈-Bicycloalkylenimino-, Morpholino-, Piperazino-, Dihydropyrazolo-, Tetrahydropyrazolo-, Tetrahydroisoxazolo-, Tetrahydropyrazinyl- oder Tetrahydropyridazinylgruppe substituiert ist,
R₃ ein Wasserstoff-, Fluor-, Chlor-, Brom- oder Jodatom, eine Formyl- oder Trifluormethylgruppe,
eine C₁₋₃-Alkoxy-, Amino-, C₁₋₃-Alkylamino-, Di-(C₁₋₃-Alkyl)-amino-, C₁₋₄-Alkanoylamino- oder N-(C₁₋₄-Alkanoyl)-C₁₋₃-alkylaminogruppe,
eine gegebenenfalls durch eine Hydroxy-, C₁₋₃-Alkoxy-, Carboxy-, Carboxy-C₁₋₃-alkoxy-, Carboxy-C₁₋₃-alkylamino-, N-(C₁₋₃-Alkyl)-carboxy-C₁₋₃-alkylamino- oder Carboxy-C₁₋₃-alkylaminocarbonylgruppe substituierte C₁₋₃-Alkylgruppe,
eine durch eine Carboxy- oder Carboxy-C₁₋₃-alkylaminocarbonylgruppe substituierte C₂₋₃-Alkenylgruppe oder
eine gegebenenfalls am Kohlenstoffatom durch eine C₁₋₃-Alkylgruppe substituierte Carbiminogruppe, die am Iminostickstoffatom durch eine Carboxy-C₁₋₃-alkoxy- oder Aminocarbonylaminogruppe substituiert ist,
oder R₂ und R₃ zusammen eine -CO-O-CH₂- oder -CO-O-CH₂CH₂-Gruppe und
R₄ ein Wasserstoff-, Fluor-, Chlor-, Brom- oder Iodatom, eine C₁₋₃-Alkyl-, C₃₋₇-Cycloalkyl-, Trifluormethyl- oder C₁₋₃-Alkoxygruppe darstellen,
oder Ar auch eine Heteroarylgruppe, die durch die vorstehend erwähnten Reste R₂ bis R₄ substituiert sein kann, welche wie vorstehend erwähnt definiert sind,
X ein Sauerstoff- oder Schwefelatom, eine gegebenenfalls durch eine oder zwei C₁₋₃-Alkylgruppen substituierte Methylengruppe, eine Carbonyl-, Sulfinyl-, Sulfonyl-, Imino-, N-(C₁₋₃-Alkyl)-imino- oder N-(Carboxy-C₁₋₃-alkyl)-iminogruppe, wobei der Alkylteil der N-(C₁₋₃-Alkyl)-iminogruppe in 2- oder 3-Stellung zusätzlich durch eine Amino-, C₁₋₃-Alkylamino-, Di-(C₁₋₃-Alkyl)-amino-, C₁₋₄-Alkanoylamino- oder N-(C₁₋₄-Alkanoyl)-C₁₋₃-alkylaminogruppe substituiert sein kann, und
Y eine durch eine Aminogruppe substituierte Cyclohexylgruppe oder eine durch den Rest R₅ substituierte Phenyl- oder Heteroarylgruppe, wobei die vorstehend erwähnte Phenylgruppe jeweils durch ein Fluor-, Chlor-, Brom- oder Jodatom oder durch eine C₁₋₃-Alkyl- oder C₁₋₃-Alkoxygruppe sowie die vorstehend erwähnte Heteroarylgruppe durch eine C₁₋₃-Alkylgruppe substituiert sein können und
R₅ ein Wasserstoffatom, eine Cyanogruppe oder eine gegebenenfalls durch eine in vivo abspaltbare Gruppe substituierte Amino-, Amino-C₁₋₃-alkyl-, Amidino-, Guanidino- oder Guanidino-C₁₋₃-alkylgruppe darstellt,
bedeuten, wobei
unter den vorstehend erwähnten Heteroarylgruppen eine gegebenenfalls durch eine oder zwei C₁₋₃-Alkylgruppen substituierte 5-gliedrige heteroaromatische Gruppe, die eine gegebenenfalls durch eine C₁₋₃-Alkylgruppe substituierte Iminogruppe, ein Sauerstoff- oder Schwefelatom oder eine gegebenenfalls durch eine C₁₋₃-Alkylgruppe substituierte Iminogruppe, ein Sauerstoff- oder Schwefelatom und eine oder zwei Stickstoffatome sowie deren partiell hydrierten Derivate, insbesondere deren Dihydroderivate, oder eine 6-gliedrige heteroaromatische Gruppe, die ein, zwei oder drei Stickstoffatome enthält, wobei zusätzlich an die vorstehend erwähnten 5- und 6-gliedrigen heteroaromatischen Ringe über zwei benachbarte Kohlenstoffatome ein Phenylring ankondensiert sein kann, zu verstehen ist,
die bei der Definition der Reste vorstehend erwähnten Carboxygruppen durch eine Tetrazolylgruppe oder durch eine in-vivo in eine Carboxygruppe überführbare Gruppe ersetzt sein können, und
die der bei der Definition der Reste erwähnten Imino- oder Aminogruppen durch einen in-vivo abspaltbaren Rest substituiert sein können,
deren Tautomere, deren Stereoisomere, deren Gemische und deren Salze.

2. Verbindungen der allgemeinen Formel I gemäß Anspruch 1, in der
A eine gegebenenfalls durch ein Chlor-, Brom- oder Jodatom substituierte Vinylengruppe, eine Ethylen- oder Ethinylengruppe,
R₁ ein Wasserstoffatom oder eine C₁₋₃-Alkylgruppe,
Ar eine durch eine Benzoylgruppe substituierte Pyridyl- oder Thienylgruppe,
eine durch eine Pyrrolidinocarbonylgruppe substituierte Bromfuranylgruppe oder
eine durch die Reste R₂ bis R₄ substituierte Phenylgruppe, wobei
R₂ eine Phenyl- oder Phenoxygruppe,
eine C₁₋₃-Alkylgruppe, die durch eine Phenyl-, Phenylamino-, N-(C₁₋₃-Alkyl)-phenylamino- oder N- (C₁₋₃-Alkanoyl)-phenylaminogruppe substituiert sein kann,
eine Carboxy- oder C₁₋₃-Alkoxycarbonylgruppe,
eine Benzoyl- oder Phenylsulfonylgruppe, in denen jeweils der Phenylteil zusätzlich durch ein Fluor-, Chlor- oder Bromatom, durch eine Methyl-, Methoxy-, Carboxy- oder C₁₋₃-Alkoxycarbonylgruppe substituiert sein kann, wobei in den vorstehend erwähnten Benzoylgruppen zusätzlich das Sauerstoffatom durch Carboxy-C₁₋₃-alkoxyimino- oder C₁₋₃-Alkoxycarbonyl-C₁₋₃-alkoxyiminogruppe ersetzt sein kann,
eine C₁₋₅-Alkylaminogruppe, die im Alkylteil durch eine Phenyl-, Carboxy-, C₁₋₃-Alkoxycarbonyl-, Carboxy-C₁₋₃-alkylaminocarbonyl-, C₁₋₃-Alkoxycarbonyl-C₁₋₃-alkylaminocarbonyl-, N-(C₁₋₃-Alkyl)-carboxy-C₁₋₃-alkylaminocarbonyl- oder N-(C₁₋₃-Alkyl)-C₁₋₃-alkoxycarbonyl-C₁₋₃-alkylaminocarbonylgruppe substituiert sein kann, oder eine C₃₋₇-Cycloalkylaminogruppe, wobei die vorstehend erwähnten Gruppen jeweils am Aminstickstoffatom zusätzlich durch eine C₃₋₇-Cycloalkanoyl-, Benzoyloder Phenylsulfonylgruppe, durch eine Carboxy-C₁₋₃-alkylcarbonyl- oder C₁₋₃-Alkoxycarbonyl-C₁₋₃-alkylcarbonylgruppe, in der der Alkylteil der Alkylcarbonylgruppe jeweils durch eine Amino- oder Trifluoracetylaminogruppe substituiert sein kann, durch eine C₂₋₄-Alkanoylgruppe, die im Alkanoylteil durch eine Amino-, Carboxy-, C₁₋₃-Alkoxycarbonyl-, Carboxy-C₁₋₃-alkoxy-, C₁₋₃-Alkoxycarbonyl-C₁₋₃-alkoxy-, Carboxy-C₁₋₃-alkylaminocarbonyl-, C₁₋₃-Alkoxycarbonyl-C₁₋₃-alkylaminocarbonyl-, N-(C₁₋₃-Alkyl)-carboxy-C₁₋₃-alkylaminocarbonyl- oder N- (C₁₋₃-Alkyl) -C₁₋₃-alkoxycarbonyl-C₁₋₃-alkylaminocarbonylgruppe substituiert sein kann; durch eine Carboxy-C₁₋₂-alkylaminocarbonyl-, C₁₋₃-Alkoxycarbonyl-C₁₋₂-alkylaminocarbonyl-, Carboxy-C₁₋₃-alkylaminocarbonyl-C₁₋₂-alkylaminocarbonyl- oder C₁₋₃-Alkoxycarbonyl-C₁₋₃-alkylaminocarbonyl-C₁₋₂-alkylaminocarbonylgruppe substituiert ist,
eine Formyl-, Pyridylcarbonyl-, Thienylcarbonyl-, Imidazolylcarbonyl-, 1-Methyl-imidazolylcarbonyl-, Thiazolylcarbonyl- oder Indolylcarbonylgruppe,
eine gegebenenfalls durch eine 1 oder 2 Methylgruppen substituierte Benzimidazol-1-yl-, Benzimidazol-1-yl-methyl- oder 5-Oxo-4,5-dihydro-pyrazol-3-ylgruppe,
eine durch eine Phenylgruppe, durch eine Phenylgruppe und eine C₁₋₄-Alkylgruppe oder durch eine oder zwei C₁₋₄-Alkylgruppen substituierte Pyrazol-1-ylgruppe, in der ein Alkylsubstituent gleichzeitig durch eine Carboxy- oder C₁₋₃-Alkoxycarbonylgruppe substituiert sein kann, oder
eine Carbonylgruppe, die
durch eine gegebenenfalls durch eine Carboxy- oder C₁₋₃-Alkoxycarbonylgruppe substituierte C₁₋₅-Alkylgruppe,
durch eine C₃₋₇-Cycloalkylgruppe,
durch eine Amino- oder C₁₋₅-Alkylaminogruppe, die jeweils am Aminstickstoffatom durch eine C₁₋₃-Alkylgruppe, die durch eine C₃₋₇-Cycloalkyl-, Phenyl-, Pyrrolidinyl- oder Pyridinylgruppe oder in 2- oder 3-Stellung durch eine Di-(C₁₋₃-Alkyl)-aminogruppe substituiert sein kann, oder durch eine Di-(C₁₋₃-Alkyl)-aminogruppe substituiert sein kann,
durch eine Carboxy-C₁₋₃-alkylamino- oder C₁₋₃-Alkoxycarbonyl-C₁₋₃-alkylaminogruppe, die jeweils am Aminstickstoffaton durch eine gegebenenfalls durch eine C₁₋₃-Alkylgruppe substituierte Pyrazolylgruppe substituiert ist,
durch eine 3- bis 7-gliedrige Cycloalkyleniminogruppe, die durch eine oder zwei C₁₋₃-Alkylgruppen substituiert sein kann, wobei die vorstehend erwähnten gegebenenfalls durch eine Methylgruppe substituierten Pyrrolidinogruppen zusätzlich durch eine Hydroxymethyl-, Carboxy-, C₁₋₃-Alkoxycarbonyl-, Carboxy-C₁₋₃-alkyl-, C₁₋₃-Alkoxycarbonyl-C₁₋₃-alkyl-, Carboxy-C₁₋₃-alkyloxy-C₁₋₃-alkyl-, C₁₋₃-Alkoxycarbonyl-C₁₋₃-alkyloxy-C₁₋₃-alkyl-, Carboxy-C₁₋₃-alkylamino-C₁₋₃-alkyl-, N- (C₁₋₃-Alkyl)-carboxy-C₁₋₃-alkylamino-C₁₋₃-alkyl-, C₁₋₃-Alkoxycarbonyl-C₁₋₃-alkylamino-C₁₋₃-alkyl-, N- (C₁₋₃-Alkyl)-carboxy-C₁₋₃-alkylamino-C₁₋₃-alkyl- oder N-(C₁₋₃-Alkyl)-C₁₋₃-alkoxycarbonyl-C₁₋₃-alkylamino-C₁₋₃-alkylgruppe substituiert sein können,
durch eine Morpholino-, Piperazino-, 4-Methyl-piperazino-, Piperazino-C₁₋₃-alkyl-, Dihydropyrazolo-, Tetrahydropyrazolo-, Tetrahydroisooxazolo- oder 7-Azabicycloheptylgruppe oder
durch eine gegebenenfalls im Alkylteil durch eine Carboxyoder C₁₋₃-Alkoxycarbonylgruppe substituierte N-(C₁₋₃-Alkyl)-phenyl- oder N-(C₁₋₃-Alkyl)-pyridylaminogruppe substituiert ist,
R₃ ein Wasserstoff-, Fluor-, Chlor- oder Bromatom,
eine Hydroxy-, C₁₋₃-Alkoxy-, Trifluormethyl-, Amino- oder C₂₋₃-Alkanoylaminogruppe,
eine C₁₋₃-Alkylgruppe, die durch eine Hydroxy-, Carboxy-, C₁₋₃-Alkoxycarbonyl-, Carboxy-C₁₋₃-alkoxy-, C₁₋₃-Alkoxycarbonyl-C₁₋₃-alkoxy-, Carboxy-C₁₋₃-alkylaminocarbonyl-, C₁₋₃-Alkoxycarbonyl-C₁₋₃-alkylaminocarbonyl-, N-(C₁₋₃-Alkyl)-carboxy-C₁₋₃-alkylaminocarbonyl- oder N- (C₁₋₃-Alkyl)-C₁₋₃-alkoxycarbonyl-C₁₋₃-alkylaminocarbonylgruppe substituiert sein kann,
eine C₁₋₃-Alkylgruppe, die durch eine Carboxy-C₁₋₃-alkylamino-, C₁₋₃-Alkoxycarbonyl-C₁₋₃-alkylamino-, N- (C₁₋₃-Alkyl)-carboxy-C₁₋₃-alkylamino- oder C₁₋₃-Alkoxycarbonyl-C₁₋₃-alkylaminocarbonylgruppe substituiert ist,
eine durch eine Carboxy- oder C₁₋₃-Alkoxycarbonylgruppe substituierte C₂₋₃-Alkenylgruppe oder
eine gegebenenfalls am Kohlenstoffatom durch eine C₁₋₃-Alkylgruppe substituierte Carbiminogruppe, die am Iminostickstoffatom durch eine Carboxy-C₁₋₃-alkoxy-, C₁₋₃-Alkoxycarbonyl-C₁₋₃-alkoxy- oder Aminocarbonylaminogruppe substituiert ist,
oder R₂ und R₃ zusammen eine -CO-O-CH₂-Gruppe und
R₄ ein Wasserstoff-, Fluor-, Chlor- oder Bromatom, eine C₁₋₃-Alkyl- oder Trifluormethylgruppe darstellen,
X ein Sauerstoff- oder Schwefelatom oder eine gegebenenfalls durch eine C₁₋₃-Alkylgruppe substituierte -NH-Gruppe und
Y eine durch eine Aminogruppe substituierte Cyclohexylgruppe, eine durch eine Amidinogruppe, welche durch eine Benzoyl- oder C₁₋₈-Alkoxycarbonylgruppe substituiert sein kann, substituierte Phenylen- oder Pyridinylengruppe, wobei die vorstehend erwähnte Phenylengruppe durch eine Methyl- oder Methoxygruppe und die vorstehend erwähnte Pyridinylengruppe durch eine Methylgruppe substituiert sein kann,
deren Tautomere, deren Stereoisomere, deren Gemische und deren Salze.

3. Verbindungen der allgemeinen Formel 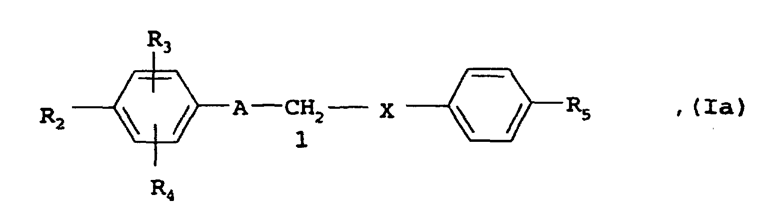 gemäß Anspruch 1, in der
A eine Ethylen- oder Ethinylengruppe,
X ein Sauerstoffatom oder eine gegebenenfalls durch eine Methylgruppe substituierte Iminogruppe,
R₂ eine C₁₋₄-Alkylcarbonylamino- oder C₃₋₅-Cycloalkylcarbonylaminogruppe, die jeweils am Aminstickstoffatom durch eine Carboxy-C₁₋₂-alkyl-, C₁₋₃-Alkoxycarbonyl-C₁₋₂-alkyl-, Carboxy-C₁₋₂-alkylaminocarbonyl-C₁₋₂-alkyl- oder C₁₋₃-Alkoxycarbonyl-C₁₋₂-alkylaminocarbonyl-C₁₋₂-alkylgruppe substituiert ist,
eine C₁₋₄-Alkylamino- oder C₃₋₅-Cycloalkylaminogruppe, die jeweils am Aminstickstoffatom durch eine durch eine gegebenenfalls im Alkylteil durch eine Aminogruppe substituierte Carboxy-C₁₋₃-alkylcarbonyl-, C₁₋₃-Alkoxycarbonyl-C₁₋₃-alkylcarbonyl-, Carboxy-C₁₋₂-alkylaminocarbonyl-C₁₋₂-alkylcarbonyl-, C₁₋₃-Alkoxycarbonyl-C₁₋₂-alkylaminocarbonyl-C₁₋₂-alkylcarbonyl-, Carboxy-C₁₋₂-alkylaminocarbonyl-, C₁₋₃-Alkoxycarbonyl-C₁₋₂-alkylaminocarbonyl-, Carboxy-C₁₋₂-alkylaminocarbonyl-C₁₋₂-alkylaminocarbonyl- oder C₁₋₃-Alkoxycarbonyl-C₁₋₂-alkylaminocarbonyl-C₁₋₂-alkylaminocarbonylgruppe, durch eine Carboxymethyloxymethylcarbonyl-, C₁₋₃-Alkoxycarbonyl-methyloxymethylcarbonyl-, Carboxymethylaminomethylcarbonyl-, C₁₋₃-Alkoxycarbonyl-methylaminomethylcarbonyl-, N-Methyl-carboxymethylaminomethylcarbonyl-, N-Methyl-C₁₋₃-alkoxycarbonyl-carboxymethylaminomethylcarbonyl-, Aminomethylcarbonyl-, 2-Amino-ethylcarbonyl-, Carboxy-C₁₋₂-alkylaminocarbonylmethyloxymethylcarbonyl-, C₁₋₃-Alkoxycarbonyl-C₁₋₂-alkylaminocarbonylmethyloxymethylcarbonyl-, Carboxy-C₁₋₂-al kylaminocarbonylmethylaminomethylcarbonyl-, C₁₋₃-Alkoxycarbonyl-C₁₋₂-alkylaminocarbonylmethylaminomethylcarbonyl-, N-Methyl-carboxy-C₁₋₂-alkylaminocarbonylmethylaminomethylcarbonyloder N-Methyl-C₁₋₃-alkoxycarbonyl-C₁₋₂-alkylaminocarbonylmethylaminomethylcarbonylgruppe substituiert ist, oder
eine Carbonylgruppe, die
durch eine Cyclopentylgruppe,
durch eine C₃₋₅-Alkylgruppe, die zusätzlich durch eine Carboxy- oder C₁₋₃-Alkoxycarbonylgruppe substituiert sein kann,
durch eine am Aminstickstoffatom durch eine C₁₋₄-Alkyl-, Carboxy-C₁₋₃-alkyl- oder C₁₋₃-Alkoxycarbonyl-C₁₋₃-alkylgruppe substituierte C₁₋₄-Alkylamino-, Phenylamino- oder Pyridylaminogruppe,
durch eine durch eine Methyl-, Hydroxymethyl-, Amino-, Carboxy-, C₁₋₃-Alkoxycarbonyl-, Carboxy-C₁₋₂-alkyl-, C₁₋₃-Alkoxycarbonyl-C₁₋₂-alkyl-, Carboxymethyloxymethyl-, C₁₋₃-Alkoxycarbonylmethyloxymethyl-, Carboxymethylaminomethyl-, C₁₋₃-Alkoxycarbonyl-methylaminomethyl-, Carboxymethylaminocarbonylmethyloxymethyl- oder C₁₋₃-Alkoxycarbonylmethylaminocarbonylmethyloxymethylgruppe substituierte Pyrrolidinogruppe substituiert ist,
R₃ ein Wasserstoff-, Fluor-, Chlor- oder Bromatom oder eine Trifluormethylgruppe,
eine gegebenenfalls durch eine Hydroxy-, Carboxy-, C₁₋₃-Alkoxycarbonyl-, Carboxymethyloxy-, C₁₋₃-Alkoxycarbonyl-methyloxy-, Carboxymethylamino-, N-Methyl-carboxymethylamino-, C₁₋₃-Alkoxycarbonylmethylamino-, N-Methyl-C₁₋₃-alkoxycarbonylmethylamino-, Carboxymethylaminocarbonyl- oder C₁₋₃-Alkoxycarbonylmethylaminocarbonylgruppe substituierte C₁₋₂-Alkylgruppe, eine durch eine Carboxy- oder C₁₋₃-Alkoxycarbonylgruppe substituierte Vinylgruppe,
R₄ ein Wasserstoff-, Fluor-, Chlor- oder Bromatom, eine Methyl-, Ethyl- oder Trifluormethylgruppe und
R₅ eine gegebenenfalls durch eine C₁₋₈-Alkoxycarbonyl- oder Benzoylgruppe substituierte Amidinogruppe bedeuten,
deren Tautomere, deren Stereoisomere, deren Gemische und deren Salze.

4. Verbindungen der allgemeinen Formel Ia gemäß Anspruch 3, in der
A eine Ethylen- oder Ethinylengruppe,
X eine Iminogruppe,
R₂ eine C₁₋₄-Alkylaminocarbonylgruppe, die jeweils am Aminstickstoffatom durch eine Carboxy-C₁₋₂-alkyl- oder C₁₋₃-Alkoxycarbonyl-C₁₋₂-alkylgruppe substituiert ist,
eine C₁₋₄-Alkylaminogruppe, die am Aminstickstoffatom durch eine Carboxy-C₁₋₃-alkylcarbonyl-, C₁₋₃-Alkoxycarbonyl-C₁₋₃-alkylcarbonyl-, Carboxy-C₁₋₂-alkylaminocarbonyl- oder C₁₋₃-Alkoxycarbonyl-C₁₋₂-alkylaminocarbonylgruppe gruppe substituiert ist, oder
eine Carbonylgruppe, die
durch eine C₃₋₅-Alkylgruppe, die zusätzlich durch eine Carboxy- oder C₁₋₃-Alkoxycarbonylgruppe substituiert sein kann,
durch eine am Aminstickstoffatom durch eine Carboxy-C₁₋₃-alkyl- oder C₁₋₃-Alkoxycarbonyl-C₁₋₃-alkylgruppe substituierte C₁₋₄-Alkylamino- oder Pyridylaminogruppe,
durch eine gegebenenfalls durch eine Methylgruppe substituierte Pyrrolidinogruppe substituiert ist,
R₃ eine gegebenenfalls durch eine Carboxy- oder C₁₋₃-Alkoxycarbonylgruppe substituierte C₁₋₂-Alkylgruppe,
R₄ ein Wasserstoffatom oder eine Methylgruppe und
R₅ eine gegebenenfalls durch eine C₁₋₈-Alkoxycarbonyl- oder Benzoylgruppe substituierte Amidinogruppe bedeuten,
deren Tautomere, deren Stereoisomere, deren Gemische und deren Salze.

5. Folgende Verbindungen der allgemeinen Formel I gemäß Anspruch 1, 3 oder 4:
(a) rac-4-{3-[5-Ethoxycarbonylmethyl-2-methyl-4-(2-methylpyrrolidinocarbonyl)-phenyl]-propargylamino}benzamidin,
(b) rac-4-{3-[2,5-Dimethyl-4-(2-methyl-pyrrolidinocarbonyl)-phenyl]-propargylamino}benzamidin,
(c) 4-[3-(2,5-Dimethyl-4-isopropylcarbonyl-phenyl)propargylamino]benzamidin,
(d) 4-{3-[2,5-Dimethyl-4-(N-methyl-N-pyridin-2-yl-aminocarbonyl)-phenyl]propargylamino}benzamidin,
(e) 4-{3-[2,5-Dimethyl-4-(N-methyl-N-pyridin-2-yl-aminocarbonyl)-phenyl]prop-1-ylamino}benzamidin,
(f) 4-[3-(3-Methyl-4-pyrrolidinocarbonyl-phenyl)-propargylamino]-benzamidin,
(g) 4-{3-[2,5-Dimethyl-4-(N-(2-methoxycarbonyl-ethyl)-N-ethylcarbonylamino)-phenyl]-propargylamino}benzamidin,
(h) 4-{3-[2,5-Dimethyl-4-(N-isopropyl-N-hydroxycarbonylmethylaminocarbonyl-amino)-phenyl]-propargylamino}-benzamidin und
(i) 4-{3-[2,5-Dimethyl-4-(N-isopropyl-N-hydroxycarbonylmethylcarbonyl-amino)-phenyl]-propargylamino}-benzamidin
sowie deren Salze.

6. Physiologisch verträgliche Salze der Verbindungen gemäß den Ansprüchen 1 bis 5, in denen Y keine Cyanogruppe enthält.

7. Arzneimittel, enthaltend eine Verbindung nach mindestens einem der Ansprüche 1 bis 5, in denen Y keine Cyanogruppe enthält, oder ein Salz gemäß Anspruch 6 neben gegebenenfalls einem oder mehreren inerten Trägerstoffen und/oder Verdünnungsmitteln.

8. Verwendung einer Verbindung nach mindestens einem der Ansprüche 1 bis 5, in denen Y keine Cyanogruppe enthält, oder ein Salz gemäß Anspruch 6 zur Herstellung eines Arzneimittels mit einer die Thrombinzeit verlängernder Wirkung, einer thrombinhemmender Wirkung und einer Hemmwirkung auf verwandte Serinproteasen.

9. Verfahren zur Herstellung eines Arzneimittels gemäß Anspruch 7, **dadurch gekennzeichnet, daß** auf nichtchemischem Wege eine Verbindung nach mindestens einem der Ansprüche 1 bis 5, in denen Y keine Cyanogruppe enthält, oder ein Salz gemäß Anspruch 6 in einen oder mehrere inerte Trägerstoffe und/oder Verdünnungsmittel eingearbeitet wird.

10. Verfahren zur Herstellung der Verbindungen gemäß den Ansprüchen 1 bis 6, **dadurch gekennzeichnet, daß**
a. eine Verbindung der allgemeinen Formel
Ar - Z₁ , (II)
in der
Ar wie in den Ansprüchen 1 bis 5 erwähnt definiert ist und
Z₁ eine Austrittsgruppe darstellt, mit einer Verbindung der allgemeinen Formel
H - A' - HCR₁ - X - Y' , (III)
in der
R₁ und X wie in den Ansprüchen 1 bis 5 erwähnt definiert sind, Y' die für Y wie in den Ansprüchen 1 bis 5 erwähnten Bedeutungen mit der Maßgabe besitzt, daß eine vorhandene Amino- oder Iminogruppe durch einen üblichen Schutzrest geschützt ist, und A' eine Ethinylgruppe darstellt, umgesetzt und eine so erhaltene Verbindung gegebenenfalls anschließend katalytisch hydriert und/oder von so einer erhaltenen Verbindung ein verwendeter Schutzrest abgespalten wird oder
b. zur Herstellung einer Verbindung der allgemeinen Formel I, in der der Ar-A-Rest eine Carboxygruppe enthält und R₅ wie in den Ansprüchen 1 bis 5 erwähnt definiert ist oder der Ar-A-Rest wie in den Ansprüchen 1 bis 5 erwähnt definiert ist und R₅ eine Amino-, Amino-C₁₋₃-alkyl-, Amidino- oder Guanidinogruppe darstellt oder der Ar-A-Rest eine Carboxygruppe enthält und R₅ eine Amino-, Amino-C₁₋₃-alkyl-, Amidino- oder Guanidinogruppe darstellt, eine Verbindung der allgemeinen Formel
Ar' - A - HCR₁ - X - Y" , (IV)
in der
A, R₁, und X wie in den Ansprüchen 1 bis 5 erwähnt definiert sind,
Ar' und Y" die für Ar und Y in den Ansprüchen 1 bis 5 erwähnten Bedeutungen mit der Maßgabe besitzen, daß
Ar' eine durch Hydrolyse, Behandeln mit einer Säure oder Base, Thermolyse oder Hydrogenolyse in eine Carboxygruppe überführbare Gruppe enthält und Y" die für Y in den Ansprüchen 1 bis 5 erwähnt Bedeutungen aufweist oder
Ar' die für Ar in den Ansprüchen 1 bis 5 erwähnten Bedeutungen aufweist und Y" eine durch Hydrolyse, Behandeln mit einer Säure oder Base, Thermolyse oder Hydrogenolyse in eine Amino-, Amino-C₁₋₃-alkyl-, Amidino- oder Guanidinogruppe überführbare Gruppe oder
Ar' eine durch Hydrolyse, Behandeln mit einer Säure oder Base, Thermolyse oder Hydrogenolyse in eine Carboxygruppe überführbare Gruppe enthält und Y" eine durch Hydrolyse, Behandeln mit einer Säure oder Base, Thermolyse oder Hydrogenolyse in eine Amino-, Amino-C₁₋₃-alkyl-, Amidino- oder Guanidinogruppe überführbare Gruppe enthalten,
mittels Hydrolyse, Behandeln mit einer Säure oder Base, Thermolyse oder Hydrogenolyse in eine Verbindung der allgemeinen Formel I übergeführt wird, in der der Ar-A-Rest eine Carboxygruppe enthält und R₅ wie in den Ansprüchen 1 bis 5 erwähnt definiert ist oder der Ar-A-Rest wie in den Ansprüchen 1 bis 5 erwähnt definiert ist und R₅ eine Amino-, Amino-C₁₋₃-alkyl-, Amidino- oder Guanidinogruppe darstellt oder der Ar-A-Rest eine Carboxygruppe enthält und R₅ eine Amino-, Amino-C₁₋₃-alkyl-, Amidino- oder Guanidinogruppe darstellt, übergeführt wird oder
c. zur Herstellung einer Verbindung der allgemeinen Formel I, in der R₅ eine Amidinogruppe darstellt, eine gegebenenfalls im Reaktionsgemisch gebildete Verbindung der allgemeinen Formel
Ar - A - HCR₁ - X - Y" , (V)
in der
A, Ar, R₁ und X wie in den Ansprüchen 1 bis 5 erwähnt definiert sind und
Y" einen der für Y in den Ansprüchen 1 bis 5 erwähnten Reste mit der Maßgabe bedeutet, daß R₅ eine Z₁-(HN=)C-Gruppe darstellt, in der
Z₁ eine Alkoxy-, Alkylthio-, Aralkoxy- oder Aralkylthiogruppe darstellt,
mit einem Ammoniumsalz umgesetzt wird, wobei gleichzeitig an eine elektronenreiche oder elektronenarme Dreifachbindung Halogenwasserstoff addiert werden kann, oder
d) zur Herstellung einer Verbindung der allgemeinen Formel I, in der R₅ eine Amidinogruppe darstellt, die durch eine Hydroxygruppe substituiert ist, eine gegebenenfalls im Reaktionsgemisch gebildete Verbindung der allgemeinen Formel
Ar - A - HCR₁ - X - Y" , (V)
in der
A, Ar, R₁ und X wie in den Ansprüchen 1 bis 5 erwähnt definiert sind und
Y" einen der für Y in den Ansprüchen 1 bis 5 erwähnten Reste mit der Maßgabe bedeutet, daß R₅ eine Z₁-(HN=)C-Gruppe darstellt, in der
Z₁ eine Alkoxy-, Alkylthio-, Aralkoxy- oder Aralkylthiogruppe darstellt,
mit Hydroxylamin oder dessen Salzen umgesetzt wird oder
e. zur Herstellung einer Verbindung der allgemeinen Formel I, in der X ein Sauerstoff- oder Schwefelatom, eine Carbonyl-, Imino- oder N-(C₁₋₃-Alkyl)-iminogruppe darstellt, eine Verbindung der allgemeinen Formel I
Ar - A - HCR₁ - Z₂ , (VI)
in der
A, Ar und R₁ wie in den Ansprüchen 1 bis 5 erwähnt definiert sind und
Z₂ eine Austrittsgruppe darstellt, mit einer Verbindung der allgemeinen Formel
U - Y , (VII)
in der
Y wie in den Ansprüchen 1 bis 5 erwähnt definiert ist und
U eine Hydroxy-, Mercapto-, Hydroxycarbonyl-, Imino- oder N-(C₁₋₃-Alkyl)-iminogruppe bedeutet, umgesetzt wird oder
f. zur Herstellung einer Verbindung der allgemeinen Formel I, in der Ar und/oder Y einen in-vivo abspaltbaren Rest enthalten, eine Verbindung der allgemeinen Formel
Ar" - A - HCR₁ - X - Y'" , (VIII)
in der
A, R₁, und X wie in den Ansprüchen 1 bis 5 erwähnt definiert sind,
Ar" und Y'" die für Ar und Y in den Ansprüchen 1 bis 5 erwähnten Bedeutungen mit der Maßgabe besitzen, daß
Ar" eine Carboxygruppe enthält und Y'" die für Y in den Ansprüchen 1 bis 5 erwähnt Bedeutungen aufweist oder
Ar" die für Ar in den Ansprüchen 1 bis 5 erwähnten Bedeutungen aufweist und
Y' " eine Amino-, Amino-C₁₋₃-alkyl-, Amidino- oder Guanidinogruppe enthält oder
Ar" eine Carboxygruppe und Y'" eine Amino-, Amino-C₁₋₃-alkyl-, Amidino- oder Guanidinogruppe überführbare Gruppe enthalten, mit einer Verbindung der allgemeinen Formel
Z₃ - R₇ , (IX)
in der
R₇ eine C₁₋₈-Alkoxycarbonylgruppe, eine RₐCO-O-(R_{b}CR_{c})-Gruppe oder den Acylrest einer der in den Ansprüchen 1 bis 5 erwähnten in vivo abspaltbaren Reste, wobei Rₐ bis R_{c} wie in den Ansprüchen 1 bis 5 erwähnt definiert sind, und
Z₃ eine nukleofuge Austrittsgruppe oder auch, wenn Ar" eine Carboxygruppe enthält, eine Hydroxygruppe bedeuten, umgesetzt wird und
gewünschtenfalls anschließend eine so erhaltene Verbindung der allgemeinen Formel I, in der R₅ eine Amidinogruppe darstellt, durch Alkylierung mit einem Halogenessigsäurederivat, durch anschließende Hydrolyse und Decarboxylierung in eine durch eine oder zwei Methylgruppen substituierte entsprechende Amidinoverbindung übergeführt wird und/oder
eine so erhaltene Verbindung der allgemeinen Formel I, in der R₅ eine Hydroxyamidinogruppe darstellt, mittels katalytischer Hydrierung in eine entsprechende Amidinoverbindung übergeführt wird und/oder
eine so erhaltene Verbindung der allgemeinen Formel I, die eine Doppel- oder Dreifachbindung enthält, mittels katalytischer Hydrierung in eine entsprechende gesättigte Verbindung übergeführt wird und/oder
eine so erhaltene Verbindung der allgemeinen Formel I, in der X ein Schwefelatom darstellt, mittels Oxidation in eine entsprechende Sulfinyl- oder Sulfonylverbindung übergeführt wird und/oder
eine so erhaltene Verbindung der allgemeinen Formel I, in der R₂ eine Tetrahydropyrazolocarbonylgruppe darstellt, mittels Oxidation in eine entsprechende 4,5-Dihydropyrazolocarbonyl-Verbindung übergeführt wird und/oder
eine so erhaltene Verbindung der allgemeinen Formel I, die eine Carbonylgruppe enthält, mittels einem entsprechenden Oxim in eine entsprechende Oximverbindung übergeführt wird und/oder
eine so erhaltene Verbindung der allgemeinen Formel I, die eine Carboxygruppe enthält, mittels eines entsprechenden Amins in ein entsprechendes Amid übergeführt wird und/oder
erforderlichenfalls ein während den Umsetzungen zum Schutze von reaktiven Gruppen verwendeter Schutzrest abgespalten wird und/oder
gewünschtenfalls anschließend eine so erhaltene Verbindung der allgemeinen Formel I in ihre Stereoisomere aufgetrennt wird und/oder
eine so erhaltene Verbindung der allgemeinen Fotmel I, die eine Doppelbindung enthält in ihre cis/trans-Isomere aufgetrennt wird und/oder
eine so erhaltene Verbindung der allgemeinen Formel I in ihre Salze, insbesondere für die pharmazeutische Anwendung in ihre physiologisch verträglichen Salze mit einer anorganischen oder organischen Säure oder Base, übergeführt wird.

## Claims

1. Substituted aryl and heteroaryl derivatives of general formula
Ar - A - (HCR₁) - X - Y , (I)
wherein
A denotes an ethynylene group, a vinylene or ethylene group optionally substituted by a C₁₋₃-alkyl or carboxy-C₁₋₃-alkyl group or by a chlorine, bromine or iodine atom,
R₁ denotes a hydrogen atom, a C₁₋₃-alkyl or carboxy-C₁₋₃-alkyl group,
Ar denotes a phenyl group substituted by the groups R₂ to R₄, wherein
R₂ denotes a C₁₋₆-alkyl- or C₃₋₇-cycloalkyl-C₁₋₃-alkyl group, which may be substituted in the C₁₋₆- and C₁₋₃-alkyl moieties by a carboxy, phenyl, amino, C₁₋₃-alkylamino, carboxy-C₁₋₃-alkylamino, di-(C₁₋₃-alkyl)-amino, N-(carboxy-C₁₋₃-alkyl)-C₁₋₃-alkylamino, C₃₋₇-cycloalkylamino, phenylamino, N- (C₁₋₃-alkyl) -phenylamino, N- (C₁₋₄-alkanoyl) -phenylamino, heteroarylamino, N-(C₁₋₃-alkyl)-heteroarylamino, N-(carboxy-C₁₋₃-alkyl) -phenylamino or N- (carboxy-C₁₋₃-alkyl)-heteroarylamino group,
a carboxy-C₁₋₅-alkyl group which is substituted in the alkyl moiety by a C₁₋₃-alkylamino, N,N-di-(C₁₋₃-alkyl)-amino, pyrrolidino, piperidino or hexamethyleneimino group,
a carboxy-C₁₋₅-alkyl group wherein the hydrogen atoms of a methylene group are replaced by a n-C₂₋₅-alkylene bridge,
a phenyl, phenyloxy or phenylsulphonyl group, which may be substituted in each case in the phenyl moiety by a fluorine, chlorine, bromine or iodine atom, by a C₁₋₃-alkyl, carboxy-C₁₋₃-alkyl or C₁₋₃-alkoxy group,
a C₁₋₅-alkylamino, carboxy-C₁₋₃-alkylamino, di-(C₁₋₅-alkyl)-amino, N-(carboxy-C₁₋₃-alkyl)-C₁₋₅-alkylamino, C₃₋₇-cycloalkylamino, N-(carboxy-C₁₋₃-alkyl)-C₃₋₇-cyclo-alkylamino, phenylamino, N-(C₁₋₃-alkyl)-phenylamino, N-(carboxy-C₁₋₃-alkyl)-phenylamino, heteroarylamino, N- (C₁₋₃-alkyl)-heteroarylamino or N- (carboxy-C₁₋₃-alkyl)-heteroarylamino group,
a C₁₋₅-alkylcarbonylamino, C₃₋₇-cycloalkylcarbonylamino, arylcarbonylamino, heteroarylcarbonylamino, C₁₋₅-alkylsulphonylamino, arylsulphonylamino, heteroarylsulphonylamino, N-(C₁₋₃-alkyl)-C₁₋₅-alkylcarbonylamino, N- (C₁₋₃-alkyl)-C₃₋₇-cycloalkylcarbonylamino, N-(C₁₋₃-alkyl)-arylcarbonylamino, N-(C₁₋₃-alkyl)-heteroarylcarbonylamino, N- (C₁₋₃-alkyl)-C₁₋₅-alkylsulphonylamino, N- (C₁₋₃-alkyl)-arylsulphonylamino or N-(C₁₋₃-alkyl)-heteroarylsulphonylamino group, whilst
the above-mentioned N-(C₁₋₃-alkyl) moieties may additionally be substituted by a carboxy, carboxy-C₁₋₃-alkylaminocarbonyl or N-(C₁₋₃-alkyl)-carboxy-C₁₋₃-alkylaminocarbonyl group or, with the exception of the α-carbon atom in relation to the nitrogen atom, by a hydroxy, carboxy-C₁₋₃-alkoxy, amino, carboxy-C₁₋₃-alkylamino or N- (C₁₋₃-alkyl)-carboxy-C₁₋₃-alkylamino group,
a 5- to 7-membered cycloalkyleneimino group,
an amino, C₁₋₅-alkylamino, C₃₋₇-cycloalkylamino, arylamino, aryl-C₁₋₃-alkylamino, heteroarylamino or heteroaryl-C₁₋₃-alkyl-amino group, which are substituted in each case at the amino nitrogen atom by a C₁₋₃-alkylcarbonyl, carboxy-C₁₋₃-alkylcarbonyl, carboxy-C₁₋₃-alkylaminocarbonyl, 2-oxopyrrolidinylcarbonyl or piperazinocarbonyl group, whilst additionally
(i) the above-mentioned amino group, which is monosubstituted by a C₁₋₃-alkylcarbonyl, carboxy-C₁₋₃-alkylcarbonyl or carboxy-C₁₋₃-alkylaminocarbonyl group, is substituted by a 5- to 7-membered cycloalkyleneimino group or by a N,N-di-(C₁₋₅-alkyl)-amino group, and
(ii) the alkyl moiety of the above-mentioned C₁₋₃-alkylcarbonyl group is substituted by a carboxy, amino, hydroxy, carboxy-C₁₋₃-alkoxy, carboxy-C₁₋₃-alkylaminocarbonyl, carboxy-C₁₋₃-alkylamino, N-(C₁₋₃-alkyl)-carboxy-C₁₋₃-alkylamino or amino-C₁₋₃-alkylcarbonylamino group or by a carboxy or hydroxy group and by an amino or trifluoroacetylamino group,
a carbimino group which is substituted at the nitrogen atom by a carboxy-C₁₋₃-alkoxy, amino, C₁₋₃-alkylamino, carboxy-C₁₋₃-alkylamino, di-(C₁₋₃-alkyl)-amino or N- (carboxy-C₁₋₃-alkyl)-C₁₋₃-alkylamino group and at the carbon atom by a C₁₋₅-alkyl group, by a phenyl group optionally substituted by a C₁₋₃-alkyl or C₁₋₃-alkoxy group or by a heteroaryl group optionally substituted by a C₁₋₃-alkyl group,
a heteroaryl or heteroaryl-C₁₋₃-alkyl group, which may in each case additionally be substituted in the heteroaryl moiety by a phenyl or heteroaryl group or by a phenyl or heteroaryl group and by a carboxy-C₁₋₃-alkyl or C₁₋₃-alkoxycarbonyl-C₁₋₃-alkyl group,
a 5-oxo-4,5-dihydro-pyrazolyl or 6-oxo-4,5-dihydropyridazinyl group optionally substituted by 1 to 3 C₁₋₃-alkyl groups wherein an alkyl substituent may at the same time be substituted by a carboxy or C₁₋₃-alkoxycarbonyl group, or a carbonyl group which is substituted
by a hydrogen atom, by a hydroxy, C₁₋₅-alkoxy or C₃₋₇-cycloalkoxy group,
by a C₁₋₅-alkyl or C₃₋₇-cycloalkyl group optionally substituted by a carboxy group,
by a C₁₋₃-alkyl group substituted by a piperazino group,
by a phenyl group which may be substituted by a fluorine, chlorine, bromine or iodine atom, by a C₁₋₃-alkyl, carboxy-C₁₋₃-alkyl, C₁₋₃-alkoxy or carboxy group,
by an amino, C₁₋₅-alkylamino, carboxy-C₁₋₃-alkylamino, C₃₋₇-cycloalkylamino, phenylamino or heteroarylamino group, each of which may additionally be substituted at the amino nitrogen atom by a C₁₋₅-alkyl, C₃₋₇-cycloalkyl, phenyl-C₁₋₃-alkyl, carboxy-C₁₋₃-alkyl, 2-[di-(C₁₋₃-alkyl)-amino]-ethyl, 3-[di-(C₁₋₃-alkyl)-amino]-propyl, di- (C₁₋₃-alkyl)-amino, 2- (N-carboxy-C₁₋₃-alkyl-C₁₋₃-alkylamino)-ethyl, 3-(N-carboxy-C₁₋₃-alkyl-C₁₋₃-alkylamino)-propyl or N-carboxy-C₁₋₃-alkyl-C₁₋₃-alkylamino, phenyl, pyridyl, pyrrolidinyl or piperidinyl group,
by a pyrrolyl, thienyl, imidazolyl, pyrazolyl, thiazolyl, pyridinyl, pyrimidinyl, pyrazinyl or pyridazinyl group optionally substituted by one or two C₁₋₃-alkyl groups onto which a phenyl ring may be fused via two adjacent carbon atoms,
by a C₃₋₆-cycloalkyleneimino, C₅₋₈-bicycloalkyleneimino, morpholino, piperazino, dihydropyrazolo, tetrahydropyrazolo, tetrahydroisoxazolo, tetrahydropyrazinyl or tetrahydropyridazinyl group optionally substituted by a C₁₋₃-alkyl or carboxy-C₁₋₃-alkyl group or
by a C₃₋₆-cycloalkyleneimino group optionally substituted by a C₁₋₃-alkyl, carboxy-C₁₋₃-alkyl, hydroxy, hydroxy-C₁₋₃-alkyl, amino, carboxy, carboxy-C₁₋₃-alkoxy-C₁₋₃-alkyl, carboxy-C₁₋₃-alkylamino-C₁₋₃-alkyl or carboxy-C₁₋₃-alkylaminocarbonyl-C₁₋₃-alkyl group,
by a C₅₋₈-bicycloalkyleneimino, morpholino, piperazino, dihydropyrazolo, tetrahydropyrazolo, tetrahydroisoxazolo, tetrahydropyrazinyl or tetrahydropyridazinyl group optionally substituted by a C₁₋₃-alkyl or carboxy-C₁₋₃-alkyl group,
R₃ denotes a hydrogen, fluorine, chlorine, bromine or iodine atom, a formyl or trifluoromethyl group,
a C₁₋₃-alkoxy, amino, C₁₋₃-alkylamino, di-(C₁₋₃-alkyl)-amino, C₁₋₄-alkanoylamino or N-(C₁₋₄-alkanoyl)-C₁₋₃-alkylamino group,
a C₁₋₃-alkyl group optionally substituted by a hydroxy, C₁₋₃-alkoxy, carboxy, carboxy-C₁₋₃-alkoxy, carboxy-C₁₋₃-alkylamino, N- (C₁₋₃-alkyl)-carboxy-C₁₋₃-alkylamino or carboxy-C₁₋₃-alkylaminocarbonyl group,
a C₂₋₃-alkenyl group substituted by a carboxy or carboxy-C₁₋₃-alkylaminocarbonyl group or
a carbimino group optionally substituted at the carbon atom by a C₁₋₃-alkyl group, which is substituted at the imino nitrogen atom by a carboxy-C₁₋₃-alkoxy or aminocarbonylamino group,
or R₂ and R₃ together denote a -CO-O-CH₂- or -CO-O-CH₂CH₂-group and
R₄ denotes a hydrogen, fluorine, chlorine, bromine or iodine atom, a C₁₋₃-alkyl, C₃₋₇-cycloalkyl, trifluoromethyl or C₁₋₃-alkoxy group,
or Ar may also denote a heteroaryl group which may be substituted by the above-mentioned groups R₂ to R₄, which are as hereinbefore defined,
X denotes an oxygen or sulphur atom, a methylene group, a carbonyl, sulphinyl, sulphonyl, imino, N-(C₁₋₃-alkyl)-imino or N-(carboxy-C₁₋₃-alkyl)-imino group optionally substituted by one or two C₁₋₃-alkyl groups, whilst the alkyl moiety of the N-(C₁₋₃-alkyl)-imino group may additionally be substituted in the 2- or 3-position by an amino, C₁₋₃-alkylamino, di-(C₁₋₃-alkyl)-amino, C₁₋₄-alkanoylamino or N-(C₁₋₄-alkanoyl)-C₁₋₃-alkylamino group, and
Y denotes a cyclohexyl group substituted by an amino group or a phenyl or heteroaryl group substituted by the group R₅, whilst the above-mentioned phenyl group may be substituted in each case by a fluorine, chlorine, bromine or iodine atom or by a C₁₋₃-alkyl or C₁₋₃-alkoxy group and the above-mentioned heteroaryl group may be substituted by a C₁₋₃-alkyl group and
R₅ denotes a hydrogen atom, a cyano group or an amino, amino-C₁₋₃-alkyl, amidino, guanidino or guanidino-C₁₋₃-alkyl group optionally substituted by a group which may be cleaved in vivo,
whilst
by the above-mentioned heteroaryl groups is meant a 5-membered heteroaromatic group, optionally substituted by one or two C₁₋₃-alkyl groups, which contains an imino group optionally substituted by a C₁₋₃-alkyl group, an oxygen or sulphur atom or an imino group optionally substituted by a C₁₋₃-alkyl group, an oxygen or sulphur atom and one or two nitrogen atoms and the partially hydrogenated derivatives thereof, particularly the dihydro derivatives thereof, or a 6-membered heteroaromatic group which contains one, two or three nitrogen atoms, whilst additionally a phenyl ring may be fused to the above-mentioned 5- and 6-membered heteroaromatic rings via two adjacent carbon atoms,
the carboxy groups mentioned above in the definition of the groups may be replaced by a tetrazolyl group or by a group which can be converted *in vivo* into a carboxy group, and
the imino or amino groups mentioned above in the definition of the groups may be substituted by a group which can be cleaved *in vivo*,
the tautomers, the stereoisomers, the mixtures thereof and the salts thereof.

2. Compounds of general formula I according to Claim 1, wherein
A denotes a vinylene group optionally substituted by a chlorine, bromine or iodine atom, or an ethylene or ethynylene group,
R₁ denotes a hydrogen atom or a C₁₋₃-alkyl group,
Ar denotes a pyridyl or thienyl group substituted by a benzoyl group,
a bromofuranyl group substituted by a pyrrolidinocarbonyl group or
a phenyl group substituted by the groups R₂ to R₄, whilst
R₂ denotes a phenyl or phenoxy group,
a C₁₋₃-alkyl group which may be substituted by a phenyl, phenylamino, N- (C₁₋₃-alkyl)-phenylamino or N- (C₁₋₃-alkanoyl)-phenylamino group,
a carboxy or C₁₋₃-alkoxycarbonyl group,
a benzoyl or phenylsulphonyl group wherein in each case the phenyl moiety may additionally be substituted by a fluorine, chlorine or bromine atom, by a methyl, methoxy, carboxy or C₁₋₃-alkoxycarbonyl group, whilst in the above-mentioned benzoyl groups the oxygen atom may additionally be replaced by a carboxy-C₁₋₃-alkoxyimino or C₁₋₃-alkoxycarbonyl-C₁₋₃-alkoxyimino group,
a C₁₋₅-alkylamino group which may be substituted in the alkyl moiety by a phenyl, carboxy, C₁₋₃-alkoxycarbonyl, carboxy-C₁₋₃-alkylaminocarbonyl, C₁₋₃-alkoxycarbonyl-C₁₋₃-alkylaminocarbonyl, N-(C₁₋₃-alkyl)-carboxy-C₁₋₃-alkylaminocarbonyl or N-(C₁₋₃-alkyl)-C₁₋₃-alkoxycarbonyl-C₁₋₃-alkylaminocarbonyl group, or a C₃₋₇-cycloalkylamino group, whilst the above-mentioned groups may each additionally be substituted at the amino nitrogen atom by a C₃₋₇-cycloalkanoyl, benzoyl or phenylsulphonyl group, by a carboxy-C₁₋₃-alkylcarbonyl or C₁₋₃-alkoxycarbonyl-C₁₋₃-alkylcarbonyl group wherein the alkyl moiety of the alkylcarbonyl group is substituted in each case by an amino or trifluoroacetylamino group, by a C₂₋₄-alkanoyl group, which may be substituted in the alkanoyl moiety by an amino, carboxy, C₁₋₃-alkoxycarbonyl, carboxy-C₁₋₃-alkoxy, C₁₋₃-alkoxycarbonyl-C₁₋₃-alkoxy, carboxy-C₁₋₃-al-kylaminocarbonyl, C₁₋₃-alkoxycarbonyl-C₁₋₃-alkylaminocarbonyl, N- (C₁₋₃-alkyl)-carboxy-C₁₋₃-alkylaminocarbonyl or N-(C₁₋₃-alkyl)-C₁₋₃-alkoxycarbonyl-C₁₋₃-alkylaminocarbonyl group, by a carboxy-C₁₋₂-alkylaminocarbonyl, C₁₋₃-alkoxycarbonyl-C₁₋₂-alkylaminocarbonyl, carboxy-C₁₋₃-alkylaminocarbonyl-C₁₋₂-alkylaminocarbonyl or C₁₋₃-alkoxycarbonyl-C₁₋₃-alkylaminocarbonyl-C₁₋₂-alkylaminocarbonyl group,
a formyl, pyridylcarbonyl, thienylcarbonyl, imidazolylcarbonyl, 1-methyl-imidazolylcarbonyl, thiazolylcarbonyl or indolylcarbonyl group,
a benzimidazol-1-yl, benzimidazol-1-yl-methyl or 5-oxo-4,5-dihydro-pyrazol-3-yl group optionally substituted by 1 or 2 methyl groups,
a pyrazol-1-yl group substituted by a phenyl group, by a phenyl group and a C₁₋₄-alkyl group or by one or two C₁₋₄-alkyl groups wherein an alkyl substituent may at the same time be substituted by a carboxy or C₁₋₃-alkoxycarbonyl group, or
a carbonyl group which is substituted
by a C₁₋₅-alkyl group optionally substituted by a carboxy or C₁₋₃-alkoxycarbonyl group,
by a C₃₋₇-cycloalkyl group,
by an amino or C₁₋₅-alkylamino group, which are in each case substituted at the amino nitrogen atom by a C₁₋₃-alkyl group which may be substituted by a C₃₋₇-cycloalkyl, phenyl, pyrrolidinyl or pyridinyl group.or in the 2 or 3 position by a di-(C₁₋₃-alkyl)-amino group, or by a di-(C₁₋₃-alkyl)-amino group,
by a carboxy-C₁₋₃-alkylamino or C₁₋₃-alkoxycarbonyl-C₁₋₃-alkylamino group which is in each case substituted at the amino nitrogen atom by a pyrazolyl group optionally substituted by a C₁₋₃-alkyl group, by a 3- to 7-membered cycloalkyleneimino group which may be substituted by one or two C₁₋₃-alkyl groups, whilst the above-mentioned pyrrolidino groups optionally substituted by a methyl group may additionally be substituted by a hydroxymethyl, carboxy, C₁₋₃-alkoxycarbonyl, carboxy-C₁₋₃-alkyl, C₁₋₃-alkoxycarbonyl-C₁₋₃-alkyl, carboxy-C₁₋₃-alkyloxy-C₁₋₃-alkyl, C₁₋₃-alkoxycarbonyl-C₁₋₃-alkyloxy-C₁₋₃-alkyl, carboxy-C₁₋₃-alkylamino-C₁₋₃-alkyl, N-(C₁₋₃-alkyl)-carboxy-C₁₋₃-alkylamino-C₁₋₃-alkyl, C₁₋₃-alkoxycarbonyl-C₁₋₃-alkylamino-C₁₋₃-alkyl, N- (C₁₋₃-alkyl) -carboxy-C₁₋₃-alkylamino-C₁₋₃-alkyl or N- (C₁₋₃-alkyl) -C₁₋₃-alkoxycarbonyl-C₁₋₃-alkylamino-C₁₋₃-alkyl group,
by a morpholino, piperazino, 4-methyl-piperazino, piperazino-C₁₋₃-alkyl, dihydropyrazolo, tetrahydropyrazolo, tetrahydroisoxazolo or 7-azabicycloheptyl group or
by a N- (C₁₋₃-alkyl)-phenyl or N-(C₁₋₃-alkyl)-pyridylamino group optionally substituted in the alkyl moiety by a carboxy or C₁₋₃-alkoxycarbonyl group,
R₃ denotes a hydrogen, fluorine, chlorine or bromine atom,
a hydroxy, C₁₋₃-alkoxy, trifluoromethyl, amino or C₂₋₃-alkanoylamino group,
a C₁₋₃-alkyl group which may be substituted by a hydroxy, carboxy, C₁₋₃-alkoxycarbonyl, carboxy-C₁₋₃-alkoxy, C₁₋₃-alkoxycarbonyl-C₁₋₃-alkoxy, carboxy-C₁₋₃-alkylaminocarbonyl, C₁₋₃-alkoxycarbonyl-C₁₋₃-alkylaminocarbonyl, N-(C₁₋₃-alkyl)-carboxy-C₁₋₃-alkylaminocarbonyl or N-(C₁₋₃-alkyl)-C₁₋₃-alkoxycarbonyl-C₁₋₃-alkylaminocarbonyl group,
a C₁₋₃-alkyl group which is substituted by a carboxy-C₁₋₃-alkylamino, C₁₋₃-alkoxycarbonyl-C₁₋₃-alkylamino, N-(C₁₋₃-alkyl)-carboxy-C₁₋₃-alkylamino or C₁₋₃-alkoxycarbonyl-C₁₋₃-alkylaminocarbonyl group,
a C₂₋₃-alkenyl group substituted by a carboxy or C₁₋₃-alkoxycarbonyl group or
a carbimino group optionally substituted at the carbon atom by a C₁₋₃-alkyl group, which is substituted at the imino nitrogen atom by a carboxy-C₁₋₃-alkoxy, C₁₋₃-alkoxycarbonyl-C₁₋₃-alkoxy or aminocarbonylamino group,
or R₂ and R₃ together denote a -CO-O-CH₂-group and
R₄ denotes a hydrogen, fluorine, chlorine or bromine atom, a C₁₋₃-alkyl or trifluoromethyl group,
X denotes an oxygen or sulphur atom or an -NH-group optionally substituted by a C₁₋₃-alkyl group and
Y denotes a cyclohexyl group substituted by an amino group, a phenylene or pyridinylene group substituted by an amidino group, which may be substituted by a benzoyl or C₁₋₈-alkoxycarbonyl group., whilst the above-mentioned phenylene group may be substituted by a methyl or methoxy group and the above-mentioned pyridinylene group may be substituted by a methyl group,
the tautomers, the stereoisomers, the mixtures thereof and the salts thereof.

3. Compounds of general formula 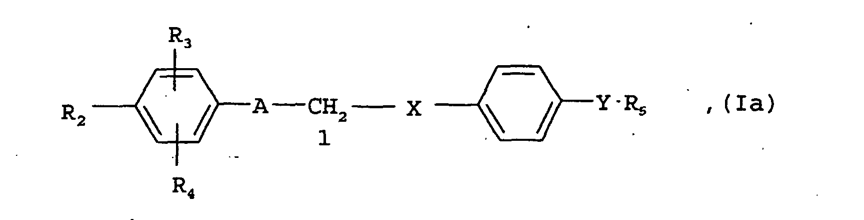 wherein
A denotes an ethylene or ethynylene group,
X denotes an oxygen atom or an imino group optionally substituted by a methyl group,
R₂ denotes a C₁₋₄-alkylcarbonylamino or C₃₋₅-cycloalkylcarbonylamino group, which is substituted in each case at the amino nitrogen atom by a carboxy-C₁₋₂-alkyl, C₁₋₃-alkoxycarbonyl-C₁₋₂-alkyl, carboxy-C₁₋₂-alkylaminocarbonyl-C₁₋₂-alkyl or C₁₋₃-alkoxycarbonyl-C₁₋₂-alkylaminocarbonyl-C₁₋₂-alkyl group,
a C₁₋₄-alkylamino or C₃₋₅-cycloalkylamino group, which is substituted in each case at the amino nitrogen atom by a carboxy-C₁₋₃-alkylcarbonyl, C₁₋₃-alkoxycarbonyl-C₁₋₃-alkylcarbonyl, carboxy-C₁₋₂-alkylaminocarbonyl-C₁₋₂-alkylcarbonyl, C₁₋₃-alkoxycarbonyl-C₁₋₂-alkylaminocarbonyl-C₁₋₂-alkylcarbonyl, carboxy-C₁₋₂-alkylaminocarbonyl, C₁₋₃-alkoxycarbonyl-C₁₋₂-alkylaminocarbonyl, carboxy-C₁₋₂-alkylaminocarbonyl-C₁₋₂-alkylaminocarbonyl or C₁₋₃-alkoxycarbonyl-C₁₋₂-alkylaminocarbonyl-C₁₋₂-alkylaminocarbonyl group optionally substituted in the alkyl moiety by an amino group, or by a carboxymethyloxymethylcarbonyl, C₁₋₃-alkoxycarbonyl-methyloxymethylcarbonyl, carboxymethylaminomethylcarbonyl, C₁₋₃-alkoxycarbonyl-methylaminomethylcarbonyl, N-methyl-carboxymethylaminomethylcarbonyl, N-methyl-C₁₋₃-alkoxycarbonyl-carboxymethylaminomethylcarbonyl, aminomethylcarbonyl, 2-amino-ethylcarbonyl, carboxy-C₁₋₂-alkylaminocarbonylmethyloxymethylcarbonyl, C₁₋₃-alkoxycarbonyl-C₁₋₂-alkylaminocarbonylmethyloxymethylcarbonyl, carboxy-C₁₋₂-alkylaminocarbonylmethylaminomethylcarbonyl, C₁₋₃-alkoxycarbonyl-C₁₋₂-alkylaminocarbonylmethylaminomethylcarbonyl, N-methylcarboxy-C₁₋₂-alkylaminocarbonylmethylaminomethylcarbonyl or N-methyl-C₁₋₃-alkoxycarbonyl-C₁₋₂-alkylaminocarbonylmethylaminomethylcarbonyl group, or
a carbonyl group which is substituted
by a cyclopentyl group,
by a C₃₋₅-alkyl group which may additionally be substituted by a carboxy or C₁₋₃-alkoxycarbonyl group,
by a C₁₋₄-alkylamino, phenylamino or pyridylamino group substituted at the amino nitrogen atom by a C₁₋₄-alkyl, carboxy-C₁₋₃-alkyl or C₁₋₃-alkoxycarbonyl-C₁₋₃-alkyl group,
by a pyrrolidino group substituted by a methyl, hydroxymethyl, amino, carboxy, C₁₋₃-alkoxycarbonyl, carboxy-C₁₋₂-alkyl, C₁₋₃-alkoxycarbonyl-C₁₋₂-alkyl, carboxymethyloxymethyl, C₁₋₃-alkoxycarbonylmethyloxymethyl, carboxymethylaminomethyl, C₁₋₃-alkoxycarbonylmethylaminomethyl, carboxymethylaminocarbonylmethyloxymethyl or C₁₋₃-alkoxycarbonylmethylaminocarbonylmethyloxymethyl group,
R₃ denotes a hydrogen, fluorine, chlorine or bromine atom or a trifluoromethyl group,
a C₁₋₂-alkyl group optionally substituted by a hydroxy, carboxy, C₁₋₃-alkoxycarbonyl, carboxymethyloxy, C₁₋₃-alkoxycarbonyl-methyloxy, carboxymethylamino, N-methylcarboxymethylamino, C₁₋₃-alkoxycarbonylmethylamino, N-methyl-C₁₋₃-alkoxycarbonylmethylamino, carboxymethylaminocarbonyl or C₁₋₃-alkoxycarbonylmethylaminocarbonyl group,
a vinyl group substituted by a carboxy or C₁₋₃-alkoxycarbonyl group,
R₄ denotes a hydrogen, fluorine, chlorine or bromine atom, a methyl, ethyl or trifluoromethyl group and
R₅ denotes an amidino group optionally substituted by a C₁₋₈-alkoxycarbonyl or benzoyl group,
the tautomers, the stereoisomers, the mixtures thereof and the salts thereof.

4. Compounds of general formula Ia according to Claim 3, wherein
A denotes an ethylene or ethynylene group,
X denotes an imino group,
R₂ denotes a C₁₋₄-alkylaminocarbonyl group, which is substituted in each case at the amino nitrogen atom by a carboxy-C₁₋₂-alkyl or C₁₋₃-alkoxycarbonyl-C₁₋₂-alkyl group,
a C₁₋₄-alkylamino group which is substituted at the amino nitrogen atom by a carboxy-C₁₋₃-alkylcarbonyl, C₁₋₃-alkoxycarbonyl-C₁₋₃-alkylcarbonyl, carboxy-C₁₋₂-alkylaminocarbonyl or C₁₋₃-alkoxycarbonyl-C₁₋₂-alkylaminocarbonyl group, or
a carbonyl group which is substituted
by a C₃₋₅-alkyl group which may additionally be substituted by a carboxy or C₁₋₃-alkoxycarbonyl group,
by a C₁₋₄-alkylamino or pyridylamino group substituted at the amino nitrogen atom by a carboxy-C₁₋₃-alkyl or C₁₋₃-alkoxycarbonyl-C₁₋₃-alkyl group,
by a pyrrolidino group optionally substituted by a methyl group,
R₃ denotes a C₁₋₂-alkyl group optionally substituted by a carboxy or C₁₋₃-alkoxycarbonyl group,
R₄ denotes a hydrogen atom or a methyl group and
R₅ denotes an amidino group optionally substituted by a C₁₋₈-alkoxycarbonyl or benzoyl group,
the tautomers, the stereoisomers, the mixtures thereof and the salts thereof.

5. The following compounds of general formula I according to Claim 1, 3 or 4:
(a) rac-4-{3-[5-ethoxycarbonylmethyl-2-methyl-4-(2-methylpyrrolidinocarbonyl)-phenyl]-propargylamino}benzamidine,
(b) rac-4-{3-[2,5-dimethyl-4-(2-methyl-pyrrolidinocarbonyl)-phenyl]-propargylamino}benzamidine,
(c) 4-[3-(2,5-dimethyl-4-isopropylcarbonyl-phenyl)propargylamino]benzamidine,
(d) 4-{3-[2,5-dimethyl-4-(N-methyl-N-pyridin-2-yl-aminocarbonyl)-phenyl]propargylamino}benzamidine,
(e) 4-{3-[2,5-dimethyl-4-(N-methyl-N-pyridin-2-yl-aminocarbonyl)-phenyl]prop-1-ylamino}benzamidine,
(f) 4-[3-(3-methyl-4-pyrrolidinocarbonyl-phenyl)-propargylamino]-benzamidine,
(g) 4-{3-[2,5-dimethyl-4-(N-(2-methoxycarbonyl-ethyl)-N-ethyl-carbonylamino)-phenyl]-propargylamino}benzamidine,
(h) 4-{3-[2,5-dimethyl-4-(N-isopropyl-N-hydroxycarbonylmethylaminocarbonyl-amino)-phenyl]-propargylamino}-benzamidine and
(i) 4-{3-[2,5-dimethyl-4-(N-isopropyl-N-hydroxycarbonylmethylcarbonyl-amino)-phenyl]-propargylamino}-benzamidine
and the salts thereof.

6. Physiologically acceptable salts of the compounds according to claims 1 to 5 wherein Y does not contain a cyano group.

7. Pharmaceutical compositions containing a compound according to at least one of claims 1 to 5 wherein Y does not contain a cyano group, or a salt according to Claim 6 optionally together with one or more inert carriers and/or diluents.

8. Use of a compound according to at least one of claims 1 to 5 wherein Y does not contain a cyano group, or a salt according to Claim 6 for preparing a pharmaceutical composition with the effect of prolonging thrombin time, a thrombin inhibiting effect and an inhibiting effect on related serine proteases.

9. Process for preparing a pharmaceutical composition according to Claim 7, **characterised in that** a compound according to at least one of claims 1 to 5 wherein Y does not contain a cyano group, or a salt according to Claim 6 is incorporated in one or more inert carriers and/or diluents by a non-chemical method.

10. Process for preparing the compounds according to claims 1 to 6, **characterised in that**
a. a compound of general formula
Ar - Z₁ , (II)
wherein
Ar is defined as in claims 1 to 5 and
Z₁ denotes a leaving group, is reacted with a compound of general formula i
H - A' - HCR₁ - X - Y' , (III)
wherein
R₁ and X are defined as in claims 1 to 5,
Y' has the meanings given for Y in claims 1 to 5 with the proviso that any amino or imino group present is protected by a conventional protecting group, and
A' denotes an ethynyl group, and a compound thus obtained is then optionally catalytically hydrogenated and/or any protecting group used is cleaved from a compound thus obtained or
b. in order to prepare a compound of general formula I wherein the Ar-A group contains a carboxy group and R₅ is defined as in claims 1 to 5 or the Ar-A group is defined as in claims 1 to 5 and R₅ denotes an amino, amino-C₁₋₃-alkyl, amidino or guanidino group or the Ar-A group contains a carboxy group and R₅ denotes an amino, amino-C₁₋₃-alkyl, amidino or guanidino group, a compound of general formula
Ar' - A - HCR₁ - X - Y" , (IV)
wherein
A, R₁, and X are defined as in claims 1 to 5,
Ar' and Y" has the meanings given for Ar and Y in claims 1 to 5 with the proviso that
Ar' contains a group which can be converted into a carboxy group by hydrolysis, treatment with an acid or base, thermolysis or hydrogenolysis and Y" has the meanings given for Y in claims 1 to 5 or
Ar' has the meanings given for Ar in claims 1 to 5 and Y" contains a group which can be converted into an amino, amino-C₁₋₃-alkyl, amidino or guanidino group by hydrolysis, treatment with an acid or base, thermolysis or hydrogenolysis or
Ar' contains a group which can be converted into a carboxy group by hydrolysis, treatment with an acid or base, thermolysis or hydrogenolysis and Y" contains a group which can be converted into an amino, amino-C₁₋₃-alkyl, amidino or guanidino group by hydrolysis, treatment with an acid or base, thermolysis or hydrogenolysis,
is converted by hydrolysis, treatment with an acid or base, thermolysis or hydrogenolysis into a compound of general formula I wherein the Ar-A group contains a carboxy group and R₅ is defined as in claims 1 to 5 or the Ar-A group is defined as in claims 1 to 5 and R₅ denotes an amino, amino-C₁₋₃-alkyl, amidino or guanidino group or the Ar-A group contains a carboxy group and R₅ denotes an amino, amino-C₁₋₃-alkyl, amidino or guanidino group, or
c. in order to prepare a compound of general formula I wherein R₅ denotes an amidino group, a compound of general formula
Ar - A - HCR₁ - X - Y" , (V)
optionally formed in the reaction mixture
wherein
A, Ar, R₁ and X are defined as in claims 1 to 5 and
Y" denotes one of the groups mentioned for Y in claims 1 to 5 with the proviso that R₅ denotes a Z₁-(HN=)C- group, wherein
Z₁ denotes an alkoxy, alkylthio, aralkoxy or aralkylthio group,
is reacted with an ammonium salt, whilst at the same time hydrohalic acid may be added to an electron-rich or electron-depleted triple bond, or
d) in order to prepare a compound of general formula I wherein R₅ denotes an amidino group which is substituted by a hydroxy group, a compound of general formula
Ar - A - HCR₁ - X - Y" , (V)
optionally formed in the reaction mixture
wherein
A, Ar, R₁ and X are defined as in claims 1 to 5 and
Y" denotes one of the groups mentioned for Y in claims 1 to 5 with the proviso that R₅ denotes a Z₁-(HN=)C- group, wherein
Z₁ denotes an alkoxy, alkylthio, aralkoxy or aralkylthio group,
is reacted with hydroxylamine or the salts thereof or
e. in order to prepare a compound of general formula I wherein X denotes an oxygen or sulphur atom, a carbonyl, imino or N-(C₁₋₃-alkyl)-imino group, a compound of general formula I
Ar - A - HCR₁ - Z₂ , (VI)
wherein
A, Ar and R₁ are defined as in claims 1 to 5 and
Z₂ denotes a leaving group, is reacted with a compound of general formula
U - Y , (VII)
wherein
Y is defined as in claims 1 to 5 and
U denotes a hydroxy, mercapto, hydroxycarbonyl, imino or N-(C₁₋₃-alkyl)-imino group, or
f. in order to prepare a compound of general formula I wherein Ar and/or Y contain a group which can be cleaved *in vivo,* a compound of general formula
Ar" - A - HCR₁ - X - Y' " , (VIII)
wherein
A, R₁, and X are defined as in claims 1 to 5,
Ar" and Y' " have the meanings given for Ar and Y in claims 1 to 5 with the proviso that
Ar" contains a carboxy group and Y' " has the meanings given for Y in claims 1 to 5 or
Ar" has the meanings given for Ar in claims 1 to 5 and Y' " contains an amino, amino-C₁₋₃-alkyl, amidino or guanidino group or
Ar" contains a carboxy group and Y' " contains a group which may be converted into an amino, amino-C₁₋₃-alkyl, amidino or guanidino group, is reacted with a compound of general formula
Z₃ - R₇ , (IX)
wherein
R₇ denotes a C₁₋₈-alkoxycarbonyl group, an RₐCO-O-(R_{b}CR_{c})-group or the acyl group of one of the groups which can be *cleaved in vivo* mentioned in claims 1 to 5, whilst Rₐ to R_{c} are defined as in claims 1 to 5, and
Z₃ denotes a nucleofugic leaving group or, if Ar" contains a carboxy group, it also denotes a hydroxy group, and
subsequently, if desired, a compound of general formula I thus obtained wherein R₅ denotes an amidino group, is converted by alkylation with a haloacetic acid derivative, by subsequent hydrolysis and decarboxylation into a corresponding amidino compound substituted by one or two methyl groups and/or
a compound of general formula I thus obtained wherein R₅ denotes a hydroxyamidino group, is converted by catalytic hydrogenation into a corresponding amidino compound and/or
a compound thus obtained of general formula I which contains a double or triple bond is converted by catalytic hydrogenation into a corresponding saturated compound and/or
a compound of general formula I thus obtained wherein X denotes a sulphur atom is converted by oxidation into a corresponding sulphinyl or sulphonyl compound and/or
a compound of general formula I thus obtained wherein R₂ denotes a tetrahydropyrazolocarbonyl group is converted by oxidation into a corresponding 4,5-dihydropyrazolocarbonyl compound and/or
a compound thus obtained of general formula I which contains a carbonyl group is converted by means of a corresponding oxime into a corresponding oxime compound and/or
a compound of general formula I thus obtained which contains a carboxy group is converted by means of a corresponding amine into a corresponding amide and/or
if necessary any protecting group used to protect reactive groups during the reactions is cleaved and/or
subsequently, if desired, a compound of general formula I thus obtained is resolved into the stereoisomers thereof and/or
a compound of general formula I thus obtained which contains a double bond is resolved into the cis/trans isomers thereof and/or
a compound of general formula I thus obtained is converted into the salts thereof, particularly, for pharmaceutical use, into the physiologically acceptable salts thereof with an inorganic or organic acid or base.

## Revendications

1. Dérivés d'aryles et d'hétéroaryles substitués de formule générale
Ar-A-(HCR₁)-X-Y (I)
dans laquelle
A est un groupe éthinylène, un groupe vinylène ou éthylène le cas échéant substitué par un groupe alkyle en C₁₋₃ ou carboxy-(alkyle en C₁₋₃) ou par un atome de chlore, brome ou iode,
R₁ est un atome d'hydrogène, un groupe alkyle en C₁₋₃ ou carboxy-(alkyle en C₁₋₃),
Ar est un groupe phényle substitué par les groupements R₂ à R₄, dans lesquels
R₂ est un groupe alkyle en C₁₋₆ ou un groupe (cycloalkyle en C₃₋₇)-(alkyle en C₁₋₃) qui peuvent être substitués respectivement dans la partie alkyle en C₁₋₆ et en C₁₋₃ par un groupe carboxy, phényle, amino, (alkyle en C₁₋₃)-amino, carboxy-(alkyle en C₁₋₃)-amino, di-(alkyle en C₁₋₃)-amino, N-(carboxy-(alkyle en C₁₋₃))-(alkyle en C₁₋₃)-amino, (cycloalkyle en C₃₋₇)-amino, phénylamino, N-(alkyle en C₁₋₃)-phénylamino, N-(alcanoyle en C₁₋₄)-phénylamino, hétéroarylamino, N-(alkyle en C₁₋₃)-hétéroarylamino, N-(carboxy-(alkyle en C₁₋₃))-phénylamino ou N-(carboxy-(alkyle en C₁₋₃))hétéroarylamino,
un groupe carboxy-(alkyle en C₁₋₅) qui est substitué dans la partie alkyle par un groupe (alkyle en C₁₋₃)-amino, N,N-di-(alkyle en C₁₋₃)-amino, pyrrolidino, pipéridino ou hexaméthylèneimino,
un groupe carboxy-(alkyle en C₁₋₅) dans lequel les atomes d'hydrogène d'un groupe méthylène sont remplacés par un pont n-alkylène en C₂₋₅,
un groupe phényle, phényloxy ou phénylsulfonyle, qui peuvent chacun être substitués dans la partie phényle par un atome de fluor, chlore, brome ou iode ou par un groupe alkyle en C₁₋₃, carboxy-(alkyle en C₁₋₃) ou alcoxy en C₁₋₃,
un groupe (alkyle en C₁₋₅)-amino, carboxy-(alkyle en C₁₋₃)-amino, di-(alkyle en C₁₋₅)-amino, N-(carboxy-(alkyle en C₁₋₃))-(alkyle en C₁₋₅)-amino, (cycloalkyle en C₃₋₇)- amino, N-(carboxy-(alkyle en C₁₋₃))-(cycloalkyle en C₃₋₇)-amino, phénylamino, N-(alkyle en C₁₋₃)-phénylamino, N-( carboxy-(alkyle en C₁₋₃))-phénylamino, hétéroarylamino, N-(alkyle en C₁₋₃)-hétéroarylamino ou N-( carboxy-(alkyle en C₁₋₃))-hétéroarylamino,
un groupe (alkyle en C₁₋₅)carbonylamino, (cycloalkyle en C₃₋₇)carbonylamino, arylcarbonylamino, hétéroarylcarbonylamino, (alkyle en C₁₋₅)sulfonylamino, arylsulfonylamino, hétéroarylsulfonylamino, N-(alkyle en C₁₋₃)-(alkyle en C₁₋₅)carbonylamino, N-(alkyle en C₁₋₃)-(cycloalkyle en C₃₋₇)-carbonylamino, N-(alkyle en C₁₋₃)-arylcarbonylamino, N-(alkyle en C₁₋₃)-hétéroarylcarbonylamino, N-(alkyle en C₁₋₃)-(alkyle en C₁₋₅)-sulfonylamino, N-(alkyle en C₁₋₃)-arylsulfonylamino ou N-(alkyle en C₁₋₃)-hétéroarylsulfonylamino, dans lesquels
les parties N-(alkyle en C₁₋₃) précitées peuvent être substitués en plus par un groupe carboxy, carboxy(alkyle en C₁₋₃)aminocarbonyle ou N-(alkyle en C₁₋₃)-carboxy-(alkyle en C₁₋₃)aminocarbonyle ou, à l'exception de l'atome de carbone en α par rapport à l'atome d'azote, également par un groupe hydroxy, carboxy-(alcoxy en C₁₋₃), amino, carboxy-(alkyle en C₁₋ ₃)-amino en C₁₋₃ ou N-(alkyle en C₁₋₃)-carboxy-(alkyle en C₁₋₃)amino,
un groupe cycloalkylèneimino de 5 à 7 chaînons,
un groupe amino, (alkyle en C₁₋₅)-amino, (cycloalkyle en C₃₋₇)-amino, arylamino, aryl-(alkyle en C₁₋₃)amino, hétéroarylamino ou hétéroaryl-(alkyle en C₁₋₃)-amino, qui sont chacun substitués sur l'atome d'azote de l'amine par un groupe (alkyle en C₁₋₃)-carbonyle, carboxy-(alkyle en C₁₋₃)-carbonyle, carboxy-(alkyle en C₁₋₃)-aminocarbonyle, 2-oxo-pyrrolidinylcarbonyle ou pipérazinocarbonyle, dans lequel, en plus
(i) le groupe amino précité, qui est monosubstitué par un groupe (alkyle en C₁₋₃)carbonyle, carboxy(alkyle en C₁₋₃)carbonyle ou carboxy(alkyle en C₁₋₃)aminocarbonyle, est substitué par un groupe cycloalkylèneimino de 5 à 7 chaînons ou par un groupe N,N-di-(alkyle en C₁₋₅)amino, et
(ii) la partie alkyle du groupe (alkyle en C₁₋₃)carbonyle précité est substituée par un groupe carboxy, amino, hydroxy, carboxy-(alcoxy en C₁₋₃), carboxy-(alkyle en C₁₋₃)-aminocarbonyle, carboxy-(alkyle en C₁₋₃)-amino, N-(alkyle en C₁₋₃)-carboxy-(alkyle en C₁₋₃)-amino ou amino-(alkyle en C₁₋₃)-carbonylamino ou par un groupe carboxy ou hydroxy et par un groupe amino ou trifluoroacétylamino,
un groupe carbimino, qui est substitué sur l'atome d'azote par un groupe carboxy-(alcoxy en C₁₋₃), amino, (alkyle en C₁₋₃)-amino, carboxy-(alkyle en C₁₋ ₃)-amino, di-(alkyle en C₁₋₃)-amino ou N-(carboxy-(alkyle en C₁₋₃))-(alkyle en C₁₋₃)-amino et sur l'atome de carbone par un groupe alkyle en C₁₋₅, par un groupe phényle le cas échéant substitué par un groupe alkyle en C₁₋₃ ou alcoxy en C₁₋₃ ou par un groupe hétéroaryle le cas échéant substitué par un groupe alkyle en C₁₋₃,
un groupe hétéroaryle ou hétéroaryl-(alkyle en C₁₋₃), qui peuvent de plus chacun être substitués dans la partie hétéroaryle également par un groupe phényle ou hétéroaryle ou par un groupe phényle ou hétéroaryle et par un groupe carboxy-(alkyle en C₁₋₃) ou (alcoxy en C₁₋₃)-carbonyl-(alkyle en C₁₋₃),
un groupe 5-oxo-4,5-dihydro-pyrazolyle ou 6-oxo-4,5-dihydro-pyridazinyle, le cas échéant substitué par 1 à 3 groupes alkyles en C₁₋₃, dans lesquels un substituant alkyle peut être substitué en même temps par un groupe carboxy ou (alcoxy en C₁₋₃)carbonyle, ou
un groupe carbonyle, qui est substitué
par un atome d'hydrogène, par un groupe hydroxy, alcoxy en C₁₋₅ ou cycloalcoxy en C₃₋₇,
par un groupe alkyle en C₁₋₅ ou cycloalkyle en C₃₋₇ le cas échéant substitué par un groupe carboxy,
par un groupe alkyle en C₁₋₃ substitué par un groupe pipérazino,
par un groupe phényle, qui peut être substitué par un atome de fluor, chlore, brome ou iode, par un groupe alkyle en C₁₋₃, carboxy-(alkyle en C₁₋₃), alcoxy en C₁₋₃ ou carboxy,
par un groupe amino, (alkyle en C₁₋₅)-amino, carboxy-(alkyle en C₁₋₃)-amino, (cycloalkyle en C₃₋₇)amino, phénylamino ou hétéroarylamino, qui peuvent chacun de plus être substitués sur l'atome d'azote de l'amine par un groupe alkyle en C₁₋₅, cycloalkyle en C₃₋₇, phényl-(alkyle en C₁₋₃), carboxy-(alkyle en C₁₋₃), 2-[di-(alkyle en C₁₋₃)-amino]-éthyle, 3-[di-(alkyle en C₁₋₃)-amino]-propyle, di-(alkyle en C₁₋₃)-amino, 2-(N-carboxy-(alkyle en C₁₋₃)-(alkyle en C₁₋₃)-amino)-éthyle, 3-(N-carboxy-(alkyle en C₁₋₃)-(alkyle en C₁₋₃)-amino)-propyle ou N-carboxy-(alkyle en C₁₋₃)-(alkyle en C₁₋₃)-amino, phényle, pyridyle, pyrrolidinyle ou pipéridinyle,
par un groupe pyrrolyle, thiényle, imidazolyle, pyrazolyle, thiazolyle, pyridinyle, pyrimidinyle, pyrazinyle ou pyridazinyle, le cas échéant substitué par un ou deux groupes alkyles en C₁₋₃, avec lequel un noyau phényle peut être condensé respectivement par l'intermédiaire de deux atomes de carbone voisins,
par un groupe (cycloalkylène en C₃₋₆)imino, (bicycloalkylène en C₅₋₈)-imino, morpholino, pipérazino, dihydropyrazolo, tétrahydropyrazolo, tétrahydroisoxazolo, tétrahydropyrazinyle ou tétrahydropyridazinyle, le cas échéant substitué par un groupe alkyle en C₁₋₃ ou carboxy-(alkyle en C₁₋₃), ou
par un groupe (cycloalkylène en C₃₋₆)imino, le cas échéant substitué par un groupe alkyle en C₁₋₃, carboxy-(alkyle en C₁₋₃), hydroxy, hydroxy-(alkyle en C₁₋₃), amino, carboxy, carboxy-(alcoxy en C₁₋₃)-(alkyle en C₁₋ ₃), carboxy-(alkyle en C₁₋₃)-amino-(alkyle en C₁₋₃) ou carboxy-(alkyle en C₁₋₃)-aminocarbonyl-(alkyle en C₁₋₃),
par un groupe (bicycloalkylène en C₅₋₈)imino, morpholino, pipérazino, dihydropyrazolo, tétrahydropyrazolo, tétrahydroisoxazolo, tétrahydropyrazinyle ou tétrahydropyridazinyle, le cas échéant substitué par un groupe alkyle en C₁₋₃ ou carboxy-(alkyle en C₁₋₃),
R₃ est un atome d'hydrogène, fluor, chlore, brome ou iode, un groupe formyle ou trifluorométhyle,
un groupe alcoxy en C₁₋₃, amino, (alkyle en C₁₋₃)-amino, di-(alkyle en C₁₋₃)-amino, (alcanoyle en C₁₋₄)amino ou N-(alcanoyle en C₁₋₄)(alkyle en C₁₋₃)-amino,
un groupe alkyle en C₁₋₃, le cas échéant substitué par un groupe hydroxy, alcoxy en C₁₋₃, carboxy, carboxy-(alcoxy en C₁₋₃), carboxy-(alkyle en C₁₋₃)-amino, N-(alkyle en C₁₋₃)-carboxy-(alkyle en C₁₋₃)-amino ou carboxy-(alkyle en C₁₋₃)-aminocarbonyle,
un groupe alcényle en C₂₋₃ substitué par un groupe carboxy ou carboxy-(alkyle en C₁₋₃)-aminocarbonyle ou
un groupe carbimino, le cas échéant substitué sur l'atome de carbone par un groupe alkyle en C₁₋₃, qui est substitué sur l'atome d'azote de l'imine par un groupe carboxy-(alcoxy en C₁₋₃) ou aminocarbonylamino, ou bien R₂ et R₃ pris ensemble représentent un groupe -CO-O-CH₂- ou -CO-O-CH₂-CH₂- et
R₄ est un atome d'hydrogène, fluor, chlore, brome ou iode ou un groupe alkyle en C₁₋₃, cycloalkyle en C₃₋₇, trifluorométhyle ou alcoxy en C₁₋₃,
ou bien Ar est également un groupe hétéroaryle, qui peut être substitué par les groupements R₂ à R₄ précités, qui sont tels que définis précédemment,
X est un atome d'oxygène ou de soufre, un groupe méthylène le cas échéant substitué par un ou deux groupes alkyle en C₁₋₃, un groupe carbonyle, sulfinyle, sulfonyle, imino, N-(alkyle en C₁₋₃)-imino ou N-(carboxy-(alkyle en C₁₋₃))imino, dans lequel la partie alkyle du groupe N-(alkyle en C₁₋₃)imino peut être substituée en plus en position 2 ou 3 par un groupe amino, (alkyle en C₁₋₃)-amino, di-(alkyle en C₁₋₃)-amino, (alcanoyle en C₁₋₄)amino ou N-(alcanoyle en C₁₋₄)-(alkyle en C₁₋₃)-amino, et
Y est un groupe cyclohexyle substitué par un groupe amino ou un groupe phényle ou hétéroaryle substitué par le groupement R₅, dans lequel le groupe phényle précité peut être substitué respectivement par un atome de fluor, chlore, brome ou iode ou par un groupe alkyle en C₁₋₃ ou alcoxy en C₁₋₃ et le groupe hétéroaryle précité peut être substitué par un groupe alkyle en C₁₋₃, et
R₅ est un atome d'hydrogène, un groupe cyano ou un groupe amino, amino-(alkyle en C₁₋₃), amidino, guanidino ou guanidino-(alkyle en C₁₋₃), le cas échéant substitué par un groupe dissociable *in vivo*,
dans lesquels
il faut comprendre par les groupes hétéroaryles précités un groupe hétéroaromatique à 5 chaînons, le cas échéant substitué par un ou deux groupes alkyles en C₁₋₃, qui contient un groupe imino le cas échéant substitué par un groupe alkyle en C₁₋₃, un atome d'oxygène ou de soufre, ou un groupe imino le cas échéant substitué par un groupe alkyle en C₁₋₃, un atome d'oxygène ou de soufre et un ou deux atomes d'azote, ainsi que leurs dérivés partiellement hydrogénés, en particulier leurs dérivés dihydro,
ou bien un groupe hétéroaromatique à six chaînons qui contient un, deux ou trois atomes d'azote, un noyau phényle pouvant être condensé en plus avec les cycles hétéro- aromatiques à 5 et 6 chaînons précités par l'intermédiaire de deux atomes de carbone voisins,
les groupes carboxy mentionnés dans la définition des groupements peuvent être remplacés par un groupe tétrazolyle ou par un groupe pouvant être converti *in vivo* en un groupe carboxy, et
les groupes imino ou amino mentionnés dans la définition des groupements peuvent être substitués par un groupement dissociable *in vivo,*
leurs tautomères, leurs stéréoisomères, leurs mélanges et leurs sels.

2. Composés de formule générale I selon la revendication 1, dans laquelle
A est un groupe vinylène, éthylène ou éthinylène, le cas échéant substitué par un atome de chlore, brome ou iode,
R₁ est un atome d'hydrogène ou un groupe alkyle en C₁₋₃,
Ar est un groupe pyridyle ou thiényle substitué par un groupe benzoyle,
un groupe bromofuranyle substitué par un groupe pyrrolidinocarbonyle ou
un groupe phényle substitué par les groupements R₂ à R₄, dans lequel
R₂ est un groupe phényle ou phénoxy,
un groupe alkyle en C₁₋₃, qui peut être substitué par un groupe phényle, phénylamino, N-(alkyle en C₁₋₃)-phénylamino- ou N-(alcanoyle en C₁₋₃)-phénylamino,
un groupe carboxy ou (alcoxy en C₁₋₃)-carbonyle,
un groupe benzoyle ou phénylsulfonyle, dans lesquels chaque partie phényle peut être de plus substituée par un atome de fluor, chlore ou brome, par un groupe méthyle, méthoxy, carboxy ou (alcoxy en C₁₋₃)-carbonyle, l'atome d'oxygène dans les groupes benzoyles précités pouvant être de plus remplacé par un groupe carboxy(alcoxy en C₁₋₃)-imino ou (alcoxy en C₁₋₃)-carbonyl-(alcoxy en C₁₋₃)-imino,
un groupe (alkyle en C₁₋₅)-amino, qui peut être substitué dans la partie alkyle par un groupe phényle, carboxy, (alcoxy en C₁₋₃)-carbonyle, carboxy-(alkyle en C₁₋₃)-aminocarbonyle, (alcoxy en C₁₋₃)-carbonyl-(alkyle en C₁₋₃)-aminocarbonyle, N-{alkyle en C₁₋₃)-carboxy-(alkyle en C₁₋₃)-aminocarbonyle ou N-(alkyle en C₁₋₃)-(alcoxy en C1-3)-carbonyl-(alkyle en C₁₋₃)-aminocarbonyle, ou un groupe (cycloalkyle en C₃₋₇)-amino, les groupes précités pouvant être chacun de plus substitués sur l'atome d'azote de l'amine par un groupe cycloalcanoyle en C₃₋₇, benzoyle ou phénylsulfonyle, par un groupe carboxy-(alkyle en C₁₋₃)-carbonyle ou (alcoxy en C₁₋₃)-carbonyl-(alkyle en C₁₋₃₋)carbonyle dans lequel la partie alkyle de chaque groupe alkylcarbonyle peut être substituée par un groupe amino ou trifluoroacétylamino, par un groupe alcanoyle en C₂₋₄ qui peut être substitué dans la partie alcanoyle par un groupe amino, carboxy, (alcoxy en C₁₋₃)-carbonyle, carboxy-(alcoxy en C₁₋₃), (alcoxy en C₁₋₃)-carbonyl-(alcoxy en C₁₋₃), carboxy-(alkyle en C₁₋₃)-aminocarbonyle, (alcoxy en C₁₋₃)-carbonyl-(alkyle en C₁₋₃)-aminocarbonyle, N-(alkyle en C₁₋₃)-carboxy-(alkyle en C₁₋₃)-aminocarbonyle ou N-(alkyle en C₁₋₃)-(alcoxy en C₁₋₃)-carbonyl-(alkyle en C₁₋₃)-aminocarbonyle, par un groupe carboxy-(alkyle en C₁₋₂)-aminocarbonyle, (alcoxy en C₁₋₃)-carbonyl-(alkyle en C₁₋₂)-aminocarbonyle, carboxy-(alkyle en C₁₋₃)-aminocarbonyl-(alkyle en C₁₋ ₂)-aminocarbonyle ou (alcoxy en C₁₋₃)-carbonyl(alkyle en C₁₋₃)-aminocarbonyl-(alkyle en C₁₋₂)-aminocarbonyle, un groupe formyle, pyridylcarbonyle, thiénylcarbonyle, imidazolylcarbonyle, 1-méthyl-imidazolylcarbonyle, thiazolylcarbonyle ou indolylcarbonyle,
un groupe benzimidazol-1-yle, benzimidazol-1-yl-méthyle ou 5-oxo-4,5-dihydro-pyrazol-3-yle, le cas échéant substitué par 1 ou 2 groupes méthyles,
un groupe pyrazol-1-yle substitué par un groupe phényle, par un groupe phényle et un groupe alkyle en C₁₋₄, ou par un ou deux groupes alkyles en C₁₋₄, dans lesquels un substituant alkyle peut être substitué en même temps par un groupe carboxy ou (alcoxy en C₁₋₃)carbonyle, ou
un groupe carbonyle qui est substitué
par un groupe alkyle en C₁₋₅ le cas échéant substitué par un groupe carboxy ou (alcoxy en C₁₋₃)carbonyle,
par un groupe cycloalkyle en C₃₋₇,
par un groupe amino ou (alkyle en C₁₋₅)amino qui peut être substitué respectivement sur l'atome d'azote de l'amine par un groupe alkyle en C₁₋₃ qui peut être substitué par un groupe cycloalkyle en C₃₋₇, phényle, pyrrolidinyle ou pyridinyle ou en position 2 ou 3 par un groupe di-(alkyle en C₁₋₃)-amino, ou par un groupe di-(alkyle en C₁₋₃)amino,
par un groupe carboxy(alkyle en C₁₋₃)amino ou (alcoxy en C₁₋₃)carbonyl-(alkyle en C₁₋₃)amino qui est chaque fois substitué sur l'atome d'azote de l'amine par un groupe pyrazolyle le cas échéant substitué par un groupe alkyle en C₁₋₃,
par un groupe cycloalkylèneimino à 3 à 7 chaînons qui peut être substitué par un ou deux groupes alkyles en C₁₋₃, les groupes pyrrolidino précités le cas échéant substitués par un groupe méthyle pouvant être de plus substitués par un groupe hydroxyméthyle, carboxy, (alcoxy en C₁₋₃)-carbonyle, carboxy-(alkyle en C₁₋₃), (alcoxy en C₁₋₃)-carbonyl-(alkyle en C₁₋₃), carboxy-(alkyle en C₁₋₃)-oxy-(alkyle en C₁₋₃), (alcoxy en C₁₋₃)-carbonyl-(alkyle en C₁₋₃)-oxy-(alkyle en C₁₋₃), carboxy-(alkyle en C₁₋₃)-amino-(alkyle en C₁₋₃), N-(alkyle en C₁₋₃)-carboxy-(alkyle en C₁₋₃)-amino-(alkyle en C₁₋₃), (alcoxy en C₁₋₃)-carbonyl-(alkyle en C₁₋₃)-amino-(alkyle en C₁₋₃), N-(alkyle en C₁₋₃)-carboxy-(alkyle en C₁₋₃)-amino-(alkyle en C₁₋₃) ou N-(alkyle en C₁₋₃)-(alcoxy en C₁₋₃)-carbonyl-(alkyle en C₁₋₃)-amino-(alkyle en C₁₋₃),
par un groupe morpholino, pipérazino, 4-méthylpipérazino, pipérazino-(alkyle en C₁₋₃), dihydropyrazolo, tétrahydropyrazolo, tétrahydroisooxazolo ou 7-azabicycloheptyle, ou
par un groupe N-(alkyle en C₁₋₃)-phényle ou N-(alkyle en C₁₋₃)pyridylamino le cas échéant substitué dans la partie alkyle par un groupe carboxy ou (alcoxy en C₁₋₃)carbonyle,
R₃ est un atome d'hydrogène, fluor, chlore ou brome,
un groupe hydroxy, alcoxy en C₁₋₃, trifluorométhyle, amino ou (alcanoyle en C₂₋₃)amino,
un groupe alkyle en C₁₋₃ qui peut être substitué par un groupe hydroxy, carboxy, (alcoxy en C₁₋₃)carbonyle, carboxy-(alcoxy en C₁₋₃), (alcoxy en C₁₋₃)-carbonyl-(alcoxy en C₁₋₃), carboxy(alkyle en C₁₋₃)aminocarbonyle, (alcoxy en C₁₋₃)carbonyl(alkyle en C₁₋₃)aminocarbonyle, N-(alkyle en C₁₋₃)carboxy(alkyle en C₁₋₃)aminocarbonyle ou N-(alkyle en C₁₋₃)(alcoxy en C₁₋₃)carbonyl(alkyle en C₁₋₃)aminocarbonyle,
un groupe alkyle en C₁₋₃ qui est substitué par un groupe carboxy(alkyle en C₁₋₃)amino, (alcoxy en C₁₋₃)carbonyl(alkyle en C₁₋₃)amino, N-(alkyle en C₁₋₃)-carboxy(alkyle en C₁₋₃)amino ou (alcoxy en C₁₋₃)carbonyl(alkyle en C₁₋₃)-aminocarbonyle,
un groupe alcényle en C₂₋₃ substitué par un groupe carboxy ou (alcoxy en C₁₋₃)-carbonyle ou
un groupe carbimino le cas échéant substitué sur l'atome de carbone par un groupe alkyle en C₁₋₃ qui est substitué sur l'atome d'azote de l'imine par un groupe carboxy-(alcoxy en C₁₋₃), (alcoxy en C₁₋₃)carbonyl(alcoxy en C₁₋₃) ou aminocarbonylamino,
ou bien R₂ et R₃ pris ensemble représentent un groupe -CO-O-CH₂-, et
R₄ représente un atome d'hydrogène, fluor, chlore ou brome ou un groupe alkyle en C₁₋₃ ou trifluorométhyle,
X est un atome d'oxygène ou de soufre ou un groupe -NH le cas échéant substitué par un groupe alkyle en C₁₋₃, et
Y est un groupe cyclohexyle substitué par un groupe amino, un groupe phénylène ou pyridinylène substitué par un groupe amidino, lequel peut être substitué par un groupe benzoyle ou (alcoxy en C₁₋₈)carbonyle, le groupe phénylène précité pouvant être substitué par un groupe méthyle ou méthoxy et le groupe pyridinylène précité par un groupe méthyle,
leurs tautomères, leurs stéréoisomères, leurs mélanges et leurs sels.

3. Composés de formule générale 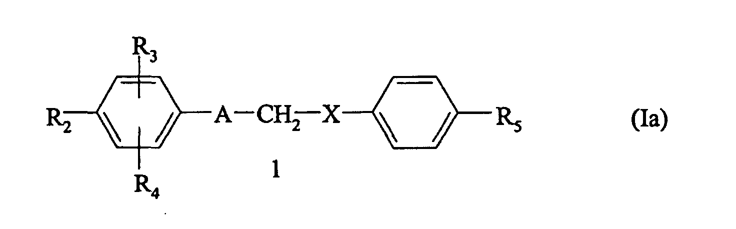 selon la revendication 1, dans laquelle
A est un groupe éthylène ou éthinylène,
X est un atome d'oxygène ou un groupe imino le cas échéant substitué par un groupe méthyle,
R₂ est un groupe (alkyle en C₁₋₄)carbonylamino ou (cycloalkyle en C₃₋₅)-carbonylamino, qui est substitué respectivement sur l'atome d'azote de l'amine par un groupe carboxy-(alkyle en C₁₋₂), (alcoxy en C₁₋₃)carbonyl(alkyle en C₁₋₂), carboxy-(alkyle en C₁₋₂)aminocarbonyl(alkyle en C₁₋₂) ou (alcoxy en C₁₋₃)carbonyl(alkyle en C₁₋₂)aminocarbonyl(alkyle en C₁₋₂),
un groupe (alkyle en C₁₋₄)-amino ou (cycloalkyle en C₃₋₅)-amino qui est substitué respectivement sur l'atome d'azote de l'amine par un groupe carboxy(alkyle en C₁₋₃)-carbonyle, (alcoxy en C₁₋₃)carbonyl(alkyle en C₁₋₃)carbonyle, carboxy(alkyle en C₁₋ ₂)aminocarbonyl(alkyle en C₁₋₂)carbonyle, (alcoxy en C₁₋₃)carbonyl(alkyle en C₁₋ ₂)aminocarbonyl(alkyle en C₁₋₂)carbonyle, carboxy(alkyle en C₁₋₂)aminocarbonyle, (alcoxy en C₁₋₃)carbonyl(alkyle en C₁₋₂)aminocarbonyle, carboxy(alkyle en C₁₋ ₂)aminocarbonyl(alkyle en C₁₋₂)aminocarbonyle ou (alcoxy en C₁₋₃)carbonyl(alkyle en C₁₋₂)aminocarbonyl(alkyle en C₁₋₂)aminocarbonyle le cas échéant substitué dans la partie alkyle par un groupe amino, par un groupe carboxyméthyloxyméthylcarbonyle, (alcoxy en C₁₋₃)carbonyl-méthyloxyméthylcarbonyle, carboxyméthylaminométhylcarbonyle, (alcoxy en C₁₋₃)carbonyl-méthylaminométhylcarbonyle, N-méthyl-carboxyméthylaminométhylcarbonyle, N-méthyl-(alcoxy en C₁₋₃)-carbonylcarboxyméthylaminométhylcarbonyle, aminométhylcarbonyle, 2-aminoéthylcarbonyle, carboxy-(alkyle en C₁₋₂)-aminocarbonylméthyloxyméthylcarbonyle, (alcoxy en C₁₋₃)carbonyl(alkyle en C₁₋₂)-aminocarbonylméthyloxyméthylcarbonyle, carboxy-(alkyle en C₁₋ ₂)aminocarbonylméthylaminométhylcarbonyle, (alcoxy en C₁₋₃)-carbonyl(alkyle en C₁₋₂)-aminocarbonylméthylaminométhylcarbonyle, N-méthyl-carboxy(alkyle en C₁₋ ₂)-aminocarbonylméthylaminométhylcarbonyle ou N-méthyl-(alcoxy en C₁₋ ₃)carbonyl(alkyle en C₁₋₂)aminocarbonylméthylaminométhylcarbonyle, ou un groupe carbonyle qui est substitué
par un groupe cyclopentyle,
par un groupe alkyle en C₃₋₅ qui peut être de plus substitué par un groupe carboxy ou (alcoxy en C₁₋₃)carbonyle,
par un groupe (alkyle en C₁₋₄)-amino, phénylamino ou pyridylamino substitué sur l'atome d'azote de l'amine par un groupe alkyle en C₁₋₄, carboxy-(alkyle en C₁₋₃) ou (alcoxy en C₁₋₃)carbonyl(alkyle en C₁₋₃),
par un groupe pyrrolidino substitué par un groupe méthyle, hydroxyméthyle, amino, carboxy, (alcoxy en C₁₋₃)carbonyle, carboxy-(alkyle en C₁₋₂), (alcoxy en C₁₋₃)carbonyl(alkyle en C₁₋₂), carboxyméthyloxyméthyle, (alcoxy en C₁₋₃)-carbonylméthyloxyméthyle, carboxyméthylaminométhyle, (alcoxy en C₁₋₃)-carbonyl-méthylaminométhyle, carboxyméthylaminocarbonylméthyloxyméthyle ou (alcoxy en C₁₋₃)carbonylméthylaminocarbonylméthyloxyméthyle,
R₃ est un atome d'hydrogène, fluor, chlore ou brome ou un groupe trifluorométhyle,
un groupe alkyle en C₁₋₂ substitué le cas échéant par un groupe hydroxy, carboxy, (alcoxy en C₁₋₃)carbonyle, carboxyméthyloxy, (alcoxy en C₁₋₃)carbonyl-méthyloxy, carboxyméthylamino, N-méthyl-carboxyméthylamino, (alcoxy en C₁₋₃)carbonyl-méthylamino, N-méthyl-(alcoxy en C₁₋₃)carbonyl-méthylamino, carboxyméthylamino-carbonyle ou (alcoxy en C₁₋₃)carbonylméthylaminocarbonyle,
un groupe vinyle substitué par un groupe carboxy ou (alcoxy en C₁₋₃)carbonyle,
R₄ est un atome d'hydrogène, fluor, chlore ou brome ou un groupe méthyle, éthyle ou trifluorométhyle et
R₅ est un groupe amidino le cas échéant substitué par un groupe (alcoxy en C₁₋₈)-carbonyle ou benzoyle,
leurs tautomères, leurs stéréoisomères, leurs mélanges et leurs sels.

4. Composés de formule générale Ia selon la revendication 3, dans laquelle
A est un groupe éthylène ou éthinylène,
X est un groupe imino,
R₂ est un groupe (alkyle en C₁₋₄)aminocarbonyle qui est substitué respectivement sur l'atome d'azote de l'amine par un groupe carboxy-(alkyle en C₁₋₂)ou (alcoxy en C₁₋₃)-carbonyl(alkyle en C₁₋₂),
un groupe (alkyle en C₁₋₄)-amino qui est substitué sur l'atome d'azote de l'amine par un groupe carboxy(alkyle en C₁₋₃)carbonyle, (alcoxy en C₁₋₃)carbonyl(alkyle en C₁₋ ₃)-carbonyle, carboxy(alkyle en C₁₋₂)aminocarbonyle ou (alcoxy en C₁₋₃)carbonyl-(alkyle en C₁₋₂)aminocarbonyle, ou
un groupe carbonyle qui est substitué
par un groupe alkyle en C₃₋₅ qui peut être de plus substitué par un groupe carboxy ou (alcoxy en C₁₋₃)carbonyle,
par un groupe (alkyle en C₁₋₄)-amino ou pyridylamino substitué sur l'atome d'azote de l'amine par un groupe carboxy-(alkyle en C₁₋₃) ou (alcoxy en C₁₋ ₃)carbonyl- (alkyle en C₁₋₃),
par un groupe pyrrolidino le cas échéant substitué par un groupe méthyle,
R₃ est un groupe alkyle en C₁₋₂ le cas échéant substitué par un groupe carboxy ou (alcoxy en C₁₋₃)carbonyle,
R₄ un atome d'hydrogène ou un groupe méthyle, et
R₅ est un groupe amidino le cas échéant substitué par un groupe (alcoxy en C₁₋₈)-carbonyle ou benzoyle,
leurs tautomères, leurs stéréoisomères, leurs mélanges et leurs sels.

5. Composés suivants de formule générale I selon la revendication 1,3 ou 4 :
(a) rac-4-{3-[5-éthoxycarbonylméthyl-2-méthyl-4-(2-méthyl-pyrrolidinocarbonyl)- phényl]-propargylamino}benzamidine,
(b) rac-4-{3-[2,5-diméthyl-4-(2-méthyl-pyrrolidinocarbonyl)-phényl]-propargylamino}benzamidine,
(c) 4-[3-(2,5-diméthyl-4-isopropylcarbonyl-phényl)propargylamino]-benzamidine,
(d) 4-{3-[2,5-diméthyl-4-(N-méthyl-N-pyridin-2-yl-aminocarbonyl)-phényl]-propargylamino}benzamidine,
(e) 4-{3-[2,5-diméthyl-4-(N-méthyl-N-pyridin-2-yl-aminocarbonyl)-phényl]-prop-1-ylamino}benzamidine,
(f) 4-[3-(3-méthyl-4-pyrrolidinocarbonyl-phényl)-propargylamino]-benzamidine,
(g) 4-{3-[2,5-diméthyl-4-(N-(2-méthoxycarbonyléthyl)-N-éthylcarbonylamino)-phényl]propargylamino}benzamidine,
(h) 4-(3-[2,5-diméthyl-4-(N-isopropyl-N-hydroxycarbonylméthylaminocarbonyl-amino)-phényl]-propargylamino)-benzamidine, et
(i) 4-{3-[2,5-diméthyl-4-(N-isopropyl-N-hydroxycarbonylméthylcarbonylamino)-phényl]-propargylamino}-benzamidine
ainsi que leurs sels.

6. Sels physiologiquement compatibles des composés selon les revendications 1 à 5, dans lesquels Y ne contient pas de groupe cyano.

7. Médicaments contenant un composé selon l'une au moins des revendications 1 à 5, dans lesquels Y ne contient pas de groupe cyano, ou un sel selon la revendication 6, le cas échéant en plus d'un ou plusieurs véhicules et/ou diluants inertes.

8. Utilisation d'un composé selon l'une au moins des revendications 1 à 5, dans laquelle Y ne contient pas de groupe cyano, ou d'un sel selon la revendication 6, pour la préparation d'un médicament ayant un effet d'allongement du temps de thrombine, un effet d'inhibition de la thrombine et un effet d'inhibition des sérines protéases apparentées.

9. Procédé de préparation d'un médicament selon la revendication 7, **caractérisé en ce qu'**un composé selon l'une au moins des revendications 1 à 5, dans lequel Y ne contient pas de groupe cyano, ou un sel selon la revendication 6, est incorporé de manière non chimique dans un ou plusieurs véhicules et/ou diluants inertes.

10. Procédé de préparation des composés selon les revendications 1 à 6, **caractérisé en ce que**
a. un composé de formule générale
Ar-Z₁ (II)
dans laquelle Ar est tel que défini dans les revendications 1 à 5 et Z₁ est un groupe partant, est mis à réagir avec un composé de formule générale
H-A'-HCR₁-X-Y' (III)
dans laquelle R₁ et X sont tels que définis dans les revendications 1 à 5, Y' a les significations indiquées pour Y dans les revendications 1 à 5, à la condition qu'un groupe amino ou imino présent soit protégé par un groupement protecteur usuel, et A' est un groupe éthinyle, et un composé ainsi obtenu est ensuite le cas échéant hydrogéné catalytiquement et/ou un groupement protecteur utilisé est dissocié d'un composé ainsi obtenu, ou
b. pour préparer un composé de formule générale I dans laquelle le groupement ArA contient un groupe carboxy et R₅ est tel que défini dans les revendications 1 à 5, ou bien le groupement Ar-A est tel que défini dans les revendications 1 à 5 et R₅ est un groupe amino, amino-(alkyle en C₁₋₃), amidino ou guanidino, ou bien le groupement Ar-A contient un groupe carboxy et R₅ est un groupe amino, amino-(alkyle en C₁₋₃), amidino ou guanidino, un composé de formule générale
Ar'-A-HCR₁-X-Y" (IV)
dans laquelle
A, R₁ et X sont tels que définis dans les revendications 1 à 5,
Ar' et Y" ont les significations indiquées pour Ar et Y dans les revendications 1 à 5, à la condition que
Ar' contienne un groupe pouvant être converti par hydrolyse, traitement avec un acide ou une base, thermolyse ou hydrogénolyse en un groupe carboxy et que Y" ait les significations indiquées pour Y dans les revendications 1 à 5, ou bien que
Ar' ait les significations indiquées pour Ar dans les revendications 1 à 5 et que Y" contienne un groupe pouvant être converti par hydrolyse, traitement avec un acide ou une base, thermolyse ou hydrogénolyse en un groupe amino, amino-(alkyle en C₁₋₃), amidino ou guanidino, ou bien que
Ar' contienne un groupe pouvant être converti par hydrolyse, traitement avec un acide ou une base, thermolyse ou hydrogénolyse en un groupe carboxy et que Y" contienne un groupe pouvant être converti par hydrolyse, traitement avec un acide ou une base, thermolyse ou hydrogénolyse en un groupe amino, amino-(alkyle en C₁₋₃), amidino ou guanidino,
est converti par hydrolyse, traitement avec un acide ou une base, thermolyse ou hydrogénolyse en un composé de formule générale I, dans lequel le groupement Ar-A contient un groupe carboxy et R₅ est tel que défini dans les revendications 1 à 5, ou bien le groupement Ar-A est tel que défini dans les revendications 1 à 5 et R₅ est un groupe amino, amino-(alkyle en C₁₋₃), amidino ou guanidino, ou bien le groupement Ar-A contient un groupe carboxy et R₅ est un groupe amino, amino-(alkyle en C₁₋₃), amidino ou guanidino, ou
c. pour préparer un composé de formule générale I dans laquelle R₅ est un groupe amidino, un composé, le cas échéant formé dans le mélange réactionnel, de formule générale
Ar-A-HCR₁-X-Y" (V)
dans laquelle
A, Ar, R₁ et X sont tels que définis dans les revendications 1 à 5 et
Y" représente un des groupements indiqués pour Y dans les revendications 1 à 5, à la condition que R₅ soit un groupe Z₁-(HN=)C, dans lequel
Z₁ est un groupe alcoxy, alkylthio, aralcoxy ou aralkylthio, est mis à réagir avec un sel d'ammonium, un hydrogène halogéné pouvant en même temps être additionné à une liaison triple riche en électrons ou pauvre en électrons, ou
d) pour préparer un composé de formule générale I, dans laquelle R₅ est un groupe amidino qui est substitué par un groupe hydroxy, un composé, le cas échéant formé dans le mélange réactionnel, de formule générale
Ar-A-HCR₁-X-Y" (V)
dans laquelle
A, Ar, R₁ et X sont tels que définis dans les revendications 1 à 5 et
Y" représente un des groupements indiqués pour Y dans les revendications 1 à 5, à la condition que R₅ soit un groupe Z₁-(HN=)C, dans lequel
Z₁ est un groupe alcoxy, alkylthio, aralcoxy ou aralkylthio,
est mis à réagir avec de l'hydroxylamine ou un de ses sels, ou
e. pour préparer un composé de formule générale I, dans laquelle X est un atome d'oxygène ou de soufre, un groupe carbonyle, imino ou N-(alkyle en C₁₋₃)imino, un composé de formule générale I
Ar-A-HCR₁-Z₂ (VI)
dans laquelle
A, Ar et R₁ sont tels que définis dans les revendications 1 à 5 et
Z₂ est un groupe partant, est mis à réagir avec un composé de formule générale
U-Y (VII)
dans laquelle
Y est tel que défini dans les revendications 1 à 5 et
U est un groupe hydroxy, mercapto, hydroxycarbonyle, imino ou N-(alkyle en C₁₋₃)-imino,ou
f. pour préparer un composé de formule générale I, dans laquelle Ar et/ou Y contiennent un groupement dissociable *in vivo,* un composé de formule générale
Ar"-A-HCR₁-X-Y"' (VIII)
dans laquelle
A, R₁ et X sont tels que définis dans les revendications 1 à 5,
Ar" et Y''' ont les significations indiquées pour Ar et Y dans les revendications 1 à 5, à la condition que
Ar" contienne un groupe carboxy et que Y''' ait les significations indiquées pour Y dans les revendications 1 à 5, ou bien que
Ar" ait les significations indiquées pour Ar dans les revendications 1 à 5 et que Y''' contienne un groupe amino, amino-(alkyle en C₁₋₃), amidino ou guanidino, ou bien que
Ar" contienne un groupe carboxy et que Y"' contienne un groupe pouvant être converti en un groupe amino, amino-(alkyle en C₁₋₃), amidino ou guanidino, est mis à réagir avec un composé de formule générale
Z₃-R₇ (IX)
dans laquelle
R₇ est un groupe (alcoxy en C₁₋₈)carbonyle, un groupe RₐCO-O-(R_{b}CRc) ou le groupement acyle d'un des groupements dissociables *in vivo* indiqués dans les revendications 1 à 5, Rₐ à R_{c} étant tels que définis dans les revendications 1 à 5, et
Z₃ est un groupe partant nucléofuge ou également, lorsque Ar" contient un groupe carboxy, un groupe hydroxy, et
si on le souhaite, un composé ainsi obtenu de formule générale I, dans laquelle R₅ représente un groupe amidino, est ensuite converti par alkylation avec un dérivé d'acide haloacétique suivie d'une hydrolyse et d'une décarboxylation en un composé amidino correspondant substitué par un ou deux groupes méthyles, et/ou
un composé ainsi obtenu de formule générale I, dans laquelle R₅ représente un groupe hydroxyamidino, est converti par hydrogénation catalytique en un composé amidino correspondant, et/ou
un composé ainsi obtenu de formule générale I qui contient une liaison double ou triple est converti par hydrogénation catalytique en un composé saturé correspondant, et/ou
un composé ainsi obtenu de formule générale I, dans laquelle X représente un atome de soufre, est converti par oxydation en un composé sulfinyle ou sulfonyle correspondant, et/ou
un composé ainsi obtenu de formule générale I, dans laquelle R₂ représente un groupe tétrahydropyrazolocarbonyle, est converti par oxydation en un composé 4,5-dihydropyrazolocarbonyle correspondant, et/ou
un composé ainsi obtenu de formule générale I qui contient un groupe carbonyle est converti à l'aide d'un oxime correspondant en un composé oxime correspondant, et/ou
un composé ainsi obtenu de formule générale I qui contient un groupe carboxy est converti à l'aide d'une amine correspondante en un amide correspondant, et/ou
si nécessaire, un groupement protecteur utilisé au cours des réactions pour protéger des groupes réactifs est dissocié, et/ou
si on le souhaite, un composé ainsi obtenu de formule générale I est ensuite séparé en ses stéréoisomères, et/ou
un composé ainsi obtenu de formule générale I qui contient une double liaison est séparé en ses isomères cis/trans, et/ou
un composé ainsi obtenu de formule générale I est converti en ses sels, en particulier, pour l'utilisation pharmaceutique, en ses sels physiologiquement compatibles, avec un acide ou une base inorganique ou organique.
